# EUROPEAN PATENT APPLICATION

(11) **EP 3 816 182 A1**
(43) Date of publication of application: **05.05.2021**
(21) Application number: 19810410.1
(22) Date of filing: 30.05.2019
(51) Int. Cl.: C07K 16/00, A61K 39/395, C07K 19/00

(54) **LIGAND-BINDING MOLECULE CONTAINING SINGLE DOMAIN ANTIBODY**

(30) Priority: 30.05.2018 JP 2018103701
(71) Applicant: CHUGAI SEIYAKU KABUSHIKI KAISHA, Tokyo 115-8543 (JP)
(72) Inventor: ISHIKAWA, Hiroyuki, Gotemba-shi, Shizuoka 412-8513 (JP); KAWA, Tatsuya, Gotemba-shi, Shizuoka 412-8513 (JP); HIRONIWA, Naoka, Synapse, 138623 (SG)
(74) Representative: Vossius & Partner Patentanwälte Rechtsanwälte mbB
(86) International application number: PCT/JP2019/021464
(87) International publication number: WO 2019/230868

(57) **Abstract**

The present invention relates to ligand-binding molecules containing a single-domain antibody in which the binding activity to the ligand is attenuated by the cleavage of a cleavage site, methods for producing the same, complexes formed by the ligand-binding molecules and ligands, fusion proteins comprising the ligand-binding molecules and ligands, and pharmaceutical compositions comprising the ligand-binding molecules or fusion proteins of the ligand-binding molecules and ligands.

## Description

### [Technical Field]

The present invention provides ligand-binding molecules comprising a single-domain antibody, methods for producing the ligand-binding molecules, and pharmaceutical compositions comprising the ligand-binding molecules. The present invention also provides fusion proteins comprising the ligand-binding molecules, methods for producing the fusion proteins, and pharmaceutical compositions comprising the fusion proteins.

### [Background Art]

Antibodies have been receiving attention as drugs because they are highly stable in plasma and cause few adverse reactions. In particular, many IgG-type antibody drugs have been on the market, and a large number of antibody drugs are currently under development (Non Patent Literatures 1 and 2).

Antibody-based cancer therapeutic drugs that have previously been approved include Rituxan, cetuximab, and Herceptin, which are directed against CD20 antigen, EGFR antigen, and HER2 antigen, respectively (Non Patent Literature 3). These antibody molecules bind to their antigens expressed on cancer cells and thereby exert cytotoxic activity against the cancer cells through ADCC, signal inhibition, etc.

Meanwhile, a method for delivering a biologically active ligand such as a cytokine to solid cancer is known, which uses an immunocytokine prepared by fusing such a ligand with an antibody molecule that binds to a cancer antigen highly expressed on cancer cells. The cytokine delivered to solid cancer by the immunocytokine activates immunity and thereby exerts an antitumor effect. Since cytokines including IL-2, IL-12, and TNF are very toxic, delivering these cytokines locally to cancer using an antibody to allow them to act locally on the cancer is expected to provide enhanced effects with alleviated adverse reactions (Non Patent Literatures 4, 5, and 6). However, none of these molecules have yet been approved as drugs because of their problems such as poor clinical effect in systemic administration, narrow therapeutic windows, and being too toxic to be administered systemically.

This is largely because cytokines, including immunocytokines, are exposed to the whole body after systemic administration and therefore may act and exhibit toxicity in a systemic manner, or can only be administered at very low doses in order to circumvent the toxicity. It has also been reported that there was no difference in antitumor effect between an immunocytokine composed of IL-2 fused with an antibody that binds to a cancer antigen and an immunocytokine composed of IL-2 fused with an antibody that does not bind to the cancer antigen (Non Patent Literature 7).

### [Citation List]

### [Non-Patent Literature]

[NPL 1] Monoclonal antibody successes in the clinic. Janice M Reichert, Clark J Rosensweig, Laura B Faden & Matthew C Dewitz, Nat. Biotechnol. (2005) 23, 1073-1078
[NPL 2] The therapeutic antibodies market to 2008. Pavlou AK, Belsey MJ., Eur. J. Pharm. Biopharm. (2005) 59 (3), 389-396
[NPL 3] Monoclonal antibodies: versatile platforms for cancer immunotherapy. Weiner LM, Surana R, Wang S., Nat. Rev. Immunol. (2010) 10 (5), 317-327
[NPL 4] Cyclophosphamide and tucotuzumab (huKS-IL2) following first-line chemotherapy in responding patients with extensive-disease small-cell lung cancer. Gladkov O, Ramlau R, Serwatowski P, Milanowski J, Tomeczko J, Komarnitsky PB, Kramer D, Krzakowski MJ. Anticancer Drugs. 2015 Nov; 26 (10): 1061-8.
[NPL 5] Defining the Pharmacodynamic Profile and Therapeutic Index of NHS-IL12 Immunocytokine in Dogs with Malignant Melanoma. Paoloni M, Mazcko C, Selting K, Lana S, Barber L, Phillips J, Skorupski K, Vail D, Wilson H, Biller B, Avery A, Kiupel M, LeBlanc A, Bernhardt A, Brunkhorst B, Tighe R, Khanna C. PLoS One. 2015 Jun 19; 10 (6): e0129954.
[NPL 6] Isolated limb perfusion with the tumor-targeting human monoclonal antibody-cytokine fusion protein L19-TNF plus melphalan and mild hyperthermia in patients with locally advanced extremity melanoma. Papadia F, Basso V, Patuzzo R, Maurichi A, Di Florio A, Zardi L, Ventura E, Gonzalez-Iglesias R, Lovato V, Giovannoni L, Tasciotti A, Neri D, Santinami M, Menssen HD, De Cian F. J Surg Oncol. 2013 Feb; 107 (2): 173-9.
[NPL 7] Antigen specificity can be irrelevant to immunocytokine efficacy and biodistribution. Tzeng A, Kwan BH, Opel CF, Navaratna T, Wittrup KD. Proc Natl Acad Sci U S A. 2015 Mar 17; 112 (11): 3320-5.

### [Summary of Invention]

### [Technical Problem]

The present invention was made in view of such circumstances, and one of the objectives is to provide single-domain antibody-containing ligand-binding molecules that selectively activate ligands such as cytokines or chemokines in target tissues, complexes of the ligand-binding molecules and their ligands, fusion proteins comprising the ligand-binding molecules, and methods for producing them. A further objective of the present invention is to provide pharmaceutical compositions comprising as active ingredient, the ligand-binding molecules, or complexes of said ligand-binding molecules and their ligands, or fusion proteins comprising the ligand-binding molecules, and methods for producing the pharmaceutical compositions.

### [Solution to Problem]

The present inventors conducted intensive research to achieve the above-mentioned objectives, and created ligand-binding molecules comprising a single-domain antibody having a cleavage site, and fusion proteins comprising the ligand-binding molecules. In addition to elucidating that the ligand-binding molecules, complexes of said ligand-binding molecules and ligands, fusion proteins comprising the ligand-binding molecules, or pharmaceutical compositions comprising them, are useful in treating diseases using the ligands, the present inventors also discovered that the ligand-binding molecules or fusion proteins comprising the ligand binding molecules are useful in methods of treating diseases that include administering the ligand-binding molecules, the complexes of the ligand-binding molecules and ligands, or the fusion proteins comprising the ligand-binding molecules, or in the manufacture of pharmaceuticals for treating diseases. The present inventors created methods for producing the ligand-binding molecules, complexes of the ligand-binding molecules and their ligands, and fusion proteins comprising the ligand-binding molecules, and thus completed the present invention.

The present invention is based on such knowledge, and specifically includes the embodiments described below as examples.
(A1) A ligand-binding molecule, wherein the ligand-binding molecule comprises a single-domain antibody, wherein the single-domain antibody can bind to a ligand and has at least one cleavage site introduced, and wherein the binding of the ligand-binding molecule to the ligand is attenuated in a state where the cleavage site is cleaved, compared to the binding of the ligand-binding molecule to the ligand in a state where the cleavage site is not cleaved.
(A2) The ligand-binding molecule according to (A1), wherein the ligand is released from the ligand-binding molecule in a state where the cleavage site is cleaved.
(A3) The ligand-binding molecule according to (A1) or (A2), wherein the cleavage site comprises a protease cleavage sequence.
(A4) The ligand-binding molecule according to (A3), wherein the protease is a target tissue specific protease.
(A5) The ligand-binding molecule according to (A4), wherein the target tissue is a cancer tissue and the target tissue specific protease is a cancer tissue specific protease.
(A6) The ligand-binding molecule according to (A4), wherein the target tissue is an inflammatory tissue, and the target tissue specific protease is an inflammatory tissue specific protease.
(A7) The ligand-binding molecule according to any one of (A3) to (A6), wherein the protease is at least one protease selected from a matriptase, urokinase (uPA), and metalloprotease.
(A8) The ligand-binding molecule according to any one of (A3) to (A7), wherein the protease cleavage sequence is a sequence comprising one or more sequences selected from sequences set forth in SEQ ID NOs: 2 to 82, 788 to 827, and sequences set forth in Table 1.
(A9) The ligand-binding molecule according to any one of (A3) to (A8), wherein a first flexible linker is further attached to one end of the protease cleavage sequence.
(A10) The ligand-binding molecule according to (A9), wherein the first flexible linker is a flexible linker consisting of a glycine-serine polymer.
(A11) The ligand-binding molecule according to (A9) or (A10), wherein a second flexible linker is further attached to the other end of the protease cleavage sequence.
(A12) The ligand-binding molecule according to (A11), wherein the second flexible linker is a flexible linker consisting of a glycine-serine polymer.
(A13) The ligand-binding molecule according to any one of (A1) to (A12), wherein the single-domain antibody is VHH, or a single-domain VH antibody, or a single-domain VL antibody.
(A14) The ligand-binding molecule according to (A13), wherein the single-domain antibody is a VHH or a single-domain VH antibody, and wherein the cleavage site, or the protease cleavage sequence, or the protease cleavage sequence and the first flexible linker, or the protease cleavage sequence and the first flexible linker and the second flexible linker, is/are introduced into one or more positions comprised in one or more sequences selected from the following sequences of the single-domain antibody:
   the sequence from amino acid 7 (Kabat Numbering) to amino acid 17 (Kabat Numbering) of the single-domain antibody; the sequence from amino acid 12 (Kabat Numbering) to amino acid 17 (Kabat Numbering) of the single-domain antibody; the sequence from amino acid 31 (Kabat Numbering) to amino acid 35b (Kabat Numbering) of the single-domain antibody; the sequence from amino acid 40 (Kabat Numbering) to amino acid 47 (Kabat Numbering) of the single-domain antibody; the sequence from amino acid 50 (Kabat Numbering) to amino acid 65 (Kabat Numbering) of the single-domain antibody; the sequence from amino acid 55 (Kabat Numbering) to amino acid 69 (Kabat Numbering) of the single-domain antibody; the sequence from amino acid 73 (Kabat Numbering) to amino acid 79 (Kabat Numbering) of the single-domain antibody; the sequence from amino acid 83 (Kabat Numbering) to amino acid 89 (Kabat Numbering) of the single-domain antibody; the sequence from amino acid 95 (Kabat Numbering) to amino acid 99 (Kabat Numbering) of the single-domain antibody; the sequence from amino acid 95 (Kabat Numbering) to amino acid 102 (Kabat Numbering) of the single-domain antibody; and the sequence from amino acid 101 (Kabat Numbering) to amino acid 113 (Kabat Numbering) of the single-domain antibody.
(A15) The ligand-binding molecule according to (A13), wherein the single-domain antibody is a single-domain VL antibody, and wherein the cleavage site, or the protease cleavage sequence, or the protease cleavage sequence and the first flexible linker, or the protease cleavage sequence and the first flexible linker and the second flexible linker, is/are introduced into one or more positions comprised in one or more sequences selected from the following sequences of the single-domain antibody:
   the sequence from amino acid 7 (Kabat Numbering) to amino acid 19 (Kabat Numbering) of the single-domain antibody; the sequence from amino acid 24 (Kabat Numbering) to amino acid 34 (Kabat Numbering) of the single-domain antibody; the sequence from amino acid 39 (Kabat Numbering) to amino acid 46 (Kabat Numbering) of the single-domain antibody; the sequence from amino acid 49 (Kabat Numbering) to amino acid 62 (Kabat Numbering) of the single-domain antibody; the sequence from amino acid 50 (Kabat Numbering) to amino acid 56 (Kabat Numbering) of the single-domain antibody; the sequence from amino acid 89 (Kabat Numbering) to amino acid 97 (Kabat Numbering) of the single-domain antibody; and the sequence from amino acid 96 (Kabat Numbering) to amino acid 107 (Kabat Numbering) of the single-domain antibody.
(A16) The ligand-binding molecule according to any one of (A1) to (A15), wherein the ligand is a molecule having biological activity, and the single-domain antibody inhibits the biological activity of the ligand by binding to the ligand.
(A17) The ligand-binding molecule according to any one of (A1) to (A16), wherein the ligand is a molecule having biological activity, and the single-domain antibody has a neutralizing activity against the ligand.
(A18) The ligand-binding molecule according to any one of (A1) to (A17), wherein the ligand-binding molecule comprises only a single-domain antibody comprising the cleavage site or the protease cleavage sequence.
(A19) The ligand-binding molecule according to any one of (A1) to (A18), wherein the ligand-binding molecule further comprises an antibody Fc region.
(A20) The ligand-binding molecule according to any one of (A1) to (A19), wherein the ligand-binding molecule comprises a series of peptide chains consisting of a single-domain antibody-antibody Fc region from the N-terminus to the C-terminus.
(A21) The ligand-binding molecule according to (A1) to (A19), wherein the ligand-binding molecule is a dimer comprising two series of peptide chains consisting of a single-domain antibody-antibody hinge region-antibody Fc region.
(A22) The ligand-binding molecule according to (A19) to (A21), wherein the antibody Fc region is an Fc region comprising one sequence selected from the amino acid sequences set forth in SEQ ID NOs: 103 to 106, or an Fc region mutant obtained by modifying these Fc regions.
(A23) The ligand-binding molecule according to any one of (A1) to (A22), wherein the ligand is a cytokine or chemokine.
(A24) The ligand-binding molecule of any one of (A1) to (A23), wherein the ligand is a ligand selected from interleukin, interferon, hematopoietic factor, TNF superfamily, chemokine, cell growth factor, and TGF-β family.
(A25) The ligand-binding molecule according to any one of (A1) to (A24), wherein the ligand is CXCL10, IL-12, PD-1, IL-6R, or IL-1Ra.
(A26) The ligand-binding molecule according to any one of (A1) to (A25), which is bound to the ligand.
(A27) The ligand-binding molecule according to any one of (A1) to (A25), which is fused with the ligand.
(A28) The ligand-binding molecule according to (A27), wherein, in a state where the ligand-binding molecule is fused with the ligand, the single-domain antibody comprised in the ligand-binding molecule does not bind to another ligand.
(A29) The ligand-binding molecule according to (A27) or (A28), wherein the ligand-binding molecule is fused with the ligand via a linker.
(A30) The ligand-binding molecule according to (A29), wherein the linker does not comprise a protease cleavage sequence.
(A31) A complex formed of the ligand and the ligand-binding molecule according to any one of (A1) to (A25).
(A32) A fusion protein in which the ligand and the ligand-binding molecule according to any one of (A1) to (A25) are fused.
(A33) The fusion protein according to (A32), wherein, in a state where the ligand-binding molecule is fused to the ligand, the single-domain antibody does not bind to another ligand.
(A34) The fusion protein according to (A32) or (A33), wherein the ligand-binding molecule is fused with the ligand via a linker.
(A35) The fusion protein according to (A34), wherein the linker does not comprise a protease cleavage sequence.
(A36) The fusion protein according to (A34) or (A35), which is fused in the order of ligand-linker-ligand-binding molecule from the N-terminus to the C-terminus.
(B1) A ligand-binding molecule, wherein the ligand-binding molecule comprises a single-domain antibody, wherein the single-domain antibody can bind to a ligand and has at least one protease cleavage sequence, wherein the protease cleavage sequence can be cleaved by a target tissue specific protease, and wherein the binding of the ligand-binding molecule to the ligand is attenuated in a state where the proteases cleavage sequence is cleaved compared to the binding of the ligand-binding molecule to the ligand in a state where the cleavage site is not cleaved.
(B2) The ligand-binding molecule according to (B1), wherein the ligand is released from the ligand-binding molecule in a state where the protease cleavage sequence is cleaved.
(B3) The ligand-binding molecule according to any one of (B1) to (B2), wherein the target tissue is a cancer tissue, and the target tissue specific protease is a cancer tissue specific protease.
(B4) The ligand-binding molecule according to (B1) to (B2), wherein the target tissue is an inflammatory tissue, and the target tissue specific protease is an inflammatory tissue specific protease.
(B5) The ligand-binding molecule according to any one of (B1) to (B4), wherein the protease is at least one protease selected from a matriptase, urokinase (uPA), and metalloprotease.
(B6) The ligand-binding molecule according to any one of (B1) to (B5), wherein the protease cleavage sequence is a sequence comprising one or more sequences selected from the sequences set forth in SEQ ID NOs: 2 to 82, 788 to 827, and sequences set forth in Table 1.
(B7) The ligand-binding molecule according to any one of (B1) to (B6), wherein a first flexible linker is further attached to one end of the protease cleavage sequence.
(B8) The ligand-binding molecule according to (B7), wherein the first flexible linker is a flexible linker consisting of a glycine-serine polymer.
(B9) The ligand-binding molecule according to (B7) or (B8), wherein a second flexible linker is further attached to the other end of the protease cleavage sequence.
(B10) The ligand-binding molecule according to (B9), wherein the second flexible linker is a flexible linker consisting of a glycine-serine polymer.
(B11) The ligand-binding molecule according to any one of (B1) to (B10), wherein the single-domain antibody is VHH, or a single-domain VH antibody, or a single-domain VL antibody.
(B12) The ligand-binding molecule according to (B11), wherein the single-domain antibody is VHH or a single-domain VH antibody, and wherein the cleavage site, or the protease cleavage sequence, or the protease cleavage sequence and the first flexible linker, or the protease cleavage sequence and the first flexible linker and the second flexible linker, is/are introduced into one or more positions comprised in one or more sequences selected from the following sequences of the single-domain antibody:
   the sequence from amino acid 7 (Kabat Numbering) to amino acid 17 (Kabat Numbering) of the single-domain antibody; the sequence from amino acid 12 (Kabat Numbering) to amino acid 17 (Kabat Numbering) of the single-domain antibody; the sequence from amino acid 31 (Kabat Numbering) to amino acid 35b (Kabat Numbering) of the single-domain antibody; the sequence from amino acid 40 (Kabat Numbering) to amino acid 47 (Kabat Numbering) of the single-domain antibody; the sequence from amino acid 50 (Kabat Numbering) to amino acid 65 (Kabat Numbering) of the single-domain antibody; the sequence from amino acid 55 (Kabat Numbering) to amino acid 69 (Kabat Numbering) of the single-domain antibody; the sequence from amino acid 73 (Kabat Numbering) to amino acid 79 (Kabat Numbering) of the single-domain antibody; the sequence from amino acid 83 (Kabat Numbering) to amino acid 89 (Kabat Numbering) of the single-domain antibody; the sequence from amino acid 95 (Kabat Numbering) to amino acid 99 (Kabat Numbering) of the single-domain antibody; the sequence from amino acid 95 (Kabat Numbering) to amino acid 102 (Kabat Numbering) of the single-domain antibody; and the sequence from amino acid 101 (Kabat Numbering) to amino acid 113 (Kabat Numbering) of the single-domain antibody.
(B13) The ligand-binding molecule according to (B11), wherein the single-domain antibody is a single-domain VL antibody, and wherein the cleavage site, or the protease cleavage sequence, or the protease cleavage sequence and the first flexible linker, or the protease cleavage sequence and the first flexible linker and the second flexible linker, is/are introduced into one or more positions comprised in one or more sequences selected from the following sequences of the single-domain antibody:
   the sequence from amino acid 7 (Kabat Numbering) to amino acid 19 (Kabat Numbering) of the single-domain antibody; the sequence from amino acid 24 (Kabat Numbering) to amino acid 34 (Kabat Numbering) of the single-domain antibody; the sequence from amino acid 39 (Kabat Numbering) to amino acid 46 (Kabat Numbering) of the single-domain antibody; the sequence from amino acid 49 (Kabat Numbering) to amino acid 62 (Kabat Numbering) of the single-domain antibody; the sequence from amino acid 50 (Kabat Numbering) to amino acid 56 (Kabat Numbering) of the single-domain antibody; the sequence from amino acid 89 (Kabat Numbering) to amino acid 97 (Kabat Numbering) of the single-domain antibody; and the sequence from amino acid 96 (Kabat Numbering) to amino acid 107 (Kabat Numbering) of the single-domain antibody.
(B14) The ligand-binding molecule according to any one of (B1) to (B13), wherein the ligand is a molecule having biological activity, and the single-domain antibody inhibits the biological activity of the ligand by binding to the ligand.
(B15) The ligand-binding molecule according to any one of (B1) to (B14), wherein the ligand is a molecule having biological activity, and the single-domain antibody has a neutralizing activity for the ligand.
(B16) The ligand-binding molecule according to any one of (B1) to (B15), wherein the ligand-binding molecule comprises only a single-domain antibody comprising the cleavage site or the protease cleavage sequence.
(B17) The ligand-binding molecule according to any one of (B1) to (B16), wherein the ligand-binding molecule further comprises an antibody Fc region.
(B18) The ligand-binding molecule according to any one of (B1) to (B17), wherein the ligand-binding molecule comprises a series of peptide chains consisting of a single-domain antibody-antibody Fc region from the N-terminus to the C-terminus.
(B19) The ligand-binding molecule according to (B1) to (B17), wherein the ligand-binding molecule is a dimer comprising two series of peptide chains consisting of a single-domain antibody-antibody hinge region-antibody Fc region.
(B20) The ligand-binding molecule according to (B17) to (B19), wherein the antibody Fc region is an Fc region comprising one sequence selected from the amino acid sequences represented by SEQ ID NOs: 103 to 106, or an Fc region mutant obtained by modifying these Fc regions.
(B21) The ligand-binding molecule according to any one of (B1) to (B20), wherein the ligand is a cytokine or chemokine.
(B22) The ligand-binding molecule according to any one of (B1) to (B21), wherein the ligand is a ligand selected from interleukin, interferon, hematopoietic factor, TNF superfamily, chemokine, cell growth factor, and TGF-β family.
(B23) The ligand-binding molecule according to any one of (B1) to (B22), wherein the ligand is CXCL10, IL-12, PD-1, IL-6R, or IL-1Ra.
(B24) The ligand-binding molecule according to any one of (B1) to (B23), which is bound to the ligand.
(B25) The ligand-binding molecule according to any one of (B1) to (B23), which is fused with the above ligand.
(B26) The ligand-binding molecule according to (B25), wherein, in a state where the ligand-binding molecule is fused with the ligand, the single-domain antibody comprised in the ligand-binding molecule does not bind to another ligand.
(B27) The ligand-binding molecule according to (B25) or (B26), wherein the ligand-binding molecule is fused with the ligand via a linker.
(B28) The ligand-binding molecule according to (B27), wherein the linker does not comprise a protease cleavage sequence.
(B29) A complex formed of the ligand and the ligand-binding molecule according to any one of (B1) to (B23).
(B30) A fusion protein in which the ligand and the ligand-binding molecule according to any one of (B1) to (B23) are fused.
(B31) The fusion protein according to (B30), wherein, in a state where the ligand-binding molecule is fused to the ligand, the single-domain antibody does not bind to another ligand.
(B32) The fusion protein according to (B30) or (B31), wherein the ligand-binding molecule is fused with the ligand via a linker.
(B33) The fusion protein according to (B32), wherein the linker does not comprise a protease cleavage sequence.
(B34) The fusion protein according to (B32) or (B33), which is fused in the order of ligand-linker-ligand-binding molecule from the N-terminus to the C-terminus.
(C1) A ligand-binding molecule comprising a single-domain antibody, wherein the single-domain antibody is VHH or a single-domain VH antibody, and the single-domain antibody has a cleavage site at one or more positions comprised in one or more sequences selected from the following sequences:
   the sequence from amino acid 7 (Kabat Numbering) to amino acid 17 (Kabat Numbering) of the single-domain antibody; the sequence from amino acid 12 (Kabat Numbering) to amino acid 17 (Kabat Numbering) of the single-domain antibody; the sequence from amino acid 31 (Kabat Numbering) to amino acid 35b (Kabat Numbering) of the single-domain antibody; the sequence from amino acid 40 (Kabat Numbering) to amino acid 47 (Kabat Numbering) of the single-domain antibody; the sequence from amino acid 50 (Kabat Numbering) to amino acid 65 (Kabat Numbering) of the single-domain antibody; the sequence from amino acid 55 (Kabat Numbering) to amino acid 69 (Kabat Numbering) of the single-domain antibody; the sequence from amino acid 73 (Kabat Numbering) to amino acid 79 (Kabat Numbering) of the single-domain antibody; the sequence from amino acid 83 (Kabat Numbering) to amino acid 89 (Kabat Numbering) of the single-domain antibody; the sequence from amino acid 95 (Kabat Numbering) to amino acid 99 (Kabat Numbering) of the single-domain antibody; the sequence from amino acid 95 (Kabat Numbering) to amino acid 102 (Kabat Numbering) of the single-domain antibody; and the sequence from amino acid 101 (Kabat Numbering) to amino acid 113 (Kabat Numbering) of the single-domain antibody.
(C2) A ligand-binding molecule comprising a single-domain antibody, wherein the single-domain antibody is a single-domain VL antibody, and the single-domain antibody has a cleavage site at one or more positions comprised in one or more sequences selected from the following sequences:
   the sequence from amino acid 7 (Kabat Numbering) to amino acid 19 (Kabat Numbering) of the single-domain antibody; the sequence from amino acid 24 (Kabat Numbering) to amino acid 34 (Kabat Numbering) of the single-domain antibody; the sequence from amino acid 39 (Kabat Numbering) to amino acid 46 (Kabat Numbering) of the single-domain antibody; the sequence from amino acid 49 (Kabat Numbering) to amino acid 62 (Kabat Numbering) of the single-domain antibody; the sequence from amino acid 50 (Kabat Numbering) to amino acid 56 (Kabat Numbering) of the single-domain antibody; the sequence from amino acid 89 (Kabat Numbering) to amino acid 97 (Kabat Numbering) of the single-domain antibody; and the sequence from amino acid 96 (Kabat Numbering) to amino acid 107 (Kabat Numbering) of the single-domain antibody.
(C3) The ligand-binding molecule according to (C1) or (C2), wherein the single-domain antibody is capable of binding to a ligand, and wherein the binding of the ligand-binding molecule to the ligand in a state where the cleavage site is cleaved is attenuated compared to the binding of the ligand-binding molecule to the ligand in a state where the cleavage site is not cleaved.
(C4) The ligand-binding molecule according to any one of (C1) to (C3), wherein the ligand is released from the ligand-binding molecule in a state where the cleavage site is cleaved.
(C5) The ligand-binding molecule according to any one of (C1) to (C4), wherein the cleavage site comprises a protease cleavage sequence.
(C6) The ligand-binding molecule according to (C5), wherein the protease is a target tissue specific protease.
(C7) The ligand-binding molecule according to (C6), wherein the target tissue is a cancer tissue and the target tissue specific protease is a cancer tissue specific protease.
(C8) The ligand-binding molecule according to (C6), wherein the target tissue is an inflammatory tissue, and the target tissue specific protease is an inflammatory tissue specific protease.
(C9) The ligand-binding molecule according to any one of (C5) to (C8), wherein the protease is at least one protease selected from a matriptase, urokinase (uPA), and metalloprotease.
(C10) A ligand-binding molecule according to any one of (C5) to (C9), wherein the protease cleavage sequence is a sequence comprising one or more sequences selected from the sequences set forth in SEQ ID NOs: 2 to 82, 788 to 827, and sequences set forth in Table 1.
(C11) The ligand-binding molecule of any one of (C5) to (C10), wherein a first flexible linker is further attached to one end of the protease cleavage sequence.
(C12) The ligand-binding molecule according to (C11), wherein the first flexible linker is a flexible linker consisting of a glycine-serine polymer.
(C13) The ligand-binding molecule according to (C11) or (C12), wherein a second flexible linker is further attached to the other end of the protease cleavage sequence.
(C14) The ligand-binding molecule according to (C13), wherein the second flexible linker is a flexible linker consisting of a glycine-serine polymer.
(C15) The ligand-binding molecule according to any one of (C1) to (C14), wherein the ligand is a molecule having biological activity, and the single-domain antibody inhibits the biological activity of the ligand by binding to the ligand.
(C16) The ligand-binding molecule according to any one of (C1) to (C15), wherein the ligand is a molecule having biological activity, and the single-domain antibody has a neutralizing activity for the ligand.
(C17) The ligand-binding molecule according to any one of (C1) to (C16), wherein the ligand-binding molecule comprises only a single-domain comprising the cleavage site or the protease cleavage sequence.
(C18) The ligand-binding molecule according to any one of (C1) to (C17), wherein the ligand-binding molecule further comprises an antibody Fc region.
(C19) The ligand-binding molecule according to any one of (C1) to (C18), wherein the ligand-binding molecule comprises a series of peptide chains consisting of a single-domain antibody-antibody Fc region from the N-terminus to the C-terminus.
(C20) The ligand-binding molecule according to (C1) to (C18), wherein the ligand-binding molecule is a dimer comprising two series of peptide chains consisting of a single-domain antibody-antibody hinge region-antibody Fc region.
(C21) The ligand-binding molecule according to (C18) to (C20), wherein the antibody Fc region is an Fc region comprising one sequence selected from the amino acid sequences represented by SEQ ID NOs: 103 to 106, or an Fc region mutant obtained by modifying these Fc regions.
(C22) The ligand-binding molecule according to any one of (C1) to (C21), wherein the ligand is a cytokine or chemokine.
(C23) The ligand-binding molecule according to any one of (C1) to (C22), wherein the ligand is a ligand selected from interleukin, interferon, hematopoietic factor, TNF superfamily, chemokine, cell growth factor, and TGF-β family.
(C24) The ligand-binding molecule according to any one of (C1) to (C23), wherein the ligand is CXCL10, IL-12, PD-1, IL-6R, or IL-1Ra.
(C25) The ligand-binding molecule according to any one of (C1) to (C24), which is bound to the ligand.
(C26) The ligand-binding molecule according to any one of (C1) to (C24), which is fused with the ligand.
(C27) The ligand-binding molecule according to (C26), wherein, in a state where the ligand-binding molecule is fused with the ligand, the single-domain antibody comprised in the ligand-binding molecule does not bind to another ligand.
(C28) The ligand-binding molecule according to (C26) or (C27), wherein the ligand-binding molecule is fused with the ligand via a linker.
(C29) The ligand-binding molecule according to (C28), wherein the linker does not comprise a protease cleavage sequence.
(C30) A complex formed of the ligand and the ligand-binding molecule according to any one of (C1) to (C24).
(C31) A fusion protein in which the ligand and the ligand-binding molecule according to any one of (C1) to (C24) are fused.
(C32) The fusion protein according to (C31), wherein, in a state where the ligand-binding molecule is fused with the ligand, the single-domain antibody does not bind to another ligand.
(C33) The fusion protein according to (C31) or (C32), wherein the ligand-binding molecule is fused with the ligand via a linker.
(C34) The fusion protein according to (C33), wherein the linker does not comprise a protease cleavage sequence.
(C35) The fusion protein according to (C33) or (C34), which is fused in the order of ligand-linker-ligand-binding molecule from the N-terminus to the C-terminus.
(D1) A pharmaceutical composition comprising the ligand-binding molecule according to any one of (A1) to (A27), (B1) to (B25), and (C1) to (C26).
(D2) A pharmaceutical composition comprising the ligand-binding molecule according to any one of (A1) to (A26), (B1) to (B24), and (C1) to (C25), and the ligand.
(D3) A pharmaceutical composition comprising the complex according to (A31) or (B29) or (C30).
(D4) A pharmaceutical composition comprising the fusion protein according to any one of (A32) to (A36), (B30) to (B34), and (C31) to (C35).
(E1) A method for producing the ligand-binding molecule according to any one of (A1) to (A25), (B1) to (B24), and (C1) to (C25).
(E2) The production method according to (E1), which comprises introducing a protease cleavage sequence into the single-domain antibody in the ligand-binding molecule comprising the single-domain antibody.
(E3) A method for producing the fusion protein according to any one of (A32) to (A36), (B30) to (B34), and (C31) to (C35), which comprises fusing a ligand-binding molecule comprising a single-domain antibody having a protease cleavage sequence introduced thereinto and a ligand capable of binding to the single-domain antibody.
(E4) A polynucleotide that encodes the ligand-binding molecule according to any one of (A1) to (A25), (B1) to (B23), and (C1) to (C24).
(E5) A vector comprising the polynucleotide according to (E4).
(E6) A host cell comprising the polynucleotide according to (E4) or the vector according to (E5).
(E7) A method for producing the ligand- binding molecule according to any one of (A1) to (A25), (B1) to (B23), and (C1) to (C24), which comprises culturing the host cell according to (E6).
(E8) A polynucleotide that encodes the fusion protein according to any one of (A32) to (A36), (B30) to (B34), and (C31) to (C35).
(E9) A vector comprising the polynucleotide according to (E8).
(E10) A host cell comprising the polynucleotide according to (E8) or the vector according to (E9).
(E11) A method for producing the fusion protein according to any one of (A32) to (A36), (B30) to (B34), and (C31) to (C35), which comprises culturing the host cell according to (E10).

### [Brief Description of Drawings]

[Fig. 1] Fig. 1 is a diagram showing an example of a ligand-binding molecule of the present invention. In a state where the cleavage site contained in the single-domain antibody is not cleaved, the single-domain antibody can bind to the ligand. In a state where the cleavage site is cleaved, the single-domain antibody is cleaved, it cannot bind to the ligand, and the ligand comes off.
[Fig. 2] Fig. 2 is a diagram showing an example of a fusion protein of a ligand-binding molecule of the present invention and the ligand. In a state where the cleavage site contained in the single-domain antibody is not cleaved, the single-domain antibody within the fusion protein can bind to the ligand within the fusion protein, and in a state where the cleavage site is cleaved, the single-domain antibody is cleaved and is no longer capable of binding to the ligand, and the portion of the fusion protein containing the ligand comes off.
[Fig. 3] Fig. 3 is a diagram showing another example of a ligand-binding molecule of the present invention. In this example, the ligand-binding molecule is a dimeric protein containing a single-domain antibody-antibody hinge region-antibody Fc region.
[Fig. 4] Fig. 4 is a diagram showing another example of a fusion protein of a ligand-binding molecule of the present invention and the ligand. In this example, the ligand-binding molecule is a dimeric protein containing a single-domain antibody-antibody hinge region-antibody Fc region.

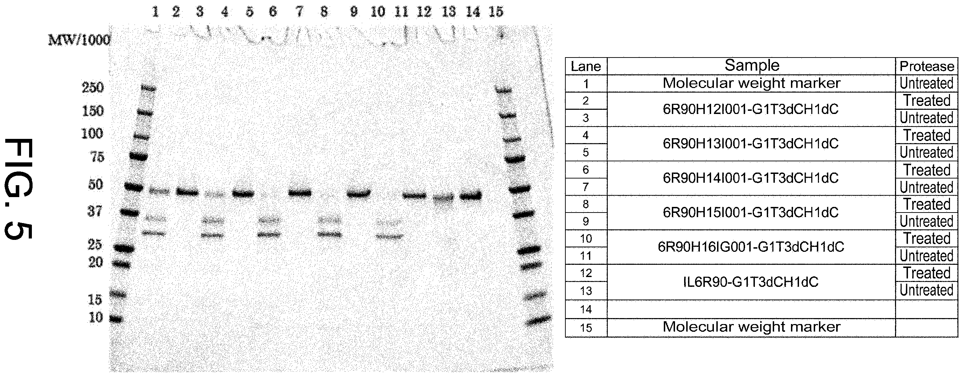

[Fig. 5] Fig. 5 shows the results of an SDS-PAGE of ligand-binding molecules treated with a protease and ligand-binding molecules not treated with the protease. The bands of control molecules (Lanes 12 and 13) not containing the protease cleavage sequence are in the same position regardless of the presence or absence of protease treatment, while for ligand-binding molecules to which each protease-cleaving sequence is introduced there is a new band that occurs only after the protease treatment, showing that single-domain antibody-containing ligand-binding molecules introduced with each protease cleavage sequence have been cleaved by the protease treatment.
[Fig. 6] Fig. 6 shows real-time binding graphs that evaluate the binding to IL-6R of protease-treated ligand-binding molecules and protease-untreated ligand-binding molecules. The title of each graph is the name of the measured sample, and the vertical axis is the relative coupling of the ligand-binding molecule and IL-6R, and the horizontal axis shows the time(s). The gray line shows data of protease-untreated samples, and the black line shows data of protease-treated samples.

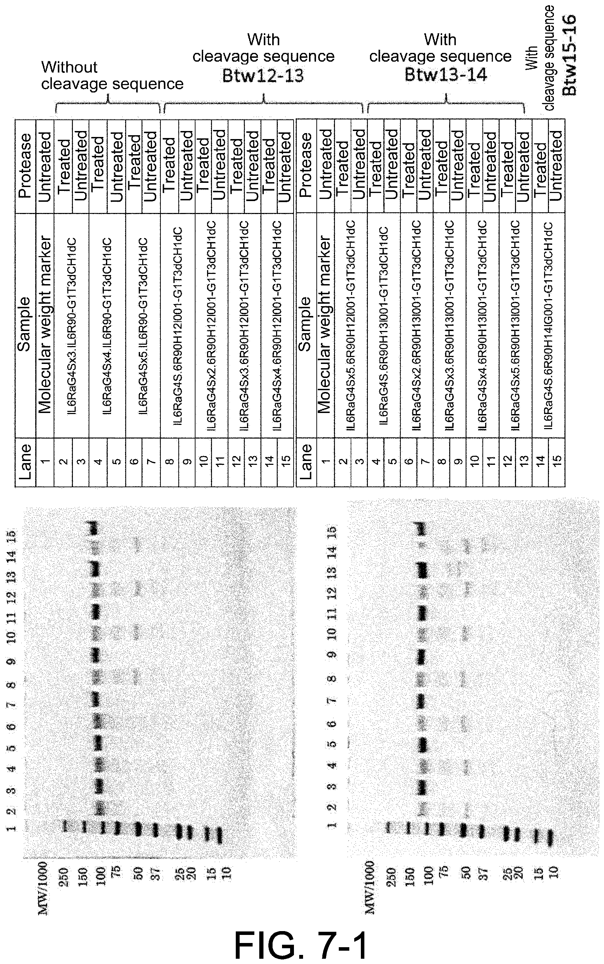

[Fig. 7-1] Fig. 7-1 shows the results of SDS-PAGE of fusion proteins treated with a protease and fusion proteins not treated with the protease. The bands of control molecules not including the protease cleavage sequence are in the same position regardless of the presence or absence of protease treatment. In contrast, for each fusion protein containing a ligand-binding molecule comprising a single-domain antibody introduced with a protease cleavage sequence there is a new band that occurs only after the protease treatment; that is, it shows that each fusion protein including a ligand-binding molecule containing a single-domain antibody introduced with each protease cleavage sequence, has been cleaved by the protease treatment.
[Fig. 7-2] Fig. 7-2 is the continuation of Fig. 7-1.
[Fig. 7-3] Fig. 7-3 is the continuation of Fig. 7-2.
[Fig. 7-4] Fig. 7-4 is the continuation of Fig. 7-3.
[Fig. 8-1] Fig. 8-1 shows real-time graphs that evaluate the binding of free IL-6R to the biotinylated anti-IL-6R single-domain antibody-containing molecule (IL6R90-bio) present in solutions containing fusion proteins treated with proteases and fusion proteins not treated with proteases. The title of each graph is the name of the measured sample. The vertical axis shows the relative binding of IL-6R and IL6R90-bio, and the horizontal axis shows the time (s). The gray line shows data of protease-untreated samples, and the black line shows data of protease-treated samples.
[Fig. 8-2] Fig. 8-2 is the continuation of Fig. 8-1.
[Fig. 9] Fig. 9 shows the results of SDS-PAGE of fusion proteins treated with a protease and fusion proteins not treated with the protease.
[Fig. 10] Fig. 10 shows real-time graphs indicating the binding of free human PD-1 to the biotinylated anti-PD-1 single-domain antibody-containing molecule (PD1-bio) present in solutions containing protease-treated fusion proteins and protease-untreated fusion proteins. The title of each graph is the name of the measured sample, the vertical axis shows the relative binding of PD-1 and PD1-bio, and the horizontal axis shows the time(s). The gray line shows data of protease-untreated samples, and the black line shows data of protease-treated samples.
[Fig. 11] Fig. 11 shows the results of protease cleavage of IgGs into which various protease cleavage sequences have been introduced.
[Fig. 12] Fig. 12 shows the results of protease cleavage of IgGs into which various protease cleavage sequences have been introduced.
[Fig. 13] Fig. 13 shows the results of protease cleavage of IgGs into which various protease cleavage sequences have been introduced.

### [Description of Embodiments]

### Polypeptide

The term polypeptide as used in the present invention usually refers to a peptide having a length of about 4 amino acids or more, and a protein. When a chain of amino acids connected by a peptide bond from the N-terminal to the C-terminal is considered as a series of peptide chains, the polypeptide according to the present invention may be a complexed protein in which multiple series of peptide chains are formed by interactions such as SS bonds, hydrophobic interactions, and ionic bonds. Furthermore, the polypeptides of the present invention are usually polypeptides consisting of artificially designed sequences, but are not particularly limited thereto, and may be, for example, polypeptides derived from living organisms. They may be any of natural polypeptides, synthetic polypeptides, recombinant polypeptides, or the like. In addition, fragments of the above-mentioned polypeptides are also included in the polypeptides of the present invention.

### Amino acid

In the present specification, each amino acid is indicated by one-letter code or three-letter code, or both, as represented by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

### Amino acid alteration

For the alteration of an amino acid in the amino acid sequence of a polypeptide, a method known in the art such as site-directed mutagenesis (Kunkel et al. (Proc. Natl. Acad. Sci. USA (1985) 82, 488-492)) or overlap extension PCR can be appropriately adopted. A plurality of methods known in the art can also be adopted as alteration methods for substituting an amino acid by an amino acid other than a natural amino acid (Annu. Rev. Biophys. Biomol. Struct. (2006) 35, 225-249; and Proc. Natl. Acad. Sci. U.S.A. (2003) 100 (11), 6353-6357). For example, a tRNA-containing cell-free translation system (Clover Direct (Protein Express)) in which a non-natural amino acid is bound to an amber suppressor tRNA complementary to UAG codon (amber codon), which is a stop codon, is also preferably used.

In the present specification, the term "and/or" used to represent amino acid alteration sites is meant to include every combination in which "and" and "or" are appropriately combined. Specifically, for example, the phrase "amino acids at positions 37, 45, and/or 47 are substituted" includes the following variations of amino acid alteration:
(a) position 37, (b) position 45, (c) position 47, (d) positions 37 and 45, (e) positions 37 and 47, (f) positions 45 and 47, and (g) positions 37, 45, and 47.

In the present specification, expression in which the one-letter codes or three-letter-codes of amino acids before and after alteration are written prior to and subsequent to a number representing a particular position can be appropriately used for representing amino acid alteration. For example, the alteration F37V or Phe37Val used for substituting an amino acid contained in an antibody variable region represents the substitution of Phe at position 37 defined by the Kabat numbering with Val. Specifically, the number represents an amino acid position defined by the Kabat numbering; the one-letter code or three-letter code of the amino acid written prior to the number represents the amino acid before the substitution; and the one-letter code or three-letter code of the amino acid subsequent to the number represents the amino acid after the substitution. Likewise, the alteration P238A or Pro238Ala used for substituting an amino acid in an Fc region contained in an antibody constant region represents the substitution of Pro at position 238 defined by the EU numbering with Ala. Specifically, the number represents an amino acid position defined by the EU numbering; the one-letter code or three-letter code of the amino acid written prior to the number represents the amino acid before the substitution; and the one-letter code or three-letter code of the amino acid subsequent to the number represents the amino acid after the substitution.

### Antibody and antibody fragment

In the present specification, the term "antibody" is used in the broadest sense and encompasses various antibody structures including, but are not limited to, a monoclonal antibody, a polyclonal antibody, a multispecific antibody (e.g., a bispecific antibody), and an antibody fragment as long as the antibody exhibits the desired antigen binding activity.

"Antibody fragment" refers to a molecule, other than a complete antibody, including a portion of a complete antibody and binding to an antigen to which the complete antibody binds. Examples of the antibody fragment include, but are not limited to, Fv, Fab, Fab', Fab'-SH, F(ab')₂, diabodies, linear antibodies, single-chain antibody molecules (e.g., scFv), and multispecific antibodies formed from antibody fragments.

The terms "full-length antibody", "complete antibody", and "whole antibody" are used interchangeably with each other in the present specification and refer to an antibody having a structure substantially similar to a natural antibody structure, or having a heavy chain containing an Fc region defined in the present specification.

### Variable region

The term "variable region" or "variable domain" refers to a domain of an antibody heavy chain or light chain involved in the binding of the antibody to its antigen. Usually, antibody heavy chain and light chain variable domains (VH and VL, respectively) have a similar structure and each domain contains 4 conserved framework regions (FRs) and 3 complementarity determining regions (CDRs) (see, e.g., Kindt et al., Kuby Immunology, 6th ed., W.H. Freeman and Co., page 91 (2007)). One VH or VL domain should be sufficient for conferring antigen binding specificity.

### CDR

The term "complementarity determining region" or "CDR" used in the present specification is hypervariable in the sequence, and/or forms a structurally determined loop ("hypervariable loop"), and/or refers to antigen contact residues ("antigen contacts") or each region of an antibody variable domain or a single-domain antibody.

Usually, an antibody contains 6 CDRs: three in VH (HI, H2, and H3), and three in VL (L1, L2, and L3). In the present specification, exemplary antibody CDRs include the following:
(a) hypervariable loops occurring at amino acid residues 26 to 32 (L1), 50 to 52 (L2), 91 to 96 (L3), 26 to 32 (HI), 53 to 55 (H2), and 96 to 101 (H3) (Chothia and Lesk, J. Mol. Biol. 196: 901-917 (1987));
(b) CDRs occurring at amino acid residues 24 to 34 (L1), 50 to 56 (L2), 89 to 97 (L3), 31 to 35b (HI), 50 to 65 (H2), and 95 to 102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c to 36 (L1), 46 to 55 (L2), 89 to 96 (L3), 30 to 35b (HI), 47 to 58 (H2), and 93 to 101 (H3) (MacCallum et al., J. Mol. Biol. 262: 732-745 (1996)); and
(d) a combination of (a), (b), and/or (c) containing CDR amino acid residues 46 to 56 (L2), 47 to 56 (L2), 48 to 56 (L2), 49 to 56 (L2), 26 to 35 (HI), 26 to 35b (HI), 49 to 65 (H2), 93 to 102 (H3), or 94 to 102 (H3).

Generally, a single-domain antibody comprises three CDRs: CDR1, CDR2, and CDR3. When a single-domain antibody is a VHH or a single-domain VH antibody, the single-domain antibody CDRs exemplarily include the following:
(a) hypervariable loops occurring at amino acid residues 26 to 32 (CDR1), 53 to 55 (CDR2), and 96 to 101 (CDR3) (Chothia and Lesk, J. Mol. Biol. 196: 901-917 (1987));
(b) CDRs occurring at amino acid residues 31 to 35b (CDR1), 50 to 65 (CDR2), and 95 to 102 (CDR3) (Kabat et al., Sequences of Proteins of Immunological Interest, 5th Ed. Public Health Service, National Institutes of Health, Bethesda, MD (1991));
(c) antigen contacts occurring at amino acid residues 30 to 35b (CDR1), 47 to 58 (CDR2), and 93 to 101 (CDR3) (MacCallum et al., J. Mol. Biol. 262: 732-745 (1996)); and
(d) a combination of (a), (b), and/or (c), including CDR amino acid residues 26 to 35 (CDR1), 26 to 35b (CDR1), 49 to 65 (CDR2), 93 to 102 (CDR3), or 94 to 102 (CDR3).

When the single-domain antibody is a single-domain VL antibody, the single-domain antibody CDRs exemplarily include the following:
(a) hypervariable loops occurring at amino acid residues 26 to 32 (CDR1), 50 to 52 (CDR2), and 91 to 96 (CDR3) (Chothia and Lesk, J. Mol. Biol. 196: 901-917 (1987));
(b) CDRs occurring at amino acid residues 24 to 34 (CDR1), 50 to 56 (CDR2), and 89 to 97 (CDR3) (Kabat et al., Sequences of Proteins of Immunological Interest, 5th Ed. Public Health Service, National Institutes of Health, Bethesda, MD (1991));
(c) antigen contacts occurring at amino acid residues 27c to 36 (CDR1), 46 to 55 (CDR2), and 89 to 96 (CDR3) (MacCallum et al., J. Mol. Biol. 262: 732-745 (1996)); and
(d) a combination of (a), (b), and/or (c), including CDR amino acid residues 46 to 56 (CDR2), 47 to 56 (CDR2), 48 to 56 (CDR2), or 49 to 56 (CDR2).

In the present specification, CDR residues and other residues (e.g., FR residues) in a variable domain are numbered according to Kabat et al. (supra), unless otherwise specified.

### FR

The term "framework" or "FR" refers to variable domain residues or single-domain antibody residues other than complementarity determining region (CDR) residues. FRs in a variable domain or a single-domain antibody generally consist of 4 FR domains: FR1, FR2, FR3, and FR4. Accordingly, the sequences of CDRs and FRs usually appear in VH (or VL) in the following order: FR1-H1 (L1)-FR2-H2 (L2)-FR3-H3 (L3)-FR4. In a single-domain antibody, the sequences of CDRs and FRs generally appear in the following order: FR1-CDR1-FR2-CDR2-FR3-CDR3 -FR4.

"Human consensus framework" is a framework that shows the most commonly-occurring amino acid residues in a selected group of human immunoglobulin VL or VH framework sequences. Usually, the selection of the human immunoglobulin VL or VH sequence is from a subgroup of variable domain sequences. Usually, the sequence subgroup is a subgroup described in Kabat et al., Sequences of Proteins of Immunological Interest, Fifth Edition, NIH Publication 91-3242, Bethesda MD (1991), vols. 1-3. In one embodiment, for VL, the subgroup is the subgroup κI by Kabat et al. above. In one embodiment, for VH, the subgroup is subgroup III by Kabat et al. above.

### Constant region

In the present specification, the term "constant region" or "constant domain" refers to a part other than variable regions in an antibody. For example, an IgG antibody is a heterotetrameric glycoprotein of approximately 150,000 Da constituted by two identical light chains and two identical heavy chains connected through disulfide bonds. Each heavy chain has a variable region (VH) also called variable heavy chain domain or heavy chain variable domain, followed by a heavy chain constant region (CH) containing a CH1 domain, a hinge region, a CH2 domain, and a CH3 domain, from the N terminus to the C terminus. Likewise, each light chain has a variable region (VL) also called variable light chain domain or light chain variable domain, followed by a constant light chain (CL) domain, from the N terminus to the C terminus. The light chains of natural antibodies may be attributed to one of two types called kappa (κ) and lambda (λ) on the basis of the amino acid sequences of their constant domains. The term "comprising/comprises a constant region" may include the entire constant region or may include a part of a constant region.

The "class" of an antibody refers to the type of a constant domain or a constant region carried by the heavy chain of the antibody. Antibodies have 5 major classes: IgA, IgD, IgE, IgG, and IgM. Some of these classes may be further divided into subclasses (isotypes), for example, IgG1, IgG2, IgG3, IgG4, IgA1, and IgA2. Heavy chain constant domains corresponding to immunoglobulins of different classes are called α, δ, ε, γ, and µ, respectively.

### Fc region

In the present specification, the term "Fc region" is used for defining the C-terminal region of immunoglobulin heavy chains, including at least a portion of constant regions. This term includes an Fc region having a natural sequence and a mutant Fc region. In one embodiment, the heavy chain Fc region of human IgG1 spans from Cys226 or from Pro230 to the carboxyl terminus of the heavy chain. However, the C-terminal lysine (Lys447) or glycine-lysine (Gly446-Lys447) of the Fc region may be present or absent. In the present specification, amino acid residues in an Fc region or a constant region are numbered according to the EU numbering system (also called EU index) described in Kabat et al., Sequences of Proteins of Immunological Interest, 5th Ed. Public Health Service, National Institutes of Health, Bethesda, MD 1991, unless otherwise specified.

### Hinge region

The term "hinge region" or "antibody hinge region" can refer to the region composed of the 216^{th} to 230^{th} amino acids according to EU numbering in the antibody heavy chain, or a part thereof.

### Single-domain antibody

In the present specification, the "single-domain antibody" is an antibody capable of exhibiting an antigen-binding activity by that domain alone. The structure of a single-domain antibody is not limited as long as it can exert an antigen-binding activity by that domain alone. Ordinary antibodies, exemplified by IgG antibodies and such, show antigen binding activity in a state where a variable region is formed by VH and VL pairing, whereas a single-domain antibody does not pair with other domains, and it is known that the domain structure of the single-domain antibody itself can exert antigen-binding activity. Single-domain antibodies usually have a relatively low molecular weight and exist in the form of monomers. In several embodiments, the form of the single-domain antibody is similar to those of an antibody heavy chain variable region or an antibody light chain variable region.

Examples of single-domain antibodies include, but are not limited to, the variable region of the heavy chain antibody from animals of the family *Camelidae* (VHH), an antigen-binding molecule congenitally lacking a light chain such as shark V_{NAR}, or antibody fragments that include all or part of an antibody VH domain or all or part of an antibody VL domain. Examples of single-domain antibodies that are antibody fragments that include all or part of an antibody VH/VL domain include, but are not limited to, an artificially-produced single-domain antibody starting from the human antibody VH or human antibody VL (hereinafter, single-domain VH antibody, single-domain VL antibody) as described in US Patent No. 6,248,516 B1, and such.

The single-domain antibody can be obtained from an animal capable of producing the single-domain antibody or by the immunization of the animal capable of producing the single-domain antibody. Examples of the animal capable of producing the single-domain antibody include, but are not limited to, animals of the family *Camelidae,* and transgenic animals harboring a gene capable of raising the single-domain antibody. The animals of the family *Camelidae* include camels, lamas, alpacas, one-hump camels and guanacos, etc. Examples of the transgenic animals harboring a gene capable of raising the single-domain antibody include, but are not limited to, transgenic animals described in WO2015/143414 and U.S. Patent Publication No. US2011/0123527 A1. The framework sequences of the single-domain antibody obtained from the animal may be converted to human germline sequences or sequences similar thereto to obtain a humanized single-domain antibody. The humanized single-domain antibody (e.g., humanized VHH) is also one embodiment of the single-domain antibody of the present invention.

The single-domain antibody can be obtained from an animal capable of producing the single-domain antibody or by the immunization of the animal capable of producing the single-domain antibody. Examples of the animal capable of producing the single-domain antibody include, but are not limited to, animals of the family *Camelidae,* and transgenic animals harboring a gene capable of raising the single-domain antibody. The animals of the family *Camelidae* include camels, lamas, alpacas, one-hump camels and guanacos, etc. Examples of the transgenic animals harboring a gene capable of raising the single-domain antibody include, but are not limited to, transgenic animals described in WO2015/143414 and U.S. Patent Publication No. US2011/0123527 A1. The framework sequences of the single-domain antibody obtained from the animal may be converted to human germline sequences or sequences similar thereto to obtain a humanized single-domain antibody. The humanized single-domain antibody (e.g., humanized VHH) is also one embodiment of the single-domain antibody of the present invention.

Alternatively, the single-domain antibody can be obtained by ELISA, panning, or the like from a polypeptide library containing single-domain antibodies. Examples of the polypeptide library containing single-domain antibodies include, but are not limited to, naive antibody libraries obtained from various animals or humans (e.g., Methods in Molecular Biology 2012 911 (65-78); and Biochimica et Biophysica Acta - Proteins and Proteomics 2006 1764: 8 (1307-1319)), antibody libraries obtained by the immunization of various animals (e.g., Journal of Applied Microbiology 2014 117: 2 (528-536)), and synthetic antibody libraries prepared from antibody genes of various animals or humans (e.g., Journal of Biomolecular Screening 2016 21: 1 (35-43); Journal of Biological Chemistry 2016 291:24 (12641-12657); and AIDS 2016 30: 11 (1691-1701)).

In the present invention, there is an embodiment in which a cleavage site/protease cleavage sequence is introduced into a single-domain antibody, but the expression "single-domain antibody" can be used regardless of whether or not a cleavage site/protease cleavage sequence is introduced.

In several embodiments, the single-domain antibody of the present invention can generally be defined as a polypeptide comprising:
a) an amino acid sequence consisting of four framework regions/sequences having three complementarity determining regions/sequences inserted therebetween (the amino acid residue at position 11 according to Kabat numbering is selected from the group consisting of L, M, S, V, and W, and is preferably L), and/or
b) an amino acid sequence consisting of four framework regions/sequences having three complementarity determining regions/sequences inserted therebetween (the amino acid residue at position 37 according to Kabat numbering is selected from the group consisting of F, Y, H, I, L, and V, and is preferably F or Y), and/or
c) an amino acid sequence consisting of four framework regions/sequences having three complementarity determining regions/sequences inserted therebetween (the amino acid residue at position 44 according to Kabat numbering is selected from the group consisting of G, E, A, D, Q, R, S, and L, and is preferably G, E or Q, and is more preferably G or E), and/or
d) an amino acid sequence consisting of four framework regions/sequences having three complementarity determining regions/sequences inserted therebetween (the amino acid residue at position 45 according to Kabat numbering is selected from the group consisting of L, R, C, I, L, P, Q, and V, and is preferably L or R), and/or
e) an amino acid sequence consisting of four framework regions/sequences having three complementarity determining regions/sequences inserted therebetween (the amino acid residue at position 47 according to Kabat numbering is selected from the group consisting of W, L, F, A, G, I, M, R, S, V, and Y, and is preferably W, L, F or R), and/or
f) an amino acid sequence consisting of four framework regions/sequences having three complementarity determining regions/sequences inserted therebetween (the amino acid residue at position 83 according to Kabat numbering is selected from the group consisting of R, K, N, E, G, I, M, Q, and T, and is preferably K or R, and is more preferably K), and/or
g) an amino acid sequence consisting of four framework regions/sequences having three complementarity determining regions/sequences inserted therebetween (the amino acid residue at position 84 according to Kabat numbering is selected from the group consisting of P, A, L, R, S, T, D, and V, and is preferably P), and/or
h) an amino acid sequence consisting of four framework regions/sequences having three complementarity determining regions/sequences inserted therebetween (the amino acid residue at position 103 according to Kabat numbering is selected from the group consisting of W, P, R and S, and is preferably W), and/or
i) an amino acid sequence consisting of four framework regions/sequences having three complementarity determining regions/sequences inserted therebetween (the amino acid residue at position 104 according to Kabat numbering is G or D, and is preferably G), and/or
j) an amino acid sequence consisting of four framework regions/sequences having three complementarity determining regions/sequences inserted therebetween (the amino acid residue at position 108 according to Kabat numbering is selected from the group consisting of Q, L and R, and is preferably Q or L).

More specifically, but not exclusively, a single-domain antibody can be defined as a polypeptide comprising any one of the amino acid sequences consisting of four framework regions/sequences having the following three complementarity determining regions/sequences inserted therebetween:
k) an amino acid sequence in which the amino acid residues at positions 43 to 46 according to Kabat numbering are KERE or KQRE;
1) an amino acid sequence in which the amino acid residues at positions 44 to 47 according to Kabat numbering are GLEW; and,
m) an amino acid sequence in which the amino acid residues at positions 83 to 84 according to Kabat numbering are KP or EP.

### Chimeric antibody

The term "chimeric" antibody refers to an antibody where a part of its heavy chain and/or light chain is derived from a particular source or species, while the rest of its heavy chain and/or light chain is derived from a different source or species. A "chimeric single-domain antibody" is a single-domain antibody in which a portion of the single-domain antibody is derived from a particular source or species, while the remaining portion of the single-domain antibody is derived from a different source or species.

### Humanized antibody

"Humanized" antibody refers to chimeric antibodies that contain amino acid residues from non-human CDRs and amino acid residues from human FRs. In some embodiments, the humanized antibody includes substantially all of, at least one, and typically two variable domains, in which all or substantially all CDRs correspond to those of a nonhuman antibody, and all or substantially all FRs correspond to those of a human antibody. "Humanized single-domain antibody" refers to a chimeric single-domain antibody that contains amino acid residues from non-human CDRs and amino acid residues from human FRs. In some embodiments, in the humanized single-domain antibody, all or substantially all CDRs correspond to those of a non-human antibody, and all or substantially all FRs correspond to those of a human antibody. In a humanized antibody, even if some of the residues in the FRs do not correspond to those of a human antibody, it is considered as an example where substantially all FRs correspond to those of a human antibody. For example, when humanizing VHH, which is one embodiment of a single-domain antibody, it is necessary to make some of the residues in FRs into residues that do not correspond to those of a human antibody (C Vincke et al., The Journal of Biological Chemistry 284, 3273-3284.).

A humanized antibody may optionally include at least a portion of an antibody constant region derived from a human antibody. The "humanized form" of an antibody (e.g., a non-human antibody) is an antibody that has undergone humanization.

### Affinity

"Affinity" refers to the total strength of a non-covalent interaction between one binding site of a molecule (e.g., antibody) and the molecule's binding partner (e.g., antigen). Unless otherwise indicated, "binding affinity" as used in the present specification refers to a unique binding affinity that reflects a 1:1 interaction between members of a binding pair (e.g., antibody and antigen). The affinity of molecule X for its partner Y can generally be represented by the dissociation constant (Kd). Affinity can be measured by conventional methods known in the art, including those described in the present specification. Specific illustrative and exemplary embodiments for measuring binding affinity are discussed below.

### Antibodies that bind to the same epitope

The term "antibody that binds to the same epitope" as a reference antibody refers to an antibody that blocks the binding of the reference antibody to its own antigen by 50% or more in a competitive assay, and conversely, the reference antibody inhibits the binding of the aforementioned antibody to its antigen by 50% or more in the competitive assay. An exemplary competitive assay is provided in the present specification.

### Isolated antibody

An "isolated" antibody is one that has been separated from components of its original environment. In some embodiments, the antibody can be, for example, measured by electrophoresis (e.g., SDS-PAGE, isoelectric focusing (IEF), capillary electrophoresis) or chromatography (e.g., ion exchange or reverse phase HPLC) and purified to >95% or >99% purity. For a review of methods for assessing antibody purity, see, for example, Flatman et al., J. Chromatogr. B 848:79-87 (2007).

### Monoclonal antibody

As used herein, the term "monoclonal antibody" refers to an antibody obtained from a substantially homogeneous population of antibodies. That is, the individual antibodies that make up the population are identical apart from possible mutant antibodies (e.g., mutant antibodies containing naturally-occurring mutations, or mutant antibodies that occur during the production of monoclonal antibody preparations, where such mutants are usually present in small amounts), and/or bind to the same epitope. In contrast to polyclonal antibody preparations that typically contain different antibodies against different determinants (epitopes), each monoclonal antibody of a monoclonal antibody preparation is against a single determinant on the antigen. The modifier "monoclonal" indicates the characteristics of an antibody of being obtained from a substantially homogeneous population of antibodies and is not to be construed as requiring the production of the antibody by any particular method. For example, monoclonal antibodies used according to the present invention include, but are not limited to, those generated by a variety of techniques including the hybridoma method, recombinant DNA method, phage display method, and methods using transgenic animals having all or a part of human immunoglobulin loci. Such methods and other exemplary methods for making monoclonal antibodies are described in the present specification.

### Sequence identity

"Percent (%) amino acid sequence identity" to a reference polypeptide sequence is defined as the percentage ratio of amino acid residues in a candidate sequence that are identical to the amino acid residues in the reference polypeptide sequence, after aligning the sequences to obtain maximal percent sequence identity, and, if necessary, introducing gaps, when no conservative substitutions are considered to be part of the sequence identity. The alignment of interest for determining the percent amino acid sequence identify can be done using various methods within the technical scope of the technical field, for example, publicly available computer software such as BLAST, BLAST-2, ALIGN, Megalign (DNASTAR) software, or GENETYX(registered trademark) (GENETYX CORPORATION), and such. Those skilled in the art can determine the appropriate parameters for taking an alignment of sequences, including any algorithms needed to achieve the maximum alignment over the entire length of the sequences being compared.

### Vector

As used in the present specification, the term "vector" refers to a nucleic acid molecule capable of multiplying another nucleic acid to which it has been linked. This term refers to a vector as a self-replicating nucleic acid structure, and includes vectors incorporated into the genome of host cells into which they have been introduced. Certain vectors are capable of effecting the expression of a nucleic acid to which they are operably linked. Such a vector is also referred to as an "expression vector" in the present specification.

### Host cell etc.

The terms "host cell", "host cell line", and "host cell culture" are used interchangeably and refer to a cell introduced with a foreign nucleic acid (including progeny of such a cell). Host cells include "transformants" and "transformed cells", which include first generation transformed cells and progenies derived from those cells regardless of passage number. Progenies need not be completely identical to the parent cell in terms of nucleic acid content and may include mutations. Mutant progenies that have the same function or biological activity as that used when screening or selecting the original transformed cells are also included in this specification.

### Protease

In the present specification, the term "protease" refers to an enzyme such as endopeptidase or exopeptidase which hydrolyzes a peptide bond, and typically to an endopeptidase. The protease used in the present invention is limited only by its capacity to cleave the protease cleavage sequence and is not particularly limited by its type. In some embodiments, target tissue specific protease is used. The target tissue specific protease can refer to, for example, any of
(1) protease that is expressed at a higher level in the target tissue than in normal tissues,
(2) protease that has higher activity in the target tissue than in normal tissues,
(3) protease that is expressed at a higher level in the target cells than in normal cells, and
(4) protease that has higher activity in the target cells than in normal cells.

In a more specific embodiment, a cancer tissue specific protease or an inflammatory tissue specific protease is used.

### Target tissue

In the present specification, the term "target tissue" means a tissue containing at least one target cell. In some embodiments of the present invention, the target tissue is a cancer tissue. In some embodiments of the present invention, the target tissue is an inflammatory tissue.

The term "cancer tissue" means a tissue containing at least one cancer cell. Thus, considering that, for example, cancer tissue contains cancer cells and vascular vessels, every cell type that contributes to the formation of tumor mass containing cancer cells and endothelial cells is implied. In the present specification, tumor mass refers to a foci of tumor tissue. The term "tumor" is generally used to mean benign neoplasm or malignant neoplasm.

In the present specification, examples of "inflammatory tissue" include the following:
a joint in rheumatoid arthritis or osteoarthritis,
a lung (alveolus) in bronchial asthma or COPD,
a digestive organ in inflammatory bowel disease, Crohn disease, or ulcerative colitis,
a fibrotic tissue in fibrosis in the liver, the kidney, or the lung,
a tissue showing rejection reaction in organ transplantation,
a vascular vessel or heart (cardiac muscle) in arteriosclerosis or heart failure,
a visceral fat in metabolic syndrome,
a skin tissue in atopic dermatitis and other dermatitides, and
a spinal nerve in disk herniation or chronic lumbago.

### Target tissue specific protease

In several types of target tissue, protease specifically expressed or specifically activated therein or protease considered to be related to the disease condition of the target tissue (target tissue specific protease) is known. For example, WO2013/128194, WO2010/081173, and WO2009/025846 disclose protease specifically expressed in cancer tissue. Also, J Inflamm (Lond). 2010; 7: 45, Nat Rev Immunol. 2006 Jul; 6 (7): 541-50, Nat Rev Drug Discov. 2014 Dec; 13 (12): 904-27, Respir Res. 2016 Mar 4; 17: 23, Dis Model Mech. 2014 Feb; 7 (2): 193-203, and Biochim Biophys Acta. 2012 Jan; 1824 (1): 133-45 disclose protease considered to be related to inflammation.

In addition to the protease specifically expressed in a target tissue, there also exists protease that is specifically activated in a target tissue. For example, protease may be expressed in an inactive form and then become an active form. In many tissues, a substance that inhibits active protease is present, and activity is controlled by the process of activation and the presence of the inhibitor (Nat Rev Cancer. 2003 Jul; 3 (7): 489-501). In a target tissue, the active protease may be specifically activated by escaping inhibition.

Active protease can be measured by use of a method using an antibody that recognizes the active protease (PNAS 2013 Jan 2; 110 (1): 93-98) or a method in which a peptide recognized by protease is fluorescently labeled and the fluorescence is quenched before cleavage but emitted after cleavage (Nat Rev Drug Discov. 2010 Sep; 9 (9): 690-701. doi: 10.1038/nrd3053).

Specific examples of the protease include, but are not limited to, cysteine protease (including cathepsin families B, L, S, etc.), aspartyl protease (cathepsins D, E, K, O, etc.), serine protease (including matriptase (including MT-SP1), cathepsins A and G, thrombin, plasmin, urokinase (uPA), tissue plasminogen activator (tPA), elastase, proteinase 3, thrombin, kallikrein, tryptase, and chymase), metalloprotease (metalloprotease (MMP1-28)) including both membrane-bound forms (MMP14-17 and MMP24-25) and secreted forms (MMP1-13, MMP18-23, and MMP26-28), A disintegrin and metalloprotease (ADAM), A disintegrin and metalloprotease with thrombospondin motifs (ADAMTS), meprin (meprin alpha and meprin beta), CD10 (CALLA), prostate-specific antigen (PSA), legumain, TMPRSS3, TMPRSS4, human neutrophil elastase (HNE), beta secretase (BACE), fibroblast activation protein alpha (FAP), granzyme B, guanidinobenzoatase (GB), hepsin, neprilysin, NS3/4A, HCV-NS3/4, calpain, ADAMDEC1, renin, cathepsin C, cathepsin V/L2, cathepsin X/Z/P, cruzipain, otubain 2, kallikrein-related peptidases (KLKs (KLK3, KLK4, KLK5, KLK6, KLK7, KLK8, KLK10, KLK11, KLK13, and KLK14)), bone morphogenetic protein 1 (BMP-1), activated protein C, blood coagulation-related protease (Factor VIIa, Factor IXa, Factor Xa, Factor XIa, and Factor XIIa), HtrA1, lactoferrin, marapsin, PACE4, DESC1, dipeptidyl peptidase 4 (DPP-4), TMPRSS2, cathepsin F, cathepsin H, cathepsin L2, cathepsin O, cathepsin S, granzyme A, Gepsin calpain 2, glutamate carboxypeptidase 2, AMSH-like proteases, AMSH, gamma secretase, antiplasmin-cleaving enzyme (APCE), decysin 1, N-acetylated alpha-linked acidic dipeptidase-like 1 (NAALADL1), and furin.

From another viewpoint, the target tissue specific protease can refer to cancer tissue specific protease or inflammatory tissue specific protease.

### Cancer tissue specific protease

Examples of the cancer tissue specific protease include protease specifically expressed in a cancer tissue disclosed in WO2013/128194, WO2010/081173, and WO2009/025846.

As for the type of the cancer tissue specific protease, protease having higher expression specificity in the cancer tissue to be treated is more effective for reducing adverse reactions. The concentration of cancer tissue specific protease in a cancer tissue is preferably 5 times or more, more preferably 10 times or more, further preferably 100 times or more, particularly preferably 500 times or more, and most preferably 1000 times or more than the concentration in normal tissues. Also, the activity of cancer tissue specific protease in a cancer tissue is preferably 2 times or more, more preferably 3 times or more, 4 times or more, 5 times or more, or 10 times or more, further preferably 100 times or more, particularly preferably 500 times or more, and most preferably 1000 times or more than the activity in normal tissues.

The cancer tissue specific protease may be bound to a cancer cell membrane or may be secreted extracellularly without being bound to a cell membrane. When the cancer tissue specific protease is not bound to a cancer cell membrane, the cancer tissue specific protease preferably exists within or in the vicinity of the cancer tissue in order for the cytotoxicity of immunocytes to be specific for cancer cells. In the present specification, "vicinity of the cancer tissue" means a position within the range that allows cleavage of the protease cleavage sequence specific for the cancer tissue to thereby exert the effect of reducing the ligand binding activity. Preferably, the range is such that damage on normal cells is minimized.

From an alternative viewpoint, cancer tissue specific protease is any of
(i) protease that is expressed at a higher level in the cancer tissue than in normal tissues,
(ii) protease that has higher activity in the cancer tissue than in normal tissues,
(iii) protease that is expressed at a higher level in the cancer cells than in normal cells, and
(iv) protease that has higher activity in the cancer cells than in normal cells.

One type of cancer tissue specific protease may be used alone, or two or more types may be used in combination. The number of types of cancer tissue specific protease can be appropriately set by those skilled in the art in consideration of the cancer type to be treated.

From the above viewpoints, the cancer tissue specific protease is preferably serine protease or metalloprotease, more preferably matriptase (including MT-SP1), urokinase (uPA), or metalloprotease, further preferably MT-SP1, uPA, MMP-2, or MMP-9, among the proteases listed above.

### Inflammatory tissue specific protease

As for the type of inflammatory tissue specific protease, protease having higher expression specificity in the inflammatory tissue to be treated is more effective for reducing adverse reactions. The concentration of inflammatory tissue specific protease in an inflammatory tissue is preferably 5 times or more, more preferably 10 times or more, further preferably 100 times or more, particularly preferably 500 times or more, and most preferably 1000 times or more than the concentration in normal tissues. Also, the activity of inflammatory tissue specific protease in an inflammatory tissue is preferably 2 times or more, more preferably 3 times or more, 4 times or more, 5 times or more, or 10 times or more, further preferably 100 times or more, particularly preferably 500 times or more, most preferably 1000 times or more than the activity in normal tissues.

The inflammatory tissue specific protease may be bound to an inflammatory cell membrane or may be secreted extracellularly without being bound to a cell membrane. When the inflammatory tissue specific protease is not bound to an inflammatory cell membrane, the inflammatory tissue specific protease preferably exists within or in the vicinity of the inflammatory tissue in order for the cytotoxicity of immunocytes to be specific for inflammatory cells. In the present specification, "vicinity of the inflammatory tissue" means a position within the range that allows cleavage of the protease cleavage sequence specific for the inflammatory tissue to thereby exert the effect of reducing the ligand binding activity. Preferably, the range is such that damage on normal cells is minimized.

From an alternative viewpoint, inflammatory tissue specific protease is any of
(i) protease that is expressed at a higher level in the inflammatory tissue than in normal tissues,
(ii) protease that has higher activity in the inflammatory tissue than in normal tissues,
(iii) protease that is expressed at a higher level in the inflammatory cells than in normal cells, and
(iv) protease that has higher activity in the inflammatory cells than in normal cells.

One type of inflammatory tissue specific protease may be used alone, or two or more types may be used in combination. The number of types of inflammatory tissue specific protease can be appropriately set by those skilled in the art in consideration of the pathological condition to be treated.

From these viewpoints, the inflammatory tissue specific protease is preferably metalloprotease among the proteases listed above, and more preferably ADAMTS5, MMP-2, MMP-7, MMP-9, or MMP-13 among the metalloprotease.

### Antibody production method

Methods for producing an antibody having a desired binding activity are known to those skilled in the art. A method for producing an antibody that binds to IL-6R (anti-IL-6R antibody) is exemplified below. Antibodies that bind to antigens other than IL-6R can also be appropriately produced according to the following examples. When producing a single-domain antibody, although there are differences in immunized animals and such, the single-domain antibody can be appropriately produced according to the following examples.

The anti-IL-6R antibody can be obtained as a polyclonal or monoclonal antibody by use of an approach known in the art. A mammal-derived monoclonal antibody can be preferably prepared as the anti-IL-6R antibody. The mammal-derived monoclonal antibody includes, for example, those produced by hybridomas and those produced by host cells transformed by a genetic engineering approach with an expression vector containing an antibody gene. The antibody described in the present application includes a "humanized antibody" and a "chimeric antibody".

The monoclonal antibody-producing hybridomas can be prepared by use of a technique known in the art, for example, as discussed below. Mammals are immunized with IL-6R protein used as a sensitizing antigen according to a usual immunization method. Immunocytes thus obtained are fused with known parental cells by a usual cell fusion method. Next, cells producing a monoclonal antibody are screened for selecting hybridomas producing the anti-IL-6R antibody by a usual screening method.

Specifically, the monoclonal antibody is prepared, for example, as discussed below. First, the IL-6R gene can be expressed to obtain the IL-6R protein which is used as a sensitizing antigen for antibody obtainment. Specifically, a gene sequence encoding IL-6R is inserted into a known expression vector with which appropriate host cells are then transformed. The desired human IL-6R protein is purified from the host cells or from a culture supernatant thereof by a method known in the art. In order to obtain soluble IL-6R from the culture supernatant, for example, soluble IL-6R as described by Mullberg et al. (J. Immunol. (1994) 152 (10), 4958-4968) is expressed. Alternatively, purified natural IL-6R protein can also be used as a sensitizing antigen.

The purified IL-6R protein can be used as the sensitizing antigen for use in the immunization of mammals. A partial peptide of IL-6R can also be used as the sensitizing antigen. The partial peptide may be obtained by chemical synthesis from the amino acid sequence of human IL-6R. Alternatively, the partial peptide may be obtained by incorporating a portion of the IL-6R gene to an expression vector followed by its expression. Furthermore, the partial peptide can also be obtained by degrading the IL-6R protein with a proteolytic enzyme. The region and size of the IL-6R peptide for use as a partial peptide are not particularly limited by specific embodiments. The number of amino acids constituting a peptide to be used as the sensitizing antigen is preferably at least 5 or more, for example, 6 or more, or 7 or more. More specifically, a peptide of 8 to 50, preferably 10 to 30 residues can be used as the sensitizing antigen.

Also, a fusion protein comprising a desired partial polypeptide or peptide of the IL-6R protein fused with a different polypeptide can be used as the sensitizing antigen. For example, an antibody Fc fragment or a peptide tag can be preferably used for producing the fusion protein for use as the sensitizing antigen. A vector for the expression of the fusion protein can be prepared by fusing in frame genes encoding two or more types of the desired polypeptide fragments, and inserting the fusion gene into an expression vector as described above. The method for preparing the fusion protein is described in Molecular Cloning 2nd ed. (Sambrook, J. et al., Molecular Cloning 2nd ed., 9.47-9.58 (1989), Cold Spring Harbor Lab. Press). The method for obtaining IL-6R for use as the sensitizing antigen and the immunization method using it are also specifically described in WO2003/000883, WO2004/022754, WO2006/006693, etc.

The mammals to be immunized with the sensitizing antigen are not limited to particular animals and are preferably selected in consideration of compatibility with the parental cells for use in cell fusion. In general, rodents (e.g., mice, rats, and hamsters), rabbits, monkeys, or the like are preferably used. When obtaining a single-domain antibody, animals of the family *Camelidae,* or transgenic animals harboring a gene capable of raising the single-domain antibody are preferably used.

The above animals are immunized with the sensitizing antigen according to a method known in the art. For example, as a general method, immunization is carried out by administering the sensitizing antigen to the mammals by intraperitoneal or subcutaneous administration. Specifically, the sensitizing antigen diluted with PBS (phosphate-buffered saline), physiological saline, or the like at an appropriate dilution ratio is mixed, if desired, with a usual adjuvant, for example, a Freund's complete adjuvant and emulsified. Then, the resulting sensitizing antigen is administered to the mammals several times at 4- to 21-day intervals. Also, an appropriate carrier can be used in the immunization with the sensitizing antigen. Particularly, in the case of using a partial peptide having a small molecular weight as the sensitizing antigen, immunization with the sensitizing antigen peptide bound with a carrier protein such as albumin or keyhole limpet hemocyanin may be desirable in some cases.

Alternatively, hybridomas producing the desired antibody can also be prepared as described below by use of DNA immunization. DNA immunization is an immunization method which involves immunostimulating immunized animals by expressing *in vivo* the sensitizing antigen in the immunized animals administered with a vector DNA that has been constructed in a form capable of expressing the gene encoding the antigenic protein in the immunized animals. DNA immunization can be expected to be superior to the general immunization method in which a protein antigen is administered to animals to be immunized as follows:
- DNA immunization can provide immunostimulation while maintaining the structure of a membrane protein (e.g., IL-6R); and
- DNA immunization does not require purification of the immunizing antigen.

In order to obtain a monoclonal antibody of the present invention by DNA immunization, first, DNA for IL-6R protein expression is administered to the animals to be immunized. The DNA encoding IL-6R can be synthesized by a method known in the art such as PCR. The obtained DNA is inserted into an appropriate expression vector, which is then administered to the animals to be immunized. For example, a commercially available expression vector such as pcDNA3.1 can be preferably used as the expression vector. A generally used method can be used as a method for administering the vector to a living body. For example, gold particles with the expression vector adsorbed thereon are transfected into the cells of animal individuals to be immunized using a gene gun to thereby perform DNA immunization. Furthermore, an antibody recognizing IL-6R can also be prepared by use of a method described in WO 2003/104453.

A rise in the titer of the antibody binding to IL-6R is confirmed in the serum of the mammals thus immunized. Then, immunocytes are collected from the mammals and subjected to cell fusion. Particularly, spleen cells can be used as preferred immunocytes.

Mammalian myeloma cells are used as cells to be fused with the immunocytes. The myeloma cells preferably have an appropriate selection marker for screening. The selection marker refers to a trait that can survive (or cannot survive) under particular culture conditions. For example, hypoxanthine guanine phosphoribosyltransferase deficiency (hereinafter, abbreviated as HGPRT deficiency) or thymidine kinase deficiency (hereinafter, abbreviated as TK deficiency) is known as the selection marker. Cells having the HGPRT or TK deficiency are sensitive to hypoxanthine aminopterin thymidine (hereinafter, abbreviated as HAT-sensitive). HAT-sensitive cells are killed in a HAT selective medium because the cells fail to synthesize DNA. By contrast, these cells, when fused with normal cells, become able to grow even in the HAT selective medium because the fused cells can continue DNA synthesis through the use of the salvage pathway of the normal cells.

Cells having HGPRT or TK deficiency can be selected in a medium containing 6-thioguanine or 8-azaguanine (hereinafter, abbreviated as 8AG) or 5'-bromodeoxyuridine, respectively. The normal cells are killed by incorporating these pyrimidine analogs into their DNAs. By contrast, the cells deficient in these enzymes can survive in the selective medium because the cells cannot incorporate the pyrimidine analogs. In addition, a selection marker called G418 resistance confers resistance to a 2-deoxystreptamine antibiotic (gentamicin analog) through a neomycin resistance gene. Various myeloma cells suitable for cell fusion are known in the art.

For example, P3 (P3x63Ag8.653) (J. Immunol. (1979)123 (4), 1548-1550), P3x63Ag8U.1 (Current Topics in Microbiology and Immunology (1978)81, 1-7), NS-1 (C. Eur. J. Immunol. (1976)6 (7), 511-519), MPC-11 (Cell (1976)8 (3), 405-415), SP2/0 (Nature (1978)276 (5685), 269-270), FO (J. Immunol. Methods (1980)35 (1-2), 1-21), S194/5.XX0.BU.1 (J. Exp. Med. (1978)148 (1), 313-323), and R210 (Nature (1979)277 (5692), 131-133) can be preferably used as such myeloma cells.

Basically, cell fusion of the immunocytes with the myeloma cells is performed according to a method known in the art, for example, the method of Kohler and Milstein et al. (Methods Enzymol. (1981) 73, 3-46).

More specifically, cell fusion can be carried out, for example, in a usual nutrient medium in the presence of a cell fusion promoter. For example, polyethylene glycol (PEG) or hemagglutinating virus of Japan (HVJ) is used as the fusion promoter. In addition, an auxiliary such as dimethyl sulfoxide is added thereto, if desired, for enhancing fusion efficiency and then used.

The ratio between the immunocytes and the myeloma cells used can be arbitrarily set. For example, the amount of the immunocytes is preferably set to 1 to 10 times the amount of the myeloma cells. For example, an RPMI1640 medium or a MEM medium suitable for the growth of the myeloma cell line as well as a usual medium for use in this kind of cell culture is used as the medium in the cell fusion. Preferably, a solution supplemented with serum (e.g., fetal calf serum (FCS)) can be further added to the medium.

For cell fusion, predetermined amounts of the immunocytes and the myeloma cells are mixed well in the medium, and a PEG solution (e.g., average molecular weight of PEG of about 1000 to 6000) preheated to approximately 37°C is added thereto usually at a concentration of 30 to 60% (w/v). The mixed solution is gently mixed and the desired fusion cells (hybridomas) are thus formed. Subsequently, appropriate medium listed above is sequentially added, and the supernatant is removed by centrifugation. This operation can be repeated to remove cell fusion agents or the like unfavorable for hybridoma growth.

The hybridomas thus obtained can be cultured in a usual selective medium, for example, a HAT medium (medium containing hypoxanthine, aminopterin, and thymidine), for selection. The culture using the HAT medium can be continued for a time sufficient (usually, sufficient time is several days to several weeks) to kill cells other than the desired hybridomas (non-fused cells). Subsequently, hybridomas producing the desired antibody are screened for and single-cell-cloned by a usual limiting dilution method.

The hybridomas thus obtained can be selected through the use of a selective medium according to the selection marker possessed by the myeloma cells used in the cell fusion. For example, cells having HGPRT or TK deficiency can be selected by culturing in a HAT medium (medium containing hypoxanthine, aminopterin, and thymidine). Specifically, in the case of using HAT-sensitive myeloma cells in cell fusion, cells successfully fused with normal cells can grow selectively in the HAT medium. The culture using the HAT medium is continued for a time long enough to kill cells other than the desired hybridomas (non-fused cells). Specifically, the culture can generally be performed for several days to several weeks to select the desired hybridomas. Subsequently, hybridomas producing the desired antibody can be screened for and single-cell-cloned by a usual limiting dilution method.

Screening and single-cell-cloning of the desired antibody can be preferably carried out by a screening method based on known antigen-antibody reactions. For example, a monoclonal antibody that binds to IL-6R can bind to IL-6R expressed on the cell surface. Such a monoclonal antibody can be screened, for example, by FACS (fluorescence activated cell sorting). FACS is a system capable of measuring the binding of an antibody to the cell surface by analyzing cells contacted with a fluorescent antibody using laser beam, and measuring the fluorescence emitted from the individual cells.

In order to screen for hybridomas that produce the monoclonal antibody of the present invention by FACS, first, IL-6R-expressing cells are prepared. Cells preferred for screening are mammalian cells forced to express IL-6R. Untransformed mammalian cells used as host cells can be used as a control to selectively detect the binding activity of an antibody against IL-6R on the cell surface. Specifically, hybridomas producing the IL-6R monoclonal antibody can be obtained by selecting hybridomas producing an antibody that binds to cells forced to express IL-6R but that does not bind to the host cells.

Alternatively, the antibody can be evaluated for its binding activity against immobilized IL-6R-expressing cells on the basis of the principle of ELISA. The IL-6R-expressing cells are immobilized onto each well of, for example, an ELISA plate. The hybridoma culture supernatant is contacted with the immobilized cells in the well to detect an antibody that binds to the immobilized cells. When the monoclonal antibody is derived from a mouse, the antibody bound with the cell can be detected using an anti-mouse immunoglobulin antibody. Hybridomas producing the desired antibody having antigen binding capacity thus selected by screening can be cloned by a limiting dilution method or the like.

The monoclonal antibody-producing hybridomas thus prepared can be passage cultured in a usual medium. Further, the hybridomas can be preserved over a long period in liquid nitrogen.

The hybridomas are cultured according to a usual method, and the desired monoclonal antibody can be obtained from the culture supernatant thereof. Alternatively, the hybridomas may be administered to mammals compatible therewith and grown, and the monoclonal antibody can be obtained from the ascitic fluids of the mammals. The former method is suitable for obtaining highly pure antibodies.

An antibody encoded by an antibody gene cloned from antibody-producing cells such as hybridomas can also be preferably used. The cloned antibody gene is integrated to an appropriate vector, which is then transfected into hosts so that the antibody encoded by the gene is expressed. Methods for isolating an antibody gene, integrating the gene to a vector, and transforming a host cell are already established by, for example, Vandamme et al. (Eur. J. Biochem. (1990) 192 (3), 767-775). A method for producing a recombinant antibody is also known, as mentioned below.

For example, cDNA encoding the variable region (V region) of the anti-IL-6R antibody is obtained from hybridoma cells producing the anti-IL-6R antibody. For this purpose, usually, total RNA is first extracted from the hybridomas. For example, the following methods can be used as a method for extracting mRNA from cells:
- a guanidine ultracentrifugation method (Biochemistry (1979) 18 (24), 5294-5299), and
- an AGPC method (Anal. Biochem. (1987) 162 (1), 156-159).

The extracted mRNA can be purified using mRNA Purification Kit (manufactured by GE Healthcare Bio-Sciences Corp.) or the like. Alternatively, a kit for directly extracting total mRNA from cells is also commercially available, such as QuickPrep mRNA Purification Kit (manufactured by GE Healthcare Bio-Sciences Corp.). The mRNA can be obtained from hybridomas using such a kit. From the obtained mRNA, cDNA encoding the antibody V region can be synthesized using reverse transcriptase. The cDNA can be synthesized using, for example, AMV Reverse Transcriptase First-strand cDNA Synthesis Kit (manufactured by Seikagaku Corp.). Alternatively, a 5'-RACE method (Proc. Natl. Acad. Sci. USA (1988) 85 (23), 8998-9002; and Nucleic Acids Res. (1989) 17 (8), 2919-2932) using SMART RACE cDNA amplification kit (manufactured by Clontech Laboratories, Inc.) and PCR can be appropriately used for cDNA synthesis and amplification. In the course of such cDNA synthesis, appropriate restriction enzyme sites mentioned later can be further introduced to both ends of the cDNA.

The cDNA fragment of interest is purified from the obtained PCR product and subsequently ligated with vector DNA. The recombinant vector thus prepared is transfected into *E. coli* or the like. After colony selection, the desired recombinant vector can be prepared from the *E. coli* that has formed the colony. Then, whether or not the recombinant vector has the nucleotide sequence of the cDNA of interest is confirmed by a method known in the art, for example, a dideoxynucleotide chain termination method.

The 5'-RACE method using primers for amplifying a variable region gene is conveniently used for obtaining a gene encoding the variable region. First, a 5'-RACE cDNA library is obtained by cDNA synthesis using RNAs extracted from hybridoma cells as templates. A commercially available kit such as SMART RACE cDNA amplification kit is appropriately used in the synthesis of the 5'-RACE cDNA library.

The antibody gene is amplified by PCR using the obtained 5'-RACE cDNA library as a template. Primers for amplifying mouse antibody genes can be designed on the basis of a known antibody gene sequence. These primers have nucleotide sequences differing depending on immunoglobulin subclasses. Thus, the subclass is desirably determined in advance using a commercially available kit such as Iso Strip mouse monoclonal antibody isotyping kit (Roche Diagnostics K.K.).

Specifically, primers capable of amplifying genes encoding γ1, γ2a, γ2b, and γ3 heavy chains and κ and λ light chains can be used, for example, for the purpose of obtaining a gene encoding mouse IgG. In order to amplify an IgG variable region gene, a primer that anneals to a moiety corresponding to a constant region close to the variable region is generally used as a 3' primer. On the other hand, a primer attached to the 5' RACE cDNA library preparation kit is used as a 5' primer.

The PCR products thus obtained by amplification can be used to reshape immunoglobulins composed of combinations of heavy and light chains. The desired antibody can be screened for using the binding activity of the reshaped immunoglobulin against IL-6R as an index. More preferably, the binding of the antibody to IL-6R is specific, for example, for the purpose of obtaining an antibody against IL-6R. An antibody that binds to IL-6R can be screened for, for example, by the following steps:
(1) contacting IL-6R-expressing cells with an antibody containing the V region encoded by the cDNA obtained from the hybridomas;
(2) detecting the binding of the antibody to the IL-6R-expressing cells; and
(3) selecting the antibody binding to the IL-6R-expressing cells.

A method for detecting the binding of an antibody (including single-domain antibody) to IL-6R-expressing cells is known in the art. Specifically, the binding of an antibody to IL-6R-expressing cells can be detected by an approach such as FACS mentioned above. A fixed preparation of IL-6R-expressing cells can be appropriately used for evaluating the binding activity of the antibody.

A panning method using phage vectors is also preferably used as a method of screening for an antibody using binding activity as an index. Also in the case of screening for a single-domain antibody, screening can be suitably carried out according to the following examples.

When antibody genes are obtained as libraries of heavy chain and light chain subclasses from a polyclonal antibody-expressing cell population, a screening method using phage vectors is advantageous. Genes encoding heavy chain and light chain variable regions can be linked via an appropriate linker sequence to form a single-chain Fv (scFv). The gene encoding scFv can be inserted into phage vectors to obtain phages expressing scFv on their surface. After contacting the phages with the desired antigen, phages bound with the antigen can be recovered to recover DNA encoding scFv having the binding activity of interest. This operation can be repeated as necessary to enrich scFvs having the desired binding activity.

After obtaining the cDNA encoding the V region of the anti-IL-6R antibody of interest, this cDNA is digested with restriction enzymes that recognize the restriction sites inserted at both ends of the cDNA. The restriction enzymes preferably recognize and digest a nucleotide sequence that appears at low frequency in the nucleotide sequence constituting the antibody gene. Preferably, sites for restriction enzymes that provide cohesive ends are inserted for inserting one copy of the digested fragment in the correct orientation into a vector. The thus-digested cDNA encoding the V region of the anti-IL-6R antibody can be inserted into an appropriate expression vector to obtain an antibody expression vector. In this case, a gene encoding an antibody constant region (C region) and the gene encoding the V region are fused in frame to obtain a chimeric antibody. In this context, a "chimeric antibody" implies that the origin of the constant and variable regions is different. Thus, heterogeneous (e.g., mouse-human) chimeric antibodies as well as human-human homogeneous chimeric antibodies are also included in the chimeric antibody according to the present invention. The V region gene can be inserted into an expression vector preliminarily having a constant region gene to construct a chimeric antibody expression vector. Specifically, for example, a recognition sequence for a restriction enzyme that digests the V region gene can be appropriately placed on the 5' side of an expression vector carrying the DNA encoding the desired antibody constant region (C region). Both are digested with the same combination of restriction enzymes and are fused in frame to construct a chimeric antibody expression vector.

In order to produce the anti-IL-6R monoclonal antibody, the antibody gene is integrated to an expression vector such that the antibody gene is expressed under the control of expression control regions. The expression control regions for antibody expression include, for example, an enhancer and a promoter. Also, an appropriate signal sequence can be added to the amino terminus such that the expressed antibody is extracellularly secreted. For example, a peptide having an amino acid sequence MGWSCIILFLVATATGVHS (SEQ ID NO: 1) can be used as the signal sequence, and other suitable signal sequences may be added. The expressed polypeptide is cleaved at the carboxyl-terminal moiety of the above sequence. The cleaved polypeptide can be extracellularly secreted as a mature polypeptide. Subsequently, appropriate host cells can be transformed with this expression vector to obtain recombinant cells expressing DNA encoding the anti-IL-6R antibody.

### Ligand-binding molecule

One aspect of a ligand-binding molecule of the present invention is a molecule capable of binding to a ligand, using a single-domain antibody contained in the ligand-binding molecule, particularly a molecule capable of binding to a ligand in an uncleaved state. "Bind" as used herein usually means binding due to an interaction that mainly includes non-covalent bonds such as an electrostatic force, van der Waals force, and hydrogen bond. Suitable examples of the ligand binding embodiment of a ligand-binding molecule of the present invention include, but are not limited to, antigen-antibody reactions in which an antigen binds with, for example, an antigen-binding region, antigen-binding molecule, antibody, and antibody fragment.

The phrase "capable of binding to a ligand" means that the ligand-binding molecule is capable of binding to the ligand even if the ligand-binding molecule and the ligand are separate molecules, and does not mean that the ligand-binding molecule and the ligand are connected through a covalent bond. For example, the fact that the ligand and the ligand-binding molecule are covalently bound via a linker is not referred to as "capable of binding to a ligand". Also, the phrase "ligand binding is attenuated" means that the capability of binding (binding capacity) described above is attenuated. For example, when the ligand and the ligand-binding molecule are covalently bound via a linker, cleavage of the linker does not mean attenuation of the ligand binding. In the present invention, the ligand-binding molecule may be connected with the ligand via a linker or the like as long as the ligand-binding molecule is capable of binding to the ligand.

The ligand-binding molecules of the present invention are limited only by the binding to the ligand through the single-domain antibody contained in the molecule in the uncleaved state, and can include molecules of any structure as long as they can bind to the target ligand through the single-domain antibody contained in the molecules in the uncleaved state.

One aspect of the ligand-binding molecule of the present invention is a polypeptide comprising a cleavage site. The cleavage site can be cleaved by, for example, an enzyme, can be reduced with a reducing agent, or can be photodegraded. The cleavage site may be placed at any position in the polypeptide as long as the ligand binding of the ligand-binding molecule can be attenuated by the cleavage of the cleavage site. The polypeptide may contain one or more cleavage sites.

In addition, the ligand-binding molecules of the present invention have a weaker (i.e., attenuated) ligand binding in the cleaved state compared to the uncleaved state. More specifically, the binding of the single-domain antibody within a ligand-binding molecule of the present invention to a ligand is weaker (i.e., attenuated) in a state where the ligand-binding molecule is cleaved compared to a state where the ligand-binding molecule is not cleaved. The weakening of the ligand binding can be evaluated by the binding activity of the ligand-binding molecule to a ligand.

### Assessment of the binding activity to a ligand

The binding activity of the ligand-binding molecule to a ligand can be assessed by a well-known method such as FACS, an ELISA format, a BIACORE method using amplified luminescent proximity homogeneous assay (ALPHA) screening or surface plasmon resonance (SPR) phenomena, or bio-layer interferometry (BLI) (Octet) (Proc. Natl. Acad. Sci. USA (2006) 103 (11), 4005-4010).

ALPHA screening is carried out based on the following principle according to ALPHA technology that uses two beads, a donor and an acceptor. Luminescence signals are detected only when molecules bound with the donor beads interact with molecules bound with the acceptor beads and when the two beads are close to one another. Laser-excited photosensitizers in the donor beads convert ambient oxygen into singlet oxygen in an excited state. The singlet oxygen molecules spread around the donor beads and when they reach the nearby acceptor beads, they induce chemiluminescent reaction in the beads to result in light emission. When the molecule bound with the donor bead and the molecule bound with the acceptor bead do not interact, chemiluminescent reaction does not occur because singlet oxygen produced by the donor bead does not reach the acceptor bead.

For example, a biotin-labeled ligand-binding molecule is bound to the donor bead, and a glutathione S transferase (GST)-tagged ligand is bound to the acceptor bead. In the absence of a competing untagged ligand-binding molecule, the ligand-binding molecule interacts with the ligand to generate signals of 520 to 620 nm. The untagged ligand-binding molecule competes with the tagged ligand-binding molecule for interaction with the ligand. Decrease in fluorescence resulting from the competition can be quantified to determine relative binding affinity. Biotinylation of a ligand-binding molecule, such as an antibody, using sulfo-NHS-biotin or the like is known in the art. A method which involves, for example, fusing a polynucleotide encoding the ligand in-frame with a polynucleotide encoding GST to form a fused gene, expressing the GST-fused ligand in cells or the like carrying a vector that permits expression of the fused gene, and purifying the GST-fused ligand using a glutathione column, can be appropriately adopted as a method for tagging a ligand with GST. The obtained signals are preferably analyzed using, for example, the software GRAPHPAD PRISM (GraphPad Software, Inc., San Diego) adapted to a one-site competition model based on non-linear regression analysis.

One (ligand) of the substances between which the interaction is to be observed is immobilized onto a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other one (analyte) of the substances between which the interaction is to be observed is poured onto the surface of the sensor chip, and when the analyte binds with the ligand the mass of the immobilized ligand molecule increases and results in the change of refractive index of the solvent on the sensor chip surface. This change in refractive index shifts the position of the SPR signal (in contrast, the position of the signal returns when dissociation occurs). The Biacore system plots on the ordinate the amount of the above-mentioned shift, i.e., change in mass on the sensor chip surface, and displays time-dependent change in mass as assay data (sensorgram). Kinetics (association rate constant (ka) and dissociation rate constant (kd)) is determined from the curve of the sensorgram, and dissociation constant (KD) is determined from the ratio between the two constants. Inhibition assay or equilibrium analysis is also preferably used in the BIACORE method. Examples of inhibition assay are described in Proc. Natl. Acad. Sci. USA (2006) 103 (11), 4005-4010, and examples of equilibrium analysis are described in Methods Enzymol. 2000; 323: 325-40.

The phrase "the function of a ligand-binding molecule to bind with a ligand is attenuated" means that the amount of ligand bound per a test ligand-binding molecule is, for example, 50% or less, preferably 45% or less, 40% or less, 35% or less, 30% or less, 20% or less, or 15% or less, particularly preferably 10% or less, 9% or less, 8% or less, 7% or less, 6% or less, 5% or less, 4% or less, 3% or less, 2% or less, or 1% or less, of the amount of ligand bound to a control ligand-binding molecule, on the basis of the measurement method described above. Desired index may be appropriately used as a binding activity index, and a dissociation constant (KD) may be used. In the case of using a dissociation constant (KD) as an index for evaluating binding activity, a larger dissociation constant (KD) of the test ligand-binding molecule for the ligand than the dissociation constant (KD) of a control ligand-binding molecule for the ligand means that the test ligand-binding molecule has weaker binding activity against the ligand than the control ligand-binding molecule. The phrase "the function of binding to a ligand is attenuated" means that the dissociation constant (KD) of the test ligand-binding molecule for the ligand is, for example, 2 times or more, preferably 5 times or more, or 10 times or more, and particularly preferably 100 times or more compared to the dissociation constant (KD) of the control ligand-binding molecule for the ligand.

Examples of the control ligand-binding molecule include an uncleaved form of a ligand-binding molecule.

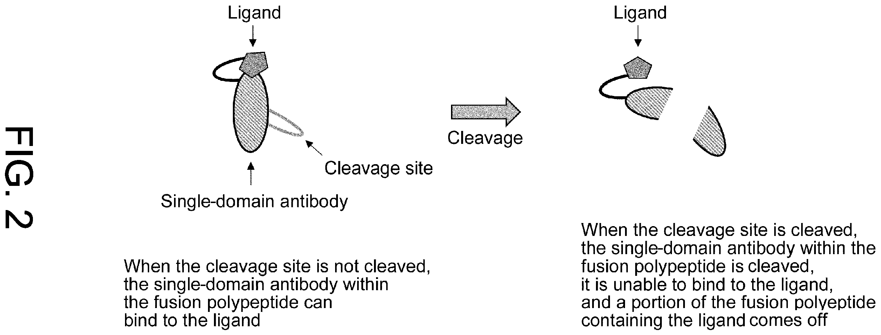

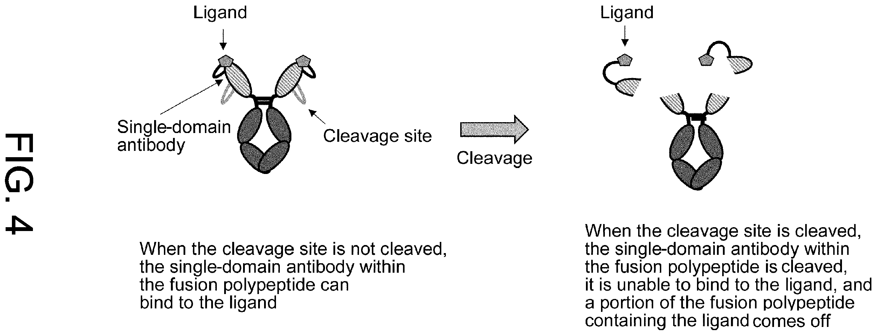

In one embodiment of the present invention, in the ligand-binding molecule of the present invention, the ligand is released from the ligand-binding molecule by the cleavage of the cleavage site. In this context, when the ligand is fused with a portion of the ligand-binding molecule via a linker and the linker does not have a cleavage site, the ligand is released while connected with the portion of the ligand-binding molecule via the linker (see e.g., Figures 2 and 4). Even when the ligand is released together with a portion of the ligand-binding molecule as mentioned above, it can be concluded that the ligand is released from the ligand-binding molecule as long as there is a portion of the ligand-binding molecule which could not maintain stable interaction with the ligand.

A method for detecting the release of the ligand from the ligand-binding molecule by the cleavage of the cleavage site includes a method of detecting the ligand using, for example, an antibody for ligand detection that recognizes the ligand. When the ligand-binding molecule is an antibody fragment, the antibody for ligand detection preferably binds to the same epitope as that for the ligand-binding molecule. The ligand detected using the antibody for ligand detection can be confirmed by a well-known method such as FACS, an ELISA format, a BIACORE method using amplified luminescent proximity homogeneous assay (ALPHA) screening or surface plasmon resonance (SPR) phenomena, or bio-layer interferometry (BLI) (Octet) (Proc. Natl. Acad. Sci. USA (2006) 103 (11), 4005-4010).

In the case of detecting the release of the ligand using, for example, Octet, the antibody for ligand detection that recognizes the ligand is biotinylated and contacted with a biosensor. Then, binding to the ligand in a sample can be measured to detect the release of the ligand. Specifically, the amount of the ligand is measured in a sample containing the ligand and the ligand-binding molecule before protease treatment or after protease treatment, using the antibody for ligand detection. The amount of the ligand detected in the sample can be compared between before and after protease treatment to detect the release of the ligand. Alternatively, the amount of the ligand is measured using the antibody for ligand detection in a sample containing protease, ligand-binding molecule, and ligand and a sample containing ligand-binding molecule and ligand without containing protease. The amount of the ligand detected in the sample with or without protease can be compared to detect the release of the ligand. More specifically, release of the ligand can be detected by a method described in the Examples of the present application. When the ligand-binding molecule is fused with the ligand to form a fusion protein, the amount of the ligand is measured using the antibody for ligand detection in a sample containing the fusion protein before protease treatment or after protease treatment. The amount of the ligand detected in the sample can be compared between before and after protease treatment to detect the release of the ligand. Alternatively, the amount of the ligand is measured using the antibody for ligand detection in a sample containing protease and the fusion protein and a sample containing the fusion protein without containing protease. The amount of the ligand detected in the sample with or without protease can be compared to detect the release of the ligand. More specifically, release of the ligand can be detected by a method described in the Examples of the present application.

In an embodiment in which the biological activity of the ligand is inhibited upon binding to the ligand-binding molecule, a method for detecting the release of the ligand from the ligand-binding molecule includes measuring the biological activity of the ligand in a sample for detecting ligand release. Specifically, the biological activity of the ligand can be measured in a sample containing the ligand and the ligand-binding molecule before protease treatment or after protease treatment and then compared to detect the release of the ligand. Alternatively, the biological activity of the ligand can be measured and compared in a sample containing protease, ligand-binding molecule, and ligand and a sample containing ligand-binding molecule and ligand without containing protease to detect the release of the ligand. When the ligand-binding molecule is fused with the ligand to form a fusion protein, the biological activity of the ligand can be measured and compared in a sample containing the fusion protein before protease treatment or after protease treatment to detect the release of the ligand. Alternatively, the biological activity of the ligand can be measured and compared in a sample containing protease and fusion protein and a sample containing fusion protein without containing protease to detect the release of the ligand.

In one embodiment of the present invention, the cleavage site comprises a protease cleavage sequence and is cleaved by protease.

### Protease cleavage sequence

The protease cleavage sequence is a particular amino acid sequence that is specifically recognized by a target tissue specific protease when a polypeptide is hydrolyzed by the target tissue specific protease in an aqueous solution.

The protease cleavage sequence is preferably an amino acid sequence that is hydrolyzed with high specificity by a target tissue specific protease that is more specifically expressed or activated in the target tissue or cells to be treated, from the viewpoint of reducing adverse reactions.

Specific examples of the protease cleavage sequence include target sequences that are specifically hydrolyzed by the above-illustrated protease specifically expressed in a cancer tissue as disclosed in WO2013/128194, WO2010/081173, and WO2009/025846, the inflammatory tissue specific protease, and the like. A sequence artificially altered by, for example, appropriately introducing an amino acid mutation to a target sequence that is specifically hydrolyzed by known protease can also be used. Alternatively, a protease cleavage sequence identified by a method known to those skilled in the art as described in Nature Biotechnology 19, 661-667 (2001) may be used.

Furthermore, a naturally occurring protease cleavage sequence may be used. For example, a protease-cleaved sequence present in a protein that changes its molecular form by protease cleavage, such as TGFβ which is converted to a latent form by protease cleavage, can also be used.

Examples of the protease cleavage sequence that can be used include, but are not limited to, sequences disclosed in WO2015/116933, WO2015/048329, WO2016/118629, WO2016/179257, WO2016/179285, WO2016/179335, WO2016/179003, WO2016/046778, WO2016/014974, US2016/0289324, US2016/0311903, PNAS (2000) 97: 7754-7759, Biochemical Journal (2010) 426: 219-228, and Beilstein J Nanotechnol. (2016) 7: 364-373.

The protease cleavage sequence is more preferably an amino acid sequence that is specifically hydrolyzed by a suitable target tissue specific protease as mentioned above. The following amino acid sequences are preferable among the amino acid sequences that are specifically hydrolyzed by a target tissue specific protease:
LSGRSDNH (SEQ ID NO: 2, cleavable by MT-SP1 or uPA),
PLGLAG (SEQ ID NO: 3, cleavable by MMP-2 or MMP-9), and
VPLSLTMG (SEQ ID NO: 4, cleavable by MMP-7).

The following sequences can also be used as the protease cleavage sequence:
TSTSGRSANPRG (SEQ ID NO: 5, cleavable by MT-SP1 or uPA),
ISSGLLSGRSDNH (SEQ ID NO: 6, cleavable by MT-SP1 or uPA),
AVGLLAPPGGLSGRSDNH (SEQ ID NO: 7, cleavable by MT-SP1 or uPA),
GAGVPMSMRGGAG (SEQ ID NO: 8, cleavable by MMP-1),
GAGIPVSLRSGAG (SEQ ID NO: 9, cleavable by MMP-2),
GPLGIAGQ (SEQ ID NO: 10, cleavable by MMP-2),
GGPLGMLSQS (SEQ ID NO: 11, cleavable by MMP-2),
PLGLWA (SEQ ID NO: 12, cleavable by MMP-2),
GAGRPFSMIMGAG (SEQ ID NO: 13, cleavable by MMP-3),
GAGVPLSLTMGAG (SEQ ID NO: 14, cleavable by MMP-7),
GAGVPLSLYSGAG (SEQ ID NO: 15, cleavable by MMP-9),
AANLRN (SEQ ID NO: 16, cleavable by MMP-11),
AQAYVK (SEQ ID NO: 17, cleavable by MMP-11),
AANYMR (SEQ ID NO: 18, cleavable by MMP-11),
AAALTR (SEQ ID NO: 19, cleavable by MMP-11),
AQNLMR (SEQ ID NO: 20, cleavable by MMP-11),
AANYTK (SEQ ID NO: 21, cleavable by MMP-11),
GAGPQGLAGQRGIVAG (SEQ ID NO: 22, cleavable by MMP-13),
PRFKIIGG (SEQ ID NO: 23, cleavable by pro-urokinase),
PRFRIIGG (SEQ ID NO: 24, cleavable by pro-urokinase),
GAGSGRSAG (SEQ ID NO: 25, cleavable by uPA),
SGRSA (SEQ ID NO: 26, cleavable by uPA),
GSGRSA (SEQ ID NO: 27, cleavable by uPA),
SGKSA (SEQ ID NO: 28, cleavable by uPA),
SGRSS (SEQ ID NO: 29, cleavable by uPA),
SGRRA (SEQ ID NO: 30, cleavable by uPA),
SGRNA (SEQ ID NO: 31, cleavable by uPA),
SGRKA (SEQ ID NO: 32, cleavable by uPA),
QRGRSA (SEQ ID NO: 33, cleavable by tPA),
GAGSLLKSRMVPNFNAG (SEQ ID NO: 34, cleavable by cathepsin B)
TQGAAA (SEQ ID NO: 35, cleavable by cathepsin B),
GAAAAA (SEQ ID NO: 36, cleavable by cathepsin B),
GAGAAG (SEQ ID NO: 37, cleavable by cathepsin B),
AAAAAG (SEQ ID NO: 38, cleavable by cathepsin B),
LCGAAI (SEQ ID NO: 39, cleavable by cathepsin B),
FAQALG (SEQ ID NO: 40, cleavable by cathepsin B),
LLQANP (SEQ ID NO: 41, cleavable by cathepsin B),
LAAANP (SEQ ID NO: 42, cleavable by cathepsin B),
LYGAQF (SEQ ID NO: 43, cleavable by cathepsin B),
LSQAQG (SEQ ID NO: 44, cleavable by cathepsin B),
ASAASG (SEQ ID NO: 45, cleavable by cathepsin B),
FLGASL (SEQ ID NO: 46, cleavable by cathepsin B),
AYGATG (SEQ ID NO: 47, cleavable by cathepsin B),
LAQATG (SEQ ID NO: 48, cleavable by cathepsin B),
GAGSGVVIATVIVITAG (SEQ ID NO: 49, cleavable by cathepsin L),
APMAEGGG (SEQ ID NO: 50, cleavable by meprin alpha or meprin beta),
EAQGDKII (SEQ ID NO: 51, cleavable by meprin alpha or meprin beta),
LAFSDAGP (SEQ ID NO: 52, cleavable by meprin alpha or meprin beta),
YVADAPK (SEQ ID NO: 53, cleavable by meprin alpha or meprin beta),
RRRRR (SEQ ID NO: 54, cleavable by furin),
RRRRRR (SEQ ID NO: 55, cleavable by furin),
GQSSRHRRAL (SEQ ID NO: 56, cleavable by furin),
SSRHRRALD (SEQ ID NO: 57),
RKSSIIIRMRDVVL (SEQ ID NO: 58, cleavable by plasminogen),
SSSFDKGKYKKGDDA (SEQ ID NO: 59, cleavable by staphylokinase),
SSSFDKGKYKRGDDA (SEQ ID NO: 60, cleavable by staphylokinase),
IEGR (SEQ ID NO: 61, cleavable by Factor IXa),
IDGR (SEQ ID NO: 62, cleavable by Factor IXa),
GGSIDGR (SEQ ID NO: 63, cleavable by Factor IXa),
GPQGIAGQ (SEQ ID NO: 64, cleavable by collagenase),
GPQGLLGA (SEQ ID NO: 65, cleavable by collagenase),
GIAGQ (SEQ ID NO: 66, cleavable by collagenase),
GPLGIAG (SEQ ID NO: 67, cleavable by collagenase),
GPEGLRVG (SEQ ID NO: 68, cleavable by collagenase),
YGAGLGVV (SEQ ID NO: 69, cleavable by collagenase),
AGLGVVER (SEQ ID NO: 70, cleavable by collagenase),
AGLGISST (SEQ ID NO: 71, cleavable by collagenase),
EPQALAMS (SEQ ID NO: 72, cleavable by collagenase),
QALAMSAI (SEQ ID NO: 73, cleavable by collagenase),
AAYHLVSQ (SEQ ID NO: 74, cleavable by collagenase),
MDAFLESS (SEQ ID NO: 75, cleavable by collagenase),
ESLPVVAV (SEQ ID NO: 76, cleavable by collagenase),
SAPAVESE (SEQ ID NO: 77, cleavable by collagenase),
DVAQFVLT (SEQ ID NO: 78, cleavable by collagenase),
VAQFVLTE (SEQ ID NO: 79, cleavable by collagenase),
AQFVLTEG (SEQ ID NO: 80, cleavable by collagenase),
PVQPIGPQ (SEQ ID NO: 81, cleavable by collagenase),
LVPRGS (SEQ ID NO: 82, cleavable by thrombin),
TSGSGRSANARG (SEQ ID NO: 170, cleavable by uPA and MT-SP1),
TSQSGRSANQRG (SEQ ID NO: 171, cleavable by uPA and MT-SP1),
TSPSGRSAYPRG (SEQ ID NO: 172, cleavable by uPA and MT-SP1),
TSGSGRSATPRG (SEQ ID NO: 173, cleavable by uPA and MT-SP1),
TSQSGRSATPRG (SEQ ID NO: 174, cleavable by uPA and MT-SP1),
TSASGRSATPRG (SEQ ID NO: 175, cleavable by uPA and MT-SP1),
TSYSGRSAVPRG (SEQ ID NO: 176, cleavable by uPA and MT-SP1),
TSYSGRSANFRG (SEQ ID NO: 177, cleavable by uPA and MT-SP1),
TSSSGRSATPRG (SEQ ID NO: 178, cleavable by uPA and MT-SP1), and
TSTTGRSASPRG (SEQ ID NO: 179, cleavable by uPA and MT-SP1).

The sequences shown in Table 1 may also be used as the protease cleavage sequence.

**[Table 1]**

| Protease Cleavage Sequences (cleavable by uPA and MT-SP1) | | | |
|---|---|---|---|
| SEQ ID NO | Cleavage sequence | SEQ ID NO | Cleavage sequence |
| 180 | TSASGRSANPRG | 479 | ASGRSANP |
| 181 | TSESGRSANPRG | 480 | ESGRSANP |
| 182 | TSFSGRSANPRG | 481 | FSGRSANP |
| 183 | TSGSGRSANPRG | 482 | GSGRSANP |
| 184 | TSHSGRSANPRG | 483 | HSGRSANP |
| 185 | TSKSGRSANPRG | 484 | KSGRSANP |
| 186 | TSMSGRSANPRG | 485 | MSGRSANP |
| 187 | TSNSGRSANPRG | 486 | NSGRSANP |
| 188 | TSPSGRSANPRG | 487 | PSGRSANP |
| 189 | TSQSGRSANPRG | 488 | QSGRSANP |
| 190 | TSWSGRSANPRG | 489 | WSGRSANP |
| 191 | TSYSGRSANPRG | 490 | YSGRSANP |
| 192 | TSTAGRSANPRG | 491 | TAGRSANP |
| 193 | TSTDGRSANPRG | 492 | TDGRSANP |
| 194 | TSTEGRSANPRG | 493 | TEGRSANP |
| 195 | TSTFGRSANPRG | 494 | TFGRSANP |
| 196 | TSTLGRSANPRG | 495 | TLGRSANP |
| 197 | TSTMGRSANPRG | 496 | TMGRSANP |
| 198 | TSTPGRSANPRG | 497 | TPGRSANP |
| 199 | TSTQGRSANPRG | 498 | TQGRSANP |
| 200 | TSTVGRSANPRG | 499 | TVGRSANP |
| 201 | TSTWGRSANPRG | 500 | TWGRSANP |
| 202 | TSTSARSANPRG | 501 | TSARSANP |
| 203 | TSTSERSANPRG | 502 | TSERSANP |
| 204 | TSTSFRSANPRG | 503 | TSFRSANP |
| 205 | TSTSHRSANPRG | 504 | TSHRSANP |
| 206 | TSTSIRSANPRG | 505 | TSIRSANP |
| 207 | TSTSKRSANPRG | 506 | TSKRSANP |
| 208 | TSTSLRSANPRG | 507 | TSLRSANP |
| 209 | TSTSMRSANPRG | 508 | TSMRSANP |
| 210 | TSTSNRSANPRG | 509 | TSNRSANP |
| 211 | TSTSPRSANPRG | 510 | TSPRSANP |
| 212 | TSTSQRSANPRG | 511 | TSQRSANP |
| 213 | TSTSRRSANPRG | 512 | TSRRSANP |
| 214 | TSTSTRSANPRG | 513 | TSTRSANP |
| 215 | TSTSVRSANPRG | 514 | TSVRSANP |
| 216 | TSTSWRSANPRG | 515 | TSWRSANP |
| 217 | TSTSYRSANPRG | 516 | TSYRSANP |
| 218 | TSTSGRAANPRG | 517 | TSGRAANP |
| 219 | TSTSGRDANPRG | 518 | TSGRDANP |
| 220 | TSTSGREANPRG | 519 | TSGREANP |
| 221 | TSTSGRGANPRG | 520 | TSGRGANP |
| 222 | TSTSGRHANPRG | 521 | TSGRHANP |
| 223 | TSTSGRIANPRG | 522 | TSGRIANP |
| 224 | TSTSGRKANPRG | 523 | TSGRKANP |
| 225 | TSTSGRLANPRG | 524 | TSGRLANP |
| 226 | TSTSGRMANPRG | 525 | TSGRMANP |
| 227 | TSTSGRNANPRG | 526 | TSGRNANP |
| 228 | TSTSGRPANPRG | 527 | TSGRPANP |
| 229 | TSTSGRQANPRG | 528 | TSGRQANP |
| 230 | TSTSGRRANPRG | 529 | TSGRRANP |
| 231 | TSTSGRTANPRG | 530 | TSGRTANP |
| 232 | TSTSGRVANPRG | 531 | TSGRVANP |
| 233 | TSTSGRWANPRG | 532 | TSGRWANP |
| 234 | TSTSGRYANPRG | 533 | TSGRYANP |
| 235 | TSTSGRSENPRG | 534 | TSGRSENP |
| 236 | TSTSGRSFNPRG | 535 | TSGRSFNP |
| 237 | TSTSGRSKNPRG | 536 | TSGRSKNP |
| 238 | TSTSGRSMNPRG | 537 | TSGRSMNP |
| 239 | TSTSGRSNNPRG | 538 | TSGRSNNP |
| 240 | TSTSGRSPNPRG | 539 | TSGRSPNP |
| 241 | TSTSGRSQNPRG | 540 | TSGRSQNP |
| 242 | TSTSGRSRNPRG | 541 | TSGRSRNP |
| 243 | TSTSGRSSNPRG | 542 | TSGRSSNP |
| 244 | TSTSGRSWNPRG | 543 | TSGRSWNP |
| 245 | TSTSGRSYNPRG | 544 | TSGRSYNP |
| 246 | TSTSGRSAAPRG | 545 | TSGRSAAP |
| 247 | TSTSGRSADPRG | 546 | TSGRSADP |
| 248 | TSTSGRSAEPRG | 547 | TSGRSAEP |
| 249 | TSTSGRSAFPRG | 548 | TSGRSAFP |
| 250 | TSTSGRSAGPRG | 549 | TSGRSAGP |
| 251 | TSTSGRSAKPRG | 550 | TSGRSAKP |
| 252 | TSTSGRSALPRG | 551 | TSGRSALP |
| 253 | TSTSGRSAMPRG | 552 | TSGRSAMP |
| 254 | TSTSGRSAPPRG | 553 | TSGRSAPP |
| 255 | TSTSGRSAQPRG | 554 | TSGRSAQP |
| 256 | TSTSGRSAVPRG | 555 | TSGRSAVP |
| 257 | TSTSGRSAWPRG | 556 | TSGRSAWP |
| 258 | TSTSGRSAYPRG | 557 | TSGRSAYP |
| 259 | TSTSGRSANARG | 558 | TSGRSANA |
| 260 | TSTSGRSANDRG | 559 | TSGRSAND |
| 261 | TSTSGRSANERG | 560 | TSGRSANE |
| 262 | TSTSGRSANFRG | 561 | TSGRSANF |
| 263 | TSTSGRSANGRG | 562 | TSGRSANG |
| 264 | TSTSGRSANIRG | 563 | TSGRSANI |
| 265 | TSTSGRSANKRG | 564 | TSGRSANK |
| 266 | TSTSGRSANNRG | 565 | TSGRSANN |
| 267 | TSTSGRSANQRG | 566 | TSGRSANQ |
| 268 | TSTSGRSANSRG | 567 | TSGRSANS |
| 269 | TSTSGRSANTRG | 568 | TSGRSANT |
| 270 | TSTSGRSANWRG | 569 | TSGRSANW |
| 271 | TSDSGRSANPRG | 570 | DSGRSANP |
| 272 | TSISGRSANPRG | 571 | ISGRSANP |
| 273 | TSSSGRSANPRG | 572 | SSGRSANP |
| 274 | TSTHGRSANPRG | 573 | THGRSANP |
| 275 | TSTKGRSANPRG | 574 | TKGRSANP |
| 276 | TSTTGRSANPRG | 575 | TTGRSANP |
| 277 | TSTYGRSANPRG | 576 | TYGRSANP |
| 278 | TSTSDRSANPRG | 577 | TSDRSANP |
| 279 | TSTSSRSANPRG | 578 | TSSRSANP |
| 280 | TSTSGRFANPRG | 579 | TSGRFANP |
| 281 | TSTSGRSDNPRG | 580 | TSGRSDNP |
| 282 | TSTSGRSHNPRG | 581 | TSGRSHNP |
| 283 | TSTSGRSINPRG | 582 | TSGRSINP |
| 284 | TSTSGRSLNPRG | 583 | TSGRSLNP |
| 285 | TSTSGRSTNPRG | 584 | TSGRSTNP |
| 286 | TSTSGRSVNPRG | 585 | TSGRSVNP |
| 287 | TSTSGRSAHPRG | 586 | TSGRSAHP |
| 288 | TSTSGRSAIPRG | 587 | TSGRSAIP |
| 289 | TSTSGRSARPRG | 588 | TSGRSARP |
| 290 | TSTSGRSASPRG | 589 | TSGRSASP |
| 291 | TSTSGRSATPRG | 590 | TSGRSATP |
| 292 | TSTSGRSANHRG | 591 | TSGRSANH |
| 293 | TSTSGRSANLRG | 592 | TSGRSANL |
| 294 | TSTSGRSANMRG | 593 | TSGRSANM |
| 295 | TSTSGRSANRRG | 594 | TSGRSANR |
| 296 | TSTSGRSANVRG | 595 | TSGRSANV |
| 297 | TSTSGRSANYRG | 596 | TSGRSANY |
| 298 | TSGSGRSAVPRG | 597 | GSGRSAVP |
| 299 | TSGSGRSAYPRG | 598 | GSGRSAYP |
| 300 | TSGSGRSANQRG | 599 | GSGRSANQ |
| 170 | TSGSGRSANARG | 600 | GSGRSANA |
| 301 | TSGSGRSANIRG | 601 | GSGRSANI |
| 302 | TSGSGRSANFRG | 602 | GSGRSANF |
| 303 | TSGSGRSANSRG | 603 | GSGRSANS |
| 304 | TSQSGRSAVPRG | 604 | QSGRSAVP |
| 305 | TSQSGRSAYPRG | 605 | QSGRSAYP |
| 171 | TSQSGRSANQRG | 606 | QSGRSANQ |
| 306 | TSQSGRSANARG | 607 | QSGRSANA |
| 307 | TSQSGRSANIRG | 608 | QSGRSANI |
| 308 | TSQSGRSANFRG | 609 | QSGRSANF |
| 309 | TSQSGRSANSRG | 610 | QSGRSANS |
| 310 | TSPSGRSAVPRG | 611 | PSGRSAVP |
| 172 | TSPSGRSAYPRG | 612 | PSGRSAYP |
| 311 | TSPSGRSANQRG | 613 | PSGRSANQ |
| 312 | TSPSGRSANARG | 614 | PSGRSANA |
| 313 | TSPSGRSANIRG | 615 | PSGRSANI |
| 314 | TSPSGRSANFRG | 616 | PSGRSANF |
| 315 | TSPSGRSANSRG | 617 | PSGRSANS |
| 316 | TSASGRSAVPRG | 618 | ASGRSAVP |
| 317 | TSASGRSAYPRG | 619 | ASGRSAYP |
| 318 | TSASGRSANQRG | 620 | ASGRSANQ |
| 319 | TSASGRSANARG | 621 | ASGRSANA |
| 320 | TSASGRSANIRG | 622 | ASGRSANI |
| 321 | TSASGRSANFRG | 623 | ASGRSANF |
| 322 | TSASGRSANSRG | 624 | ASGRSANS |
| 323 | TSYSGRSENPRG | 625 | YSGRSENP |
| 324 | TSGSGRSENPRG | 626 | GSGRSENP |
| 325 | TSQSGRSENPRG | 627 | QSGRSENP |
| 326 | TSPSGRSENPRG | 628 | PSGRSENP |
| 327 | TSASGRSENPRG | 629 | ASGRSENP |
| 328 | TSHSGRSENPRG | 630 | HSGRSENP |
| 329 | TSTSGRSENQRG | 631 | TSGRSENQ |
| 330 | TSTSGRSENARG | 632 | TSGRSENA |
| 331 | TSTSGRSENIRG | 633 | TSGRSENI |
| 332 | TSTSGRSENFRG | 634 | TSGRSENF |
| 333 | TSTSGRSENSRG | 635 | TSGRSENS |
| 334 | TSYSGRSAEPRG | 636 | YSGRSAEP |
| 335 | TSGSGRSAEPRG | 637 | GSGRSAEP |
| 336 | TSQSGRSAEPRG | 638 | QSGRSAEP |
| 337 | TSPSGRSAEPRG | 639 | PSGRSAEP |
| 338 | TSASGRSAEPRG | 640 | ASGRSAEP |
| 339 | TSHSGRSAEPRG | 641 | HSGRSAEP |
| 340 | TSTSGRSAEQRG | 642 | TSGRSAEQ |
| 341 | TSTSGRSAEARG | 643 | TSGRSAEA |
| 342 | TSTSGRSAEIRG | 644 | TSGRSAEI |
| 343 | TSTSGRSAEFRG | 645 | TSGRSAEF |
| 344 | TSTSGRSAESRG | 646 | TSGRSAES |
| 345 | TSGTGRSANPRG | 647 | GTGRSANP |
| 346 | TSGKGRSANPRG | 648 | GKGRSANP |
| 347 | TSGSGRSAIPRG | 649 | GSGRSAIP |
| 173 | TSGSGRSATPRG | 650 | GSGRSATP |
| 348 | TSGSGRSASPRG | 651 | GSGRSASP |
| 349 | TSGSGRSAHPRG | 652 | GSGRSAHP |
| 350 | TSGSGRSANYRG | 653 | GSGRSANY |
| 351 | TSGSGRSANVRG | 654 | GSGRSANV |
| 352 | TSGSGRSANHRG | 655 | GSGRSANH |
| 353 | TSQTGRSANPRG | 656 | QTGRSANP |
| 354 | TSQKGRSANPRG | 657 | QKGRSANP |
| 355 | TSQSGRSAIPRG | 658 | QSGRSAIP |
| 174 | TSQSGRSATPRG | 659 | QSGRSATP |
| 356 | TSQSGRSASPRG | 660 | QSGRSASP |
| 357 | TSQSGRSAHPRG | 661 | QSGRSAHP |
| 358 | TSQSGRSANYRG | 662 | QSGRSANY |
| 359 | TSQSGRSANVRG | 663 | QSGRSANV |
| 360 | TSQSGRSANHRG | 664 | QSGRSANH |
| 361 | TSPTGRSANPRG | 665 | PTGRSANP |
| 362 | TSPKGRSANPRG | 666 | PKGRSANP |
| 363 | TSPSGRSAIPRG | 667 | PSGRSAIP |
| 364 | TSPSGRSATPRG | 668 | PSGRSATP |
| 365 | TSPSGRSASPRG | 669 | PSGRSASP |
| 366 | TSPSGRSAHPRG | 670 | PSGRSAHP |
| 367 | TSPSGRSANYRG | 671 | PSGRSANY |
| 368 | TSPSGRSANVRG | 672 | PSGRSANV |
| 369 | TSPSGRSANHRG | 673 | PSGRSANH |
| 370 | TSATGRSANPRG | 674 | ATGRSANP |
| 371 | TSAKGRSANPRG | 675 | AKGRSANP |
| 372 | TSASGRSAIPRG | 676 | ASGRSAIP |
| 175 | TSASGRSATPRG | 677 | ASGRSATP |
| 373 | TSASGRSASPRG | 678 | ASGRSASP |
| 374 | TSASGRSAHPRG | 679 | ASGRSAHP |
| 375 | TSASGRSANYRG | 680 | ASGRSANY |
| 376 | TSASGRSANVRG | 681 | ASGRSANV |
| 377 | TSASGRSANHRG | 682 | ASGRSANH |
| 378 | TSYTGRSANPRG | 683 | YTGRSANP |
| 379 | TSYKGRSANPRG | 684 | YKGRSANP |
| 176 | TSYSGRSAVPRG | 685 | YSGRSAVP |
| 380 | TSYSGRSAIPRG | 686 | YSGRSAIP |
| 381 | TSYSGRSATPRG | 687 | YSGRSATP |
| 382 | TSYSGRSASPRG | 688 | YSGRSASP |
| 383 | TSYSGRSAHPRG | 689 | YSGRSAHP |
| 384 | TSYSGRSANARG | 690 | YSGRSANA |
| 177 | TSYSGRSANFRG | 691 | YSGRSANF |
| 385 | TSYSGRSANYRG | 692 | YSGRSANY |
| 386 | TSYSGRSANVRG | 693 | YSGRSANV |
| 387 | TSYSGRSANHRG | 694 | YSGRSANH |
| 388 | TSSTGRSANPRG | 695 | STGRSANP |
| 389 | TSSKGRSANPRG | 696 | SKGRSANP |
| 390 | TSSSGRSAVPRG | 697 | SSGRSAVP |
| 391 | TSSSGRSAIPRG | 698 | SSGRSAIP |
| 178 | TSSSGRSATPRG | 699 | SSGRSATP |
| 392 | TSSSGRSASPRG | 700 | SSGRSASP |
| 393 | TSSSGRSAHPRG | 701 | SSGRSAHP |
| 394 | TSSSGRSANARG | 702 | SSGRSANA |
| 395 | TSSSGRSANFRG | 703 | SSGRSANF |
| 396 | TSSSGRSANYRG | 704 | SSGRSANY |
| 397 | TSSSGRSANVRG | 705 | SSGRSANV |
| 398 | TSSSGRSANHRG | 706 | SSGRSANH |
| 399 | TSITGRSANPRG | 707 | ITGRSANP |
| 400 | TSIKGRSANPRG | 708 | IKGRSANP |
| 401 | TSISGRSAVPRG | 709 | ISGRSAVP |
| 402 | TSISGRSAIPRG | 710 | ISGRSAIP |
| 403 | TSISGRSATPRG | 711 | ISGRSATP |
| 404 | TSISGRSASPRG | 712 | ISGRSASP |
| 405 | TSISGRSAHPRG | 713 | ISGRSAHP |
| 406 | TSISGRSANARG | 714 | ISGRSANA |
| 407 | TSISGRSANFRG | 715 | ISGRSANF |
| 408 | TSISGRSANYRG | 716 | ISGRSANY |
| 409 | TSISGRSANVRG | 717 | ISGRSANV |
| 410 | TSISGRSANHRG | 718 | ISGRSANH |
| 411 | TSTTGRSAVPRG | 719 | TTGRSAVP |
| 412 | TSTTGRSAIPRG | 720 | TTGRSAIP |
| 413 | TSTTGRSATPRG | 721 | TTGRSATP |
| 179 | TSTTGRSASPRG | 722 | TTGRSASP |
| 414 | TSTTGRSAHPRG | 723 | TTGRSAHP |
| 415 | TSTTGRSANARG | 724 | TTGRSANA |
| 416 | TSTTGRSANFRG | 725 | TTGRSANF |
| 417 | TSTTGRSANYRG | 726 | TTGRSANY |
| 418 | TSTTGRSANVRG | 727 | TTGRSANV |
| 419 | TSTTGRSANHRG | 728 | TTGRSANH |
| 420 | TSTKGRSAVPRG | 729 | TKGRSAVP |
| 421 | TSTKGRSAIPRG | 730 | TKGRSAIP |
| 422 | TSTKGRSATPRG | 731 | TKGRSATP |
| 423 | TSTKGRSASPRG | 732 | TKGRSASP |
| 424 | TSTKGRSAHPRG | 733 | TKGRSAHP |
| 425 | TSTKGRSANARG | 734 | TKGRSANA |
| 426 | TSTKGRSANFRG | 735 | TKGRSANF |
| 427 | TSTKGRSANYRG | 736 | TKGRSANY |
| 428 | TSTKGRSANVRG | 737 | TKGRSANV |
| 429 | TSTKGRSANHRG | 738 | TKGRSANH |
| 430 | TSTSGRSAVYRG | 739 | TSGRSAVY |
| 431 | TSTSGRSAVVRG | 740 | TSGRSAVV |
| 432 | TSTSGRSAVHRG | 741 | TSGRSAVH |
| 433 | TSTSGRSAIYRG | 742 | TSGRSAIY |
| 434 | TSTSGRSAIVRG | 743 | TSGRSAIV |
| 435 | TSTSGRSAIHRG | 744 | TSGRSAIH |
| 436 | TSTSGRSASYRG | 745 | TSGRSASY |
| 437 | TSTSGRSASVRG | 746 | TSGRSASV |
| 438 | TSTSGRSASHRG | 747 | TSGRSASH |
| 439 | TSTSGRSAHYRG | 748 | TSGRSAHY |
| 440 | TSTSGRSAHVRG | 749 | TSGRSAHV |
| 441 | TSTSGRSAHHRG | 750 | TSGRSAHH |
| 442 | TSPSGRSEVPRG | 751 | PSGRSEVP |
| 443 | TSPSGRSAEPRG | 752 | PSGRSAEP |
| 444 | TSPSGRSAGPRG | 753 | PSGRSAGP |
| 445 | TSASGRSENARG | 754 | ASGRSENA |
| 446 | TSASGRSAEARG | 755 | ASGRSAEA |
| 447 | TSASGRSAGARG | 756 | ASGRSAGA |
| 448 | TSGTGRSATPRG | 757 | GTGRSATP |
| 449 | TSGSGRSATYRG | 758 | GSGRSATY |
| 450 | TSGSGRSATVRG | 759 | GSGRSATV |
| 451 | TSGSGRSATHRG | 760 | GSGRSATH |
| 452 | TSGTGRSATYRG | 761 | GTGRSATY |
| 453 | TSGTGRSATVRG | 762 | GTGRSATV |
| 454 | TSGTGRSATHRG | 763 | GTGRSATH |
| 455 | TSGSGRSETPRG | 764 | GSGRSETP |
| 456 | TSGTGRSETPRG | 765 | GTGRSETP |
| 457 | TSGSGRSETYRG | 766 | GSGRSETY |
| 458 | TSGSGRSETVRG | 767 | GSGRSETV |
| 459 | TSGSGRSETHRG | 768 | GSGRSETH |
| 460 | TSYTGRSAVPRG | 769 | YTGRSAVP |
| 461 | TSYSGRSAVYRG | 770 | YSGRSAVY |
| 462 | TSYSGRSAVVRG | 771 | YSGRSAVV |
| 463 | TSYSGRSAVHRG | 772 | YSGRSAVH |
| 464 | TSYTGRSAVYRG | 773 | YTGRSAVY |
| 465 | TSYTGRSAVVRG | 774 | YTGRSAVV |
| 466 | TSYTGRSAVHRG | 775 | YTGRSAVH |
| 467 | TSYSGRSEVPRG | 776 | YSGRSEVP |
| 468 | TSYTGRSEVPRG | 777 | YTGRSEVP |
| 469 | TSYSGRSEVYRG | 778 | YSGRSEVY |
| 470 | TSYSGRSEVVRG | 779 | YSGRSEVV |
| 471 | TSYSGRSEVHRG | 780 | YSGRSEVH |
| 472 | TSYTGRSAVPGG | 781 | YTGRSAVP |
| 473 | TSYSGRSAVYGG | 782 | YSGRSAVY |
| 474 | TSYSGRSAVVGG | 783 | YSGRSAVV |
| 475 | TSYSGRSAVHGG | 784 | YSGRSAVH |
| 476 | TSYTGRSAVYGG | 785 | YTGRSAVY |
| 477 | TSYTGRSAVVGG | 786 | YTGRSAVV |
| 478 | TSYTGRSAVHGG | 787 | YTGRSAVH |
| 828 | TSTSGRSANPAG | 877 | TSPTGRSANPLG |
| 829 | TSTSGRSANPHG | 878 | TSPSGRSAIPLG |
| 830 | TSTSGRSANPIG | 879 | TSYTGRSANPLG |
| 831 | TSTSGRSANPLG | 880 | TSYSGRSAIPLG |
| 832 | TSTSGRSANPSG | 881 | TSISGRSANYLG |
| 833 | ISTSGRSANPIG | 882 | TSPSGRSAGPLG |
| 834 | YSTSGRSANPIG | 883 | TSYTGRSAVPLG |
| 835 | TSYSGRSAVPAG | 884 | TSYTGRSAVYLG |
| 836 | TSPSGRSANIAG | 885 | TSYTGRSAVVLG |
| 837 | TSPSGRSANFAG | 886 | TSYTGRSAVHLG |
| 838 | TSPTGRSANPAG | 887 | TSYSGRSAVPSG |
| 839 | TSPSGRSAIPAG | 888 | TSPSGRSANISG |
| 840 | TSYTGRSANPAG | 889 | TSPSGRSANFSG |
| 841 | TSYSGRSAIPAG | 890 | TSPTGRSANPSG |
| 842 | TSISGRSANYAG | 891 | TSPSGRSAIPSG |
| 843 | TSPSGRSAGPAG | 892 | TSYTGRSANPSG |
| 844 | TSYTGRSAVPAG | 893 | TSYSGRSAIPSG |
| 845 | TSYTGRSAVYAG | 894 | TSISGRSANYSG |
| 846 | TSYTGRSAVVAG | 895 | TSPSGRSAGPSG |
| 847 | TSYTGRSAVHAG | 896 | TSYTGRSAVPSG |
| 848 | TSYSGRSAVPHG | 897 | TSYTGRSAVYSG |
| 849 | TSPSGRSANIHG | 898 | TSYTGRSAVVSG |
| 850 | TSPSGRSANFHG | 899 | TSYTGRSAVHSG |
| 851 | TSPTGRSANPHG | 900 | ISYSGRSAVPIG |
| 852 | TSPSGRSAIPHG | 901 | ISPSGRSANIIG |
| 853 | TSYTGRSANPHG | 902 | ISPSGRSANFIG |
| 854 | TSYSGRSAIPHG | 903 | ISPTGRSANPIG |
| 855 | TSISGRSANYHG | 904 | ISPSGRSAIPIG |
| 856 | TSPSGRSAGPHG | 905 | ISYTGRSANPIG |
| 857 | TSYTGRSAVPHG | 906 | ISYSGRSAIPIG |
| 858 | TSYTGRSAVYHG | 907 | ISISGRSANYIG |
| 859 | TSYTGRSAVVHG | 908 | ISPSGRSAGPIG |
| 860 | TSYTGRSAVHHG | 909 | ISYTGRSAVPIG |
| 861 | TSYSGRSAVPIG | 910 | ISYTGRSAVYIG |
| 862 | TSPSGRSANIIG | 911 | ISYTGRSAVVIG |
| 863 | TSPSGRSANFIG | 912 | ISYTGRSA VHIG |
| 864 | TSPTGRSANPIG | 913 | YSYSGRSAVPIG |
| 865 | TSPSGRSAIPIG | 914 | YSPSGRSANIIG |
| 866 | TSYTGRSANPIG | 915 | YSPSGRSANFIG |
| 867 | TSYSGRSAIPIG | 916 | YSPTGRSANPIG |
| 868 | TSISGRSANYIG | 917 | YSPSGRSAIPIG |
| 869 | TSPSGRSAGPIG | 918 | YSYTGRSANPIG |
| 870 | TSYTGRSA VPIG | 919 | YSYSGRSAIPIG |
| 871 | TSYTGRSA VYIG | 920 | YSISGRSANYIG |
| 872 | TSYTGRSA VVIG | 921 | YSPSGRSAGPIG |
| 873 | TSYTGRSA VHIG | 922 | YSYTGRSA VPIG |
| 874 | TSYSGRSAVPLG | 923 | YSYTGRSA VYIG |
| 875 | TSPSGRSANILG | 924 | YSYTGRSA VVIG |
| 876 | TSPSGRSANFLG | 925 | YSYTGRSA VHIG |

The following sequence may also be used as a protease cleavage sequence:
X1-X2-X3-X4-X5-X6-X7-X8 (SEQ ID NO: 788)
wherein, X1 to X8 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; and X8 is an amino acid selected from A, D, E, F, G, H, I, K, L, M, N, P, Q, R, S, T, V, W and Y.

The following sequence may also be used as a protease cleavage sequence:
X1-X2-X3-X4-X5-X6-X7-X8 (SEQ ID NO: 789)
wherein, X1 to X8 each represent a single amino acid, X1 is an amino acid selected from A, E, F, G, H, K, M, N, P, Q, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X1-X2-X3-X4-X5-X6-X7-X8 (SEQ ID NO: 790)
wherein, X1 to X8 each represent a single amino acid, X1 is an amino acid selected from A, D, E, F, G, H, I, K, M, N, P, Q, S, T, W and Y; X2 is an amino acid selected from A, D, F, L, M, P, Q, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X1-X2-X3-X4-X5-X6-X7-X8 (SEQ ID NO: 791)
wherein, X1 to X8 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, E, F, H, I, K, L, M, N, P, Q, R, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X1-X2-X3-X4-X5-X6-X7-X8 (SEQ ID NO: 792)
wherein, X1 to X8 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, G, H, I, K, L, M, N, Q, R, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X1-X2-X3-X4-X5-X6-X7-X8 (SEQ ID NO: 793)
wherein, X1 to X8 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from E, F, K, M, N, P, Q, R, S and W; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X1-X2-X3-X4-X5-X6-X7-X8 (SEQ ID NO: 794)
wherein, X1 to X8 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, F, G, L, M, P, Q, V and W; X8 is an amino acid selected from A, D, E, F, G, H, I, K, L, M, N, P, Q, R, S, T, V, W and Y.

The following sequence may also be used as a protease cleavage sequence:
X1-X2-X3-X4-X5-X6-X7-X8 (SEQ ID NO: 795)
wherein, X1 to X8 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, I, K, N, T and W.

The following sequence may also be used as a protease cleavage sequence:
X1-X2-X3-X4-X5-X6-X7-X8 (SEQ ID NO: 796)
wherein, X1 to X8 each represent a single amino acid, X1 is an amino acid selected from A, G, I, P, Q, S and Y; X2 is an amino acid selected from K or T; X3 is G; X4 is R; X5 is S; X6 is A; X7 is an amino acid selected from H, I and V; X8 is an amino acid selected from H, V and Y.

The following sequence may also be used as a protease cleavage sequence:
X1-X2-X3-X4-X5-X6-X7-X8 (SEQ ID NO: 797)
wherein, X1 to X8 each represent a single amino acid, X1 is Y; X2 is an amino acid selected from S and T; X3 is G; X4 is R; X5 is S; X6 is an amino acid selected from A and E; X8 is an amino acid selected from H, P, V and Y.

The following sequence may also be used as a protease cleavage sequence:
X1-X2-X3-X4-X5-X6-X7-X8-X9 (SEQ ID NO: 798)
wherein, X1 to X9 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X1-X2-X3-X4-X5-X6-X7-X8-X9 (SEQ ID NO: 799)
wherein, X1 to X9 each represent a single amino acid, X1 is an amino acid selected from A, E, F, G, H, K, M, N, P, Q, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X1-X2-X3-X4-X5-X6-X7-X8-X9 (SEQ ID NO: 800)
wherein, X1 to X9 each represent a single amino acid, X1 is an amino acid selected from A, D, E, F, G, H, I, K, M, N, P, Q, S, T, W and Y; X2 is an amino acid selected from A, D, F, L, M, P, Q, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X1-X2-X3-X4-X5-X6-X7-X8-X9 (SEQ ID NO: 801)
wherein, X1 to X9 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, E, F, H, I, K, L, M, N, P, Q, R, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X1-X2-X3-X4-X5-X6-X7-X8-X9 (SEQ ID NO: 802)
wherein, X1 to X9 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, G, H, I, K, L, M, N, Q, R, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X1-X2-X3-X4-X5-X6-X7-X8-X9 (SEQ ID NO: 803)
wherein, X1 to X9 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from E, F, K, M, N, P, Q, R, S and W ; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X1-X2-X3-X4-X5-X6-X7-X8-X9 (SEQ ID NO: 804) wherein, X1 to X9 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, F, G, L, M, P, Q, V and W; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X1-X2-X3-X4-X5-X6-X7-X8-X9 (SEQ ID NO: 805)
wherein, X1 to X9 each represent a single amino acid, X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, I, K, N, T and W; X9 is an amino acid selected from A, G, H, I, L and R.

The following sequence may also be used as a protease cleavage sequence:
X1-X2-X3-X4-X5-X6-X7-X8-X9 (SEQ ID NO: 806)
wherein, X1 to X9 each represent a single amino acid, X1 is an amino acid selected from A, G, I, P, Q, S and Y; X2 is an amino acid selected from K or T; X3 is G; X4 is R; X5 is S; X6 is A; X7 is an amino acid selected from H, I and V; X8 is an amino acid selected from H, V and Y; X9 is an amino acid selected from A, G, H, I, L and R.

The following sequence may also be used as a protease cleavage sequence:
X1-X2-X3-X4-X5-X6-X7-X8-X9 (SEQ ID NO: 807)
wherein, X1 to X9 each represent a single amino acid, X1 is Y; X2 is an amino acid selected from S and T; X3 is G; X4 is R; X5 is S; X6 is an amino acid selected from A and E; X8 is an amino acid selected from H, P, V and Y; X9 is an amino acid selected from A, G, H, I, L and R.

The following sequence may also be used as a protease cleavage sequence:
X10-X11-X1-X2-X3-X4-X5-X6-X7-X8 (SEQ ID NO: 808)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X10-X11-X1-X2-X3-X4-X5-X6-X7-X8 (SEQ ID NO: 809)
wherein, X1 to X11 each represent a single amino acid, X10 is an amino acid selected from I, T and Y; X11 is S; X1 is an amino acid selected from A, E, F, G, H, K, M, N, P, Q, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X10-X11-X1-X2-X3-X4-X5-X6-X7-X8 (SEQ ID NO: 810)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, F, L, M, P, Q, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X10-X11-X1-X2-X3-X4-X5-X6-X7-X8 (SEQ ID NO: 811)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, E, F, H, I, K, L, M, N, P, Q, R, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X10-X11-X1-X2-X3-X4-X5-X6-X7-X8 (SEQ ID NO: 812)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, G, H, I, K, L, M, N, Q, R, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X10-X11-X1-X2-X3-X4-X5-X6-X7-X8 (SEQ ID NO: 813)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from E, F, K, M, N, P, Q, R, S and W; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y.

The following sequence may also be used as a protease cleavage sequence:
X10-X11-X1-X2-X3-X4-X5-X6-X7-X8 (SEQ ID NO: 814)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, F, G, L, M, P, Q, V and W; X8 is an amino acid selected from A, D, E, F, G, H, I, K, L, M, N, P, Q, R, S, T, V, W and Y.

The following sequence may also be used as a protease cleavage sequence:
X10-X11-X1-X2-X3-X4-X5-X6-X7-X8 (SEQ ID NO: 815)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, I, K, N, T and W.

The following sequence may also be used as a protease cleavage sequence:
X10-X11-X1-X2-X3-X4-X5-X6-X7-X8 (SEQ ID NO: 816)
wherein, X1 to X11 each represent a single amino acid, X10 is an amino acid selected from I, T and Y; X11 is S; X1 is an amino acid selected from A, G, I, P, Q, S and Y; X2 is an amino acid selected from K or T; X3 is G; X4 is R; X5 is S; X6 is A; X7 is an amino acid selected from H, I and V; X8 is an amino acid selected from H, V and Y.

The following sequence may also be used as a protease cleavage sequence:
X10-X11-X1-X2-X3-X4-X5-X6-X7-X8 (SEQ ID NO: 817)
wherein, X1 to X11 each represent a single amino acid, X10 is an amino acid selected from I, T and Y; X11 is S; X1 is Y; X2 is an amino acid selected from S and T; X3 is G; X4 is R; X5 is S; X6 is an amino acid selected from A and E; X7 is an amino acid selected from N and V; X8 is an amino acid selected from H, P, V and Y.

The following sequence may also be used as a protease cleavage sequence:
X10-X11-X1-X2-X3-X4-X5-X6-X7-X8-X9 (SEQ ID NO: 818)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 819)
wherein, X1 to X11 each represent a single amino acid, X10 is an amino acid selected from I, T and Y; X11 is S; X1 is an amino acid selected from A, E, F, G, H, K, M, N, P, Q, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 820)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, F, L, M, P, Q, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 821)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, E, F, H, I, K, L, M, N, P, Q, R, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 822) wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, G, H, I, K, L, M, N, Q, R, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 823) wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from E, F, K, M, N, P, Q, R, S and W ; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 824)
wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, F, G, L, M, P, Q, V and W; X8 is an amino acid selected from A, D, E, F, G, H, I, K, L, M, N, P, Q, R, S, T, V, W and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 825) wherein, X1 to X11 each represent a single amino acid, X10 is an amino acid selected from I, T and Y; X11 is S; X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, I, K, N, T and W; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 826)
wherein, X1 to X11 each represent a single amino acid, X10 is an amino acid selected from I, T and Y; X11 is S; X1 is an amino acid selected from A, G, I, P, Q, S and Y; X2 is an amino acid selected from K or T; X3 is G; X4 is R; X5 is S; X6 is A; X7 is an amino acid selected from H, I and V; X8 is an amino acid selected from H, V and Y; X9 is an amino acid selected from A, G, H, I, L and R.

The following sequence may also be used as a protease cleavage sequence:
X10-X11-X1-X2-X3-X4-X5-X6-X7-X8-X9 (SEQ ID NO: 827)
wherein, X1 to X11 each represent a single amino acid, X10 is an amino acid selected from I, T and Y; X11 is S; X1 is Y; X2 is an amino acid selected from S and T; X3 is G; X4 is R; X5 is S; X6 is an amino acid selected from A and E; X7 is an amino acid selected from N and V; X8 is an amino acid selected from H, P, V and Y; X9 is an amino acid selected from A, G, H, I, L and R.

In addition to using the above-mentioned protease cleavage sequences, novel protease cleavage sequences may also be obtained by screening. For example, based on the result of crystal structure analysis of a known protease cleavage sequence, novel protease cleavage sequences can be explored by changing the interaction of active residues/recognition residues of the cleavage sequence and the enzyme. Novel protease cleavage sequences can also be explored by altering amino acids in a known protease cleavage sequence and examining interaction between the altered sequence and the protease. As another example, protease cleavage sequences can be explored by examining interaction of the protease with a library of peptides displayed using an *in vitro* display method such as phage display and ribosome display, or with an array of peptides immobilized on a chip or beads.

Interaction between a protease cleavage sequence and a protease can be examined by testing cleavage of the sequence by the protease *in vitro* or *in vivo.*

For example, polypeptides containing the protease cleavage sequences shown in Table 1 are all useful as protease substrates which are hydrolyzed by the action of proteases. The protease substrates shown as SEQ ID NOs: 788-827, and the protease substrates of Table 1 can be utilized as, for example, a library from which one with properties that suit the purpose can be selected to incorporate into a ligand-binding molecule. Specifically, in order to cleave the ligand-binding molecule selectively by a protease localized in the lesion, the substrates can be evaluated for sensitivity to that protease. When a ligand-binding molecule bound with a ligand is administered *in vivo,* the molecule may come in contact with various proteases before reaching the lesion. Therefore, the molecule should preferably have sensitivity to the protease localized to the lesion and also as high resistance as possible to the other proteases. In order to select a desired protease cleavage sequence depending on the purpose, each protease substrate can be analyzed in advance for sensitivity to various proteases comprehensively to find its protease resistance. Based on the obtained protease resistance spectra, it is possible to find a protease cleavage sequence with necessary sensitivity and resistance.

Alternatively, a ligand-binding molecule into which a protease cleavage sequence has been incorporated undergoes not only enzymatic actions by proteases but also various environmental stresses such as pH changes, temperature, and oxidative/reductive stress, before reaching the lesion. Based on the comparative information about resistance to these external factors among the protease substrates, protease cleavage sequence with desired properties can be selected.

In one embodiment of the present invention, a flexible linker is further attached to one end or both ends of the protease cleavage sequence. The flexible linker at one end of the protease cleavage sequence can be referred to as "first flexible linker", and the flexible linker at the other end can be referred to as "second flexible linker". In a particular embodiment, the protease cleavage sequence and the flexible linker have any of the following formulas:
(protease cleavage sequence),
(first flexible linker)-(protease cleavage sequence),
(protease cleavage sequence)-(second flexible linker), and
(first flexible linker)-(protease cleavage sequence)-(second flexible linker).

The flexible linker according to the present embodiment is preferably a peptide linker. The first flexible linker and the second flexible linker each exist independently and arbitrarily and are identical or different flexible linkers containing at least one flexible amino acid (Gly, etc.). The flexible linker contains, for example, number of residues (amino acids arbitrarily selected from Arg, Ile, Gln, Glu, Cys, Tyr, Trp, Thr, Val, His, Phe, Pro, Met, Lys, Gly, Ser, Asp, Asn, Ala, etc., particularly Gly, Ser, Asp, Asn, and Ala, more particularly, Gly and Ser, especially Gly, etc.) sufficient for the protease cleavage sequence to obtain the desired protease accessibility.

The flexible linker suitable for use at both ends of the protease cleavage sequence is usually a flexible linker that improves the access of protease to the protease cleavage sequence and elevates the cleavage efficiency of the protease. A suitable flexible linker may be readily selected and can be preferably selected from among different lengths such as 1 amino acid (Gly, etc.) to 20 amino acids, 2 amino acids to 15 amino acids, or 3 amino acids to 12 amino acids including 4 amino acids to 10 amino acids, 5 amino acids to 9 amino acids, 6 amino acids to 8 amino acids, or 7 amino acids to 8 amino acids. In some embodiments of the present invention, the flexible linker is a peptide linker of 1 to 7 amino acids.

Examples of the flexible linker include, but are not limited to, glycine polymers (G)n, glycine-serine polymers (including e.g., (GS)n, (GSGGS: SEQ ID NO: 92)n, and (GGGS: SEQ ID NO: 83)n, wherein n is an integer of at least 1), glycine-alanine polymers, alanine-serine polymers, and other flexible linkers well known in conventional techniques.

Among them, glycine and glycine-serine polymers are receiving attention because these amino acids are relatively unstructured and can easily function as neutral tethers between components.

Examples of the flexible linker consisting of the glycine-serine polymer can include, but are not limited to,
Ser
Gly·Ser (GS)
Ser·Gly (SG)
Gly·Gly·Ser (GGS)
Gly·Ser·Gly (GSG)
Ser·Gly·Gly (SGG)
Gly·Ser·Ser (GSS)
Ser·Ser·Gly (SSG)
Ser·Gly·Ser (SGS)
Gly·Gly·Gly·Ser (GGGS, SEQ ID NO: 83)
Gly·Gly·Ser·Gly (GGSG, SEQ ID NO: 84)
Gly·Ser·Gly·Gly (GSGG, SEQ ID NO: 85)
Ser·Gly·Gly·Gly (SGGG, SEQ ID NO: 86)
Gly·Ser·Ser·Gly (GSSG, SEQ ID NO: 87)
Gly·Gly·Gly·Gly·Ser (GGGGS, SEQ ID NO: 88)
Gly·Gly·Gly·Ser·Gly (GGGSG, SEQ ID NO: 89)
Gly·Gly·Ser·Gly·Gly (GGSGG, SEQ ID NO: 90)
Gly·Ser·Gly·Gly·Gly (GSGGG, SEQ ID NO: 91)
Gly·Ser·Gly·Gly·Ser (GSGGS, SEQ ID NO: 92)
Ser·Gly·Gly·Gly·Gly (SGGGG, SEQ ID NO: 93)
Gly·Ser·Ser·Gly·Gly (GSSGG, SEQ ID NO: 94)
Gly·Ser·Gly·Ser·Gly (GSGSG, SEQ ID NO: 95)
Ser·Gly·Gly·Ser·Gly (SGGSG, SEQ ID NO: 96)
Gly·Ser·Ser·Ser·Gly (GSSSG, SEQ ID NO: 97)
Gly·Gly·Gly·Gly·Gly·Ser (GGGGGS, SEQ ID NO: 98)
Ser·Gly·Gly·Gly·Gly·Gly (SGGGGG, SEQ ID NO: 99)
Gly·Gly·Gly·Gly·Gly·Gly·Ser (GGGGGGS, SEQ ID NO: 100)
Ser·Gly·Gly·Gly·Gly·Gly·Gly (SGGGGGG, SEQ ID NO: 101)
(Gly·Gly·Gly·Gly·Ser (GGGGS, SEQ ID NO: 88))n
(Ser·Gly·Gly·Gly·Gly (SGGGG, SEQ ID NO: 93))n
wherein n is an integer of 1 or larger.

However, the length and sequence of the peptide linker can be appropriately selected by those skilled in the art according to the purpose.

In some embodiments of the present invention, the cleavage site/protease cleavage sequence in the ligand-binding molecule is cleaved, thus fragmenting the single-domain antibody in the ligand-binding molecule, and the binding to the ligand is attenuated.

In one embodiment of the present invention, the cleavage site/protease cleavage sequence is introduced into the single-domain antibody within the ligand-binding molecule. In some more specific embodiments, the cleavage site/protease cleavage sequence is introduced at the position of residues forming a loop structure, and at the position of residues close to a loop structure, within the single-domain antibody. The loop structure within a single-domain antibody refers to a part that does not form a secondary structure such as an α-helix and β-sheet within the single-domain antibody.

In an embodiment in which the single-domain antibody is VHH or a single-domain VH antibody, the position of residues forming a loop structure and residues close to a loop structure may specifically refer to the range of: amino acid 7 (Kabat numbering) to amino acid 17 (Kabat numbering); amino acid 12 (Kabat numbering) to amino acid 17 (Kabat numbering); amino acid 31 (Kabat numbering) to amino acid 35b (Kabat numbering); amino acid 40 (Kabat numbering) to amino acid 47 (Kabat numbering); amino acid 50 (Kabat numbering) to amino acid 65 (Kabat numbering); amino acid 55 (Kabat numbering) to amino acid 69 (Kabat numbering); amino acid 73 (Kabat numbering) to amino acid 79 (Kabat numbering); amino acid 83 (Kabat numbering) to amino acid 89 (Kabat numbering); amino acid 95 (Kabat numbering) to amino acid 99 (Kabat numbering); amino acid 95 (Kabat numbering) to amino acid 102 (Kabat numbering); and amino acid 101 (Kabat numbering) to amino acid 113 (Kabat numbering) of the single-domain antibody.

In an embodiment where the single-domain antibody is VHH or a single-domain VH antibody, the cleavage site/protease cleavage sequence is introduced into one or more positions contained in one or more sequences selected from the following sequences of the single-domain antibody:
the sequence from amino acid 7 (Kabat numbering) to amino acid 17 (Kabat numbering) of the single-domain antibody; the sequence from amino acid 12 (Kabat numbering) to amino acid 17 (Kabat numbering) of the single-domain antibody; the sequence from amino acid 31 (Kabat numbering) to amino acid 35b (Kabat numbering) of the single-domain antibody; the sequence from amino acid 40 (Kabat numbering) to amino acid 47 (Kabat numbering) of the single-domain antibody; the sequence from amino acid 50 (Kabat numbering) to amino acid 65 (Kabat numbering) of the single-domain antibody; the sequence from amino acid 55 (Kabat numbering) to amino acid 69 (Kabat numbering) of the single-domain antibody; the sequence from amino acid 73 (Kabat numbering) to amino acid 79 (Kabat numbering) of the single-domain antibody; the sequence from amino acid 83 (Kabat numbering) to amino acid 89 (Kabat numbering) of the single-domain antibody; the sequence from amino acid 95 (Kabat numbering) to amino acid 99 (Kabat numbering) of the single-domain antibody; the sequence from amino acid 95 (Kabat numbering) to amino acid 102 (Kabat numbering) of the single-domain antibody; and the sequence from amino acid 101 (Kabat numbering) to amino acid 113 (Kabat numbering) of the single-domain antibody.

In an embodiment where the single-domain antibody is a single-domain VL antibody, the position of residues forming a loop structure and residues close to a loop structure may specifically refer to the range of: amino acid 7 (Kabat numbering) to amino acid 19 (Kabat numbering); amino acid 24 (Kabat numbering) to amino acid 34 (Kabat numbering); amino acid 39 (Kabat numbering) to amino acid 46 (Kabat numbering); amino acid 49 (Kabat numbering) to amino acid 62 (Kabat numbering); amino acid 50 (Kabat numbering) to amino acid 56 (Kabat numbering); amino acid 89 (Kabat numbering) to amino acid 97 (Kabat numbering); and amino acid 96 (Kabat numbering) to amino acid 107 (Kabat numbering) of the single-domain antibody.

In an embodiment where the single-domain antibody is a single-domain VL antibody, the cleavage site/protease cleavage sequence is introduced into one or more positions contained in one or more sequences selected from the following sequences of the single-domain antibody: the sequence from amino acid 7 (Kabat numbering) to amino acid 19 (Kabat numbering) of the single-domain antibody; the sequence from amino acid 24 (Kabat numbering) to amino acid 34 (Kabat numbering) of the single-domain antibody; the sequence from amino acid 39 (Kabat numbering) to amino acid 46 (Kabat numbering) of the single-domain antibody; the sequence from amino acid 49 (Kabat numbering) to amino acid 62 (Kabat numbering) of the single-domain antibody; the sequence from amino acid 50 (Kabat numbering) to amino acid 56 (Kabat numbering) of the single-domain antibody; the sequence from amino acid 89 (Kabat numbering) to amino acid 97 (Kabat numbering) of the single-domain antibody; and the sequence from amino acid 96 (Kabat numbering) to amino acid 107 (Kabat numbering) of the single-domain antibody.

Multiple cleavage sites/protease cleavage sequences can be provided within a ligand-binding molecule, for example, in multiple positions within the single-domain antibody in the ligand-binding molecule.

In some embodiments of the present invention, the biological activity of the ligand is inhibited by binding to the uncleaved ligand-binding molecule. Examples of the embodiments in which the biological activity of the ligand is inhibited include, but are not limited to, embodiments in which the binding of the ligand to the uncleaved ligand-binding molecule substantially or significantly interferes or competes with the binding of the ligand to its binding partner. In the case of using an antibody or a fragment thereof having ligand neutralizing activity as the ligand-binding molecule, the ligand-binding molecule bound with the ligand is capable of inhibiting the biological activity of the ligand by exerting its neutralizing activity.

In one embodiment of the present invention, preferably, the uncleaved ligand-binding molecule can sufficiently neutralize the biological activity of the ligand by binding to the ligand. That is, the biological activity of the ligand bound with the uncleaved ligand-binding molecule is preferably lower than that of the ligand unbound with the uncleaved ligand-binding molecule. The biological activity of the ligand bound with the uncleaved ligand-binding molecule can be, for example, 90% or less, preferably 80% or less, 70% or less, 60% or less, 50% or less, 40% or less, or 30% or less, particularly preferably 20% or less, 10% or less, 9% or less, 8% or less, 7% or less, 6% or less, 5% or less, 4% or less, 3% or less, 2% or less, or 1% or less, of the biological activity of the ligand unbound with the uncleaved ligand-binding molecule, though not limited thereto. The administration of the ligand-binding molecule can be expected to prevent the ligand from exerting its biological activity before arriving at a target tissue, by sufficiently neutralizing the biological activity of the ligand.

In one embodiment of the present invention, the binding activity of the cleaved ligand-binding molecule against the ligand is preferably lower than that of an *in vivo* natural ligand binding partner (e.g., natural receptor for the ligand) against the ligand. The binding activity of the cleaved ligand-binding molecule against the ligand exhibits, for example, 90% or less, preferably 80% or less, 70% or less, 60% or less, 50% or less, 40% or less, or 30% or less, particularly preferably 20% or less, 10% or less, 9% or less, 8% or less, 7% or less, 6% or less, 5% or less, 4% or less, 3% or less, 2% or less, or 1% or less, of the amount of the ligand bound with the *in vivo* natural binding partner (per unit binding partner), though not limited thereto. The desired index may be appropriately used as an index for binding activity. For example, a dissociation constant (KD) may be used. In the case of using a dissociation constant (KD) as an index for evaluating binding activity, a larger dissociation constant (KD) of the cleaved ligand-binding molecule for the ligand than that of the *in vivo* natural binding partner for the ligand means that the cleaved ligand-binding molecule has weaker binding activity against the ligand than that of the *in vivo* natural binding partner. The dissociation constant (KD) of the cleaved ligand-binding molecule for the ligand is, for example, 1.1 times or more, preferably 1.5 times or more, 2 times or more, 5 times or more, or 10 times or more, particularly preferably 100 times or more of the dissociation constant (KD) of the *in vivo* natural binding partner for the ligand. The ligand-binding molecule having only low binding activity against the ligand or hardly having binding activity against the ligand after cleavage guarantees that the ligand is released by the cleavage of the ligand-binding molecule, and can be expected to be prevented from binding to another ligand molecule again.

The ligand desirably restores the suppressed biological activity after cleavage of the ligand-binding molecule. Attenuation of the binding of the cleaved ligand-binding molecule to the ligand desirably results in attenuation of the function of the ligand-binding molecule to inhibit the biological activity of the ligand. Those skilled in the art can confirm the biological activity of the ligand by a known method, for example, a method of detecting the binding of the ligand to its binding partner.

In some embodiments of the present invention, an uncleaved ligand-binding molecule has a longer half-life than the single-domain antibody alone. An example of an embodiment for making the blood half-life of a ligand-binding molecule longer than the blood half-life of a single-domain antibody alone include, but are not limited to, increasing the molecular weight of the ligand-binding molecule, or fusing a portion having an FcRn binding ability with the single-domain antibody, or fusing a portion having albumin binding ability with the single-domain antibody, or fusing a PEGylated portion with the single-domain antibody.

In the present invention, it is preferable to use the blood half-life in humans when comparing the half-life of the single-domain antibody alone and the half-life of the ligand-binding molecule. When it is difficult to measure the blood half-life in humans, it is possible to predict the blood half-life in humans based on the blood half-life in mice (e.g., normal mice, transgenic mice expressing a human antigen, transgenic mice with human FcRn expression, etc.), or in monkeys (for example, cynomolgus monkeys).

One embodiment of increasing the blood half-life of the ligand-binding molecule over the blood half-life of the single-domain antibody alone includes increasing the molecular weight of the ligand-binding molecule. In a preferred embodiment, the molecular weight of the ligand-binding molecule is 60 kDa or more. Molecules with a molecular weight of 60 kDa or more are usually unlikely to undergo clearance by the kidney when it is present in blood, and is likely to have a long blood half-life (see J Biol Chem. 1988 Oct. 15;263(29): 15064-70).

One embodiment of increasing the blood half-life of the ligand-binding molecule compared to the blood half-life of the single-domain antibody alone includes fusing the single-domain antibody with a portion having FcRn binding ability. A portion having FcRn binding ability usually has an FcRn-binding region. An FcRn-binding region is a region having the ability to bind to FcRn, and any structure can be used as long as there is an ability to bind to FcRn.

A carrying moiety containing an FcRn-binding region can be taken up into cells by the FcRn salvage pathway and then returned to the plasma again. For example, the retention of IgG molecule in plasma is relatively long (elimination is slow) because FcRn, which is known as a salvage receptor for IgG molecules, is functioning. IgG molecules taken up into endosomes by pinocytosis bind to FcRn expressed in the endosomes under acidic conditions within the endosomes. IgG molecules that could not bind to FcRn proceed to lysosomes and are degraded there, but IgG molecules bound to FcRn move to the cell surface and return to the plasma again by dissociating from FcRn under the neutral conditions in the plasma.

An FcRn-binding region is preferably a region that directly binds to FcRn. A preferable example of an FcRn-binding region includes the Fc region of an antibody. However, regions capable of binding to polypeptides such as albumin and IgG that have the ability to bind to FcRn are capable of binding to FcRn indirectly via the albumin and IgG and such, and therefore, the FcRn binding regions of the present invention may be such regions that bind to polypeptides having the ability to bind to FcRn.

The activity of an FcRn-binding region of the present invention to bind to FcRn, particularly human FcRn, can be measured by a method known to those skilled in the art, as described in the above-mentioned binding activity section, and the conditions can be suitably determined by one skilled in the art. The binding activity to human FcRn can be assessed as KD (dissociation constant), apparent KD (apparent dissociation constant), dissociation rate kd, or apparent kd (apparent dissociation), etc. These can be measured by a method known to those skilled in the art. For example, Biacore (GE healthcare), Scatchard plot, flow cytometer, and such can be used.

Conditions for measuring the FcRn-binding activity of an FcRn-binding region can be appropriately selected by those skilled in the art and are not particularly limited. For example, as described in WO2009/125825, the measurement can be done under the conditions of MES buffer and 37°C. Furthermore, the binding activity of an FcRn-binding region of the present invention to FcRn can be measured by a method known to those skilled in the art, and can be measured using, for example, Biacore (GE Healthcare), and such. To measure the binding activity between an FcRn-binding region and an FcRn, an FcRn, or an FcRn binding region, or a carrying moiety containing the FcRn-binding region, is flowed as an analyte on a chip onto which an FcRn-binding region, or a carrying moiety containing an FcRn-binding region, or an FcRn is immobilized, respectively.

As the pH used for the measurement conditions, the binding affinity between the FcRn-binding region and FcRn may be evaluated at any pH from pH 4.0 to pH 6.5. Preferably, in order to determine the binding affinity between the FcRn-binding region and human FcRn, a pH of 5.8 to 6.0, which is close to the pH in early endosomes *in vivo,* is used. As the temperature used for the measurement conditions, the binding affinity between the FcRn-binding region and FcRn may be evaluated at any temperature from 10°C to 50°C. Preferably, a temperature of 15°C to 40°C is used to determine the binding affinity between the FcRn-binding region and human FcRn. More preferably, an arbitrary temperature from 20°C to 35°C, such as any one of 20, 21, 22, 23, 24, 25, 26, 27, 28, 29, 30, 31, 32, 33, 34, and 35°C may be used to determine the binding affinity between the FcRn-binding region and FcRn. A temperature of 25°C is a non-limiting example of an embodiment of the present invention.

An example of the FcRn-binding region is, but not limited to, an IgG antibody Fc region. When an IgG antibody Fc region is used, the type is not limited, and it is possible to use an Fc region of IgG1, IgG2, IgG3, IgG4, etc. For example, it is possible to use an Fc region containing one sequence selected from the amino acid sequences represented by SEQ ID NOs: 103, 104, 105 and 106.

In addition to an Fc region of a natural IgG antibody, a modified Fc region in which one or more amino acids are substituted can also be used as long as it has an FcRn-binding ability.

For example, it is possible to use an Fc region variant comprising an amino acid sequence where at least one amino acid selected from the following amino acids in the IgG antibody Fc region is substituted to another amino acid:
237th, 238th, 239th, 248th, 250th, 252nd, 254th, 255th, 256th, 257th, 258th, 265th, 270th, 286th, 289th, 297th, 298th, 303rd, 305th, 307th, 308th, 309th, 311th, 312th, 314th, 315th, 317th, 325th, 332nd, 334th, 360th, 376th, 380th, 382nd, 384th, 385th, 386th, 387th, 389th, 424th, 428th, 433th, 434th and 436th, according to EU numbering.

More specifically, it is possible to use an Fc region variant comprising at least one amino acid substitution selected from the following substitutions in the IgG antibody Fc region (EU numbering):
an amino acid substitution to substitute Gly at position 237 with Met,
an amino acid substitution to substitute Pro at position 238 with Ala,
an amino acid substitution to substitute Ser at position 239 with Lys,
an amino acid substitution to substitute Lys at position 248 with Ile,
an amino acid substitution to substitute Thr at position 250 with Ala, Phe, Ile, Met, Gln, Ser, Val, Trp, or Tyr,
an amino acid substitution to substitute Met at position 252 with Phe, Trp, or Tyr,
an amino acid substitution to substitute Ser at position 254 with Thr,
an amino acid substitution to substitute Arg at position 255 with Glu,
an amino acid substitution to substitute Thr at position 256 with Asp, Glu, or Gln,
an amino acid substitution to substitute Pro at position 257 with Ala, Gly, Ile, Leu, Met, Asn, Ser, Thr, or Val,
an amino acid substitution to substitute Glu at position 258 with His,
an amino acid substitution to substitute Asp at position 265 with Ala,
an amino acid substitution to substitute Asp at position 270 with Phe,
an amino acid substitution to substitute Asn at position 286 with Ala or Glu,
an amino acid substitution to substitute Thr at position 289 with His,
an amino acid substitution to substitute Asn at position 297 with Ala,
an amino acid substitution to substitute Ser at position 298 with Gly,
an amino acid substitution to substitute Val at position 303 with Ala,
an amino acid substitution to substitute Val at position 305 with Ala,
an amino acid substitution to substitute Thr at position 307 with Ala, Asp, Phe, Gly, His, Ile, Lys, Leu, Met, Asn, Pro, Gln, Arg, Ser, Val, Trp, or Tyr.
an amino acid substitution to substitute Val at position 308 with Ala, Phe, Ile, Leu, Met, Pro, Gln, or Thr.
an amino acid substitution to substitute Leu or Val at position 309 with Ala, Asp, Glu, Pro, or Arg,
an amino acid substitution to substitute Gln at position 311 with Ala, His, or Ile,
an amino acid substitution to substitute Asp at position 312 with Ala or His,
an amino acid substitution to substitute Leu at position 314 with Lys or Arg,
an amino acid substitution to substitute Asn at position 315 with Ala or His,
an amino acid substitution to substitute Lys at position 317 with Ala,
an amino acid substitution to substitute Asn at position 325 with Gly,
an amino acid substitution to substitute Ile at position 332 with Val,
an amino acid substitution to substitute Lys at position 334 with Leu,
an amino acid substitution to substitute Lys at position 360 with His,
an amino acid substitution to substitute Asp at position 376 with Ala,
an amino acid substitution to substitute Glu at position 380 with Ala,
an amino acid substitution to substitute Glu at position 382 with Ala
an amino acid substitution to substitute Asn at position 384 with Ser or Ala,
an amino acid substitution to substitute Gly at position 385 with Asp or His,
an amino acid substitution to substitute Gln at position 386 with Pro,
an amino acid substitution to substitute Pro at position 387 with Glu,
an amino acid substitution to substitute Asn at position 389 with Ala or Ser,
an amino acid substitution to substitute Ser at position 424 with Ala,
an amino acid substitution to substitute Met at position 428 with Ala, Asp, Phe, Gly, His, Ile, Lys, Leu, Asn, Pro, Gln, Ser, Thr, Val, Trp, or Tyr,
an amino acid substitution to substitute His at position 433 with Lys,
an amino acid substitution to substitute Asn at position 434 with Ala, Phe, His, Ser, Trp, or Tyr, and
an amino acid substitution to substitute Tyr or Phe at position 436 with His.

From another viewpoint, an Fc region containing at least one amino acid selected from
Met as the amino acid at position 237,
Ala as the amino acid at position 238,
Lys as the amino acid at position 239,
Ile as the amino acid at position 248,
Ala, Phe, Ile, Met, Gln, Ser, Val, Trp, or Tyr as the amino acid at position 250,
Phe, Trp, or Tyr as the amino acid at position 252,
Thr as the amino acid at position 254,
Glu as the amino acid at position 255,
Asp, Glu, or Gln as the amino acid at position 256,
Ala, Gly, Ile, Leu, Met, Asn, Ser, Thr, or Val as the amino acid at position 257, His as the amino acid at position 258,
Ala as the amino acid at position 265,
Phe as the amino acid at position 270,
Ala or Glu as the amino acid at position 286,
His as the amino acid at position 289,
Ala as the amino acid at position 297,
Gly as the amino acid at position 298,
Ala as the amino acid at position 303,
Ala as the amino acid at position 305,
Ala, Asp, Phe, Gly, His, Ile, Lys, Leu, Met, Asn, Pro, Gln, Arg, Ser, Val, Trp, or Tyr as the amino acid at position 307,
Ala, Phe, Ile, Leu, Met, Pro, Gln, or Thr as the amino acid at position 308,
Ala, Asp, Glu, Pro, or Arg as the amino acid at position 309,
Ala, His, or Ile as the amino acid at position 311,
Ala or His as the amino acid at position 312,
Lys or Arg as the amino acid at position 314,
Ala or His as the amino acid at position 315,
Ala as the amino acid at position 317,
Gly as the amino acid at position 325,
Val as the amino acid at position 332,
Leu as the amino acid at position 334,
His as the amino acid at position 360,
Ala as the amino acid at position 376,
Ala as the amino acid at position 380,
Ala as the amino acid at position 382,
Ala as the amino acid at position 384,
Asp or His as the amino acid at position 385,
Pro as the amino acid at position 386,
Glu as the amino acid at position 387,
Ala or Ser as the amino acid at position 389,
Ala as the amino acid at position 424,
Ala, Asp, Phe, Gly, His, Ile, Lys, Leu, Asn, Pro, Gln, Ser, Thr, Val, Trp, or Tyr as the amino acid at position 428,
Lys as the amino acid at position 433,
Ala, Phe, His, Ser, Trp, or Tyr as the amino acid at position 434, and
His as the amino acid at position 436
(all according to the EU numbering)
in an IgG antibody Fc region may be used.

Furthermore, as one embodiment for increasing the blood half-life of the ligand-binding molecule compared to the blood half-life of the single-domain antibody alone, there is the method of fusing the single-domain antibody with a portion having an albumin-binding property. Albumin does not undergo renal excretion and also has FcRn binding ability, it has a long half-life in blood of 17 to 19 days (J Clin Invest. 1953 Aug; 32(8): 746-768). Therefore, a protein bound to albumin becomes bulky and can also indirectly bind to FcRn, and it is reported that the half-life in blood is increased (Antibodies 2015, 4(3), 141-156).

Moreover, as one embodiment for increasing the blood half-life of the ligand-binding molecule compared to the blood half-life of the single-domain antibody alone, there is the method of fusing the single-domain antibody with a PEGylated portion. It is thought that PEGylating a protein makes the protein bulky, and at the same time suppresses the decomposition by blood proteases, thus prolongs the blood half-life of the protein (J Pharm Sci. 2008 Oct;97(10):4167-83).

In some embodiments of the present invention, the ligand-binding molecule comprises an antibody Fc region. In one specific embodiment, the ligand-binding molecule comprises the CH2 domain and the CH3 domain of the human IgG antibody. In one specific embodiment, the ligand-binding molecule contains a part that extends from Cys226 of the heavy chain of the human IgG1 antibody or from Pro230 to the carboxyl terminal of the heavy chain. However, C-terminal lysine (Lys447) or glycine-lysine (Gly446-Lys447) in the Fc region may or may not be present.

### Ligand

In the present specification, the term "ligand" is a molecule having biological activity. The molecule having biological activity usually functions by interacting with a receptor on cell surface and thereby performing biological stimulation, inhibition, or modulation in other modes. These functions are usually thought to participate in the intracellular signaling pathways of cells carrying the receptor.

In the present specification, the ligand encompasses a desired molecule that exerts biological activity through interaction with a biomolecule. For example, the ligand not only means a molecule that interacts with a receptor but also includes a molecule that exerts biological activity through interaction with the molecule, such as a receptor that interacts with the molecule, or a binding fragment thereof. For example, a ligand binding site of a protein known as a receptor, and a protein containing a site of the receptor which interacts with another molecule are included in the ligand according to the present invention. Specifically, a soluble receptor, a soluble fragment of a receptor, an extracellular domain of a transmembrane receptor, and polypeptides containing them and such are included in the ligand according to the present invention.

The ligand of the present invention can usually exert desirable biological activity by binding to one or more binding partners. The binding partner of the ligand can be an extracellular, intracellular, or transmembrane protein. In one embodiment, the binding partner of the ligand is an extracellular protein, for example, a soluble receptor. In another embodiment, the binding partner of the ligand is a membrane-bound receptor.

The ligand of the present invention can specifically bind to the binding partner with a dissociation constant (KD) of 10 µM, 1 µM, 100 nM, 50 nM, 10 nM, 5 nM, 1 nM, 500 pM, 400 pM, 350 pM, 300 pM, 250 pM, 200 pM, 150 pM, 100 pM, 50 pM, 25 pM, 10 pM, 5 pM, 1 pM, 0.5 pM, or 0.1 pM or less.

Examples of the molecule having biological activity include, but are not limited to, cytokines, chemokines, polypeptide hormones, growth factors, apoptosis inducing factors, PAMPs, DAMPs, nucleic acids, and fragments thereof. In a specific embodiment, an interleukin, an interferon, a hematopoietic factor, a member of the TNF superfamily, a chemokine, a cell growth factor, a member of the TGF-β family, a myokine, an adipokine, or a neurotrophic factor can be used as the ligand. In a more specific embodiment, CXCL10, IL-2, IL-7, IL-12, IL-15, IL-18, IL-21, IFN-α, IFN-β, IFN-g, MIG, I-TAC, RANTES, MIP-1a, MIP-1b, IL-1R1 (Interleukin-1 receptor, type I), IL-1R2 (Interleukin-1 receptor, type II), IL-1RAcP (Interleukin-1 receptor accessory protein), or IL-IRa (Protein Accession No. NP_776214, mRNA Accession No. NM_173842.2) can be used as the ligand.

Chemokines are a family of homogeneous serum proteins of 7 to 16 kDa originally characterized by their ability to induce leukocyte migration. Most of chemokines have four characteristic cysteines (Cys) and are classified into CXC or alpha, CC or beta, C or gamma and CX3C or delta chemokine classes, according to motifs displayed by the first two cysteines. Two disulfide bonds are formed between the first and third cysteines and between the second and fourth cysteines. In general, the disulfide bridges are considered necessary. Clark-Lewis and collaborators have reported that the disulfide bonds are crucial for the chemokine activity of at least CXCL10 (Clark-Lewis et al., J. Biol. Chem. 269: 16075-16081, 1994). The only one exception to having four cysteines is lymphotactin, which has only two cysteine residues. Thus, lymphotactin narrowly maintains its functional structure by only one disulfide bond.

Subfamilies of CXC or alpha are further classified, according to the presence of an ELR motif (Glu-Leu-Arg) preceding the first cysteine, into two groups: ELR-CXC chemokines and non-ELR-CXC chemokines (see e.g., Clark-Lewis, supra; and Belperio et al., "CXC Chemokines in Angiogenesis", J. Leukoc. Biol. 68: 1-8, 2000).

Interferon-inducible protein-10 (IP-10 or CXCL10) is induced by interferon-y and TNF-α and produced by keratinocytes, endothelial cells, fibroblasts and monocytes. IP-10 is considered to play a role in mobilizing activated T cells to an inflammatory site of a tissue (Dufour, et al., "IFN-gamma-inducible protein 10 (IP-10; CXCL10)-deficient mice reveal a role for IP-10 in effector T cell generation and trafficking", J Immunol., 168: 3195-204, 2002). Furthermore, there is a possibility that IP-10 plays a role in hypersensitive reaction. In addition, there is a possibility that IP-10 also plays a role in the occurrence of inflammatory demyelinating neuropathies (Kieseier, et al., "Chemokines and chemokine receptors in inflammatory demyelinating neuropathies: a central role for IP-10", Brain 125: 823-34, 2002).

Researches indicate that IP-10 may be useful in the engraftment of stem cells following transplantation (Nagasawa, T., Int. J. Hematol. 72: 408-11, 2000), in the mobilization of stem cells (Gazitt, Y., J. Hematother Stem Cell Res 10: 229-36, 2001; and Hattori et al., Blood 97: 3354-59, 2001) and in an enhancement of antitumor immunity (Nomura et al., Int. J. Cancer 91: 597-606, 2001; and Mach and Dranoff, Curr. Opin. Immunol. 12: 571-75, 2000). For example, previous reports known to those skilled in the art discuss the biological activity of chemokine (Bruce, L. et al., "Radiolabeled Chemokine binding assays", Methods in Molecular Biology (2000) vol. 138, pp. 129-134; Raphaele, B. et al., "Calcium Mobilization", Methods in Molecular Biology (2000) vol. 138, pp. 143-148; and Paul D. Ponath et al., "Transwell Chemotaxis", Methods in Molecular Biology (2000) vol. 138, pp. 113-120 Humana Press. Totowa, New Jersey).

Examples of the biological activity of CXCL10 include binding to a CXCL10 receptor (CXCR3), CXCL10-induced calcium flux, CXCL10-induced cellular chemotaxis, binding of CXCL10 to glycosaminoglycan and CXCL10 oligomerization.

Examples of the method for measuring the biological activity of CXCL10 include a method of measuring the cell migration activity of CXCL10, reporter assay using a cell line stably expressing CXCR3 (see PLoS One. 2010 Sep 13; 5 (9): e12700), and PathHunter™ β-Arrestin recruitment assay using B-arrestin recruitment induced at the early stage of GPCR signal transduction.

Interleukin 12 (IL-12) is a heterodimeric cytokine consisting of disulfide-linked glycosylated polypeptide chains of 30 and 40 kD. Cytokines are synthesized by and then secreted from dendritic cells, monocytes, macrophages, B cells, Langerhans cells and keratinocytes, and antigen-presenting cells including natural killer (NK) cells. IL-12 mediates various biological processes and has been mentioned as a NK cell stimulatory factor (NKSF), a T cell stimulatory factor, a cytotoxic T lymphocyte maturation factor and an EBV-transformed B cell line factor.

Interleukin 12 can bind to an IL-12 receptor expressed on the cytoplasmic membranes of cells (e.g., T cells and NK cells) and thereby change (e.g., start or block) a biological process. For example, the binding of IL-12 to an IL-12 receptor stimulates the growth of preactivated T cells and NK cells, promotes the cytolytic activity of cytotoxic T cells (CTL), NK cells and LAK (lymphokine-activated killer) cells, induces the production of y interferon (IPNγ) by T cells and NK cells, and induces the differentiation of naive Th0 cells into Th1 cells producing IPNγ and IL-2. In particular, IL-12 is absolutely necessary for setting the production and cellular immune response (e.g., Th1 cell-mediated immune response) of cytolytic cells (e.g., NK and CTL). Thus, IL-12 is absolutely necessary for generating and regulating both protective immunity (e.g., eradication of infectious disease) and pathological immune response (e.g., autoimmunity).

Examples of the method for measuring the biological activity of IL-12 include a method of measuring the cell growth activity of IL-12, STAT4 reporter assay, a method of measuring cell activation (cell surface marker expression, cytokine production, etc.) by IL-12, and a method of measuring the promotion of cell differentiation by IL-12.

Programmed death 1 (PD-1) protein is an inhibitory member of the CD28 family of receptors. The CD28 family also includes CD28, CTLA-4, ICOS and BTLA. PD-1 is expressed on activated B cells, T cells and bone marrow cells (Okazaki et al., (2002) Curr. Opin. Immunol. 14: 391779-82; and Bennett et al., (2003) J Immunol 170: 711-8). CD28 and ICOS, the initial members of the family, were discovered on the basis of functional influence on the elevation of T cell growth after monoclonal antibody addition (Hutloff et al., (1999) Nature 397: 263-266; and Hansen et al., (1980) Immunogenics 10: 247-260). PD-1 was discovered by screening for differential expression in apoptotic cells (Ishida et al., (1992) EMBO J 11: 3887-95). CTLA-4 and BTLA, the other members of the family, were discovered by screening for differential expression in cytotoxic T lymphocytes and TH1 cells, respectively. CD28, ICOS and CTLA-4 all have an unpaired cysteine residue which permits homodimerization. In contrast, PD-1 is considered to exist as a monomer and lacks an unpaired cysteine residue characteristic of other members of the CD28 family.

The PD-1 gene encodes for a 55 kDa type I transmembrane protein which is part of the Ig gene superfamily. PD-1 contains a membrane-proximal immunoreceptor tyrosine inhibitory motif (ITIM) and a membrane-distal tyrosine-based switch motif (ITSM). PD-1 is structurally similar to CTLA-4, but lacks a MYPPPY motif (SEQ ID NO: 102) important for B7-1 and B7-2 binding. Two ligands for PD-1, PD-L1 and PD-L2, have been identified and have been shown to negatively regulate T-cell activation upon binding to PD-1 (Freeman et al., (2000) J Exp Med 192: 1027-34; Latchman et al., (2001) Nat Immunol 2: 261-8; and Carter et al., (2002) Eur J Immunol 32: 634-43). Both PD-L1 and PD-L2 are B7 homologs that bind to PD-1, but do not bind to the other members of the CD28 family. PD-L1, one of the PD-1 ligands, is abundant in various human cancers (Dong et al., (2002) Nat. Med. 8: 787-9). The interaction between PD-1 and PD-L1 results in decrease in tumor-infiltrating lymphocytes, reduction in T cell receptor-mediated growth, and immune evasion by the cancerous cells (Dong et al., (2003) J. Mol. Med. 81: 281-7; Blank et al., (2005) Cancer Immunol. Immunother. 54: 307-314; and Konishi et al., (2004) Clin. Cancer Res. 10: 5094-100). Immunosuppression can be reversed by inhibiting the local interaction of PD-1 with PD-L1, and this effect is additive when the interaction of PD-2 with PD-L2 is also inhibited (Iwai et al., (2002) Proc. Natl. Acad. Sci. USA 99: 12293-7; and Brown et al., (2003) J. Immunol. 170: 1257-66).

PD-1 is an inhibitory member of the CD28 family expressed on activated B cells, T-cells, and bone marrow cells. Animals deficient in PD-1 develop various autoimmune phenotypes, including autoimmune cardiomyopathy and lupus-like syndrome with arthritis and nephritis (Nishimura et al., (1999) Immunity 11: 141-51; and Nishimura et al., (2001) Science 291: 319-22). PD-1 has been further found to play an important role in autoimmune encephalomyelitis, systemic lupus erythematosus, graft-versus-host disease (GVHD), type I diabetes mellitus, and rheumatoid arthritis (Salama et al., (2003) J Exp Med 198: 71-78; Prokunia and Alarcon-Riquelme (2004) Hum Mol Genet 13: R143; and Nielsen et al., (2004) Lupus 13: 510). In a mouse B cell tumor line, the ITSM of PD-1 has been shown to be essential for inhibiting BCR-mediated Ca²⁺ flux and tyrosine phosphorylation of downstream effector molecules (Okazaki et al., (2001) PNAS 98: 13866-71).

In some embodiments of the present invention, the ligand is a cytokine.

Cytokines are a secretory cell signaling protein family involved in immunomodulatory and inflammatory processes. These cytokines are secreted by glial cells of the nervous system and by many cells of the immune system. The cytokines can be classified into proteins, peptides and glycoproteins, and encompass large and diverse family of regulatory factors. The cytokines can induce intracellular signal transduction through binding to their cell surface receptors, thereby causing the regulation of enzyme activity, upregulation or downregulation of some genes and transcriptional factors thereof, or feedback inhibition, etc.

In some embodiments, the cytokine of the present invention includes immunomodulatory factors such as interleukins (IL) and interferons (IFN). A suitable cytokine can contain a protein derived from one or more of the following types: four α-helix bundle families (which include the IL-2 subfamily, the IFN subfamily and IL-10 subfamily); the IL-1 family (which includes IL-1 and IL-8); and the IL-17 family. The cytokine can also include those classified into type 1 cytokines (e.g., IFN-γ and TGF-β) which enhance cellular immune response, or type 2 cytokines (e.g., IL-4, IL-10, and IL-13) which work advantageously for antibody reaction.

In some embodiments of the present invention, the ligand is a chemokine. Chemokines generally act as chemoattractants that mobilize immune effector cells to chemokine expression sites. This is considered beneficial for expressing a particular chemokine gene together with, for example, a cytokine gene, for the purpose of mobilizing other immune system components to a treatment site. Such chemokines include CXCL10, RANTES, MCAF, MIP1-α, and MIP1-β. Those skilled in the art should know that certain cytokines also have a chemoattractive effect and acknowledge that such cytokines can be classified by the term "chemokine".

In some embodiments of the present invention, a cytokine variant, a chemokine variant, or the like (e.g., Annu Rev Immunol. 2015; 33: 139-67) or a fusion protein containing them (e.g., Stem Cells Transl Med. 2015 Jan; 4 (1): 66-73) can be used as the ligand.

In some embodiments of the present invention, the ligand is selected from CXCL10, PD-1, IL-12, IL-6R, IL-1R1, IL-1R2, IL-1RAcP, and IL-1Ra. The CXCL10, PD-1, IL-12, IL-6R, IL-1R1, IL-1R2, IL-1RAcP, and IL-1Ra may have the same sequences as those of naturally occurring CXCL10, PD-1, IL-12, IL-6R, IL-1R1, IL-1R2, IL-1RAcP, and IL-1Ra, respectively, or may be a ligand variant that differs in sequence from naturally occurring CXCL10, PD-1, IL-12, IL-6R, IL-1R1, IL-1R2, IL-1RAcP, and IL-1Ra, but retains the physiological activity of the corresponding natural ligand. In order to obtain the ligand variant, an alteration may be artificially added to the ligand sequence for various purposes. Preferably, an alteration to resist protease cleavage (protease resistance alteration) is added thereto to obtain a ligand variant.

### Examples of ligand-binding molecules

In some embodiments of the present invention, the ligand-binding molecule comprises only a single-domain antibody having a protease cleavage sequence introduced. In some other embodiments of the present invention, the ligand-binding molecule comprises an Fc region. When using the Fc region of an IgG antibody, the type is not limited, and it is possible to use an Fc region of IgG1, IgG2, IgG3, IgG4, etc. For example, it is possible to use an Fc region comprising one sequence selected from the amino acid sequence as set forth in SEQ ID NOs: 103, 104, 105, 106, or an Fc region mutant obtained by modifying these Fc regions. Also, in some embodiments of the present invention, the ligand-binding molecule contains an antibody constant region.

In some embodiments of the present invention, the ligand-binding molecule is a fusion protein of an Fc region and a single-domain antibody introduced with a protease cleavage sequence. In a more specific embodiment, the ligand-binding molecule comprises a series of peptide chains consisting of, from the N-terminal to C-terminal, a single-domain antibody introduced with a protease cleavage sequence-antibody Fc region. In another specific embodiment, the ligand-binding molecule is a dimeric protein containing two series of peptide chains consisting of a single-domain antibody introduced with a protease cleavage sequence-antibody hinge region-antibody Fc region. In another specific embodiment, the ligand-binding molecule is a dimeric protein containing two series of peptide chains consisting of a single-domain antibody introduced with a protease cleavage sequence-antibody hinge region-antibody CH2-antibody CH3.

### Complex of ligand and ligand-binding molecule

In some embodiments of the present invention, the ligand-binding molecule in an uncleaved state forms a complex with the ligand by antigen-antibody binding. In a more specific embodiment, the complex of the ligand-binding molecule and ligand are formed by a non-covalent bond between the ligand-binding molecule and ligand, for example, an antigen-antibody bond.

Therefore, the present invention also provides a method for producing a complex, which comprises the step of contacting a ligand-binding molecule with a ligand, and the step of recovering a complex in which the ligand is bound to the ligand-binding molecule. In the present invention, the ligand-binding molecule and the ligand can be brought into contact under conditions capable of binding the two. The obtained complex can be made into a pharmaceutical composition, for example by combining with a pharmaceutically acceptable carrier.

### Fusion protein in which a ligand and a ligand-binding molecule are fused

In some embodiments of the present invention, the uncleaved ligand-binding molecule is fused with the ligand to form a fusion protein. The ligand-binding molecule moiety and the ligand moiety in the fusion protein further interact with each other through antigen-antibody binding. The ligand-binding molecule and the ligand can be fused via a linker or without a linker. Even when the ligand-binding molecule and the ligand in the fusion protein are fused via or without a linker, the noncovalent bond still exists between the ligand-binding molecule moiety and the ligand moiety. In other words, even in the embodiments in which the ligand-binding molecule is fused with the ligand, the noncovalent bond between the ligand-binding molecule moiety and the ligand moiety is similar to that in embodiments in which the ligand-binding molecule is not fused with the ligand. The noncovalent bond is attenuated by the cleavage of the ligand-binding molecule. In short, the ligand binding of the ligand-binding molecule is attenuated.

In a preferred embodiment of the present invention, the ligand-binding molecule and the ligand are fused via a linker. For example, an arbitrary peptide linker that can be introduced by genetic engineering, or a linker disclosed as a synthetic compound linker (see e.g., Protein Engineering, 9 (3), 299-305, 1996) can be used as the linker in the fusion of the ligand-binding molecule with the ligand. In the present embodiment, a peptide linker is preferred. The length of the peptide linker is not particularly limited and may be appropriately selected by those skilled in the art according to the purpose. Examples of the peptide linker can include, but are not limited to:
Ser
Gly·Ser (GS)
Ser·Gly (SG)
Gly·Gly·Ser (GGS)
Gly·Ser·Gly (GSG)
Ser·Gly·Gly (SGG)
Gly·Ser·Ser (GSS)
Ser·Ser·Gly (SSG)
Ser·Gly·Ser (SGS)
Gly·Gly·Gly·Ser (GGGS, SEQ ID NO: 83)
Gly·Gly·Ser·Gly (GGSG, SEQ ID NO: 84)
Gly·Ser·Gly·Gly (GSGG, SEQ ID NO: 85)
Ser·Gly·Gly·Gly (SGGG, SEQ ID NO: 86)
Gly·Ser·Ser·Gly (GSSG, SEQ ID NO: 87)
Gly·Gly·Gly·Gly·Ser (GGGGS, SEQ ID NO: 88)
Gly·Gly·Gly·Ser·Gly (GGGSG, SEQ ID NO: 89)
Gly·Gly·Ser·Gly·Gly (GGSGG, SEQ ID NO: 90)
Gly·Ser·Gly·Gly·Gly (GSGGG, SEQ ID NO: 91)
Gly·Ser·Gly·Gly·Ser (GSGGS, SEQ ID NO: 92)
Ser·Gly·Gly·Gly·Gly (SGGGG, SEQ ID NO: 93)
Gly·Ser·Ser·Gly·Gly (GSSGG, SEQ ID NO: 94)
Gly·Ser·Gly·Ser·Gly (GSGSG, SEQ ID NO: 95)
Ser·Gly·Gly·Ser·Gly (SGGSG, SEQ ID NO: 96)
Gly·Ser·Ser·Ser·Gly (GSSSG, SEQ ID NO: 97)
Gly·Gly·Gly·Gly·Gly·Ser (GGGGGS, SEQ ID NO: 98)
Ser·Gly·Gly·Gly·Gly·Gly (SGGGGG, SEQ ID NO: 99)
Gly·Gly·Gly·Gly·Gly·Gly·Ser (GGGGGGS, SEQ ID NO: 100)
Ser·Gly·Gly·Gly·Gly·Gly·Gly (SGGGGGG, SEQ ID NO: 101)
(Gly·Gly·Gly·Gly·Ser (GGGGS, SEQ ID NO: 88))n
(Ser·Gly·Gly·Gly·Gly (SGGGG, SEQ ID NO: 93))n
wherein n is an integer of 1 or larger.

However, the length and sequence of the peptide linker can be appropriately selected by those skilled in the art according to the purpose.

The synthetic compound linker (chemical cross-linking agent) is a cross-linking agent usually used in peptide cross-linking, for example, N-hydroxysuccinimide (NHS), disuccinimidyl suberate (DSS), bis(sulfosuccinimidyl) suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl]sulfone (BSOCOES), or bis[2-(sulfosuccinimidoxycarbonyloxy)ethyl]sulfone (sulfo-BSOCOES).
These cross-linking agents are commercially available.

In some embodiments of the present invention, the fusion protein of the ligand-binding molecule and the ligand is a fusion protein in which the N-terminus of the ligand-binding molecule and the C-terminus of the ligand are fused with or without a linker.

Some embodiments of the structure of the fusion protein of a ligand-binding molecule and a ligand of the present invention are listed below. The embodiments below are described in order from N-terminal to C-terminal.
Ligand-single-domain antibody
Ligand-linker-single-domain antibody
Ligand-single-domain antibody-antibody constant region (or a fragment thereof)
Ligand-linker-single-domain antibody-antibody constant region (or a fragment thereof)
Ligand-single-domain antibody-antibody hinge region-antibody CH2-antibody CH3
Ligand-linker-single-domain antibody-antibody hinge region-antibody CH2-antibody CH3

### Treatment

The "treatment" (and its grammatically derived words, for example, "treat" and "treating") used in the present specification means clinical intervention that intends to alter the natural course of an individual to be treated, and it can be carried out both for prevention and during the course of a clinical pathological condition. The desirable effects of the treatment include, but not limited to, the prevention of the development or recurrence of a disease, the alleviation of symptoms, the attenuation of any direct or indirect pathological influence of the disease, the prevention of metastasis, reduction in the rate of progression of the disease, recovery from or alleviation of a disease condition, and ameliorated or improved prognosis. In some embodiments, the ligand-binding molecule containing a single-domain-antibody of the present invention can control the biological activity of the ligand and is used for delaying the onset of a disease or delaying the progression of the disease.

### Pharmaceutical composition

The present invention also relates to a pharmaceutical composition (drug) comprising a ligand-binding molecule comprising a single-domain antibody and a pharmaceutically acceptable carrier, a pharmaceutical composition (drug) comprising a ligand-binding molecule comprising a single-domain antibody, a ligand, and a pharmaceutically acceptable carrier, and a pharmaceutical composition (drug) comprising a fusion protein in which a ligand-binding molecule comprising a single-domain antibody and a ligand are fused, and a pharmaceutically acceptable carrier.

In the present invention, the pharmaceutical composition usually refers to a drug for the treatment or prevention of a disease or for examination or diagnosis.

In the present invention, the term "pharmaceutical composition comprising the ligand-binding molecule" may be used interchangeably with a "method for treating a disease, comprising administering the ligand-binding molecule to a subject to be treated" and may be used interchangeably with "use of the ligand-binding molecule for the production of a medicament for the treatment of a disease". Also, the term "pharmaceutical composition comprising the ligand-binding molecule" may be used interchangeably with "use of the ligand-binding molecule for treating a disease".

The term "pharmaceutical composition comprising the ligand-binding molecule and a ligand" may be used interchangeably with a "method for treating a disease, comprising administering the ligand-binding molecule and a ligand to a subject to be treated" and may be used interchangeably with "use of the ligand-binding molecule and a ligand for the production of a medicament for the treatment of a disease". Also, the term "pharmaceutical composition comprising the ligand-binding molecule and a ligand" may be used interchangeably with "use of the ligand-binding molecule and a ligand for treating a disease".

The term "pharmaceutical composition comprising a fusion protein" may be used interchangeably with a "method for treating a disease, comprising administering a fusion protein to a subject to be treated" and may be used interchangeably with "use of a fusion protein for the production of a medicament for the treatment of a disease". Also, the term "pharmaceutical composition comprising a fusion protein" may be used interchangeably with "use of a fusion protein for treating a disease".

The pharmaceutical composition of the present invention can be formulated by use of a method known to those skilled in the art. For example, the pharmaceutical composition can be parenterally used in a form of an injection of a sterile solution or suspension with water or any other pharmaceutically acceptable liquids. The pharmaceutical composition can be formulated, for example, by appropriately combining with a pharmacologically acceptable carrier or medium, specifically, sterile water or physiological saline, a vegetable oil, an emulsifier, a suspending agent, a surfactant, a stabilizer, a flavoring agent, an excipient, a vehicle, an antiseptic, a binder, etc. and mixing them into a unit dosage form required for generally accepted pharmaceutical practice. The amount of the active ingredient in these formulations is set so as to give an appropriate volume in a prescribed range.

A sterile composition for injection can be formulated according to usual pharmaceutical practice using a vehicle such as injectable distilled water. Examples of the injectable aqueous solution include isotonic solutions containing physiological saline, glucose, or other adjuvants (e.g., D-sorbitol, D-mannose, D-mannitol, and sodium chloride). The aqueous solution can be used in combination with an appropriate solubilizer, for example, an alcohol (ethanol, etc.), a polyalcohol (propylene glycol, polyethylene glycol, etc.), or a nonionic surfactant (Polysorbate 80™, HCO-50, etc.).

Examples of the oily liquid include sesame oil and soybean oil, and benzyl benzoate and/or benzyl alcohol can be used in combination as a solubilizer. The oily liquid can be combined with a buffer (e.g., a phosphate buffer solution and a sodium acetate buffer solution), a soothing agent (e.g., procaine hydrochloride), a stabilizer (e.g., benzyl alcohol and phenol), or an antioxidant. The prepared injection solution is usually filled into an appropriate ampule.

The pharmaceutical composition of the present invention is preferably administered through a parenteral route. For example, a composition for injection, transnasal administration, transpulmonary administration, or percutaneous administration is administered. The pharmaceutical composition can be administered systemically or locally by, for example, intravenous injection, intramuscular injection, intraperitoneal injection, or subcutaneous injection.

The administration method can be appropriately selected according to the age and symptoms of a patient. The dose of the pharmaceutical composition containing the ligand-binding molecule can be determined to the range of, for example, 0.0001 mg to 1000 mg per kg body weight per dose. Alternatively, the dose can be determined to, for example, 0.001 to 100000 mg per patient. However, the present invention is not necessarily limited by these numerical values. The dose and the administration method vary depending on the body weight, age, symptoms, and such of a patient, and those skilled in the art can determine an appropriate dose and administration method in consideration of these conditions.

In some embodiments of the present invention, a composition containing the ligand-binding molecule can be administered to an individual. The ligand-binding molecule administered to an individual binds to a ligand originally present in the individual, for example, in blood or in a tissue, and the ligand-binding molecule in a state bound with the ligand is further transported *in vivo.* The ligand-binding molecule transported to a target tissue can be cleaved in the target tissue so that its binding to the ligand can be attenuated to release the bound ligand in the target tissue. The released ligand can exert biological activity in the target tissue and treat a disease originated in the target tissue. In the embodiments in which the ligand-binding molecule suppresses the biological activity of the ligand when bound with the ligand, and is cleaved specifically in a target tissue, the ligand does not exert biological activity during transport and exerts biological activity only when the ligand-binding molecule is cleaved in the target tissue. As a result, the disease can be treated with reduced systemic adverse reactions.

Through the above embodiments, a method for suppressing or inhibiting the systemic action of an endogenous ligand is provided, wherein the method comprises the step of administering the ligand-binding molecule of the present invention. Alternatively, the present invention provides a pharmaceutical composition for suppressing or inhibiting the systemic action of an endogenous ligand, which comprises a ligand-binding molecule as an active ingredient. Alternatively, the present invention relates to the use of a ligand-binding molecule for suppressing or inhibiting the systemic action of an endogenous ligand. The present invention also relates to the use of a ligand-binding molecule in the manufacture of a pharmaceutical composition for suppressing or inhibiting the systemic action of an endogenous ligand. In these embodiments, the systemic action of the endogenous ligand is once suppressed or inhibited by the ligand-binding molecule of the present invention, and then expressed as a tissue-specific action in the target tissue.

In some embodiments of the present invention, a composition containing the ligand-binding molecule and a composition containing the ligand can be administered separately or concurrently to an individual. Alternatively, a composition containing both the ligand-binding molecule and the ligand may be administered to an individual. In the case of administering a composition containing both the ligand-binding molecule and the ligand to an individual, the ligand-binding molecule and the ligand in the composition may form a complex. In the case of administering both the ligand-binding molecule and the ligand to an individual, the ligand-binding molecule binds to the ligand administered to the individual, and the ligand-binding molecule in a state bound with the ligand is transported *in vivo.* The ligand-binding molecule transported to a target tissue can be cleaved in the target tissue so that its binding to the ligand is attenuated to release the bound ligand in the target tissue. The released ligand can exert biological activity in the target tissue and treat a disease originated in the target tissue. In the embodiments in which the ligand-binding molecule suppresses the biological activity of the ligand when bound with the ligand, and is cleaved specifically in a target tissue, the ligand does not exert biological activity during transport and exerts biological activity only when the ligand-binding molecule is cleaved in the target tissue. As a result, the disease can be treated with systemic adverse reactions reduced. The ligand-binding molecule administered to the individual is also capable of binding to a ligand originally present in the individual, in addition to the ligand administered to the individual. The ligand-binding molecule in a state bound with the ligand originally present in the individual or the ligand administered to the individual can be transported *in vivo.*

Thus, the present invention also provides methods for producing a complex consisting of a ligand-binding molecule and a ligand, wherein the methods comprise contacting the ligand-binding single-domain antibody-containing molecule with the ligand and collecting the complex consisting of the single-domain antibody-containing molecule and the ligand. The complexes of the present invention can be formulated, for example, with a pharmaceutically acceptable carrier to make a pharmaceutical composition.

In some embodiments of the present invention, the fusion protein of the ligand-binding molecule fused with a ligand can be administered to an individual. In these embodiments, the ligand-binding molecule and the ligand in the fusion protein are fused via or without a linker. The noncovalent bond still exists between the ligand-binding molecule moiety and the ligand moiety. In the case of administering the fusion protein of the ligand-binding molecule fused with a ligand to an individual, the fusion protein is transported *in vivo,* and then the ligand-binding molecule moiety in the fusion protein is cleaved in a target tissue so that the noncovalent bond of the ligand-binding molecule moiety to the ligand is attenuated to release the ligand and a portion of the ligand-binding molecule from the fusion protein. The released ligand and the released portion of the ligand-binding molecule can exert the biological activity of the ligand in the target tissue, and treat a disease originated in the target tissue. In the embodiments in which the ligand-binding molecule suppresses the biological activity of the ligand when bound with the ligand, and is cleaved specifically in a target tissue, the ligand in the fusion protein does not exert biological activity during transport and exerts biological activity only when the fusion protein is cleaved in the target tissue. As a result, the disease can be treated with reduced systemic adverse reactions.

Through the above embodiments, a method for delivering a ligand is provided. That is, the present invention provides a method for delivering a ligand to a target tissue, which comprises the step of administering a ligand bound to a ligand conjugate of the present invention. Alternatively, the present invention provides a composition for delivering a ligand to a target tissue, the composition comprising a ligand bound to a ligand conjugate. Further, the present invention relates to the use of a ligand bound to a ligand conjugate in the delivery of a ligand to a target tissue. Furthermore, the present invention relates to the use of a ligand bound to a ligand conjugate in the manufacture of a composition for delivering a ligand to a target tissue.

### Methods for producing a ligand-binding molecule and a fusion protein

The present invention also relates to a method for producing a ligand-binding molecule comprising a single-domain antibody, the binding of which to the ligand is attenuated in a cleaved state, and a method for producing a fusion protein in which the ligand-binding molecule and the ligand are fused. One embodiment of the present invention provides a method for producing a ligand-binding molecule or a fusion protein, which comprises introducing a protease cleavage sequence into a single-domain antibody contained in the ligand-binding molecule.

Examples of a method of introducing a protease cleavage sequence into a single-domain antibody contained in a ligand-binding molecule includes a method of inserting a protease cleavage sequence into the amino acid sequence of a single-domain antibody contained in a ligand-binding molecule, and a method of replacing a part of the amino acid sequence of the single-domain antibody contained in the ligand-binding molecule with a protease cleavage sequence.

To "insert" amino acid sequence A into amino acid sequence B refers to splitting amino acid sequence B into two parts without deletion, and linking the two parts with amino acid sequence A (that is, producing such an amino acid sequence as "first half of amino acid sequence B - amino acid sequence A - second half of amino acid sequence B"). To "introduce" amino acid sequence A into amino acid sequence B refers to splitting amino acid sequence B into two parts and linking the two parts with amino acid sequence A. This encompasses not only "inserting" amino acid sequence A into amino acid sequence B as mentioned above, but also linking the two parts with amino acid sequence A after deleting one or more amino acid residues of amino acid sequence B including those adjacent to amino acid sequence A (that is, replacing a portion of amino acid sequence B with amino acid sequence A).

An example of a method for obtaining a ligand-binding molecule comprising a single-domain antibody includes a method for obtaining a single-domain antibody having the ability of binding to a ligand. Single-domain antibodies can be obtained, for example, by using a known method for producing a single-domain antibody.

A single-domain antibody obtained by such a production method may be used as is as a ligand-binding molecule, or may be used after further linking the obtained single-domain antibody to another amino acid sequence.

The methods for preparing single-domain antibodies are similar to ordinary antibody preparation methods and are well known to those skilled in the art. For example, monoclonal antibodies may be produced by a hybridoma method (Kohler and Milstein, Nature 256: 495 (1975)) or a recombination method (U.S. Patent No. 4,816,567). Alternatively, monoclonal antibodies may be isolated from phage-displayed antibody libraries (Clackson et al., Nature 352: 624-628 (1991); and Marks et al., J. Mol. Biol. 222: 581-597 (1991)). Also, monoclonal antibodies may be isolated from single B cell clones (N. Biotechnol. 28 (5): 253-457 (2011)).

The ligand-binding molecule of the present invention can be produced by introducing a protease cleavage sequence into a ligand-binding molecule. More specifically, the ligand-binding molecule of the present invention can be produced by introducing a protease cleavage sequence into a single-domain antibody in the ligand-binding molecule. Whether the ligand-binding molecule is cleaved by treatment with protease appropriate for the protease cleavage sequence can be optionally confirmed. The presence or absence of the cleavage of the protease cleavage sequence can be confirmed, for example, by contacting the protease with the molecule in which a protease cleavage sequence is introduced into the ligand-binding molecule, and determining the molecular weight of the protease-treated product by an electrophoresis method such as SDS-PAGE.

The present invention also relates to a polynucleotide encoding a ligand-binding molecule containing a single-domain antibody whose binding to the ligand is attenuated by cleavage, or a polynucleotide encoding a fusion protein in which the ligand-binding molecule is fused with a ligand.

The polynucleotide according to the present invention is usually carried by (or inserted in) an appropriate vector and transfected into host cells. The vector is not particularly limited as long as the vector can stably retain an inserted nucleic acid. For example, when *E. coli* is used as the host, a pBluescript vector (manufactured by Stratagene Corp.) or the like is preferred as a vector for cloning, although various commercially available vectors can be used. In the case of using a vector for the purpose of producing the ligand-binding molecule or the fusion protein of the present invention, an expression vector is particularly useful. The expression vector is not particularly limited as long as the vector permits expression of the ligand-binding molecule *in vitro,* in *E. coli,* in cultured cells, or in individual organisms. The expression vector is preferably, for example, a pBEST vector (manufactured by Promega Corp.) for *in vitro* expression, a pET vector (manufactured by Invitrogen Corp.) for expression in *E. coli,* a pME18S-FL3 vector (GenBank Accession No. AB009864) for expression in cultured cells, and a pME18S vector (Mol Cell Biol. 8: 466-472 (1988)) for expression in individual organisms. The insertion of the DNA of the present invention into the vector can be performed by a routine method, for example, ligase reaction using restriction sites (Current protocols in Molecular Biology edit. Ausubel et al. (1987) Publish. John Wiley & Sons. Section 11.4-11.11).

The host cells are not particularly limited, and various host cells are used according to the purpose. Examples of the cells for expressing the ligand-binding molecule or the fusion protein may include bacterial cells (e.g., *Streptococcus, Staphylococcus, E. coli, Streptomyces,* and *Bacillus subtilis*)*,* fungal cells (e.g., yeasts and *Aspergillus*)*,* insect cells (e.g., *Drosophila* S2 and *Spodoptera* SF9), animal cells (e.g., CHO, COS, HeLa, C127, 3T3, BHK, HEK293, and Bowes melanoma cells) and plant cells. The transfection of the vector to the host cells may be performed by a method known in the art, for example, a calcium phosphate precipitation method, an electroporation method (Current protocols in Molecular Biology edit. Ausubel et al., (1987) Publish. John Wiley & Sons. Section 9.1-9.9), a Lipofectamine method (manufactured by GIBCO-BRL/Thermo Fisher Scientific Inc.), or a microinjection method.

An appropriate secretory signal can be incorporated into the ligand-binding molecule or the fusion protein of interest, in order to secrete the ligand-binding molecule or the fusion protein expressed in the host cells to the endoplasmic reticulum lumen, periplasmic space, or an extracellular environment. The signal may be endogenous to the ligand-binding molecule or the fusion protein of interest, or may be a foreign signal.

The method for producing a ligand-binding molecule or a fusion protein can usually include the step of recovering the ligand-binding molecule or the fusion protein. When the ligand-binding molecule or the fusion protein of the present invention is secreted into a medium, the recovery of the ligand-binding molecule or the fusion protein in the above production method includes recovery of the medium. When the ligand-binding molecule or the fusion protein of the present invention is produced in cells, the cells are first lysed, followed by the recovery of the ligand-binding molecule or the fusion protein.

A method known in the art including ammonium sulfate or ethanol precipitation, acid extraction, anion- or cation-exchange chromatography, phosphocellulose chromatography, hydrophobic interaction chromatography, affinity chromatography, hydroxylapatite chromatography, and lectin chromatography can be used for recovering and purifying the ligand-binding molecule or the fusion protein of the present invention from the recombinant cell cultures.

It should be naturally understood by those skilled in the art that any combinations of one or more embodiments described in the present specification are also included in the present invention unless there is technical contradiction on the basis of the common knowledge in the art. Also, the present invention excluding an arbitrary combination of one or more embodiments described in the present specification and should be interpreted as being contemplated by and described in the specification, unless there is technical contradiction on the basis of the common knowledge in the art.

### [Examples]

Hereinafter, Examples of the method and the composition of the present invention will be described. It shall be understood that various other embodiments can be carried out in light of the general description mentioned above.

### Example 1 Problem of previously reported immunocytokine and protease-activated cytokine

Immunocytokines targeting antigens expressed in cancer tissues have generally been prepared by fusing the cytokine of interest to the terminus of targeting IgG or scFv (Expert Opin Investig Drugs. 2009 Jul; 18 (7): 991-1000; and Curr Opin Immunol. 2016 Jun; 40: 96-102). Since cytokines including IL-2, IL-12, and TNF are very toxic, delivering these cytokines locally to cancer using an antibody to allow them to act locally on the cancer is expected to provide enhanced effects with alleviated adverse reactions (Non Patent Literatures 4, 5, and 6). However, all of these molecules present problems such as poor clinical effect in systemic administration; narrow therapeutic windows; and being too toxic to be administered systemically. This is largely because cytokines, including immunocytokines, are exposed to the whole body after systemic administration, and therefore may act and exhibit toxicity in a systemic manner, or can only be administered at very low doses in order to circumvent the toxicity. Moreover, immunocytokines binding to cancer antigens disappear in tumors through internalization by cancer cells, and therefore it is sometimes difficult to locally expose tumor to cytokines. It has also been reported that there was no difference in antitumor effect between an immunocytokine composed of IL-2 fused with an antibody that binds to a cancer antigen and an immunocytokine composed of IL-2 fused with an antibody that does not bind to the cancer antigen (Non Patent Literature 7).

As a means to reduce the systemic action of immunocytokines, which is a major problem of immunocytokines, a molecule composed of a cytokine connected with a cytokine receptor via a linker that is cleaved by protease highly expressed in cancer has been reported. The cytokine is inhibited by the cytokine receptor connected therewith via the linker, but upon protease cleavage of the linker, the cytokine is released and thereby becomes an active form. For example, a molecule in which TNF-alpha and TNFR are connected via a linker that is cleaved by uPA (Cancer Immunol Immunother. 2006 Dec;55(12): 1590-600.), and a molecule in which IL-2 and IL-2R are connected via a linker that is cleaved by MMP-2 (Immunology. 2011 Jun;133(2):206-20) have been reported. However, the cytokines in these molecules have biological activity even before cleavage of the linker, and the cleavage of the linker improves the activity by only approximately 10 times.

The following two points may be raised as their cause: the cytokines do not have strong affinity for their receptors and therefore are active to some extent even before protease cleavage; or the cytokine receptors can bind to the cytokines even after protease cleavage of the linker and therefore inhibit the biological activity of the cytokines.

### Example 2 Problem associated with application of chemokine to cancer immunotherapy

Chemokines (Nature Immunology 9, 949-952 (2008)) are basic proteins that exert their effects via G protein-coupled receptors and are a group of cytokines. The chemokines act on particular leukocytes that express its receptor, and have the activity of causing migration (chemotaxis) of the leukocytes in the direction of the concentration gradients thereof (Nat Cell Biol. 2016 Jan; 18 (1): 43-53). The chemokines are produced in large amounts at inflamed areas and are known to bring about leukocyte migration from blood vessels into inflammatory tissues.

The chemokines are considered to be exploitable in cancer immunotherapy because leukocyte migration can be controlled by controlling the chemokines. If the local migration of T cells, antigen-presenting cells, M1 macrophages, etc. to solid cancer is attained, it may be possible to elicit an antitumor effect. Cytokines can function even by systemic administration, whereas the chemokines guide the cells towards tissues of increasing concentration through their concentration gradients and therefore cannot achieve an expected effect by systemic administration. Hence, cancer immunotherapy with the chemokines by systemic administration (chemokine therapy) is considered unpractical.

### Example 3 Concept of ligand-binding molecule with introduced protease cleavage sequence, which is capable of releasing target tissue-specific ligand

As shown in Examples 1 and 2, previously reported cytokine or chemokine therapies present the following problems:
1. Immunocytokines cause adverse reactions even if the cytokine is targeted to a solid cancer by the antibody, because cytokines act systemically to produce adverse reactions, or can be administered only at low doses in order to circumvent such adverse reactions, and therefore, tumors cannot be highly exposed to immunocytokines.
2. Protease-activated cytokines, in which a cytokine receptor (or an antibody) and a cytokine are connected via a protease-cleavable linker, are active to some extent even before protease cleavage due to the insufficient neutralization of the cytokine activity.
3. The cytokine receptors (or the antibodies) can bind to the protease-activated cytokines even after protease cleavage of the linker and therefore inhibits the biological activity of the cytokine.

It is considered important to satisfy the following conditions in order to solve these problems:
1. A ligand such as a cytokine or a chemokine is sufficiently inhibited (the biological activity thereof is minimized) by a ligand-binding molecule in the whole body.
2. The ligand restores its biological activity (becomes an active ligand) by protease cleavage.
3. The ligand-binding molecule loses its ligand binding activity by protease cleavage.

The present inventors devised a molecule whose binding to a ligand is attenuated by the cleavage of a cleavage site, as a pharmaceutical composition that satisfies the conditions described above. Such a ligand-binding molecule containing a single-domain antibody comprising the cleavage site can be prepared by first obtaining a single-domain antibody against the ligand and subsequently introducing a cleavage site into the single-domain antibody.

### Example 4 Example of a ligand-binding molecule comprising a single-domain antibody having a cleavage site introduced therein

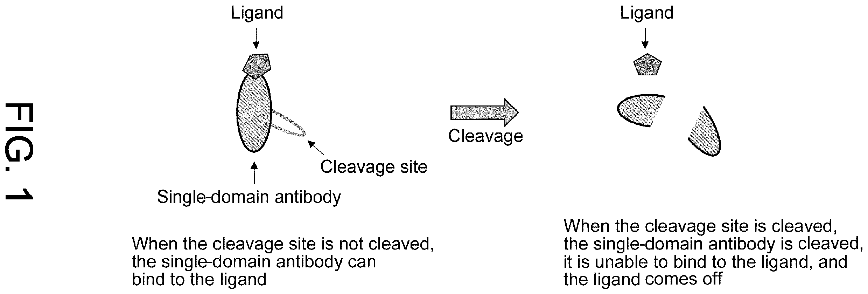

Figures 1 to 4 show examples of ligand-binding molecules comprising a single-domain antibody with a cleavage site introduced.

Figure 1 shows an example of a ligand-binding molecule containing only a single-domain antibody with a cleavage site introduced. The single-domain antibody can bind to the ligand in a state where the cleavage site is not cleaved, and if the affinity of the single-domain antibody for the ligand is sufficiently strong, the biological activity of the ligand is sufficiently inhibited. Even if the ligand-binding molecule and the ligand are systemically administered, as the ligand in the ligand-binding molecule-ligand complex is neutralized, its biological activity is not exerted, and the ligand-binding molecule-ligand complex has a longer half-life than the ligand alone. In the case where the cleavage site is a protease cleavage sequence cleaved by a tumor tissue specific protease, when the protease cleavage sequence in the single-domain antibody is cleaved by a protease highly expressed in tumor tissue regarding the complex formed of a ligand-binding molecule and a ligand that is systemically administered, the single-domain antibody can no longer bind to the ligand, neutralization of the ligand is canceled, and biological action can be exerted on the tumor tissue.

Figure 2 shows an example of a fusion polypeptide in which a ligand is fused with a ligand-binding molecule containing only a single-domain antibody into which a cleavage site has been introduced. In the state where the cleavage site is not cleaved, the single-domain antibody in the fusion polypeptide is able to bind to the ligand, and if the affinity of the single-domain antibody for the ligand is sufficiently strong, the biological activity of the ligand is sufficiently inhibited. Even if this fusion polypeptide is systemically administered, since the ligand in the fusion polypeptide is neutralized, it does not exert its biological activity, and the fusion polypeptide has a longer half-life than the ligand alone. In the case where the cleavage site is a protease cleavage sequence cleaved by a tumor tissue specific protease, when the protease cleavage sequence in the single-domain antibody is cleaved by a protease highly expressed in tumor tissue regarding the systemically administered fusion polypeptide, the single-domain antibody within the fusion polypeptide can no longer bind to the ligand, neutralization of the ligand is canceled, a portion of the fusion polypeptide including the ligand comes off, and biological action can be exerted on the tumor tissue.

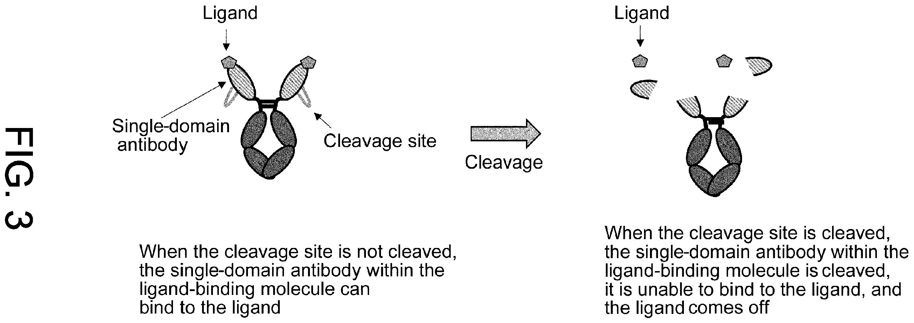

Figures 3 and 4 show an example of a ligand-binding molecule containing a single-domain antibody with a cleavage site introduced, an antibody hinge region, and an antibody Fc region, and of a fusion polypeptide of the ligand-binding molecule and ligand. Similar to the embodiments of Fig. 1 and Fig. 2, the ligand can be neutralized by the single-domain antibody in the uncleaved state, and the ligand comes off in the cleaved state to exert its biological effect. In addition, in the examples of Fig. 3 and Fig. 4, the half-life in the uncleaved state is expected to be even longer than that of the examples of Fig. 1 and Fig. 2 because an antibody Fc region is contained in the uncleaved complex or fusion polypeptide.

### Example 5 Ligand-binding molecules comprising an anti-human IL-6R single-domain antibody having an introduced protease cleavage sequence

### 5-1. Preparation of ligand-binding molecules by introducing a protease cleavage sequence into polypeptides containing an anti-human IL-6R single-domain antibody

A ligand-binding molecule comprising a single-domain antibody into which a protease cleavage sequence was introduced was prepared by introducing a protease cleavage sequence into the single-domain antibody within Polypeptide IL6R90-G1T3dCHdC (SEQ ID NO: 122), in which the N- terminus of an antibody Fc region sequence and the antibody hinge region represented by SEQ ID NO: 121 are fused to the C-terminus of a single-domain antibody against human IL-6R (SEQ ID NO: 120). First, a sequence containing the peptide sequence (LSGRSDNH, SEQ ID NO: 3), which was reported to be cleaved by a matriptase (MT-SP1) that is specifically expressed in cancer, was inserted into Polypeptide IL6R90-G1T3dCHdC to prepare the ligand-binding molecules shown in Table 2, these were expressed by transient expression using Expi293 (Life Technologies), and purified by a method known to those skilled in the art using Protein A. IL6R90-G1T3dCHdC was expressed and purified as a control molecule containing no protease cleavage sequence. The prepared ligand-binding molecules and the control molecule are dimeric proteins as shown in Figure 3.

**[Table 2]**

| Ligand-binding molecules | | |
|---|---|---|
| Ligand-binding molecule | | Insertion position of the protease cleavage sequence in the single-domain antibody contained in the ligand-binding molecule (Kabat numbering) |
| Name | SEQ ID NO | |
| 6R90H121001-G1T3dCH1dC | 123 | Between 12 and 13 |
| 6R90H131001-G1T3dCH1dC | 124 | Between 13 and 14 |
| 6R90H141001-G1T3dCH1dC | 125 | Between 14 and 15 |
| 6R90H151001-G1T3dCH1 dC | 126 | Between 15 and 16 |
| 6R90H16IG001-G1T3dCH1dC | 127 | Between 16 and 17 |

### 5-2. Evaluation of the binding with human IL-6R of the ligand-binding molecules containing an anti-human IL-6R single-domain antibody introduced with a protease cleavage sequence

### 5-2-1. Protease treatment

Recombinant Human Matriptase/ST14 Catalytic Domain (hMT-SP1, R&D systemsm 3946-SE-010) was added to 0.1 mg/mL of ligand-binding molecules prepared in Example 5-1 to a final concentration of 25 nM, this was incubated overnight at 37°C to prepare solutions containing protease-treated ligand-binding molecules. The conditions for preparing solutions containing protease-untreated ligand-binding molecules were conditions in which PBS instead of the protease was added at the same volume and stored under the same conditions.

### 5-2-2. Confirmation of protease cleavage of ligand-binding molecules (SDS-PAGE)

SDS-PAGE was used to confirm whether or not the ligand-binding molecules were cleaved by the protease treatment carried out in Example 5-2-1. 9 µL each of the solutions containing the protease-treated ligand-binding molecules/protease-untreated ligand-binding molecules prepared in Example 5-2-1 were mixed with 3 µL of sample buffer and incubated at 95°C for 1 minute. Then, electrophoresis was conducted using Mini-PROTEAN TGX gel (4-20% 15-well) (Bio-Rad #456-1096), and the proteins were stained with Sample Blue Safe Stain (Novex, LC6065). The results are shown in Fig. 5. As shown in Fig. 5, the bands of control molecules (Lanes 12 and 13) not containing the protease cleavage sequence are in the same position regardless of the presence or absence of protease treatment, while for ligand-binding molecules to which each protease cleavage sequence is introduced there is a new band that occurs only after the protease treatment. Namely, it was demonstrated that ligand-binding molecules containing a single-domain antibody introduced with each of the protease cleavage sequences were cleaved by the protease treatment.

### 5-2-3. Evaluation of binding of protease-treated ligand-binding molecules/protease-untreated ligand-binding molecules to human IL-6R

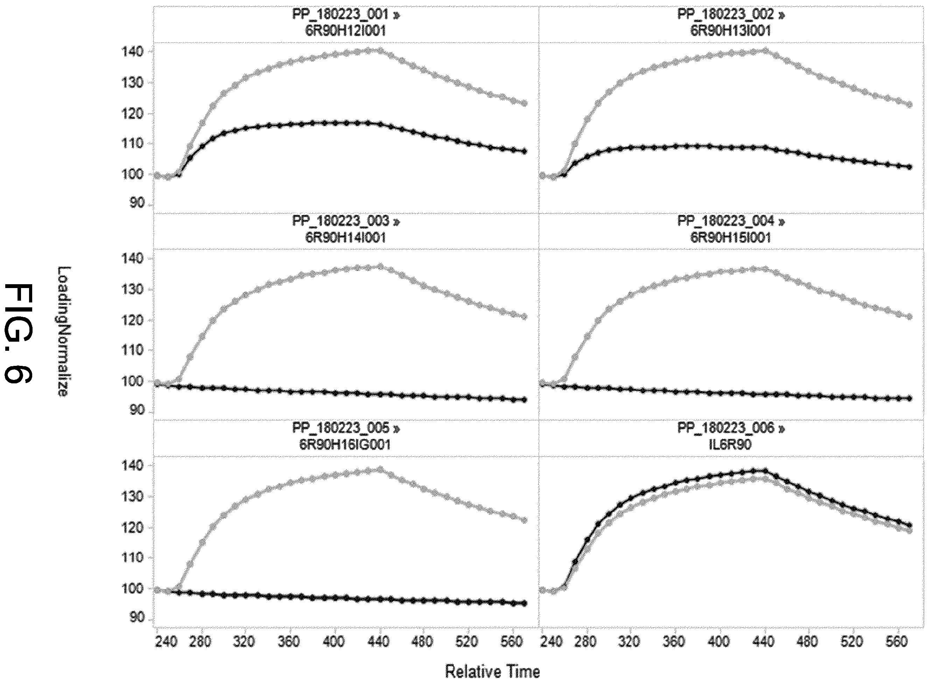

20 µL each of solutions containing the protease-treated ligand-binding molecules/protease-untreated ligand-binding molecules prepared in Example 5-2-1 were added with 60 µL of PBS to prepare IL-6R binding evaluation samples. IL-6R binding in the samples was evaluated by the Bio-Layer Interferometry method (BLI method). IL-6R binding evaluation samples and IL-6R were dispensed into a Tilted bottom (TW384) Microplate (Forte bio, 18-5076). A Protein G sensor (ForteBio, 18-0022) was hydrated with PBS and measurement was performed with Octet RED 384 at the 25°C setting. The baseline measurement was performed for 30 seconds with PBS-containing wells, and then the antibody was allowed to bind to the Protein G sensor for 200 seconds. The baseline measurement was performed again for 30 seconds with PBS-containing wells, and then binding was measured for 180 seconds with wells containing human 500 nM IL-6R, and dissociation was measured for 180 seconds with PBS-containing wells. Fig. 6 shows real-time binding graphs that show the binding state. As shown in Fig. 6, when each ligand-binding molecule introduced with a protease cleavage sequence was used, the amount of human IL-6R bound was reduced as compared to the case where a protease-untreated ligand-binding molecule was used.

### Example 6 Preparation and evaluation of fusion proteins of human IL-6R and a ligand-binding molecule containing an anti-human IL-6R single-domain antibody introduced with a protease cleavage sequence (ligand-binding molecule-IL-6R fusion proteins)

### 6-1. Preparation of fusion proteins of human IL-6R and a ligand-binding molecule containing an anti-human IL-6R single-domain antibody introduced with a protease cleavage sequence

The ligand-binding molecule-IL-6R fusion proteins (Table 3) were prepared by fusing the N-terminus of the ligand-binding molecules prepared in Example 5-1 with the C-terminal of human IL-6R (SEQ ID NO: 128) via various linkers consisting of glycine and serine. These fusion proteins were expressed by transient expression using Expi293 (Life Technologies) by a method known to those skilled in the art, and purified by a method known to those skilled in the art using protein A. The fusion proteins produced were each dimeric proteins having two peptide chains with the sequences set forth in Table 3.

**[Table 3]**

| Ligand-binding molecule-IL-6R fusion proteins | |
|---|---|
| Name | SEQ ID NO |
| IL6RaG4S.6R90H12I001-G1 T3dCH1 dC | 129 |
| IL6RaG4Sx2.6R90H12I001-G1T3dCH1dC | 130 |
| IL6RaG4Sx3.6R90H12I001-G1T3dCH1dC | 131 |
| IL6RaG4Sx4.6R90H120I001-G1T3dCH1dC | 132 |
| IL6RaG4Sx5.6R90H12I001-G1T3dCH1dC | 133 |
| IL6RaG4S.6R90H13I001-G1T3dCH1 dC | 134 |
| IL6RaG4Sx2.6R90H13I001-G1T3dCH1dC | 135 |
| IL6RaG4Sx3.6R90H13I001-G1T3dCH1dC | 136 |
| IL6RaG4Sx4.6R90H13I001-G1T3dCH1dC | 137 |
| IL6RaG4Sx5.6R90H13I001-G1T3dCH1dC | 138 |
| IL6RaG4S.6R90H14IG001-G1T3dCH1dC | 139 |
| IL6RaG4Sx2.6R90H14IG001-G1T3dCH1dC | 140 |
| IL6RaG4Sx3.6R90H14IG001-G1 T3dCH1 dC | 141 |
| IL6RaG4Sx4.6R90H14IG001-G1 T3dCH1 dC | 142 |
| IL6RaG4Sx5.6R90H14IG001-G1T3dCH1dC | 143 |
| IL6RaG4S.6R90H15I001-G1T3dCH1 dC | 144 |
| IL6RaG4Sx2.6R90H15I001-G1T3dCH1dC | 145 |
| IL6RaG4Sx3.6R90H15I001-G1T3dCH1dC | 146 |
| IL6RaG4Sx4.6R90H15I001-G1T3dCH1dC | 147 |
| IL6RaG4Sx5.6R90H15I001-G1T3dCH1dC | 148 |
| IL6RaG4S.6R90H16IG001-G1T3dCH1dC | 149 |
| IL6RaG4Sx2.6R90H16IG001-G1T3dCH1dC | 150 |
| IL6RaG4Sx3.6R90H 16IG001-G1T3dCH 1 dC | 151 |
| IL6RaG4Sx4.6R90H16IG001-G1T3dCH1dC | 152 |
| IL6RaG4Sx5.6R90H16IG001-G1T3dCH1dC | 153 |

As control molecules containing no protease cleavage sequence, molecules in which the N-terminal of IL6R90-G1T3dCHdC (SEQ ID NO: 122) was fused with the C-terminal of IL-6R via a linker were prepared (Table 4), expressed, and purified. Each of the prepared control molecules were also dimeric proteins having two peptide chains with the sequences set forth in Table 4.

**[Table 4]**

| Control molecule without protease cleavage sequence | |
|---|---|
| Name | SEQ ID NO |
| IL6RaG4S.IL6R90-G 1T3dCH1 dC | 154 |
| IL6RaG4Sx2.IL6R90-G1T3dCH1dC | 155 |
| IL6RaG4Sx3.IL6R90-G1 T3dCH1 dC | 156 |
| IL6RaG4Sx4.IL6R90-G1 T3dCH1dC | 157 |
| IL6RaG4Sx5.IL6R90-G1 T3dCH1dC | 158 |

### 6-2. Evaluation of the cleavage of ligand-binding molecule-IL-6R fusion proteins by protease

### 6-2-1. Protease treatment

Recombinant Human Matriptase/ST14 Catalytic Domain (hMT-SP1, R&D systemsm 3946-SE-010) was added to a final concentration of 25 nM to 0.1 mg/mL of the ligand-binding molecule-IL-6R fusion proteins prepared in Example 6-1. The mixture was incubated at 37°C overnight to give solutions containing protease-treated fusion proteins. The conditions for preparing solutions containing protease-untreated fusion proteins were conditions in which PBS was added instead of the protease at the same volume and stored at the same conditions.

### 6-2-2. Confirmation of protease cleavage of anti-IL-6R single-domain antibody-IL-6R fusion proteins (SDS-PAGE)

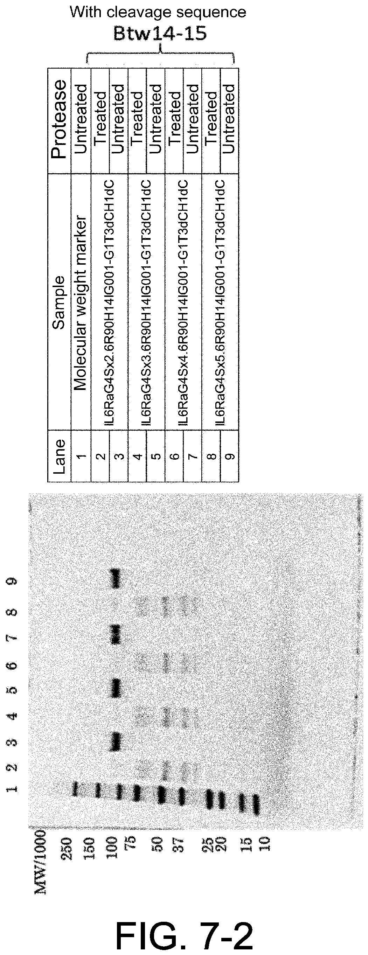

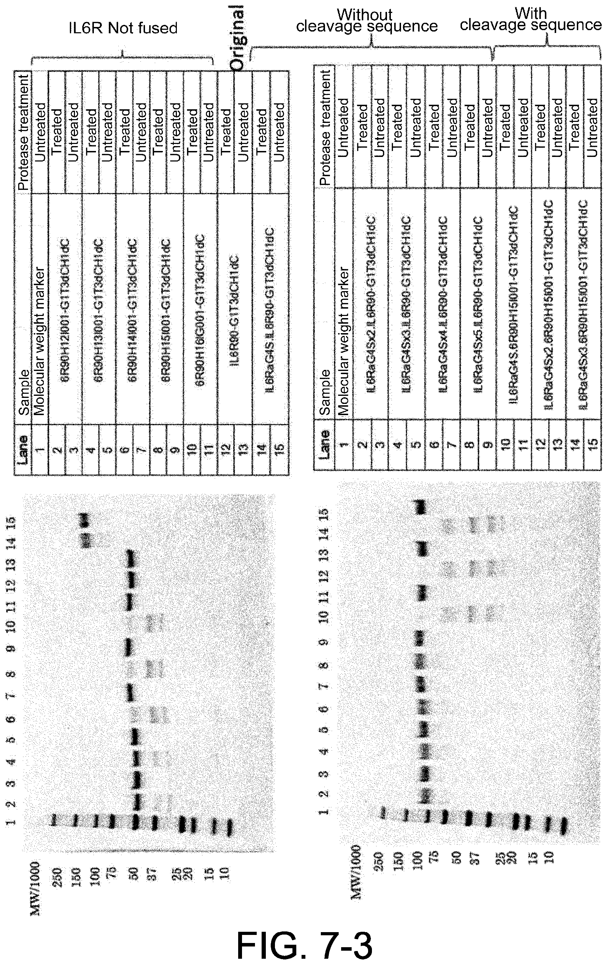

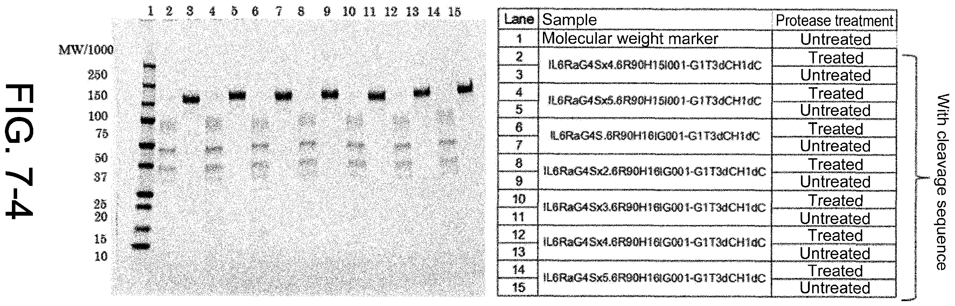

SDS-PAGE was used to confirm whether the ligand-binding molecule-IL-6R fusion proteins were cleaved by the protease treatment carried out in Example 6-2-1. 9 µL each of solutions containing protease-treated fusion proteins/protease-untreated fusion proteins prepared in Example 6-2-1were mixed with 3 µL of sample buffer and incubated at 95°C for 1 minute. Next, electrophoresis was done using Mini-PROTEAN TGX gel (4-20% 15-well) (Bio-Rad #456-1096) and the proteins were stained with Sample Blue Safe Stain (Novex, LC6065). The results are shown in Fig. 7. As shown in Fig. 7, bands of control molecules not including the protease cleavage sequence are in the same position regardless of the presence or absence of protease treatment. In contrast, for each fusion protein containing a ligand-binding molecule comprising a single-domain antibody introduced with a protease cleavage sequence there is a new band that occurs only after the protease treatment. Namely, it was shown that each fusion protein containing a ligand-binding molecule comprising a single-domain antibody introduced with each protease cleavage sequence was cleaved by the protease treatment.

### 6-2-3. Preparation of biotinylated anti-IL-6R single-domain antibody-containing molecules

As a method of detecting the release of IL-6R fused to a ligand-binding molecule, biotin was added to a molecule containing a single-domain antibody that recognizes IL-6R, and a method for detecting the binding of free IL-6R to the biotinylated anti-IL-6R single-domain antibody-containing molecule was used. The biotinylated anti-IL-6R single-domain antibody-containing molecule was prepared as follows. An anti-IL-6R single-domain antibody-containing molecule having the same single-domain-antibody partial sequence as IL6R90-G1T3dCH1dC (SEQ ID NO: 122) was prepared, and to the C terminus thereof a biotin addition sequence (AviTag sequence, SEQ ID NO: 159) was added to prepare IL6R90-FcBAPdC (SEQ ID NO: 160). A gene fragment coding for IL6R90-FcBAPdC was prepared and introduced into a vector for animal cell expression by a method known to those skilled in the art. At this time, a gene expressing EBNA1 and a gene expressing biotin ligase were simultaneously introduced and Biotin was added for the purpose of biotin labelling. The gene-introduced cells were cultured at 37°C and 8% CO₂ to secrete the biotinylated anti-IL-6R single-domain antibody-containing molecule (IL6R90-bio) of interest into the culture supernatant. IL6R90-bio was purified from the culture supernatant by a method known to those skilled in the art.

### 6-2-4. Evaluation of IL-6R release from fusion proteins by protease treatment

Release of IL-6R by protease treatment of fusion proteins was evaluated by Bio-Layer Interference method (BLI method) using the biotinylated anti-IL-6R single-domain antibody-containing molecules (IL6R90-bio) prepared in Example 6-2-3.

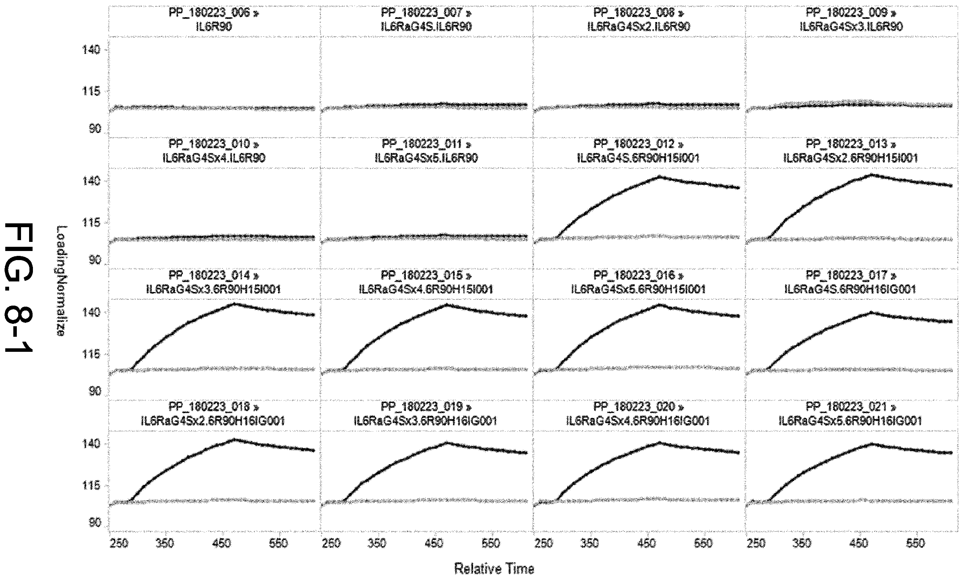

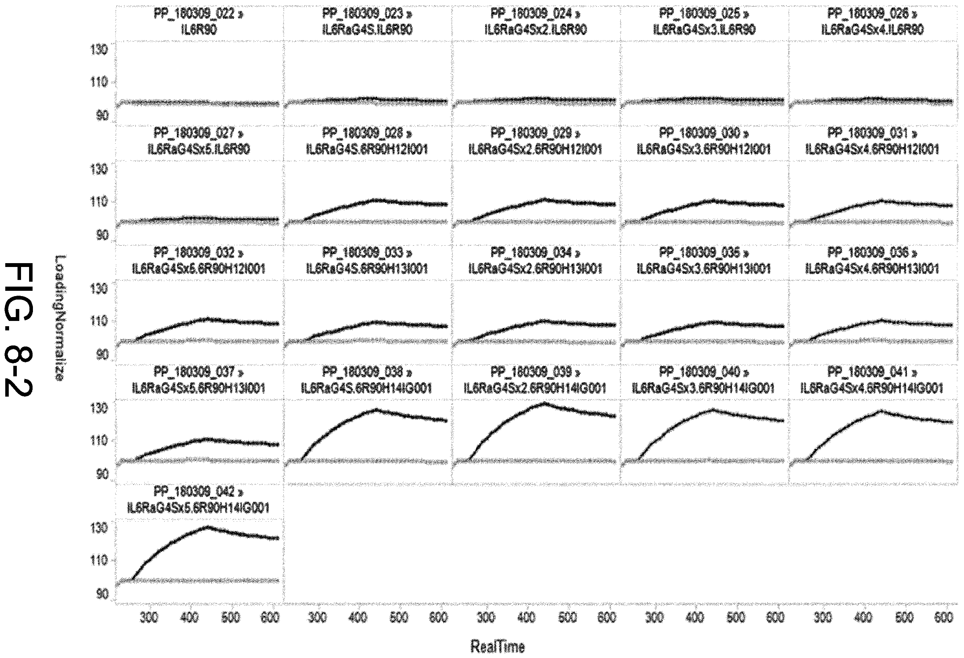

The protease-treated fusion proteins, protease-untreated fusion proteins prepared in Example 6-2-1 and IL6R90-bio were dispensed into different wells of a Tilted bottom (TW384) Microplate (ForteBio, 18-5076). A Streptavidin biosensor (ForteBio, 18-5021) was hydrated with PBS and measurement was done using Octet RED 384 at the 27°C setting. The baseline measurement was carried out for 30 seconds in wells containing PBS, and then IL6R90-bio was allowed to bind to the Streptavidin sensor for 200 seconds. The baseline measurement was done again for 30 seconds in PBS-containing wells, and then binding was measured for 180 seconds in wells containing protease-treated fusion proteins or protease-untreated fusion proteins. Dissociation was measured for 180 seconds with wells containing PBS. The real-time binding graphs showing the state of binding are shown in Fig. 8. As shown in Fig. 8, in the case of fusion proteins of IL-6R and a ligand-binding molecule containing a single-domain antibody introduced with a protease cleavage sequence, the amount of human IL-6R that binds to IL6R90-bio increased in the case of protease treatment compared to the case of no protease treatment. In other words, the protease treatment attenuates the binding activity of the single-domain antibody within a fusion protein to IL-6R, and IL-6R is released from the fusion protein.

### Example 7 Evaluation of introducing various protease cleavage sequences

### 7-1. Preparation of IgG antibodies introduced with various protease cleavage sequences

An expression vector of MRA (heavy chain: MRAH-G1T4 (SEQ ID NO: 119), light chain: MRAL-k0 (SEQ ID NO: 118)), which is a neutralizing antibody (IgG antibody) against human IL-6R, was prepared according to a method known to those skilled in the art.

Table 5 shows peptide sequences known to be cleaved by MMP-2, MMP-7, or MMP-9, and peptide sequences containing a flexible linker consisting of a glycine-serine polymer in the vicinity of these sequences.

**[Table 5]**

| Protease | Inserted sequence | SEQ ID NO |
|---|---|---|
| MMP-2, MMP-9 | PLGLAG | 3 |
| MMP-2 | GAGIPVSLRSGAG | 9 |
| MMP-2 | GPLGIAGQ | 1 0 |
| MMP-2 | GGPLGMLSQS | 1 1 |
| MMP-2 | PLGLWA | 1 2 |
| MMP-7 | VPLSLTMG | 4 |
| MMP-7 | GAGVPLSLTMGAG | 1 4 |
| MMP-9 | GAGVPLSLYSGAG | 15 |
| MMP-2, MMP-9 | GGGGSPLGLAGGGGGS | 1 0 7 |
| MMP-2 | GGGGSGPLGIAGQGGGGS | 1 0 8 |
| MMP-2 | GGGGSPLGLWAGGGGS | 1 0 9 |
| MMP-9 | GGGGSGAGVPLSLYSGAGGGGGS | 1 1 0 |

Heavy chain variants: MEIVHG4SMP2MP9G4S-MEIVHG4SMP2MP9G4SG1T4 (SEQ ID NO: 111), MEIVHG4SMP2.2G4S-MEIVHG4SMP2.2G4SG1T4 (SEQ ID NO: 112), MEIVHG4SMP2.4G4S-MEIVHG4SMP2.4G4SG1T4 (SEQ ID NO: 113), MEIVHG4SMP9G4S-MEIVHG4SMP9G4SG1T4 (SEQ ID NO: 114), MEIVHMP2.1-MEIVHMP2.1G1T4 (SEQ ID NO: 115), MEIVHMP2.3-MEIVHMP2.3G1T4 (SEQ ID NO: 116), and MEIVHMP7.2-MEIVHMP7.2G1T4 (heavy chain: SEQ ID NO: 117), in which these inserted sequences were inserted near the boundary between the heavy chain variable region and constant region of the MRA antibody, were designed. Expression vectors encoding these heavy chain variants were prepared according to a method known to those skilled in the art.

MRA variants shown in Table 6, prepared by combining the above heavy chain variant with a MRA light chain, were transiently expressed using FreeStyle 293 cells (Invitrogen Corp.) or Expi293 cells (Life Technologies Corp.) according to a method known to those skilled in the art, and purified using protein A according to a method known to those skilled in the art.

**[Table 6]**

| MRA Variants | | | |
|---|---|---|---|
| Protease | Antibody variant name | Heavy chain SEQ ID NO | Light chain SEQ ID NO |
| MMP-2 | MEIVHG4SMP2MP9G4S-MEICHG4SMP2MP9G4SG1T4/MRAL-k0 | 111 | 118 |
| MMP-2 | MEIVHG4SMP2.2G4S-MEICHG4SMP2.2G4SG1 T4/MRAL-k0 | 112 | 118 |
| MMP-2 | MEIVHG4SMP2.4G4S-MEICHG4SMP2.4G4SG1T4/MRAL-k0 | 113 | 118 |
| MMP-9 | MEIVHG4SMP2MP9G4S-MEICHG4SMP2MP9G4SG1 T4/MRAL-k0 | 111 | 118 |
| MMP-9 | MEIVHG4SMP9G4S-MEICHG4SMP9G4SG1 T4/MRAL-k0 | 114 | 118 |
| MMP-2 | MEIVHMP2.1-MEIVHMP2.1G1T4/MRAL-k0 | 115 | 118 |
| MMP-2 | MEIVHMP2.3-MEIVHMP2.3G1T4/MRAL-k0 | 116 | 118 |
| MMP-7 | MEIVHMP7.2-MEIVHMP7.2G1T4/MRAL-k0 | 117 | 118 |

### 7-2. Evaluation of protease cleavage of IgG antibodies with introduced various protease cleavage sequence

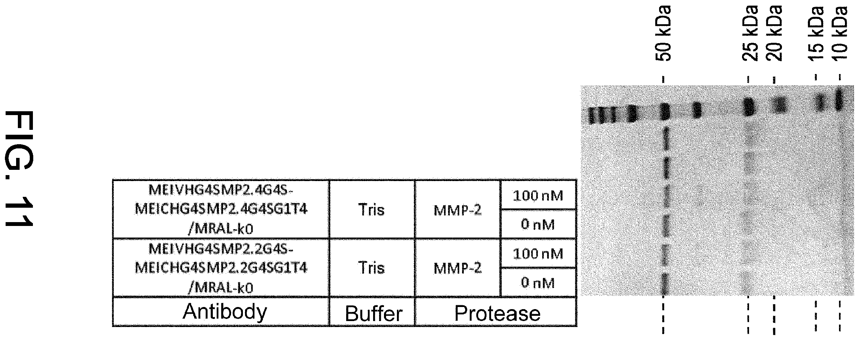

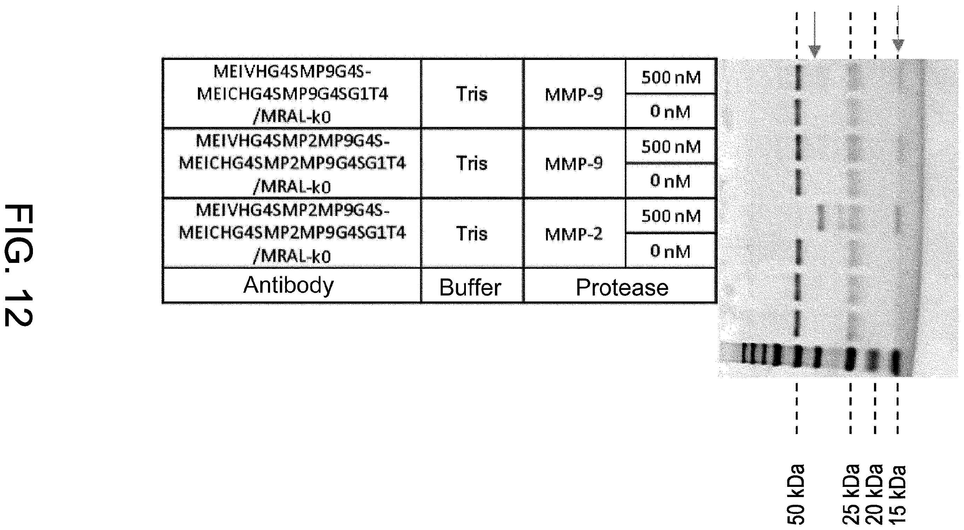

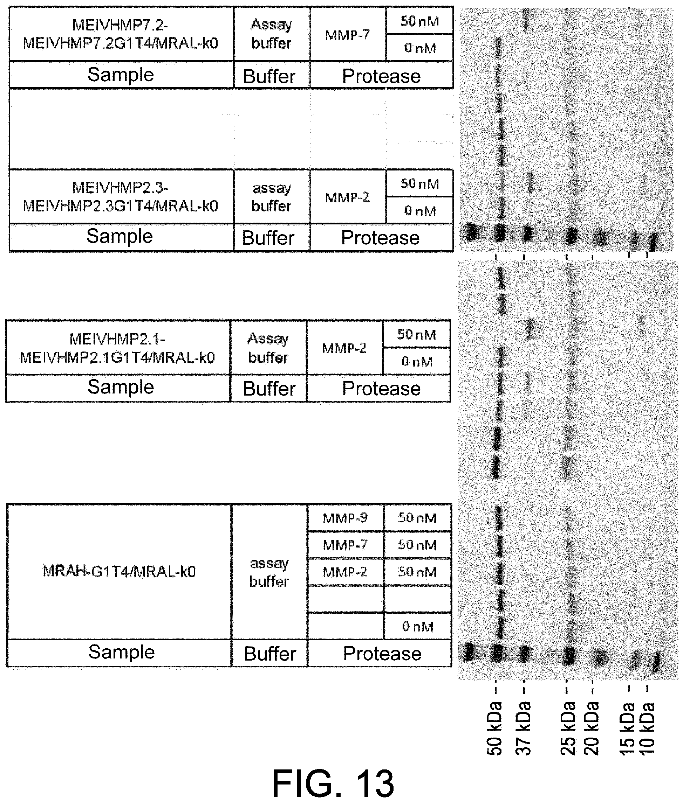

Whether the MRA variants prepared in 7-1 would be cleaved by protease was verified. Recombinant human MMP-2 (R&D Systems, Inc., 902-MP-010), recombinant human MMP-7 (R&D Systems, Inc., 907-MP-010), or recombinant human MMP-9 (R&D Systems, Inc., 911-MP-010) was used as the protease. Each protease was used after being mixed with 1 mM p-aminophenylmercuric acetate (APMA; Abcam PLC, ab112146) and activated at 37°C for 1 or 24 hours. 50 nM, 100 nM, or 500 nM protease and 50 µg/mL of each antibody were reacted in an assay buffer (MMP Activity Assay Kit (Fluorometric - Green) (ab112146), Component C: Assay Buffer) or 20 mM Tris-HCl, 150 mM NaCl, and 5 mM CaCl₂ (pH 7.2) (hereinafter, referred to as Tris) under a condition of 37°C for 20 hours. Then, cleavage by the protease was evaluated by reducing SDS-PAGE. The results are shown in Figures 11, 12, and 13. The MRA antibody variants were each reacted with the protease shown in Table 2. The cleavage by MMP-2 was observed in MEIVHG4SMP2MP9G4S-MEIVHG4SMP2MP9G4SG1T4/MRAL-k0, MEIVHG4SMP2.2G4S-MEIVHG4SMP2.2G4SG1T4/MRAL-k0, MEIVHG4SMP2.4G4S-MEIVHG4SMP2.4G4SG1T4/MRAL-k0, MEIVHMP2.1-MEIVHMP2.1G1T4/MRAL-k0, and MEIVHMP2.3-MEIVHMP2.3G1T4/MRAL-k0. The cleavage by MMP-7 was observed in MEIVHMP7.2-MEIVHMP7.2G1T4/MRAL-k0. The cleavage by MMP-9 was observed in MEIVHG4SMP2MP9G4S-MEIVHG4SMP2MP9G4SG1T4/MRAL-k0 and MEIVHG4SMP9G4S-MEIVHG4SMP9G4SG1T4/MRAL-k0.

### Example 8 Preparation and evaluation of fusion proteins of human PD-1 and a ligand-binding molecule containing an anti-human PD-1 single-domain antibody introduced with a protease cleavage sequence

### 8-1. Preparation of fusion proteins of human PD-1 and a ligand-binding molecule containing an anti-human PD-1 single-domain antibody introduced with a protease cleavage sequence

PD102C3-G1T3noCyshinge (SEQ ID NO: 161) and PD102C12-G1T3noCyshinge (SEQ ID NO: 162) in which a human antibody constant region was fused to a single-domain antibody that binds to human PD-1 were prepared by a method known to those skilled in the art. Next, a protease cleavage sequence was introduced into PD102C3-G1T3noCyshinge and PD102C12-G1T3noCyshinge, and thereafter, the N-terminal of each was fused to the C-terminal of the extracellular domain of human PD-1 (SEQ ID NO: 163) via various linkers consisting of glycine and serine to prepare ligand-binding molecule-PD-1 fusion proteins (Table 7). These fusion proteins were expressed by transient expression using Expi293 (Life Technologies) by a method known to those skilled in the art, and purified by a method known to those skilled in the art using Protein A. The fusion proteins produced were all dimeric proteins having two peptide chains of the sequences set forth in Table 7.

**[Table 7]**

| Ligand-binding molecule-human PD-1 fusion proteins | |
|---|---|
| Name | SEQ ID NO |
| hPD1 PD102C3H12-G1T3noCyshinge | 164 |
| hPD1PD102C3H13-G1T3noCyshinge | 165 |
| hPD1PD102C12H12-G1T3noCyshinge | 166 |
| hPD1PD102C12H14-G1T3noCyshinge | 167 |

### 8-2. Evaluation of the cleavage of ligand-binding molecule-PD-1 fusion proteins by protease

### 8-2-1. Protease treatment

Recombinant Human Matriptase/ST14 Catalytic Domain (hMT-SP1, R&D systemsm 3946-SE-010) was added to 0.1 mg/mL of ligand-binding molecule-PD-1 fusion proteins prepared in Example 8-1 to a final concentration of 25 nM. The mixture was incubated overnight at 37°C to give solutions containing protease-treated fused proteins. The conditions for preparing solutions containing the protease-untreated fused proteins were conditions in which PBS was added in the same volume instead of the protease and stored under the same conditions.

### 8-2-2. Confirmation of protease cleavage of anti-PD-1 single-domain antibody-PD-1 fusion proteins (SDS-PAGE)

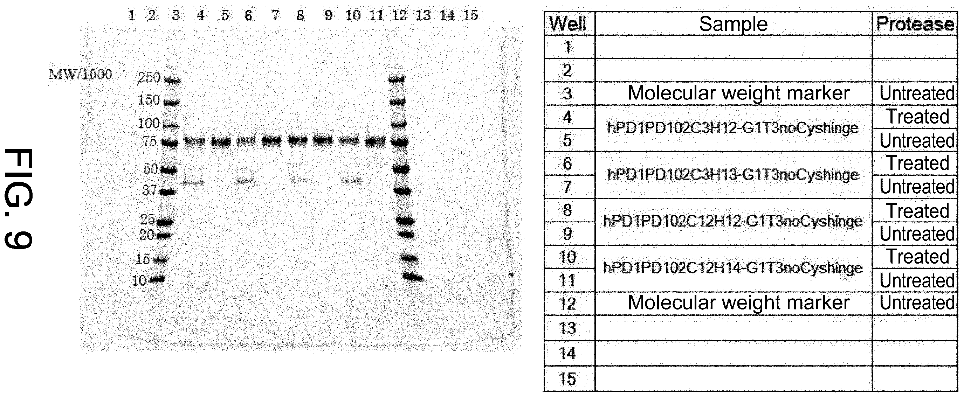

SDS-PAGE was used to confirm whether the ligand-binding molecule-PD-1 fusion proteins were cleaved by the protease treatment carried out in Example 8-2-1. 9 µL each of solutions containing the protease-treated fusion proteins/protease-untreated fusion proteins prepared in Example 8-2-1 was mixed with 3 µL of sample buffer and incubated at 95°C for 1 minute. Next, electrophoresis was performed using Mini-PROTEAN TGX gel (4-20% 15-well) (Bio-Rad #456-1096) and the proteins were stained with Sample Blue Safe Stain (Novex, LC6065). The results are shown in Fig. 9. As shown in Fig. 9, each fusion protein containing a ligand-binding molecule comprising a single-domain antibody introduced with each protease cleavage sequence has a new band that occurs only after the protease treatment. Namely, it was shown that each fusion protein containing a ligand-binding molecule comprising a single-domain antibody introduced with each protease cleaving sequence was cleaved by the protease treatment.

### 8-2-3. Preparation of biotinylated anti-PD-1 single-domain antibody-containing molecules

As a method of detecting the release of PD-1 fused to a ligand-binding molecule, biotin was added to a molecule containing a single-domain antibody that recognizes PD-1, and a method for detecting the binding of free PD-1 to the biotinylated anti-PD-1 single-domain antibody-containing molecule was used. The biotinylated anti-PD-1 single-domain antibody-containing molecule was prepared as follows. A single-domain-antibody-containing molecule having the same single-domain antibody partial sequence as PD102C3-G1T3noCyshinge was prepared, and to the C terminus thereof a biotin addition sequence (AviTag sequence, SEQ ID NO: 159) was added to prepare PD102C3-FcBAPdC (SEQ ID NO: 168).

Furthermore, an anti-PD-1 single-domain antibody-containing molecule having the same single-domain antibody partial sequence as PD102C12-G1T3noCyshinge was prepared, and to the C terminus thereof a biotin addition sequence (AviTag sequence, SEQ ID NO: 159) was added to prepare PD102C12-FcBAPdC (SEQ ID NO: 169). Gene fragments coding for PD102C3-FcBAPdC and PD102C12-FcBAPdC were prepared and introduced into a vector for animal cell expression by a method known to those skilled in the art. At this time, a gene expressing EBNA1 and a gene expressing biotin ligase were simultaneously introduced and Biotin was added for the purpose of biotin labelling. The gene-introduced cells were cultured at 37°C and 8% CO₂ to secrete the biotinylated anti-PD-1 single-domain antibody-containing molecule (PD1-bio) of interest into the culture supernatant. PD1-bio was purified from the culture supernatant by a method known to those skilled in the art.

### 8-2-4. Evaluation of PD-1 release from fusion proteins by protease treatment

PD-1 release by protease treatment of fusion proteins was evaluated by Bio-Layer Interference method (BLI method) using biotinylated anti-PD-1 single-domain antibody-containing molecule (PD1-bio) prepared in Example 8-2-3.

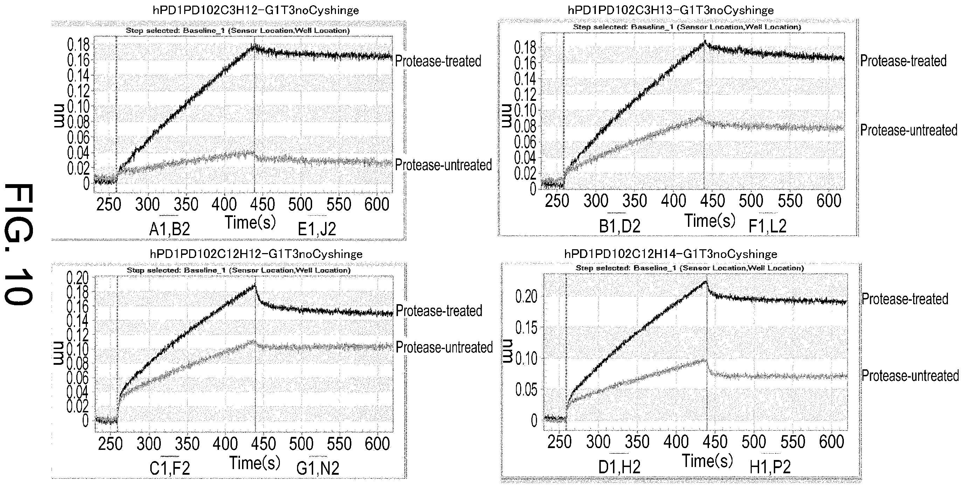

The protease-treated fusion proteins, protease-untreated fusion proteins prepared in Example 8-2-1 and PD1-bio were dispensed into different wells of a Tilted bottom (TW384) Microplate (ForteBio, 18-5076). For PD1-Bio, PD102C3-FcBAPdC or PD102C12-FcBAPdC is appropriately selected so that it binds to the same epitope as the single-domain antibody in the measurement sample. A streptavidin biosensor (ForteBio, 18-5021) was hydrated with PBS, and the measurement was done with Octet RED 384 at the 27°C setting. The baseline measurement was performed for 30 seconds with wells containing PBS, and then PD1-bio was allowed to bind to the Streptavidin sensor for 200 seconds. The baseline measurement was done again on wells containing PBS for 30 seconds, and then binding was measured for 180 seconds on wells containing protease-treated fused proteins or protease-untreated fusion proteins, and dissociation was measured for 180 seconds on PBS-containing wells. Fig. 10 shows real-time binding graphs showing the state of binding. As shown in Fig. 10, in the case of fusion proteins of PD1 and ligand-binding molecules containing a single-domain antibody introduced with a protease cleavage sequence, the amount of human PD1 that binds to PD1-bio increased in the case of protease treatment compared to the case of no protease treatment. In other words, the protease treatment attenuates the binding activity of the single-domain antibody within a fusion protein to PD1, and PD1 is released from the fusion protein.

The invention mentioned above are described in detail with reference to actual examples and illustrated examples with the aim of helping clear understanding. However, the description and illustration in the present specification should not be interpreted as limiting the scope of the present invention. The disclosure of all patent literatures and scientific literatures cited herein is explicitly incorporated herein by reference in its entirety.

### [Industrial Applicability]

The ligand-binding molecule of the present invention in a state bound with the ligand can be transported *in vivo* and cleaved in a disease tissue, so that its binding to the ligand is attenuated to release the ligand specifically in the disease tissue. Therefore, the disease tissue can be specifically exposed to the ligand. Furthermore, the ligand-binding molecule suppresses the biological activity of the ligand during transport, and therefore decreases the risk of systemic action of the ligand, which makes the ligand-binding molecule very useful in the treatment of a disease.

## Claims

1. A ligand-binding molecule, wherein the ligand-binding molecule comprises a single-domain antibody, wherein the single-domain antibody can bind to a ligand and has at least one cleavage site introduced, and wherein the binding of the ligand-binding molecule to the ligand in a state where the cleavage site is cleaved is attenuated compared to the binding of the ligand-binding molecule to the ligand in a state where the cleavage site is not cleaved.

2. The ligand-binding molecule of claim 1, wherein the cleavage site comprises a protease cleavage sequence.

3. The ligand-binding molecule of claim 2, wherein the protease is a target tissue specific protease.

4. A ligand-binding molecule, wherein the ligand-binding molecule comprises a single-domain antibody, wherein the single-domain antibody can bind to a ligand and comprises at least one protease cleavage sequence, wherein the protease cleavage sequence can be cleaved by a target tissue specific protease, and wherein the binding of the ligand-binding molecule to the ligand in a state where the protease cleavage sequence is cleaved is attenuated compared to the binding of the ligand-binding molecule to the ligand in a state where the cleavage site is not cleaved.

5. The ligand-binding molecule of any one of claims 2 to 4, wherein the protease is a cancer tissue specific protease or an inflammatory tissue specific protease.

6. The ligand-binding molecule of any one of claims 2 to 5, wherein the protease is at least one protease selected from a matriptase, urokinase (uPA), and metalloprotease.

7. The ligand-binding molecule of any one of claims 2 to 5, wherein the protease cleavage sequence is a sequence comprising one or more sequences selected from the sequences set forth in SEQ ID NOs: 2 to 82 and 788 to 827, and the sequences set forth in Table 1.

8. The ligand-binding molecule of any one of claims 1 to 7, wherein the ligand is released from the ligand-binding molecule in a state where the cleavage site or the protease cleavage sequence is cleaved.

9. The ligand-binding molecule of any one of claims 1 to 8, wherein the single-domain antibody is VHH, a single-domain VH antibody, or a single-domain VL antibody.

10. The ligand-binding molecule of any one of claims 1 to 10, wherein the ligand is a molecule having a biological activity, and the single-domain antibody has a neutralizing activity for the ligand.

11. The ligand-binding molecule of any one of claims 1 to 10, wherein the single-domain antibody is VHH or a single-domain VH antibody, and wherein the cleavage site or the protease cleavage sequence is introduced into one or more positions comprised in one or more sequences selected from the following sequences of the single-domain antibody:
the sequence from amino acid 7 (Kabat numbering) to amino acid 17 (Kabat numbering) of the single-domain antibody; the sequence from amino acid 12 (Kabat numbering) to amino acid 17 (Kabat numbering) of the single-domain antibody; the sequence from amino acid 31 (Kabat numbering) to amino acid 35b (Kabat numbering) of the single-domain antibody; the sequence from amino acid 40 (Kabat numbering) to amino acid 47 (Kabat numbering) of the single-domain antibody; the sequence from amino acid 50 (Kabat numbering) to amino acid 65 (Kabat numbering) of the single-domain antibody; the sequence from amino acid 55 (Kabat numbering) to amino acid 69 (Kabat numbering) of the single-domain antibody; the sequence from amino acid 73 (Kabat numbering) to amino acid 79 (Kabat numbering) of the single-domain antibody;
the sequence from amino acid 83 (Kabat numbering) to amino acid 89 (Kabat numbering) of the single-domain antibody; the sequence from amino acid 95 (Kabat numbering) to amino acid 99 (Kabat numbering) of the single-domain antibody; the sequence from amino acid 95 (Kabat numbering) to amino acid 102 (Kabat numbering) of the single-domain antibody; and the sequence from amino acid 101 (Kabat numbering) to amino acid 113 (Kabat numbering) of the single-domain antibody.

12. The ligand-binding molecule of any one of claims 1 to 10, wherein the single-domain antibody is a single-domain VL antibody, and wherein the cleavage site or the protease cleavage sequence is introduced into one or more positions comprised in one or more sequences selected from the following sequences of the single-domain antibody:
the sequence from amino acid 7 (Kabat numbering) to amino acid 19 (Kabat numbering) of the single-domain antibody; the sequence from amino acid 24 (Kabat numbering) to amino acid 34 (Kabat numbering) of the single-domain antibody; the sequence from amino acid 39 (Kabat numbering) to amino acid 46 (Kabat numbering) of the single-domain antibody; the sequence from amino acid 49 (Kabat numbering) to amino acid 62 (Kabat numbering) of the single-domain antibody; the sequence from amino acid 50 (Kabat numbering) to amino acid 56 (Kabat numbering) of the single-domain antibody; the sequence from amino acid 89 (Kabat numbering) to amino acid 97 (Kabat numbering) of the single-domain antibody; and the sequence from amino acid 96 (Kabat numbering) to amino acid 107 (Kabat numbering) of the single-domain antibody.

13. A complex formed of the ligand and the ligand-binding molecule of any one of claims 1 to 12.

14. A fusion protein, wherein the ligand and the ligand-binding molecule of any one of claims 1 to 12 are fused.

15. A pharmaceutical composition comprising the ligand-binding molecule of any one of claims 1 to 12, the complex of claim 13, or the fusion protein of claim 14.


SEQUENCE LISTING

<110>  CHUGAI SEIYAKU KABUSHIKI KAISHA

<120>  Ligand binding molecule comprising single domain antibody

<130>  C1-A1815P

<150>  JP 2018-103701
<151>  2018-05-30

<160>  925

<170>  PatentIn version 3.5

<210>  1
<211>  19
<212>  PRT
<213>  Artificial Sequence

<220>
<223>  an artificially synthesized sequence

<400>  1

Met Gly Trp Ser Cys Ile Ile Leu Phe Leu Val Ala Thr Ala Thr Gly
1               5                   10                  15

Val His Ser

<210>  2
<211>  8
<212>  PRT
<213>  Artificial Sequence

<220>
<223>  an artificially synthesized sequence

<400>  2

Leu Ser Gly Arg Ser Asp Asn His
1               5

<210>  3
<211>  6
<212>  PRT
<213>  Artificial Sequence

<220>
<223>  an artificially synthesized sequence

<400>  3

Pro Leu Gly Leu Ala Gly
1               5

<210>  4
<211>  8

<212>  PRT
<213>  Artificial Sequence

<220>
<223>  an artificially synthesized sequence

<400>  4

Val Pro Leu Ser Leu Thr Met Gly
1               5


<210>  5
<211>  12
<212>  PRT
<213>  Artificial Sequence

<220>
<223>  an artificially synthesized sequence

<400>  5

Thr Ser Thr Ser Gly Arg Ser Ala Asn Pro Arg Gly
1               5                   10


<210>  6
<211>  13
<212>  PRT
<213>  Artificial Sequence

<220>
<223>  an artificially synthesized sequence

<400>  6

Ile Ser Ser Gly Leu Leu Ser Gly Arg Ser Asp Asn His
1               5                   10


<210>  7
<211>  18
<212>  PRT
<213>  Artificial Sequence

<220>
<223>  an artificially synthesized sequence

<400>  7

Ala Val Gly Leu Leu Ala Pro Pro Gly Gly Leu Ser Gly Arg Ser Asp
1               5                   10                  15


Asn His


<210>  8
<211>  13
<212>  PRT
<213>  Artificial Sequence

<220>
<223>  an artificially synthesized sequence

<400>  8

Gly Ala Gly Val Pro Met Ser Met Arg Gly Gly Ala Gly
1               5                   10


<210>  9
<211>  13
<212>  PRT
<213>  Artificial Sequence

<220>
<223>  an artificially synthesized sequence

<400>  9

Gly Ala Gly Ile Pro Val Ser Leu Arg Ser Gly Ala Gly
1               5                   10


<210>  10
<211>  8
<212>  PRT
<213>  Artificial Sequence

<220>
<223>  an artificially synthesized sequence

<400>  10

Gly Pro Leu Gly Ile Ala Gly Gln
1               5


<210>  11
<211>  10
<212>  PRT
<213>  Artificial Sequence

<220>
<223>  an artificially synthesized sequence

<400>  11

Gly Gly Pro Leu Gly Met Leu Ser Gln Ser
1               5                   10


<210>  12
<211>  6
<212>  PRT
<213>  Artificial Sequence

<220>
<223>  an artificially synthesized sequence

<400>  12

Pro Leu Gly Leu Trp Ala
1               5

<210>  13
<211>  13
<212>  PRT
<213>  Artificial Sequence

<220>
<223>  an artificially synthesized sequence

<400>  13

Gly Ala Gly Arg Pro Phe Ser Met Ile Met Gly Ala Gly
1               5                   10


<210>  14
<211>  13
<212>  PRT
<213>  Artificial Sequence

<220>
<223>  an artificially synthesized sequence

<400>  14

Gly Ala Gly Val Pro Leu Ser Leu Thr Met Gly Ala Gly
1               5                   10


<210>  15
<211>  13
<212>  PRT
<213>  Artificial Sequence

<220>
<223>  an artificially synthesized sequence

<400>  15

Gly Ala Gly Val Pro Leu Ser Leu Tyr Ser Gly Ala Gly
1               5                   10


<210>  16
<211>  6
<212>  PRT
<213>  Artificial Sequence

<220>
<223>  an artificially synthesized sequence

<400>  16

Ala Ala Asn Leu Arg Asn
1               5


<210>  17
<211>  6
<212>  PRT
<213>  Artificial Sequence

<220>
<223>  an artificially synthesized sequence

<400>  17

Ala Gln Ala Tyr Val Lys
1               5


<210>  18
<211>  6
<212>  PRT
<213>  Artificial Sequence

<220>
<223>  an artificially synthesized sequence

<400>  18

Ala Ala Asn Tyr Met Arg
1               5


<210>  19
<211>  6
<212>  PRT
<213>  Artificial Sequence

<220>
<223>  an artificially synthesized sequence

<400>  19

Ala Ala Ala Leu Thr Arg
1               5


<210>  20
<211>  6
<212>  PRT
<213>  Artificial Sequence

<220>
<223>  an artificially synthesized sequence

<400>  20

Ala Gln Asn Leu Met Arg
1               5


<210>  21
<211>  6
<212>  PRT
<213>  Artificial Sequence

<220>
<223>  an artificially synthesized sequence

<400>  21

Ala Ala Asn Tyr Thr Lys
1               5

<210>  22
<211>  16
<212>  PRT
<213>  Artificial Sequence

<220>
<223>  an artificially synthesized sequence

<400>  22

Gly Ala Gly Pro Gln Gly Leu Ala Gly Gln Arg Gly Ile Val Ala Gly
1               5                   10                  15


<210>  23
<211>  8
<212>  PRT
<213>  Artificial Sequence

<220>
<223>  an artificially synthesized sequence

<400>  23

Pro Arg Phe Lys Ile Ile Gly Gly
1               5


<210>  24
<211>  8
<212>  PRT
<213>  Artificial Sequence

<220>
<223>  an artificially synthesized sequence

<400>  24

Pro Arg Phe Arg Ile Ile Gly Gly
1               5


<210>  25
<211>  9
<212>  PRT
<213>  Artificial Sequence

<220>
<223>  an artificially synthesized sequence

<400>  25

Gly Ala Gly Ser Gly Arg Ser Ala Gly
1               5


<210>  26
<211>  5
<212>  PRT
<213>  Artificial Sequence

<220>
<223>  an artificially synthesized sequence

<400>  26

Ser Gly Arg Ser Ala
1               5


<210>  27
<211>  6
<212>  PRT
<213>  Artificial Sequence

<220>
<223>  an artificially synthesized sequence

<400>  27

Gly Ser Gly Arg Ser Ala
1               5


<210>  28
<211>  5
<212>  PRT
<213>  Artificial Sequence

<220>
<223>  an artificially synthesized sequence

<400>  28

Ser Gly Lys Ser Ala
1               5


<210>  29
<211>  5
<212>  PRT
<213>  Artificial Sequence

<220>
<223>  an artificially synthesized sequence

<400>  29

Ser Gly Arg Ser Ser
1               5


<210>  30
<211>  5
<212>  PRT
<213>  Artificial Sequence

<220>
<223>  an artificially synthesized sequence

<400>  30

Ser Gly Arg Arg Ala
1               5

<210>  31
<211>  5
<212>  PRT
<213>  Artificial Sequence

<220>
<223>  an artificially synthesized sequence

<400>  31

Ser Gly Arg Asn Ala
1               5


<210>  32
<211>  5
<212>  PRT
<213>  Artificial Sequence

<220>
<223>  an artificially synthesized sequence

<400>  32

Ser Gly Arg Lys Ala
1               5


<210>  33
<211>  6
<212>  PRT
<213>  Artificial Sequence

<220>
<223>  an artificially synthesized sequence

<400>  33

Gln Arg Gly Arg Ser Ala
1               5


<210>  34
<211>  17
<212>  PRT
<213>  Artificial Sequence

<220>
<223>  an artificially synthesized sequence

<400>  34

Gly Ala Gly Ser Leu Leu Lys Ser Arg Met Val Pro Asn Phe Asn Ala
1               5                   10                  15


Gly



<210>  35

<211>  6
<212>  PRT
<213>  Artificial Sequence

<220>
<223>  an artificially synthesized sequence

<400>  35

Thr Gln Gly Ala Ala Ala
1               5


<210>  36
<211>  6
<212>  PRT
<213>  Artificial Sequence

<220>
<223>  an artificially synthesized sequence

<400>  36

Gly Ala Ala Ala Ala Ala
1               5


<210>  37
<211>  6
<212>  PRT
<213>  Artificial Sequence

<220>
<223>  an artificially synthesized sequence

<400>  37

Gly Ala Gly Ala Ala Gly
1               5


<210>  38
<211>  6
<212>  PRT
<213>  Artificial Sequence

<220>
<223>  an artificially synthesized sequence

<400>  38

Ala Ala Ala Ala Ala Gly
1               5


<210>  39
<211>  6
<212>  PRT
<213>  Artificial Sequence

<220>
<223>  an artificially synthesized sequence

<400>  39

Leu Cys Gly Ala Ala Ile
1               5



<210>  40
<211>  6
<212>  PRT
<213>  Artificial Sequence

<220>
<223>  an artificially synthesized sequence

<400>  40

Phe Ala Gln Ala Leu Gly
1               5



<210>  41
<211>  6
<212>  PRT
<213>  Artificial Sequence

<220>
<223>  an artificially synthesized sequence

<400>  41

Leu Leu Gln Ala Asn Pro
1               5



<210>  42
<211>  6
<212>  PRT
<213>  Artificial Sequence

<220>
<223>  an artificially synthesized sequence

<400>  42

Leu Ala Ala Ala Asn Pro
1               5



<210>  43
<211>  6
<212>  PRT
<213>  Artificial Sequence

<220>
<223>  an artificially synthesized sequence

<400>  43

Leu Tyr Gly Ala Gln Phe
1               5



<210>  44

<211>  6
<212>  PRT
<213>  Artificial Sequence

<220>
<223>  an artificially synthesized sequence

<400>  44

Leu Ser Gln Ala Gln Gly
1               5


<210>  45
<211>  6
<212>  PRT
<213>  Artificial Sequence

<220>
<223>  an artificially synthesized sequence

<400>  45

Ala Ser Ala Ala Ser Gly
1               5


<210>  46
<211>  6
<212>  PRT
<213>  Artificial Sequence

<220>
<223>  an artificially synthesized sequence

<400>  46

Phe Leu Gly Ala Ser Leu
1               5


<210>  47
<211>  6
<212>  PRT
<213>  Artificial Sequence

<220>
<223>  an artificially synthesized sequence

<400>  47

Ala Tyr Gly Ala Thr Gly
1               5


<210>  48
<211>  6
<212>  PRT
<213>  Artificial Sequence

<220>
<223>  an artificially synthesized sequence

<400>  48

Leu Ala Gln Ala Thr Gly
1               5


<210>  49
<211>  17
<212>  PRT
<213>  Artificial Sequence

<220>
<223>  an artificially synthesized sequence

<400>  49

Gly Ala Gly Ser Gly Val Val Ile Ala Thr Val Ile Val Ile Thr Ala
1               5                   10                  15


Gly




<210>  50
<211>  8
<212>  PRT
<213>  Artificial Sequence

<220>
<223>  an artificially synthesized sequence

<400>  50

Ala Pro Met Ala Glu Gly Gly Gly
1               5


<210>  51
<211>  8
<212>  PRT
<213>  Artificial Sequence

<220>
<223>  an artificially synthesized sequence

<400>  51

Glu Ala Gln Gly Asp Lys Ile Ile
1               5


<210>  52
<211>  8
<212>  PRT
<213>  Artificial Sequence

<220>
<223>  an artificially synthesized sequence

<400>  52

Leu Ala Phe Ser Asp Ala Gly Pro

1               5


<210>  53
<211>  7
<212>  PRT
<213>  Artificial Sequence

<220>
<223>  an artificially synthesized sequence

<400>  53

Tyr Val Ala Asp Ala Pro Lys
1               5


<210>  54
<211>  5
<212>  PRT
<213>  Artificial Sequence

<220>
<223>  an artificially synthesized sequence

<400>  54

Arg Arg Arg Arg Arg
1               5


<210>  55
<211>  6
<212>  PRT
<213>  Artificial Sequence

<220>
<223>  an artificially synthesized sequence

<400>  55

Arg Arg Arg Arg Arg Arg
1               5


<210>  56
<211>  10
<212>  PRT
<213>  Artificial Sequence

<220>
<223>  an artificially synthesized sequence

<400>  56

Gly Gln Ser Ser Arg His Arg Arg Ala Leu
1               5                   10


<210>  57
<211>  9
<212>  PRT
<213>  Artificial Sequence

<220>
<223>  an artificially synthesized sequence

<400>  57

Ser Ser Arg His Arg Arg Ala Leu Asp
1               5


<210>  58
<211>  14
<212>  PRT
<213>  Artificial Sequence

<220>
<223>  an artificially synthesized sequence

<400>  58

Arg Lys Ser Ser Ile Ile Ile Arg Met Arg Asp Val Val Leu
1               5                   10


<210>  59
<211>  15
<212>  PRT
<213>  Artificial Sequence

<220>
<223>  an artificially synthesized sequence

<400>  59

Ser Ser Ser Phe Asp Lys Gly Lys Tyr Lys Lys Gly Asp Asp Ala
1               5                   10                  15


<210>  60
<211>  15
<212>  PRT
<213>  Artificial Sequence

<220>
<223>  an artificially synthesized sequence

<400>  60

Ser Ser Ser Phe Asp Lys Gly Lys Tyr Lys Arg Gly Asp Asp Ala
1               5                   10                  15


<210>  61
<211>  4
<212>  PRT
<213>  Artificial Sequence

<220>
<223>  an artificially synthesized sequence

<400>  61

Ile Glu Gly Arg

1


<210>  62
<211>  4
<212>  PRT
<213>  Artificial Sequence

<220>
<223>  an artificially synthesized sequence

<400>  62

Ile Asp Gly Arg
1


<210>  63
<211>  7
<212>  PRT
<213>  Artificial Sequence

<220>
<223>  an artificially synthesized sequence

<400>  63

Gly Gly Ser Ile Asp Gly Arg
1               5


<210>  64
<211>  8
<212>  PRT
<213>  Artificial Sequence

<220>
<223>  an artificially synthesized sequence

<400>  64

Gly Pro Gln Gly Ile Ala Gly Gln
1               5


<210>  65
<211>  8
<212>  PRT
<213>  Artificial Sequence

<220>
<223>  an artificially synthesized sequence

<400>  65

Gly Pro Gln Gly Leu Leu Gly Ala
1               5


<210>  66
<211>  5
<212>  PRT
<213>  Artificial Sequence

<220>
<223>  an artificially synthesized sequence

<400>  66

Gly Ile Ala Gly Gln
1               5


<210>  67
<211>  7
<212>  PRT
<213>  Artificial Sequence

<220>
<223>  an artificially synthesized sequence

<400>  67

Gly Pro Leu Gly Ile Ala Gly
1               5


<210>  68
<211>  8
<212>  PRT
<213>  Artificial Sequence

<220>
<223>  an artificially synthesized sequence

<400>  68

Gly Pro Glu Gly Leu Arg Val Gly
1               5


<210>  69
<211>  8
<212>  PRT
<213>  Artificial Sequence

<220>
<223>  an artificially synthesized sequence

<400>  69

Tyr Gly Ala Gly Leu Gly Val Val
1               5


<210>  70
<211>  8
<212>  PRT
<213>  Artificial Sequence

<220>
<223>  an artificially synthesized sequence

<400>  70

Ala Gly Leu Gly Val Val Glu Arg

1               5


<210>  71
<211>  8
<212>  PRT
<213>  Artificial Sequence

<220>
<223>  an artificially synthesized sequence

<400>  71

Ala Gly Leu Gly Ile Ser Ser Thr
1               5


<210>  72
<211>  8
<212>  PRT
<213>  Artificial Sequence

<220>
<223>  an artificially synthesized sequence

<400>  72

Glu Pro Gln Ala Leu Ala Met Ser
1               5


<210>  73
<211>  8
<212>  PRT
<213>  Artificial Sequence

<220>
<223>  an artificially synthesized sequence

<400>  73

Gln Ala Leu Ala Met Ser Ala Ile
1               5


<210>  74
<211>  8
<212>  PRT
<213>  Artificial Sequence

<220>
<223>  an artificially synthesized sequence

<400>  74

Ala Ala Tyr His Leu Val Ser Gln
1               5


<210>  75
<211>  8
<212>  PRT
<213>  Artificial Sequence

<220>
<223>  an artificially synthesized sequence

<400>  75

Met Asp Ala Phe Leu Glu Ser Ser
1               5


<210>  76
<211>  8
<212>  PRT
<213>  Artificial Sequence

<220>
<223>  an artificially synthesized sequence

<400>  76

Glu Ser Leu Pro Val Val Ala Val
1               5


<210>  77
<211>  8
<212>  PRT
<213>  Artificial Sequence

<220>
<223>  an artificially synthesized sequence

<400>  77

Ser Ala Pro Ala Val Glu Ser Glu
1               5


<210>  78
<211>  8
<212>  PRT
<213>  Artificial Sequence

<220>
<223>  an artificially synthesized sequence

<400>  78

Asp Val Ala Gln Phe Val Leu Thr
1               5


<210>  79
<211>  8
<212>  PRT
<213>  Artificial Sequence

<220>
<223>  an artificially synthesized sequence

<400>  79

Val Ala Gln Phe Val Leu Thr Glu

1               5


<210>  80
<211>  8
<212>  PRT
<213>  Artificial Sequence

<220>
<223>  an artificially synthesized sequence

<400>  80

Ala Gln Phe Val Leu Thr Glu Gly
1               5


<210>  81
<211>  8
<212>  PRT
<213>  Artificial Sequence

<220>
<223>  an artificially synthesized sequence

<400>  81

Pro Val Gln Pro Ile Gly Pro Gln
1               5


<210>  82
<211>  6
<212>  PRT
<213>  Artificial Sequence

<220>
<223>  an artificially synthesized sequence

<400>  82

Leu Val Pro Arg Gly Ser
1               5


<210>  83
<211>  4
<212>  PRT
<213>  Artificial Sequence

<220>
<223>  an artificially synthesized sequence

<400>  83

Gly Gly Gly Ser
1


<210>  84
<211>  4
<212>  PRT
<213>  Artificial Sequence

<220>
<223>  an artificially synthesized sequence

<400>  84

Gly Gly Ser Gly
1


<210>  85
<211>  4
<212>  PRT
<213>  Artificial Sequence

<220>
<223>  an artificially synthesized sequence

<400>  85

Gly Ser Gly Gly
1


<210>  86
<211>  4
<212>  PRT
<213>  Artificial Sequence

<220>
<223>  an artificially synthesized sequence

<400>  86

Ser Gly Gly Gly
1


<210>  87
<211>  4
<212>  PRT
<213>  Artificial Sequence

<220>
<223>  an artificially synthesized sequence

<400>  87

Gly Ser Ser Gly
1


<210>  88
<211>  5
<212>  PRT
<213>  Artificial Sequence

<220>
<223>  an artificially synthesized sequence

<400>  88

Gly Gly Gly Gly Ser

1               5


<210>  89
<211>  5
<212>  PRT
<213>  Artificial Sequence

<220>
<223>  an artificially synthesized sequence

<400>  89

Gly Gly Gly Ser Gly
1               5


<210>  90
<211>  5
<212>  PRT
<213>  Artificial Sequence

<220>
<223>  an artificially synthesized sequence

<400>  90

Gly Gly Ser Gly Gly
1               5


<210>  91
<211>  5
<212>  PRT
<213>  Artificial Sequence

<220>
<223>  an artificially synthesized sequence

<400>  91

Gly Ser Gly Gly Gly
1               5


<210>  92
<211>  5
<212>  PRT
<213>  Artificial Sequence

<220>
<223>  an artificially synthesized sequence

<400>  92

Gly Ser Gly Gly Ser
1               5


<210>  93
<211>  5
<212>  PRT
<213>  Artificial Sequence

<220>
<223>  an artificially synthesized sequence

<400>  93

Ser Gly Gly Gly Gly
1               5


<210>  94
<211>  5
<212>  PRT
<213>  Artificial Sequence

<220>
<223>  an artificially synthesized sequence

<400>  94

Gly Ser Ser Gly Gly
1               5


<210>  95
<211>  5
<212>  PRT
<213>  Artificial Sequence

<220>
<223>  an artificially synthesized sequence

<400>  95

Gly Ser Gly Ser Gly
1               5


<210>  96
<211>  5
<212>  PRT
<213>  Artificial Sequence

<220>
<223>  an artificially synthesized sequence

<400>  96

Ser Gly Gly Ser Gly
1               5


<210>  97
<211>  5
<212>  PRT
<213>  Artificial Sequence

<220>
<223>  an artificially synthesized sequence

<400>  97

Gly Ser Ser Ser Gly

1               5


<210>  98
<211>  6
<212>  PRT
<213>  Artificial Sequence

<220>
<223>  an artificially synthesized sequence

<400>  98

Gly Gly Gly Gly Gly Ser
1               5


<210>  99
<211>  6
<212>  PRT
<213>  Artificial Sequence

<220>
<223>  an artificially synthesized sequence

<400>  99

Ser Gly Gly Gly Gly Gly
1               5


<210>  100
<211>  7
<212>  PRT
<213>  Artificial Sequence

<220>
<223>  an artificially synthesized sequence

<400>  100

Gly Gly Gly Gly Gly Gly Ser
1               5


<210>  101
<211>  7
<212>  PRT
<213>  Artificial Sequence

<220>
<223>  an artificially synthesized sequence

<400>  101

Ser Gly Gly Gly Gly Gly Gly
1               5


<210>  102
<211>  6
<212>  PRT
<213>  Artificial Sequence

<220>
<223>  an artificially synthesized sequence

<400>  102

Met Tyr Pro Pro Pro Tyr
1               5


<210>  103
<211>  330
<212>  PRT
<213>  Homo sapiens

<400>  103

<210>  104
<211>  326
<212>  PRT
<213>  Homo sapiens

<400>  104

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys

                325

<210>  105
<211>  377
<212>  PRT
<213>  Homo sapiens

<400>  105

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu

210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210>  106
<211>  327
<212>  PRT
<213>  Homo sapiens

<400>  106

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser

50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  107
<211>  16
<212>  PRT
<213>  Artificial Sequence

<220>
<223>  an artificially synthesized sequence

<400>  107

Gly Gly Gly Gly Ser Pro Leu Gly Leu Ala Gly Gly Gly Gly Gly Ser
1               5                   10                  15

<210>  108
<211>  18
<212>  PRT
<213>  Artificial Sequence

<220>
<223>  an artificially synthesized sequence

<400>  108

Gly Gly Gly Gly Ser Gly Pro Leu Gly Ile Ala Gly Gln Gly Gly Gly
1               5                   10                  15

Gly Ser

<210>  109
<211>  16
<212>  PRT
<213>  Artificial Sequence

<220>
<223>  an artificially synthesized sequence

<400>  109

Gly Gly Gly Gly Ser Pro Leu Gly Leu Trp Ala Gly Gly Gly Gly Ser
1               5                   10                  15

<210>  110
<211>  23
<212>  PRT
<213>  Artificial Sequence

<220>
<223>  an artificially synthesized sequence

<400>  110

Gly Gly Gly Gly Ser Gly Ala Gly Val Pro Leu Ser Leu Tyr Ser Gly
1               5                   10                  15

Ala Gly Gly Gly Gly Gly Ser
            20

<210>  111
<211>  463
<212>  PRT
<213>  Artificial Sequence

<220>
<223>  an artificially synthesized sequence

<400>  111

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Gly Gly Gly Gly Ser Pro Leu Gly Leu
        115                 120                 125

Ala Gly Gly Gly Gly Gly Ser Ala Ser Thr Lys Gly Pro Ser Val Phe
    130                 135                 140

Pro Leu Ala Pro Ser Ser Lys Ser Thr Ser Gly Gly Thr Ala Ala Leu
145                 150                 155                 160

Gly Cys Leu Val Lys Asp Tyr Phe Pro Glu Pro Val Thr Val Ser Trp
                165                 170                 175

Asn Ser Gly Ala Leu Thr Ser Gly Val His Thr Phe Pro Ala Val Leu
        180                 185                 190

Gln Ser Ser Gly Leu Tyr Ser Leu Ser Ser Val Val Thr Val Pro Ser
    195                 200                 205

Ser Ser Leu Gly Thr Gln Thr Tyr Ile Cys Asn Val Asn His Lys Pro
    210                 215                 220

Ser Asn Thr Lys Val Asp Lys Arg Val Glu Pro Lys Ser Cys Asp Lys
225                 230                 235                 240

Thr His Thr Cys Pro Pro Cys Pro Ala Pro Glu Leu Arg Arg Gly Pro
                245                 250                 255

Ser Val Phe Leu Phe Pro Pro Lys Pro Lys Asp Thr Leu Met Ile Ser
        260                 265                 270

Arg Thr Pro Glu Val Thr Cys Val Val Val Asp Val Ser His Glu Asp
    275                 280                 285

Pro Glu Val Lys Phe Asn Trp Tyr Val Asp Gly Val Glu Val His Asn
    290                 295                 300

Ala Lys Thr Lys Pro Arg Glu Glu Gln Tyr Asn Ser Thr Tyr Arg Val
305                 310                 315                 320

Val Ser Val Leu Thr Val Leu His Gln Asp Trp Leu Asn Gly Lys Glu
                325                 330                 335

Tyr Lys Cys Lys Val Ser Asn Lys Gly Leu Pro Ser Ser Ile Glu Lys
        340                 345                 350

Thr Ile Ser Lys Ala Lys Gly Gln Pro Arg Glu Pro Gln Val Tyr Thr
    355                 360                 365

Leu Pro Pro Ser Arg Glu Glu Met Thr Lys Asn Gln Val Ser Leu Thr
    370                 375                 380

Cys Leu Val Lys Gly Phe Tyr Pro Ser Asp Ile Ala Val Glu Trp Glu
385                 390                 395                 400

Ser Asn Gly Gln Pro Glu Asn Asn Tyr Lys Thr Thr Pro Pro Val Leu
                405                 410                 415

Asp Ser Asp Gly Ser Phe Phe Leu Tyr Ser Lys Leu Thr Val Asp Lys
        420                 425                 430

Ser Arg Trp Gln Glu Gly Asn Val Phe Ser Cys Ser Val Met His Glu
        435                 440                 445

Ala Leu His Asn His Tyr Thr Gln Lys Ser Leu Ser Leu Ser Pro
    450                 455                 460

<210> 112
<211> 465
<212> PRT
<213> Artificial Sequence

<220>
<223> an artificially synthesized sequence

<400> 112

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Gly Gly Gly Gly Ser Gly Pro Leu Gly
        115                 120                 125

Ile Ala Gly Gln Gly Gly Gly Gly Ser Ala Ser Thr Lys Gly Pro Ser
    130                 135                 140

Val Phe Pro Leu Ala Pro Ser Ser Lys Ser Thr Ser Gly Gly Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
        180                 185                 190

Val Leu Gln Ser Ser Gly Leu Tyr Ser Leu Ser Ser Val Val Thr Val
    195                 200                 205

Pro Ser Ser Ser Leu Gly Thr Gln Thr Tyr Ile Cys Asn Val Asn His
    210                 215                 220

Lys Pro Ser Asn Thr Lys Val Asp Lys Arg Val Glu Pro Lys Ser Cys
225                 230                 235                 240

Asp Lys Thr His Thr Cys Pro Pro Cys Pro Ala Pro Glu Leu Arg Arg
                245                 250                 255

Gly Pro Ser Val Phe Leu Phe Pro Pro Lys Pro Lys Asp Thr Leu Met
            260                 265                 270

Ile Ser Arg Thr Pro Glu Val Thr Cys Val Val Val Asp Val Ser His
        275                 280                 285

Glu Asp Pro Glu Val Lys Phe Asn Trp Tyr Val Asp Gly Val Glu Val
    290                 295                 300

His Asn Ala Lys Thr Lys Pro Arg Glu Glu Gln Tyr Asn Ser Thr Tyr
305                 310                 315                 320

Arg Val Val Ser Val Leu Thr Val Leu His Gln Asp Trp Leu Asn Gly
                325                 330                 335

Lys Glu Tyr Lys Cys Lys Val Ser Asn Lys Gly Leu Pro Ser Ser Ile
            340                 345                 350

Glu Lys Thr Ile Ser Lys Ala Lys Gly Gln Pro Arg Glu Pro Gln Val
        355                 360                 365

Tyr Thr Leu Pro Pro Ser Arg Glu Glu Met Thr Lys Asn Gln Val Ser
    370                 375                 380

Leu Thr Cys Leu Val Lys Gly Phe Tyr Pro Ser Asp Ile Ala Val Glu
385                 390                 395                 400

Trp Glu Ser Asn Gly Gln Pro Glu Asn Asn Tyr Lys Thr Thr Pro Pro
                405                 410                 415

Val Leu Asp Ser Asp Gly Ser Phe Phe Leu Tyr Ser Lys Leu Thr Val
            420                 425                 430

Asp Lys Ser Arg Trp Gln Glu Gly Asn Val Phe Ser Cys Ser Val Met
        435                 440                 445

His Glu Ala Leu His Asn His Tyr Thr Gln Lys Ser Leu Ser Leu Ser
    450                 455                 460

Pro
465

<210>  113
<211>  463
<212>  PRT
<213>  Artificial Sequence

<220>
<223>  an artificially synthesized sequence

<400>  113

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Gly Gly Gly Gly Ser Pro Leu Gly Leu
        115                 120                 125

Trp Ala Gly Gly Gly Gly Ser Ala Ser Thr Lys Gly Pro Ser Val Phe
    130                 135                 140

Pro Leu Ala Pro Ser Ser Lys Ser Thr Ser Gly Gly Thr Ala Ala Leu
145                 150                 155                 160

Gly Cys Leu Val Lys Asp Tyr Phe Pro Glu Pro Val Thr Val Ser Trp
                165                 170                 175

Asn Ser Gly Ala Leu Thr Ser Gly Val His Thr Phe Pro Ala Val Leu
            180                 185                 190

Gln Ser Ser Gly Leu Tyr Ser Leu Ser Ser Val Val Thr Val Pro Ser
        195                 200                 205

Ser Ser Leu Gly Thr Gln Thr Tyr Ile Cys Asn Val Asn His Lys Pro
    210                 215                 220

Ser Asn Thr Lys Val Asp Lys Arg Val Glu Pro Lys Ser Cys Asp Lys
225                 230                 235                 240

Thr His Thr Cys Pro Pro Cys Pro Ala Pro Glu Leu Arg Arg Gly Pro
                245                 250                 255

Ser Val Phe Leu Phe Pro Pro Lys Pro Lys Asp Thr Leu Met Ile Ser
            260                 265                 270

Arg Thr Pro Glu Val Thr Cys Val Val Val Asp Val Ser His Glu Asp
        275                 280                 285

Pro Glu Val Lys Phe Asn Trp Tyr Val Asp Gly Val Glu Val His Asn
    290                 295                 300

Ala Lys Thr Lys Pro Arg Glu Glu Gln Tyr Asn Ser Thr Tyr Arg Val
305                 310                 315                 320

Val Ser Val Leu Thr Val Leu His Gln Asp Trp Leu Asn Gly Lys Glu
                325                 330                 335

Tyr Lys Cys Lys Val Ser Asn Lys Gly Leu Pro Ser Ser Ile Glu Lys
            340                 345                 350

Thr Ile Ser Lys Ala Lys Gly Gln Pro Arg Glu Pro Gln Val Tyr Thr
        355                 360                 365

Leu Pro Pro Ser Arg Glu Glu Met Thr Lys Asn Gln Val Ser Leu Thr
    370                 375                 380

Cys Leu Val Lys Gly Phe Tyr Pro Ser Asp Ile Ala Val Glu Trp Glu
385                 390                 395                 400

Ser Asn Gly Gln Pro Glu Asn Asn Tyr Lys Thr Thr Pro Pro Val Leu
                405                 410                 415

Asp Ser Asp Gly Ser Phe Phe Leu Tyr Ser Lys Leu Thr Val Asp Lys
                420                 425                 430

Ser Arg Trp Gln Glu Gly Asn Val Phe Ser Cys Ser Val Met His Glu
            435                 440                 445

Ala Leu His Asn His Tyr Thr Gln Lys Ser Leu Ser Leu Ser Pro
    450                 455                 460

<210>  114
<211>  470
<212>  PRT
<213>  Artificial Sequence

<220>
<223>  an artificially synthesized sequence

<400>  114

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Gly Gly Gly Gly Ser Gly Ala Gly Val
        115                 120                 125

Pro Leu Ser Leu Tyr Ser Gly Ala Gly Gly Gly Gly Gly Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Arg Arg Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Gly
            340                 345                 350

Leu Pro Ser Ser Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
                420                 425                 430

Ser Lys Leu Thr Val Asp Lys Ser Arg Trp Gln Glu Gly Asn Val Phe
            435                 440                 445

Ser Cys Ser Val Met His Glu Ala Leu His Asn His Tyr Thr Gln Lys
    450                 455                 460

Ser Leu Ser Leu Ser Pro
465                 470

<210>  115
<211>  460
<212>  PRT
<213>  Artificial Sequence

<220>
<223>  an artificially synthesized sequence

<400>  115

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Gly Ala Gly Ile Pro Val Ser Leu Arg
        115                 120                 125

Ser Gly Ala Gly Ala Ser Thr Lys Gly Pro Ser Val Phe Pro Leu Ala
    130                 135                 140

Pro Ser Ser Lys Ser Thr Ser Gly Gly Thr Ala Ala Leu Gly Cys Leu
145                 150                 155                 160

Val Lys Asp Tyr Phe Pro Glu Pro Val Thr Val Ser Trp Asn Ser Gly
                165                 170                 175

Ala Leu Thr Ser Gly Val His Thr Phe Pro Ala Val Leu Gln Ser Ser
            180                 185                 190

Gly Leu Tyr Ser Leu Ser Ser Val Val Thr Val Pro Ser Ser Ser Leu
        195                 200                 205

Gly Thr Gln Thr Tyr Ile Cys Asn Val Asn His Lys Pro Ser Asn Thr
    210                 215                 220

Lys Val Asp Lys Lys Val Glu Pro Lys Ser Cys Asp Lys Thr His Thr
225                 230                 235                 240

Cys Pro Pro Cys Pro Ala Pro Glu Leu Leu Gly Gly Pro Ser Val Phe
                245                 250                 255

Leu Phe Pro Pro Lys Pro Lys Asp Thr Leu Met Ile Ser Arg Thr Pro
            260                 265                 270

Glu Val Thr Cys Val Val Val Asp Val Ser His Glu Asp Pro Glu Val
        275                 280                 285

Lys Phe Asn Trp Tyr Val Asp Gly Val Glu Val His Asn Ala Lys Thr
    290                 295                 300

Lys Pro Arg Glu Glu Gln Tyr Asn Ser Thr Tyr Arg Val Val Ser Val
305                 310                 315                 320

Leu Thr Val Leu His Gln Asp Trp Leu Asn Gly Lys Glu Tyr Lys Cys
                325                 330                 335

Lys Val Ser Asn Lys Ala Leu Pro Ala Pro Ile Glu Lys Thr Ile Ser
            340                 345                 350

Lys Ala Lys Gly Gln Pro Arg Glu Pro Gln Val Tyr Thr Leu Pro Pro
        355                 360                 365

Ser Arg Glu Glu Met Thr Lys Asn Gln Val Ser Leu Thr Cys Leu Val
    370                 375                 380

Lys Gly Phe Tyr Pro Ser Asp Ile Ala Val Glu Trp Glu Ser Asn Gly
385                 390                 395                 400

Gln Pro Glu Asn Asn Tyr Lys Thr Thr Pro Pro Val Leu Asp Ser Asp
                405                 410                 415

Gly Ser Phe Phe Leu Tyr Ser Lys Leu Thr Val Asp Lys Ser Arg Trp
            420                 425                 430

Gln Gln Gly Asn Val Phe Ser Cys Ser Val Met His Glu Ala Leu His
        435                 440                 445

Asn His Tyr Thr Gln Lys Ser Leu Ser Leu Ser Pro
    450                 455                 460

<210>  116
<211>  457
<212>  PRT
<213>  Artificial Sequence

<220>
<223>  an artificially synthesized sequence

<400>  116

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Gly Gly Pro Leu Gly Met Leu Ser Gln
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Lys Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Lys Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Ser Arg Glu
        355                 360                 365

Glu Met Thr Lys Asn Gln Val Ser Leu Thr Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro
    450                 455

<210> 117
<211> 460
<212> PRT
<213> Artificial Sequence

<220>
<223> an artificially synthesized sequence

<400> 117

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Gly Ala Gly Val Pro Leu Ser Leu Thr
        115                 120                 125

Met Gly Ala Gly Ala Ser Thr Lys Gly Pro Ser Val Phe Pro Leu Ala
    130                 135                 140

Pro Ser Ser Lys Ser Thr Ser Gly Gly Thr Ala Ala Leu Gly Cys Leu
145                 150                 155                 160

Val Lys Asp Tyr Phe Pro Glu Pro Val Thr Val Ser Trp Asn Ser Gly
                165                 170                 175

Ala Leu Thr Ser Gly Val His Thr Phe Pro Ala Val Leu Gln Ser Ser
            180                 185                 190

Gly Leu Tyr Ser Leu Ser Ser Val Val Thr Val Pro Ser Ser Ser Leu
        195                 200                 205

Gly Thr Gln Thr Tyr Ile Cys Asn Val Asn His Lys Pro Ser Asn Thr
    210                 215                 220

Lys Val Asp Lys Lys Val Glu Pro Lys Ser Cys Asp Lys Thr His Thr
225                 230                 235                 240

Cys Pro Pro Cys Pro Ala Pro Glu Leu Leu Gly Gly Pro Ser Val Phe
                245                 250                 255

Leu Phe Pro Pro Lys Pro Lys Asp Thr Leu Met Ile Ser Arg Thr Pro
            260                 265                 270

Glu Val Thr Cys Val Val Val Asp Val Ser His Glu Asp Pro Glu Val
        275                 280                 285

Lys Phe Asn Trp Tyr Val Asp Gly Val Glu Val His Asn Ala Lys Thr
    290                 295                 300

Lys Pro Arg Glu Glu Gln Tyr Asn Ser Thr Tyr Arg Val Val Ser Val
305                 310                 315                 320

Leu Thr Val Leu His Gln Asp Trp Leu Asn Gly Lys Glu Tyr Lys Cys
                325                 330                 335

Lys Val Ser Asn Lys Ala Leu Pro Ala Pro Ile Glu Lys Thr Ile Ser
            340                 345                 350

Lys Ala Lys Gly Gln Pro Arg Glu Pro Gln Val Tyr Thr Leu Pro Pro
        355                 360                 365

Ser Arg Glu Glu Met Thr Lys Asn Gln Val Ser Leu Thr Cys Leu Val
    370                 375                 380

Lys Gly Phe Tyr Pro Ser Asp Ile Ala Val Glu Trp Glu Ser Asn Gly
385                 390                 395                 400

Gln Pro Glu Asn Asn Tyr Lys Thr Thr Pro Pro Val Leu Asp Ser Asp
                405                 410                 415

Gly Ser Phe Phe Leu Tyr Ser Lys Leu Thr Val Asp Lys Ser Arg Trp
            420                 425                 430

Gln Gln Gly Asn Val Phe Ser Cys Ser Val Met His Glu Ala Leu His
        435                 440                 445

Asn His Tyr Thr Gln Lys Ser Leu Ser Leu Ser Pro
    450                 455                 460

<210> 118
<211> 214
<212> PRT
<213> Artificial Sequence

<220>
<223> an artificially synthesized sequence

<400> 118

<210>  119
<211>  447
<212>  PRT
<213>  Artificial Sequence

<220>
<223>  an artificially synthesized sequence

<400>  119

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu

130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Arg Arg Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Gly Leu Pro Ser Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210>  120
<211>  127
<212>  PRT
<213>  Artificial Sequence

<220>
<223>  an artificially synthesized sequence

<400>  120

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asp Asp Tyr
            20                  25                  30

Gly Met Ser Trp Val Arg Gln Ala Pro Gly Lys Ala Leu Glu Trp Val
        35                  40                  45

Ser Ala Ile Ser Trp Asn Gly Asn Asn Thr Tyr Tyr Thr Glu Ser Met
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Pro Glu Asp Thr Ala Val Tyr Tyr Cys
                85                  90                  95

Val Lys Gly Ser Thr Ala Ile Val Gly Val Pro Pro Thr Tyr Pro Asp
            100                 105                 110

Glu Tyr Asp Tyr Trp Gly Gln Gly Thr Leu Val Thr Val Ser Ser
        115                 120                 125

<210>  121
<211>  230
<212>  PRT
<213>  Artificial Sequence

<220>
<223>  an artificially synthesized sequence

<400>  121

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Glu Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro
225                 230

<210>  122
<211>  357
<212>  PRT
<213>  Artificial Sequence

<220>
<223>  an artificially synthesized sequence

<400>  122

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asp Asp Tyr
            20                  25                  30

Gly Met Ser Trp Val Arg Gln Ala Pro Gly Lys Ala Leu Glu Trp Val
        35                  40                  45

Ser Ala Ile Ser Trp Asn Gly Asn Asn Thr Tyr Tyr Thr Glu Ser Met
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Pro Glu Asp Thr Ala Val Tyr Tyr Cys
                85                  90                  95

Val Lys Gly Ser Thr Ala Ile Val Gly Val Pro Pro Thr Tyr Pro Asp
            100                 105                 110

Glu Tyr Asp Tyr Trp Gly Gln Gly Thr Leu Val Thr Val Ser Ser Glu
        115                 120                 125

Pro Lys Ser Ser Asp Lys Thr His Thr Cys Pro Pro Cys Pro Ala Pro
    130                 135                 140

Glu Leu Leu Gly Gly Pro Ser Val Phe Leu Phe Pro Pro Lys Pro Lys
145                 150                 155                 160

Asp Thr Leu Met Ile Ser Arg Thr Pro Glu Val Thr Cys Val Val Val
                165                 170                 175

Asp Val Ser His Glu Asp Pro Glu Val Lys Phe Asn Trp Tyr Val Asp
            180                 185                 190

Gly Val Glu Val His Asn Ala Lys Thr Lys Pro Arg Glu Glu Gln Tyr
        195                 200                 205

Asn Ser Thr Tyr Arg Val Val Ser Val Leu Thr Val Leu His Gln Asp
    210                 215                 220

Trp Leu Asn Gly Lys Glu Tyr Lys Cys Lys Val Ser Asn Lys Ala Leu
225                 230                 235                 240

Pro Ala Pro Ile Glu Lys Thr Ile Ser Lys Ala Lys Gly Gln Pro Arg
                245                 250                 255

Glu Pro Gln Val Tyr Thr Leu Pro Pro Ser Arg Glu Glu Met Thr Lys
            260                 265                 270

Asn Gln Val Ser Leu Thr Cys Leu Val Lys Gly Phe Tyr Pro Ser Asp
        275                 280                 285

Ile Ala Val Glu Trp Glu Ser Asn Gly Gln Pro Glu Asn Asn Tyr Lys
    290                 295                 300

Thr Thr Pro Pro Val Leu Asp Ser Asp Gly Ser Phe Phe Leu Tyr Ser
305                 310                 315                 320

Lys Leu Thr Val Asp Lys Ser Arg Trp Gln Glu Gly Asn Val Phe Ser
                325                 330                 335

Cys Ser Val Met His Glu Ala Leu His Asn His Tyr Thr Gln Lys Ser
            340                 345                 350

Leu Ser Leu Ser Pro
        355

<210>  123
<211>  365
<212>  PRT
<213>  Artificial Sequence

<220>
<223>  an artificially synthesized sequence

<400>  123

Glu Val Gln Leu Val Glu Ser Gly Gly Gly Leu Val Leu Ser Gly Arg
1               5                   10                  15

Ser Asp Asn His Gln Pro Gly Gly Ser Leu Arg Leu Ser Cys Ala Ala
            20                  25                  30

Ser Gly Phe Thr Phe Asp Asp Tyr Gly Met Ser Trp Val Arg Gln Ala
        35                  40                  45

Pro Gly Lys Ala Leu Glu Trp Val Ser Ala Ile Ser Trp Asn Gly Asn
    50                  55                  60

Asn Thr Tyr Tyr Thr Glu Ser Met Lys Gly Arg Phe Thr Ile Ser Arg
65                  70                  75                  80

Asp Asn Ala Lys Asn Thr Leu Tyr Leu Gln Met Asn Ser Leu Arg Pro
                85                  90                  95

Glu Asp Thr Ala Val Tyr Tyr Cys Val Lys Gly Ser Thr Ala Ile Val
            100                 105                 110

Gly Val Pro Pro Thr Tyr Pro Asp Glu Tyr Asp Tyr Trp Gly Gln Gly
        115                 120                 125

Thr Leu Val Thr Val Ser Ser Glu Pro Lys Ser Ser Asp Lys Thr His
    130                 135                 140

Thr Cys Pro Pro Cys Pro Ala Pro Glu Leu Leu Gly Gly Pro Ser Val
145                 150                 155                 160

Phe Leu Phe Pro Pro Lys Pro Lys Asp Thr Leu Met Ile Ser Arg Thr
                165                 170                 175

Pro Glu Val Thr Cys Val Val Val Asp Val Ser His Glu Asp Pro Glu
            180                 185                 190

Val Lys Phe Asn Trp Tyr Val Asp Gly Val Glu Val His Asn Ala Lys
        195                 200                 205

Thr Lys Pro Arg Glu Glu Gln Tyr Asn Ser Thr Tyr Arg Val Val Ser
    210                 215                 220

Val Leu Thr Val Leu His Gln Asp Trp Leu Asn Gly Lys Glu Tyr Lys
225                 230                 235                 240

Cys Lys Val Ser Asn Lys Ala Leu Pro Ala Pro Ile Glu Lys Thr Ile
                245                 250                 255

Ser Lys Ala Lys Gly Gln Pro Arg Glu Pro Gln Val Tyr Thr Leu Pro
            260                 265                 270

Pro Ser Arg Glu Glu Met Thr Lys Asn Gln Val Ser Leu Thr Cys Leu
        275                 280                 285

Val Lys Gly Phe Tyr Pro Ser Asp Ile Ala Val Glu Trp Glu Ser Asn
    290                 295                 300

Gly Gln Pro Glu Asn Asn Tyr Lys Thr Thr Pro Pro Val Leu Asp Ser
305                 310                 315                 320

Asp Gly Ser Phe Phe Leu Tyr Ser Lys Leu Thr Val Asp Lys Ser Arg
                325                 330                 335

Trp Gln Glu Gly Asn Val Phe Ser Cys Ser Val Met His Glu Ala Leu
            340                 345                 350

His Asn His Tyr Thr Gln Lys Ser Leu Ser Leu Ser Pro
        355                 360                 365

<210>  124
<211>  365
<212>  PRT
<213>  Artificial Sequence

<220>
<223>  an artificially synthesized sequence

<400>  124

Glu Val Gln Leu Val Glu Ser Gly Gly Gly Leu Val Gln Leu Ser Gly
1               5                   10                  15

Arg Ser Asp Asn His Pro Gly Gly Ser Leu Arg Leu Ser Cys Ala Ala
            20                  25                  30

Ser Gly Phe Thr Phe Asp Asp Tyr Gly Met Ser Trp Val Arg Gln Ala
        35                  40                  45

Pro Gly Lys Ala Leu Glu Trp Val Ser Ala Ile Ser Trp Asn Gly Asn
    50                  55                  60

Asn Thr Tyr Tyr Thr Glu Ser Met Lys Gly Arg Phe Thr Ile Ser Arg
65                  70                  75                  80

Asp Asn Ala Lys Asn Thr Leu Tyr Leu Gln Met Asn Ser Leu Arg Pro
                85                  90                  95

Glu Asp Thr Ala Val Tyr Tyr Cys Val Lys Gly Ser Thr Ala Ile Val
            100                 105                 110

Gly Val Pro Pro Thr Tyr Pro Asp Glu Tyr Asp Tyr Trp Gly Gln Gly
        115                 120                 125

Thr Leu Val Thr Val Ser Ser Glu Pro Lys Ser Ser Asp Lys Thr His
    130                 135                 140

Thr Cys Pro Pro Cys Pro Ala Pro Glu Leu Leu Gly Gly Pro Ser Val
145                 150                 155                 160

Phe Leu Phe Pro Pro Lys Pro Lys Asp Thr Leu Met Ile Ser Arg Thr
                165                 170                 175

Pro Glu Val Thr Cys Val Val Val Asp Val Ser His Glu Asp Pro Glu
            180                 185                 190

Val Lys Phe Asn Trp Tyr Val Asp Gly Val Glu Val His Asn Ala Lys
        195                 200                 205

Thr Lys Pro Arg Glu Glu Gln Tyr Asn Ser Thr Tyr Arg Val Val Ser
    210                 215                 220

Val Leu Thr Val Leu His Gln Asp Trp Leu Asn Gly Lys Glu Tyr Lys
225                 230                 235                 240

Cys Lys Val Ser Asn Lys Ala Leu Pro Ala Pro Ile Glu Lys Thr Ile
                245                 250                 255

Ser Lys Ala Lys Gly Gln Pro Arg Glu Pro Gln Val Tyr Thr Leu Pro
            260                 265                 270

Pro Ser Arg Glu Glu Met Thr Lys Asn Gln Val Ser Leu Thr Cys Leu
        275                 280                 285

Val Lys Gly Phe Tyr Pro Ser Asp Ile Ala Val Glu Trp Glu Ser Asn
    290                 295                 300

Gly Gln Pro Glu Asn Asn Tyr Lys Thr Thr Pro Pro Val Leu Asp Ser
305                 310                 315                 320

Asp Gly Ser Phe Phe Leu Tyr Ser Lys Leu Thr Val Asp Lys Ser Arg
                325                 330                 335

Trp Gln Glu Gly Asn Val Phe Ser Cys Ser Val Met His Glu Ala Leu
            340                 345                 350

His Asn His Tyr Thr Gln Lys Ser Leu Ser Leu Ser Pro
        355                 360                 365

<210>  125
<211>  365
<212>  PRT
<213>  Artificial Sequence

<220>
<223>  an artificially synthesized sequence

<400>  125

Glu Val Gln Leu Val Glu Ser Gly Gly Gly Leu Val Gln Pro Leu Ser
1               5                   10                  15

Gly Arg Ser Asp Asn His Gly Gly Ser Leu Arg Leu Ser Cys Ala Ala
            20                  25                  30

Ser Gly Phe Thr Phe Asp Asp Tyr Gly Met Ser Trp Val Arg Gln Ala
        35                  40                  45

Pro Gly Lys Ala Leu Glu Trp Val Ser Ala Ile Ser Trp Asn Gly Asn
    50                  55                  60

Asn Thr Tyr Tyr Thr Glu Ser Met Lys Gly Arg Phe Thr Ile Ser Arg
65                  70                  75                  80

Asp Asn Ala Lys Asn Thr Leu Tyr Leu Gln Met Asn Ser Leu Arg Pro
                85                  90                  95

Glu Asp Thr Ala Val Tyr Tyr Cys Val Lys Gly Ser Thr Ala Ile Val
            100                 105                 110

Gly Val Pro Pro Thr Tyr Pro Asp Glu Tyr Asp Tyr Trp Gly Gln Gly
        115                 120                 125

Thr Leu Val Thr Val Ser Ser Glu Pro Lys Ser Ser Asp Lys Thr His
    130                 135                 140

Thr Cys Pro Pro Cys Pro Ala Pro Glu Leu Leu Gly Gly Pro Ser Val
145                 150                 155                 160

Phe Leu Phe Pro Pro Lys Pro Lys Asp Thr Leu Met Ile Ser Arg Thr
                165                 170                 175

Pro Glu Val Thr Cys Val Val Val Asp Val Ser His Glu Asp Pro Glu
            180                 185                 190

Val Lys Phe Asn Trp Tyr Val Asp Gly Val Glu Val His Asn Ala Lys
        195                 200                 205

Thr Lys Pro Arg Glu Glu Gln Tyr Asn Ser Thr Tyr Arg Val Val Ser
    210                 215                 220

Val Leu Thr Val Leu His Gln Asp Trp Leu Asn Gly Lys Glu Tyr Lys
225                 230                 235                 240

Cys Lys Val Ser Asn Lys Ala Leu Pro Ala Pro Ile Glu Lys Thr Ile
                245                 250                 255

Ser Lys Ala Lys Gly Gln Pro Arg Glu Pro Gln Val Tyr Thr Leu Pro
            260                 265                 270

Pro Ser Arg Glu Glu Met Thr Lys Asn Gln Val Ser Leu Thr Cys Leu
        275                 280                 285

Val Lys Gly Phe Tyr Pro Ser Asp Ile Ala Val Glu Trp Glu Ser Asn
    290                 295                 300

Gly Gln Pro Glu Asn Asn Tyr Lys Thr Thr Pro Pro Val Leu Asp Ser
305                 310                 315                 320

Asp Gly Ser Phe Phe Leu Tyr Ser Lys Leu Thr Val Asp Lys Ser Arg
                325                 330                 335

Trp Gln Glu Gly Asn Val Phe Ser Cys Ser Val Met His Glu Ala Leu
            340                 345                 350

His Asn His Tyr Thr Gln Lys Ser Leu Ser Leu Ser Pro
        355                 360                 365

<210>  126
<211>  365
<212>  PRT
<213>  Artificial Sequence

<220>
<223>  an artificially synthesized sequence

<400>  126

Glu Val Gln Leu Val Glu Ser Gly Gly Gly Leu Val Gln Pro Gly Leu
1               5                   10                  15

Ser Gly Arg Ser Asp Asn His Gly Ser Leu Arg Leu Ser Cys Ala Ala
            20                  25                  30

Ser Gly Phe Thr Phe Asp Asp Tyr Gly Met Ser Trp Val Arg Gln Ala
        35                  40                  45

Pro Gly Lys Ala Leu Glu Trp Val Ser Ala Ile Ser Trp Asn Gly Asn
    50                  55                  60

Asn Thr Tyr Tyr Thr Glu Ser Met Lys Gly Arg Phe Thr Ile Ser Arg
65                  70                  75                  80

Asp Asn Ala Lys Asn Thr Leu Tyr Leu Gln Met Asn Ser Leu Arg Pro

                85                  90                  95

Glu Asp Thr Ala Val Tyr Tyr Cys Val Lys Gly Ser Thr Ala Ile Val
            100                 105                 110

Gly Val Pro Pro Thr Tyr Pro Asp Glu Tyr Asp Tyr Trp Gly Gln Gly
        115                 120                 125

Thr Leu Val Thr Val Ser Ser Glu Pro Lys Ser Ser Asp Lys Thr His
    130                 135                 140

Thr Cys Pro Pro Cys Pro Ala Pro Glu Leu Leu Gly Gly Pro Ser Val
145                 150                 155                 160

Phe Leu Phe Pro Pro Lys Pro Lys Asp Thr Leu Met Ile Ser Arg Thr
                165                 170                 175

Pro Glu Val Thr Cys Val Val Val Asp Val Ser His Glu Asp Pro Glu
            180                 185                 190

Val Lys Phe Asn Trp Tyr Val Asp Gly Val Glu Val His Asn Ala Lys
        195                 200                 205

Thr Lys Pro Arg Glu Glu Gln Tyr Asn Ser Thr Tyr Arg Val Val Ser
    210                 215                 220

Val Leu Thr Val Leu His Gln Asp Trp Leu Asn Gly Lys Glu Tyr Lys
225                 230                 235                 240

Cys Lys Val Ser Asn Lys Ala Leu Pro Ala Pro Ile Glu Lys Thr Ile
                245                 250                 255

Ser Lys Ala Lys Gly Gln Pro Arg Glu Pro Gln Val Tyr Thr Leu Pro
            260                 265                 270

Pro Ser Arg Glu Glu Met Thr Lys Asn Gln Val Ser Leu Thr Cys Leu
        275                 280                 285

Val Lys Gly Phe Tyr Pro Ser Asp Ile Ala Val Glu Trp Glu Ser Asn
    290                 295                 300

Gly Gln Pro Glu Asn Asn Tyr Lys Thr Thr Pro Pro Val Leu Asp Ser
305                 310                 315                 320

Asp Gly Ser Phe Phe Leu Tyr Ser Lys Leu Thr Val Asp Lys Ser Arg
                325                 330                 335

Trp Gln Glu Gly Asn Val Phe Ser Cys Ser Val Met His Glu Ala Leu
            340                 345                 350

His Asn His Tyr Thr Gln Lys Ser Leu Ser Leu Ser Pro
        355                 360                 365

<210>  127
<211>  366
<212>  PRT
<213>  Artificial Sequence

<220>
<223>  an artificially synthesized sequence

<400>  127

Glu Val Gln Leu Val Glu Ser Gly Gly Gly Leu Val Gln Pro Gly Gly
1               5                   10                  15

Leu Ser Gly Arg Ser Asp Asn His Gly Ser Leu Arg Leu Ser Cys Ala
            20                  25                  30

Ala Ser Gly Phe Thr Phe Asp Asp Tyr Gly Met Ser Trp Val Arg Gln
        35                  40                  45

Ala Pro Gly Lys Ala Leu Glu Trp Val Ser Ala Ile Ser Trp Asn Gly
    50                  55                  60

Asn Asn Thr Tyr Tyr Thr Glu Ser Met Lys Gly Arg Phe Thr Ile Ser
65                  70                  75                  80

Arg Asp Asn Ala Lys Asn Thr Leu Tyr Leu Gln Met Asn Ser Leu Arg
                85                  90                  95

Pro Glu Asp Thr Ala Val Tyr Tyr Cys Val Lys Gly Ser Thr Ala Ile
            100                 105                 110

Val Gly Val Pro Pro Thr Tyr Pro Asp Glu Tyr Asp Tyr Trp Gly Gln
        115                 120                 125

Gly Thr Leu Val Thr Val Ser Ser Glu Pro Lys Ser Ser Asp Lys Thr
    130                 135                 140

His Thr Cys Pro Pro Cys Pro Ala Pro Glu Leu Leu Gly Gly Pro Ser
145                 150                 155                 160

Val Phe Leu Phe Pro Pro Lys Pro Lys Asp Thr Leu Met Ile Ser Arg
                165                 170                 175

Thr Pro Glu Val Thr Cys Val Val Val Asp Val Ser His Glu Asp Pro

180                 185                 190

Glu Val Lys Phe Asn Trp Tyr Val Asp Gly Val Glu Val His Asn Ala
        195                 200                 205

Lys Thr Lys Pro Arg Glu Glu Gln Tyr Asn Ser Thr Tyr Arg Val Val
    210                 215                 220

Ser Val Leu Thr Val Leu His Gln Asp Trp Leu Asn Gly Lys Glu Tyr
225                 230                 235                 240

Lys Cys Lys Val Ser Asn Lys Ala Leu Pro Ala Pro Ile Glu Lys Thr
                245                 250                 255

Ile Ser Lys Ala Lys Gly Gln Pro Arg Glu Pro Gln Val Tyr Thr Leu
            260                 265                 270

Pro Pro Ser Arg Glu Glu Met Thr Lys Asn Gln Val Ser Leu Thr Cys
        275                 280                 285

Leu Val Lys Gly Phe Tyr Pro Ser Asp Ile Ala Val Glu Trp Glu Ser
    290                 295                 300

Asn Gly Gln Pro Glu Asn Asn Tyr Lys Thr Thr Pro Pro Val Leu Asp
305                 310                 315                 320

Ser Asp Gly Ser Phe Phe Leu Tyr Ser Lys Leu Thr Val Asp Lys Ser
                325                 330                 335

Arg Trp Gln Glu Gly Asn Val Phe Ser Cys Ser Val Met His Glu Ala
            340                 345                 350

Leu His Asn His Tyr Thr Gln Lys Ser Leu Ser Leu Ser Pro
        355                 360                 365

<210>  128
<211>  338
<212>  PRT
<213>  Homo sapiens

<400>  128

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala

35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln

<210>  129
<211>  708
<212>  PRT
<213>  Artificial Sequence

<220>
<223>  an artificially synthesized sequence

<400>  129

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala

145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Glu Val Gln Leu Val Glu Ser Gly Gly
            340                 345                 350

Gly Leu Val Leu Ser Gly Arg Ser Asp Asn His Gln Pro Gly Gly Ser
        355                 360                 365

Leu Arg Leu Ser Cys Ala Ala Ser Gly Phe Thr Phe Asp Asp Tyr Gly
    370                 375                 380

Met Ser Trp Val Arg Gln Ala Pro Gly Lys Ala Leu Glu Trp Val Ser
385                 390                 395                 400

Ala Ile Ser Trp Asn Gly Asn Asn Thr Tyr Tyr Thr Glu Ser Met Lys
                405                 410                 415

Gly Arg Phe Thr Ile Ser Arg Asp Asn Ala Lys Asn Thr Leu Tyr Leu
            420                 425                 430

Gln Met Asn Ser Leu Arg Pro Glu Asp Thr Ala Val Tyr Tyr Cys Val
        435                 440                 445

Lys Gly Ser Thr Ala Ile Val Gly Val Pro Pro Thr Tyr Pro Asp Glu
    450                 455                 460

Tyr Asp Tyr Trp Gly Gln Gly Thr Leu Val Thr Val Ser Ser Glu Pro
465                 470                 475                 480

Lys Ser Ser Asp Lys Thr His Thr Cys Pro Pro Cys Pro Ala Pro Glu
                485                 490                 495

Leu Leu Gly Gly Pro Ser Val Phe Leu Phe Pro Pro Lys Pro Lys Asp
            500                 505                 510

Thr Leu Met Ile Ser Arg Thr Pro Glu Val Thr Cys Val Val Val Asp
        515                 520                 525

Val Ser His Glu Asp Pro Glu Val Lys Phe Asn Trp Tyr Val Asp Gly
    530                 535                 540

Val Glu Val His Asn Ala Lys Thr Lys Pro Arg Glu Glu Gln Tyr Asn
545                 550                 555                 560

Ser Thr Tyr Arg Val Val Ser Val Leu Thr Val Leu His Gln Asp Trp
                565                 570                 575

Leu Asn Gly Lys Glu Tyr Lys Cys Lys Val Ser Asn Lys Ala Leu Pro
            580                 585                 590

Ala Pro Ile Glu Lys Thr Ile Ser Lys Ala Lys Gly Gln Pro Arg Glu
        595                 600                 605

Pro Gln Val Tyr Thr Leu Pro Pro Ser Arg Glu Glu Met Thr Lys Asn
    610                 615                 620

Gln Val Ser Leu Thr Cys Leu Val Lys Gly Phe Tyr Pro Ser Asp Ile
625                 630                 635                 640

Ala Val Glu Trp Glu Ser Asn Gly Gln Pro Glu Asn Asn Tyr Lys Thr
                645                 650                 655

Thr Pro Pro Val Leu Asp Ser Asp Gly Ser Phe Phe Leu Tyr Ser Lys
            660                 665                 670

Leu Thr Val Asp Lys Ser Arg Trp Gln Glu Gly Asn Val Phe Ser Cys
        675                 680                 685

Ser Val Met His Glu Ala Leu His Asn His Tyr Thr Gln Lys Ser Leu
    690                 695                 700

Ser Leu Ser Pro
705

<210>  130
<211>  713
<212>  PRT
<213>  Artificial Sequence

<220>
<223>  an artificially synthesized sequence

<400>  130

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Glu Val Gln Leu
            340                 345                 350

Val Glu Ser Gly Gly Gly Leu Val Leu Ser Gly Arg Ser Asp Asn His
        355                 360                 365

Gln Pro Gly Gly Ser Leu Arg Leu Ser Cys Ala Ala Ser Gly Phe Thr
    370                 375                 380

Phe Asp Asp Tyr Gly Met Ser Trp Val Arg Gln Ala Pro Gly Lys Ala
385                 390                 395                 400

Leu Glu Trp Val Ser Ala Ile Ser Trp Asn Gly Asn Asn Thr Tyr Tyr
                405                 410                 415

Thr Glu Ser Met Lys Gly Arg Phe Thr Ile Ser Arg Asp Asn Ala Lys
            420                 425                 430

Asn Thr Leu Tyr Leu Gln Met Asn Ser Leu Arg Pro Glu Asp Thr Ala
        435                 440                 445

Val Tyr Tyr Cys Val Lys Gly Ser Thr Ala Ile Val Gly Val Pro Pro
    450                 455                 460

Thr Tyr Pro Asp Glu Tyr Asp Tyr Trp Gly Gln Gly Thr Leu Val Thr
465                 470                 475                 480

Val Ser Ser Glu Pro Lys Ser Ser Asp Lys Thr His Thr Cys Pro Pro
                485                 490                 495

Cys Pro Ala Pro Glu Leu Leu Gly Gly Pro Ser Val Phe Leu Phe Pro
            500                 505                 510

Pro Lys Pro Lys Asp Thr Leu Met Ile Ser Arg Thr Pro Glu Val Thr
        515                 520                 525

Cys Val Val Val Asp Val Ser His Glu Asp Pro Glu Val Lys Phe Asn
    530                 535                 540

Trp Tyr Val Asp Gly Val Glu Val His Asn Ala Lys Thr Lys Pro Arg
545                 550                 555                 560

Glu Glu Gln Tyr Asn Ser Thr Tyr Arg Val Val Ser Val Leu Thr Val
                565                 570                 575

Leu His Gln Asp Trp Leu Asn Gly Lys Glu Tyr Lys Cys Lys Val Ser
            580                 585                 590

Asn Lys Ala Leu Pro Ala Pro Ile Glu Lys Thr Ile Ser Lys Ala Lys
        595                 600                 605

Gly Gln Pro Arg Glu Pro Gln Val Tyr Thr Leu Pro Pro Ser Arg Glu
    610                 615                 620

Glu Met Thr Lys Asn Gln Val Ser Leu Thr Cys Leu Val Lys Gly Phe
625                 630                 635                 640

Tyr Pro Ser Asp Ile Ala Val Glu Trp Glu Ser Asn Gly Gln Pro Glu
                645                 650                 655

Asn Asn Tyr Lys Thr Thr Pro Pro Val Leu Asp Ser Asp Gly Ser Phe
            660                 665                 670

Phe Leu Tyr Ser Lys Leu Thr Val Asp Lys Ser Arg Trp Gln Glu Gly
        675                 680                 685

Asn Val Phe Ser Cys Ser Val Met His Glu Ala Leu His Asn His Tyr
    690                 695                 700

Thr Gln Lys Ser Leu Ser Leu Ser Pro
705                 710

<210>  131
<211>  718
<212>  PRT
<213>  Artificial Sequence

<220>
<223>  an artificially synthesized sequence

<400>  131

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Glu Val Gln Leu Val Glu Ser Gly Gly Gly Leu Val Leu Ser Gly
        355                 360                 365

Arg Ser Asp Asn His Gln Pro Gly Gly Ser Leu Arg Leu Ser Cys Ala
    370                 375                 380

Ala Ser Gly Phe Thr Phe Asp Asp Tyr Gly Met Ser Trp Val Arg Gln
385                 390                 395                 400

Ala Pro Gly Lys Ala Leu Glu Trp Val Ser Ala Ile Ser Trp Asn Gly
                405                 410                 415

Asn Asn Thr Tyr Tyr Thr Glu Ser Met Lys Gly Arg Phe Thr Ile Ser
            420                 425                 430

Arg Asp Asn Ala Lys Asn Thr Leu Tyr Leu Gln Met Asn Ser Leu Arg
        435                 440                 445

Pro Glu Asp Thr Ala Val Tyr Tyr Cys Val Lys Gly Ser Thr Ala Ile
    450                 455                 460

Val Gly Val Pro Pro Thr Tyr Pro Asp Glu Tyr Asp Tyr Trp Gly Gln
465                 470                 475                 480

Gly Thr Leu Val Thr Val Ser Ser Glu Pro Lys Ser Ser Asp Lys Thr
                485                 490                 495

His Thr Cys Pro Pro Cys Pro Ala Pro Glu Leu Leu Gly Gly Pro Ser
            500                 505                 510

Val Phe Leu Phe Pro Pro Lys Pro Lys Asp Thr Leu Met Ile Ser Arg
        515                 520                 525

Thr Pro Glu Val Thr Cys Val Val Val Asp Val Ser His Glu Asp Pro
    530                 535                 540

Glu Val Lys Phe Asn Trp Tyr Val Asp Gly Val Glu Val His Asn Ala
545                 550                 555                 560

Lys Thr Lys Pro Arg Glu Glu Gln Tyr Asn Ser Thr Tyr Arg Val Val
                565                 570                 575

Ser Val Leu Thr Val Leu His Gln Asp Trp Leu Asn Gly Lys Glu Tyr
            580                 585                 590

Lys Cys Lys Val Ser Asn Lys Ala Leu Pro Ala Pro Ile Glu Lys Thr
        595                 600                 605

Ile Ser Lys Ala Lys Gly Gln Pro Arg Glu Pro Gln Val Tyr Thr Leu
    610                 615                 620

Pro Pro Ser Arg Glu Glu Met Thr Lys Asn Gln Val Ser Leu Thr Cys
625                 630                 635                 640

Leu Val Lys Gly Phe Tyr Pro Ser Asp Ile Ala Val Glu Trp Glu Ser

                645                 650                 655

Asn Gly Gln Pro Glu Asn Asn Tyr Lys Thr Thr Pro Pro Val Leu Asp
            660                 665                 670

Ser Asp Gly Ser Phe Phe Leu Tyr Ser Lys Leu Thr Val Asp Lys Ser
        675                 680                 685

Arg Trp Gln Glu Gly Asn Val Phe Ser Cys Ser Val Met His Glu Ala
    690                 695                 700

Leu His Asn His Tyr Thr Gln Lys Ser Leu Ser Leu Ser Pro
705                 710                 715

<210> 132
<211> 723
<212> PRT
<213> Artificial Sequence

<220>
<223> an artificially synthesized sequence

<400> 132

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Glu Val Gln Leu Val Glu Ser Gly Gly Gly
        355                 360                 365

Leu Val Leu Ser Gly Arg Ser Asp Asn His Gln Pro Gly Gly Ser Leu
    370                 375                 380

Arg Leu Ser Cys Ala Ala Ser Gly Phe Thr Phe Asp Asp Tyr Gly Met

385                 390                 395                 400

Ser Trp Val Arg Gln Ala Pro Gly Lys Ala Leu Glu Trp Val Ser Ala
                405                 410                 415

Ile Ser Trp Asn Gly Asn Asn Thr Tyr Tyr Thr Glu Ser Met Lys Gly
            420                 425                 430

Arg Phe Thr Ile Ser Arg Asp Asn Ala Lys Asn Thr Leu Tyr Leu Gln
        435                 440                 445

Met Asn Ser Leu Arg Pro Glu Asp Thr Ala Val Tyr Tyr Cys Val Lys
    450                 455                 460

Gly Ser Thr Ala Ile Val Gly Val Pro Pro Thr Tyr Pro Asp Glu Tyr
465                 470                 475                 480

Asp Tyr Trp Gly Gln Gly Thr Leu Val Thr Val Ser Ser Glu Pro Lys
                485                 490                 495

Ser Ser Asp Lys Thr His Thr Cys Pro Pro Cys Pro Ala Pro Glu Leu
            500                 505                 510

Leu Gly Gly Pro Ser Val Phe Leu Phe Pro Pro Lys Pro Lys Asp Thr
        515                 520                 525

Leu Met Ile Ser Arg Thr Pro Glu Val Thr Cys Val Val Val Asp Val
    530                 535                 540

Ser His Glu Asp Pro Glu Val Lys Phe Asn Trp Tyr Val Asp Gly Val
545                 550                 555                 560

Glu Val His Asn Ala Lys Thr Lys Pro Arg Glu Glu Gln Tyr Asn Ser
                565                 570                 575

Thr Tyr Arg Val Val Ser Val Leu Thr Val Leu His Gln Asp Trp Leu
            580                 585                 590

Asn Gly Lys Glu Tyr Lys Cys Lys Val Ser Asn Lys Ala Leu Pro Ala
        595                 600                 605

Pro Ile Glu Lys Thr Ile Ser Lys Ala Lys Gly Gln Pro Arg Glu Pro
    610                 615                 620

Gln Val Tyr Thr Leu Pro Pro Ser Arg Glu Glu Met Thr Lys Asn Gln
625                 630                 635                 640

Val Ser Leu Thr Cys Leu Val Lys Gly Phe Tyr Pro Ser Asp Ile Ala
                645                 650                 655

Val Glu Trp Glu Ser Asn Gly Gln Pro Glu Asn Asn Tyr Lys Thr Thr
            660                 665                 670

Pro Pro Val Leu Asp Ser Asp Gly Ser Phe Phe Leu Tyr Ser Lys Leu
        675                 680                 685

Thr Val Asp Lys Ser Arg Trp Gln Glu Gly Asn Val Phe Ser Cys Ser
    690                 695                 700

Val Met His Glu Ala Leu His Asn His Tyr Thr Gln Lys Ser Leu Ser
705                 710                 715                 720

Leu Ser Pro

<210>  133
<211>  728
<212>  PRT
<213>  Artificial Sequence

<220>
<223>  an artificially synthesized sequence

<400>  133

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val

115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Gly Gly Gly Gly Ser Glu Val Gln Leu Val
        355                 360                 365

Glu Ser Gly Gly Gly Leu Val Leu Ser Gly Arg Ser Asp Asn His Gln
    370                 375                 380

Pro Gly Gly Ser Leu Arg Leu Ser Cys Ala Ala Ser Gly Phe Thr Phe
385                 390                 395                 400

Asp Asp Tyr Gly Met Ser Trp Val Arg Gln Ala Pro Gly Lys Ala Leu
                405                 410                 415

Glu Trp Val Ser Ala Ile Ser Trp Asn Gly Asn Asn Thr Tyr Tyr Thr
            420                 425                 430

Glu Ser Met Lys Gly Arg Phe Thr Ile Ser Arg Asp Asn Ala Lys Asn
        435                 440                 445

Thr Leu Tyr Leu Gln Met Asn Ser Leu Arg Pro Glu Asp Thr Ala Val
    450                 455                 460

Tyr Tyr Cys Val Lys Gly Ser Thr Ala Ile Val Gly Val Pro Pro Thr
465                 470                 475                 480

Tyr Pro Asp Glu Tyr Asp Tyr Trp Gly Gln Gly Thr Leu Val Thr Val
                485                 490                 495

Ser Ser Glu Pro Lys Ser Ser Asp Lys Thr His Thr Cys Pro Pro Cys
            500                 505                 510

Pro Ala Pro Glu Leu Leu Gly Gly Pro Ser Val Phe Leu Phe Pro Pro
        515                 520                 525

Lys Pro Lys Asp Thr Leu Met Ile Ser Arg Thr Pro Glu Val Thr Cys
    530                 535                 540

Val Val Val Asp Val Ser His Glu Asp Pro Glu Val Lys Phe Asn Trp
545                 550                 555                 560

Tyr Val Asp Gly Val Glu Val His Asn Ala Lys Thr Lys Pro Arg Glu
                565                 570                 575

Glu Gln Tyr Asn Ser Thr Tyr Arg Val Val Ser Val Leu Thr Val Leu
            580                 585                 590

His Gln Asp Trp Leu Asn Gly Lys Glu Tyr Lys Cys Lys Val Ser Asn
        595                 600                 605

Lys Ala Leu Pro Ala Pro Ile Glu Lys Thr Ile Ser Lys Ala Lys Gly
    610                 615                 620

Gln Pro Arg Glu Pro Gln Val Tyr Thr Leu Pro Pro Ser Arg Glu Glu
625                 630                 635                 640

Met Thr Lys Asn Gln Val Ser Leu Thr Cys Leu Val Lys Gly Phe Tyr
                645                 650                 655

Pro Ser Asp Ile Ala Val Glu Trp Glu Ser Asn Gly Gln Pro Glu Asn
            660                 665                 670

Asn Tyr Lys Thr Thr Pro Pro Val Leu Asp Ser Asp Gly Ser Phe Phe
        675                 680                 685

Leu Tyr Ser Lys Leu Thr Val Asp Lys Ser Arg Trp Gln Glu Gly Asn
    690                 695                 700

Val Phe Ser Cys Ser Val Met His Glu Ala Leu His Asn His Tyr Thr
705                 710                 715                 720

Gln Lys Ser Leu Ser Leu Ser Pro
                725

<210>  134
<211>  708
<212>  PRT
<213>  Artificial Sequence

<220>
<223>  an artificially synthesized sequence

<400>  134

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Glu Val Gln Leu Val Glu Ser Gly Gly
            340                 345                 350

Gly Leu Val Gln Leu Ser Gly Arg Ser Asp Asn His Pro Gly Gly Ser
        355                 360                 365

Leu Arg Leu Ser Cys Ala Ala Ser Gly Phe Thr Phe Asp Asp Tyr Gly
    370                 375                 380

Met Ser Trp Val Arg Gln Ala Pro Gly Lys Ala Leu Glu Trp Val Ser
385                 390                 395                 400

Ala Ile Ser Trp Asn Gly Asn Asn Thr Tyr Tyr Thr Glu Ser Met Lys
                405                 410                 415

Gly Arg Phe Thr Ile Ser Arg Asp Asn Ala Lys Asn Thr Leu Tyr Leu
            420                 425                 430

Gln Met Asn Ser Leu Arg Pro Glu Asp Thr Ala Val Tyr Tyr Cys Val
        435                 440                 445

Lys Gly Ser Thr Ala Ile Val Gly Val Pro Pro Thr Tyr Pro Asp Glu
    450                 455                 460

Tyr Asp Tyr Trp Gly Gln Gly Thr Leu Val Thr Val Ser Ser Glu Pro
465                 470                 475                 480

Lys Ser Ser Asp Lys Thr His Thr Cys Pro Pro Cys Pro Ala Pro Glu
                485                 490                 495

Leu Leu Gly Gly Pro Ser Val Phe Leu Phe Pro Pro Lys Pro Lys Asp
            500                 505                 510

Thr Leu Met Ile Ser Arg Thr Pro Glu Val Thr Cys Val Val Val Asp
        515                 520                 525

Val Ser His Glu Asp Pro Glu Val Lys Phe Asn Trp Tyr Val Asp Gly
    530                 535                 540

Val Glu Val His Asn Ala Lys Thr Lys Pro Arg Glu Glu Gln Tyr Asn
545                 550                 555                 560

Ser Thr Tyr Arg Val Val Ser Val Leu Thr Val Leu His Gln Asp Trp
                565                 570                 575

Leu Asn Gly Lys Glu Tyr Lys Cys Lys Val Ser Asn Lys Ala Leu Pro
            580                 585                 590

Ala Pro Ile Glu Lys Thr Ile Ser Lys Ala Lys Gly Gln Pro Arg Glu
        595                 600                 605

Pro Gln Val Tyr Thr Leu Pro Pro Ser Arg Glu Glu Met Thr Lys Asn
    610                 615                 620

Gln Val Ser Leu Thr Cys Leu Val Lys Gly Phe Tyr Pro Ser Asp Ile
625                 630                 635                 640

Ala Val Glu Trp Glu Ser Asn Gly Gln Pro Glu Asn Asn Tyr Lys Thr
                645                 650                 655

Thr Pro Pro Val Leu Asp Ser Asp Gly Ser Phe Phe Leu Tyr Ser Lys
            660                 665                 670

Leu Thr Val Asp Lys Ser Arg Trp Gln Glu Gly Asn Val Phe Ser Cys
        675                 680                 685

Ser Val Met His Glu Ala Leu His Asn His Tyr Thr Gln Lys Ser Leu
    690                 695                 700

Ser Leu Ser Pro
705

<210>  135
<211>  713
<212>  PRT
<213>  Artificial Sequence

<220>
<223>  an artificially synthesized sequence

<400>  135

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
        100                 105                 110

Cys Glu Trp Gly Pro Arg Ser Thr Pro Ser Leu Thr Thr Lys Ala Val
    115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
        180                 185                 190

Gly Ile Leu Gln Pro Asp Pro Pro Ala Asn Ile Thr Val Thr Ala Val
    195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
        260                 265                 270

Leu Arg Ala Gln Glu Glu Phe Gly Gln Gly Glu Trp Ser Glu Trp Ser
    275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Glu Val Gln Leu
        340                 345                 350

Val Glu Ser Gly Gly Gly Leu Val Gln Leu Ser Gly Arg Ser Asp Asn
        355                 360                 365

His Pro Gly Gly Ser Leu Arg Leu Ser Cys Ala Ala Ser Gly Phe Thr
    370                 375                 380

Phe Asp Asp Tyr Gly Met Ser Trp Val Arg Gln Ala Pro Gly Lys Ala
385                 390                 395                 400

Leu Glu Trp Val Ser Ala Ile Ser Trp Asn Gly Asn Asn Thr Tyr Tyr
                405                 410                 415

Thr Glu Ser Met Lys Gly Arg Phe Thr Ile Ser Arg Asp Asn Ala Lys
            420                 425                 430

Asn Thr Leu Tyr Leu Gln Met Asn Ser Leu Arg Pro Glu Asp Thr Ala
        435                 440                 445

Val Tyr Tyr Cys Val Lys Gly Ser Thr Ala Ile Val Gly Val Pro Pro
    450                 455                 460

Thr Tyr Pro Asp Glu Tyr Asp Tyr Trp Gly Gln Gly Thr Leu Val Thr
465                 470                 475                 480

Val Ser Ser Glu Pro Lys Ser Ser Asp Lys Thr His Thr Cys Pro Pro
                485                 490                 495

Cys Pro Ala Pro Glu Leu Leu Gly Gly Pro Ser Val Phe Leu Phe Pro
            500                 505                 510

Pro Lys Pro Lys Asp Thr Leu Met Ile Ser Arg Thr Pro Glu Val Thr
        515                 520                 525

Cys Val Val Val Asp Val Ser His Glu Asp Pro Glu Val Lys Phe Asn
    530                 535                 540

Trp Tyr Val Asp Gly Val Glu Val His Asn Ala Lys Thr Lys Pro Arg
545                 550                 555                 560

Glu Glu Gln Tyr Asn Ser Thr Tyr Arg Val Val Ser Val Leu Thr Val
                565                 570                 575

Leu His Gln Asp Trp Leu Asn Gly Lys Glu Tyr Lys Cys Lys Val Ser
            580                 585                 590

Asn Lys Ala Leu Pro Ala Pro Ile Glu Lys Thr Ile Ser Lys Ala Lys

595                 600                 605

Gly Gln Pro Arg Glu Pro Gln Val Tyr Thr Leu Pro Pro Ser Arg Glu
    610                 615                 620

Glu Met Thr Lys Asn Gln Val Ser Leu Thr Cys Leu Val Lys Gly Phe
625                 630                 635                 640

Tyr Pro Ser Asp Ile Ala Val Glu Trp Glu Ser Asn Gly Gln Pro Glu
                645                 650                 655

Asn Asn Tyr Lys Thr Thr Pro Pro Val Leu Asp Ser Asp Gly Ser Phe
            660                 665                 670

Phe Leu Tyr Ser Lys Leu Thr Val Asp Lys Ser Arg Trp Gln Glu Gly
        675                 680                 685

Asn Val Phe Ser Cys Ser Val Met His Glu Ala Leu His Asn His Tyr
    690                 695                 700

Thr Gln Lys Ser Leu Ser Leu Ser Pro
705                 710

<210>  136
<211>  718
<212>  PRT
<213>  Artificial Sequence

<220>
<223>  an artificially synthesized sequence

<400>  136

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly

340                 345                 350

Ser Glu Val Gln Leu Val Glu Ser Gly Gly Gly Leu Val Gln Leu Ser
        355                 360                 365

Gly Arg Ser Asp Asn His Pro Gly Gly Ser Leu Arg Leu Ser Cys Ala
    370                 375                 380

Ala Ser Gly Phe Thr Phe Asp Asp Tyr Gly Met Ser Trp Val Arg Gln
385                 390                 395                 400

Ala Pro Gly Lys Ala Leu Glu Trp Val Ser Ala Ile Ser Trp Asn Gly
                405                 410                 415

Asn Asn Thr Tyr Tyr Thr Glu Ser Met Lys Gly Arg Phe Thr Ile Ser
            420                 425                 430

Arg Asp Asn Ala Lys Asn Thr Leu Tyr Leu Gln Met Asn Ser Leu Arg
        435                 440                 445

Pro Glu Asp Thr Ala Val Tyr Tyr Cys Val Lys Gly Ser Thr Ala Ile
    450                 455                 460

Val Gly Val Pro Pro Thr Tyr Pro Asp Glu Tyr Asp Tyr Trp Gly Gln
465                 470                 475                 480

Gly Thr Leu Val Thr Val Ser Ser Glu Pro Lys Ser Ser Asp Lys Thr
                485                 490                 495

His Thr Cys Pro Pro Cys Pro Ala Pro Glu Leu Leu Gly Gly Pro Ser
            500                 505                 510

Val Phe Leu Phe Pro Pro Lys Pro Lys Asp Thr Leu Met Ile Ser Arg
        515                 520                 525

Thr Pro Glu Val Thr Cys Val Val Val Asp Val Ser His Glu Asp Pro
    530                 535                 540

Glu Val Lys Phe Asn Trp Tyr Val Asp Gly Val Glu Val His Asn Ala
545                 550                 555                 560

Lys Thr Lys Pro Arg Glu Glu Gln Tyr Asn Ser Thr Tyr Arg Val Val
                565                 570                 575

Ser Val Leu Thr Val Leu His Gln Asp Trp Leu Asn Gly Lys Glu Tyr
            580                 585                 590

Lys Cys Lys Val Ser Asn Lys Ala Leu Pro Ala Pro Ile Glu Lys Thr
        595                 600                 605

Ile Ser Lys Ala Lys Gly Gln Pro Arg Glu Pro Gln Val Tyr Thr Leu
    610                 615                 620

Pro Pro Ser Arg Glu Glu Met Thr Lys Asn Gln Val Ser Leu Thr Cys
625                 630                 635                 640

Leu Val Lys Gly Phe Tyr Pro Ser Asp Ile Ala Val Glu Trp Glu Ser
                645                 650                 655

Asn Gly Gln Pro Glu Asn Asn Tyr Lys Thr Thr Pro Pro Val Leu Asp
            660                 665                 670

Ser Asp Gly Ser Phe Phe Leu Tyr Ser Lys Leu Thr Val Asp Lys Ser
        675                 680                 685

Arg Trp Gln Glu Gly Asn Val Phe Ser Cys Ser Val Met His Glu Ala
    690                 695                 700

Leu His Asn His Tyr Thr Gln Lys Ser Leu Ser Leu Ser Pro
705                 710                 715

<210> 137
<211> 723
<212> PRT
<213> Artificial Sequence

<220>
<223> an artificially synthesized sequence

<400> 137

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu

                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
        340                 345                 350

Ser Gly Gly Gly Gly Ser Glu Val Gln Leu Val Glu Ser Gly Gly Gly
    355                 360                 365

Leu Val Gln Leu Ser Gly Arg Ser Asp Asn His Pro Gly Gly Ser Leu
    370                 375                 380

Arg Leu Ser Cys Ala Ala Ser Gly Phe Thr Phe Asp Asp Tyr Gly Met
385                 390                 395                 400

Ser Trp Val Arg Gln Ala Pro Gly Lys Ala Leu Glu Trp Val Ser Ala
                405                 410                 415

Ile Ser Trp Asn Gly Asn Asn Thr Tyr Tyr Thr Glu Ser Met Lys Gly
            420                 425                 430

Arg Phe Thr Ile Ser Arg Asp Asn Ala Lys Asn Thr Leu Tyr Leu Gln
        435                 440                 445

Met Asn Ser Leu Arg Pro Glu Asp Thr Ala Val Tyr Tyr Cys Val Lys
    450                 455                 460

Gly Ser Thr Ala Ile Val Gly Val Pro Pro Thr Tyr Pro Asp Glu Tyr
465                 470                 475                 480

Asp Tyr Trp Gly Gln Gly Thr Leu Val Thr Val Ser Ser Glu Pro Lys
                485                 490                 495

Ser Ser Asp Lys Thr His Thr Cys Pro Pro Cys Pro Ala Pro Glu Leu
            500                 505                 510

Leu Gly Gly Pro Ser Val Phe Leu Phe Pro Pro Lys Pro Lys Asp Thr
        515                 520                 525

Leu Met Ile Ser Arg Thr Pro Glu Val Thr Cys Val Val Val Asp Val
    530                 535                 540

Ser His Glu Asp Pro Glu Val Lys Phe Asn Trp Tyr Val Asp Gly Val
545                 550                 555                 560

Glu Val His Asn Ala Lys Thr Lys Pro Arg Glu Glu Gln Tyr Asn Ser
                565                 570                 575

Thr Tyr Arg Val Val Ser Val Leu Thr Val Leu His Gln Asp Trp Leu
            580                 585                 590

Asn Gly Lys Glu Tyr Lys Cys Lys Val Ser Asn Lys Ala Leu Pro Ala
        595                 600                 605

Pro Ile Glu Lys Thr Ile Ser Lys Ala Lys Gly Gln Pro Arg Glu Pro
    610                 615                 620

Gln Val Tyr Thr Leu Pro Pro Ser Arg Glu Glu Met Thr Lys Asn Gln
625                 630                 635                 640

Val Ser Leu Thr Cys Leu Val Lys Gly Phe Tyr Pro Ser Asp Ile Ala
                645                 650                 655

Val Glu Trp Glu Ser Asn Gly Gln Pro Glu Asn Asn Tyr Lys Thr Thr
            660                 665                 670

Pro Pro Val Leu Asp Ser Asp Gly Ser Phe Phe Leu Tyr Ser Lys Leu
        675                 680                 685

Thr Val Asp Lys Ser Arg Trp Gln Glu Gly Asn Val Phe Ser Cys Ser
    690                 695                 700

Val Met His Glu Ala Leu His Asn His Tyr Thr Gln Lys Ser Leu Ser
705                 710                 715                 720

Leu Ser Pro

<210>  138
<211>  728
<212>  PRT
<213>  Artificial Sequence

<220>
<223>  an artificially synthesized sequence

<400>  138

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Gly Gly Gly Gly Ser Glu Val Gln Leu Val
        355                 360                 365

Glu Ser Gly Gly Gly Leu Val Gln Leu Ser Gly Arg Ser Asp Asn His
    370                 375                 380

Pro Gly Gly Ser Leu Arg Leu Ser Cys Ala Ala Ser Gly Phe Thr Phe
385                 390                 395                 400

Asp Asp Tyr Gly Met Ser Trp Val Arg Gln Ala Pro Gly Lys Ala Leu
                405                 410                 415

Glu Trp Val Ser Ala Ile Ser Trp Asn Gly Asn Asn Thr Tyr Tyr Thr
            420                 425                 430

Glu Ser Met Lys Gly Arg Phe Thr Ile Ser Arg Asp Asn Ala Lys Asn
        435                 440                 445

Thr Leu Tyr Leu Gln Met Asn Ser Leu Arg Pro Glu Asp Thr Ala Val
    450                 455                 460

Tyr Tyr Cys Val Lys Gly Ser Thr Ala Ile Val Gly Val Pro Pro Thr
465                 470                 475                 480

Tyr Pro Asp Glu Tyr Asp Tyr Trp Gly Gln Gly Thr Leu Val Thr Val
                485                 490                 495

Ser Ser Glu Pro Lys Ser Ser Asp Lys Thr His Thr Cys Pro Pro Cys
            500                 505                 510

Pro Ala Pro Glu Leu Leu Gly Gly Pro Ser Val Phe Leu Phe Pro Pro
        515                 520                 525

Lys Pro Lys Asp Thr Leu Met Ile Ser Arg Thr Pro Glu Val Thr Cys
    530                 535                 540

Val Val Val Asp Val Ser His Glu Asp Pro Glu Val Lys Phe Asn Trp
545                 550                 555                 560

Tyr Val Asp Gly Val Glu Val His Asn Ala Lys Thr Lys Pro Arg Glu
                565                 570                 575

Glu Gln Tyr Asn Ser Thr Tyr Arg Val Val Ser Val Leu Thr Val Leu
            580                 585                 590

His Gln Asp Trp Leu Asn Gly Lys Glu Tyr Lys Cys Lys Val Ser Asn
        595                 600                 605

Lys Ala Leu Pro Ala Pro Ile Glu Lys Thr Ile Ser Lys Ala Lys Gly
    610                 615                 620

Gln Pro Arg Glu Pro Gln Val Tyr Thr Leu Pro Pro Ser Arg Glu Glu
625                 630                 635                 640

Met Thr Lys Asn Gln Val Ser Leu Thr Cys Leu Val Lys Gly Phe Tyr
                645                 650                 655

Pro Ser Asp Ile Ala Val Glu Trp Glu Ser Asn Gly Gln Pro Glu Asn
            660                 665                 670

Asn Tyr Lys Thr Thr Pro Pro Val Leu Asp Ser Asp Gly Ser Phe Phe
        675                 680                 685

Leu Tyr Ser Lys Leu Thr Val Asp Lys Ser Arg Trp Gln Glu Gly Asn
    690                 695                 700

Val Phe Ser Cys Ser Val Met His Glu Ala Leu His Asn His Tyr Thr
705                 710                 715                 720

Gln Lys Ser Leu Ser Leu Ser Pro
                725

<210>  139
<211>  708
<212>  PRT
<213>  Artificial Sequence

<220>
<223>  an artificially synthesized sequence

<400>  139

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Glu Val Gln Leu Val Glu Ser Gly Gly
            340                 345                 350

Gly Leu Val Gln Pro Leu Ser Gly Arg Ser Asp Asn His Gly Gly Ser
        355                 360                 365

Leu Arg Leu Ser Cys Ala Ala Ser Gly Phe Thr Phe Asp Asp Tyr Gly
    370                 375                 380

Met Ser Trp Val Arg Gln Ala Pro Gly Lys Ala Leu Glu Trp Val Ser
385                 390                 395                 400

Ala Ile Ser Trp Asn Gly Asn Asn Thr Tyr Tyr Thr Glu Ser Met Lys
                405                 410                 415

Gly Arg Phe Thr Ile Ser Arg Asp Asn Ala Lys Asn Thr Leu Tyr Leu
            420                 425                 430

Gln Met Asn Ser Leu Arg Pro Glu Asp Thr Ala Val Tyr Tyr Cys Val
        435                 440                 445

Lys Gly Ser Thr Ala Ile Val Gly Val Pro Pro Thr Tyr Pro Asp Glu
    450                 455                 460

Tyr Asp Tyr Trp Gly Gln Gly Thr Leu Val Thr Val Ser Ser Glu Pro
465                 470                 475                 480

Lys Ser Ser Asp Lys Thr His Thr Cys Pro Pro Cys Pro Ala Pro Glu
                485                 490                 495

Leu Leu Gly Gly Pro Ser Val Phe Leu Phe Pro Pro Lys Pro Lys Asp
            500                 505                 510

Thr Leu Met Ile Ser Arg Thr Pro Glu Val Thr Cys Val Val Val Asp
        515                 520                 525

Val Ser His Glu Asp Pro Glu Val Lys Phe Asn Trp Tyr Val Asp Gly
    530                 535                 540

Val Glu Val His Asn Ala Lys Thr Lys Pro Arg Glu Glu Gln Tyr Asn

545                 550                 555                 560

Ser Thr Tyr Arg Val Val Ser Val Leu Thr Val Leu His Gln Asp Trp
                565                 570                 575

Leu Asn Gly Lys Glu Tyr Lys Cys Lys Val Ser Asn Lys Ala Leu Pro
            580                 585                 590

Ala Pro Ile Glu Lys Thr Ile Ser Lys Ala Lys Gly Gln Pro Arg Glu
        595                 600                 605

Pro Gln Val Tyr Thr Leu Pro Pro Ser Arg Glu Glu Met Thr Lys Asn
    610                 615                 620

Gln Val Ser Leu Thr Cys Leu Val Lys Gly Phe Tyr Pro Ser Asp Ile
625                 630                 635                 640

Ala Val Glu Trp Glu Ser Asn Gly Gln Pro Glu Asn Asn Tyr Lys Thr
                645                 650                 655

Thr Pro Pro Val Leu Asp Ser Asp Gly Ser Phe Phe Leu Tyr Ser Lys
            660                 665                 670

Leu Thr Val Asp Lys Ser Arg Trp Gln Glu Gly Asn Val Phe Ser Cys
        675                 680                 685

Ser Val Met His Glu Ala Leu His Asn His Tyr Thr Gln Lys Ser Leu
    690                 695                 700

Ser Leu Ser Pro
705

<210>  140
<211>  713
<212>  PRT
<213>  Artificial Sequence

<220>
<223>  an artificially synthesized sequence

<400>  140

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala

290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Glu Val Gln Leu
            340                 345                 350

Val Glu Ser Gly Gly Gly Leu Val Gln Pro Leu Ser Gly Arg Ser Asp
        355                 360                 365

Asn His Gly Gly Ser Leu Arg Leu Ser Cys Ala Ala Ser Gly Phe Thr
    370                 375                 380

Phe Asp Asp Tyr Gly Met Ser Trp Val Arg Gln Ala Pro Gly Lys Ala
385                 390                 395                 400

Leu Glu Trp Val Ser Ala Ile Ser Trp Asn Gly Asn Asn Thr Tyr Tyr
                405                 410                 415

Thr Glu Ser Met Lys Gly Arg Phe Thr Ile Ser Arg Asp Asn Ala Lys
            420                 425                 430

Asn Thr Leu Tyr Leu Gln Met Asn Ser Leu Arg Pro Glu Asp Thr Ala
        435                 440                 445

Val Tyr Tyr Cys Val Lys Gly Ser Thr Ala Ile Val Gly Val Pro Pro
    450                 455                 460

Thr Tyr Pro Asp Glu Tyr Asp Tyr Trp Gly Gln Gly Thr Leu Val Thr
465                 470                 475                 480

Val Ser Ser Glu Pro Lys Ser Ser Asp Lys Thr His Thr Cys Pro Pro
                485                 490                 495

Cys Pro Ala Pro Glu Leu Leu Gly Gly Pro Ser Val Phe Leu Phe Pro
            500                 505                 510

Pro Lys Pro Lys Asp Thr Leu Met Ile Ser Arg Thr Pro Glu Val Thr
        515                 520                 525

Cys Val Val Val Asp Val Ser His Glu Asp Pro Glu Val Lys Phe Asn
    530                 535                 540

Trp Tyr Val Asp Gly Val Glu Val His Asn Ala Lys Thr Lys Pro Arg
545                 550                 555                 560

Glu Glu Gln Tyr Asn Ser Thr Tyr Arg Val Val Ser Val Leu Thr Val
                565                 570                 575

Leu His Gln Asp Trp Leu Asn Gly Lys Glu Tyr Lys Cys Lys Val Ser
            580                 585                 590

Asn Lys Ala Leu Pro Ala Pro Ile Glu Lys Thr Ile Ser Lys Ala Lys
        595                 600                 605

Gly Gln Pro Arg Glu Pro Gln Val Tyr Thr Leu Pro Pro Ser Arg Glu
    610                 615                 620

Glu Met Thr Lys Asn Gln Val Ser Leu Thr Cys Leu Val Lys Gly Phe
625                 630                 635                 640

Tyr Pro Ser Asp Ile Ala Val Glu Trp Glu Ser Asn Gly Gln Pro Glu
                645                 650                 655

Asn Asn Tyr Lys Thr Thr Pro Pro Val Leu Asp Ser Asp Gly Ser Phe
            660                 665                 670

Phe Leu Tyr Ser Lys Leu Thr Val Asp Lys Ser Arg Trp Gln Glu Gly
        675                 680                 685

Asn Val Phe Ser Cys Ser Val Met His Glu Ala Leu His Asn His Tyr
    690                 695                 700

Thr Gln Lys Ser Leu Ser Leu Ser Pro
705                 710

<210>  141
<211>  718
<212>  PRT
<213>  Artificial Sequence

<220>
<223>  an artificially synthesized sequence

<400>  141

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala

35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Glu Val Gln Leu Val Glu Ser Gly Gly Gly Leu Val Gln Pro Leu
        355                 360                 365

Ser Gly Arg Ser Asp Asn His Gly Gly Ser Leu Arg Leu Ser Cys Ala
    370                 375                 380

Ala Ser Gly Phe Thr Phe Asp Asp Tyr Gly Met Ser Trp Val Arg Gln
385                 390                 395                 400

Ala Pro Gly Lys Ala Leu Glu Trp Val Ser Ala Ile Ser Trp Asn Gly
                405                 410                 415

Asn Asn Thr Tyr Tyr Thr Glu Ser Met Lys Gly Arg Phe Thr Ile Ser
            420                 425                 430

Arg Asp Asn Ala Lys Asn Thr Leu Tyr Leu Gln Met Asn Ser Leu Arg
        435                 440                 445

Pro Glu Asp Thr Ala Val Tyr Tyr Cys Val Lys Gly Ser Thr Ala Ile
    450                 455                 460

Val Gly Val Pro Pro Thr Tyr Pro Asp Glu Tyr Asp Tyr Trp Gly Gln
465                 470                 475                 480

Gly Thr Leu Val Thr Val Ser Ser Glu Pro Lys Ser Ser Asp Lys Thr
                485                 490                 495

His Thr Cys Pro Pro Cys Pro Ala Pro Glu Leu Leu Gly Gly Pro Ser
            500                 505                 510

Val Phe Leu Phe Pro Pro Lys Pro Lys Asp Thr Leu Met Ile Ser Arg
        515                 520                 525

Thr Pro Glu Val Thr Cys Val Val Val Asp Val Ser His Glu Asp Pro
    530                 535                 540

Glu Val Lys Phe Asn Trp Tyr Val Asp Gly Val Glu Val His Asn Ala
545                 550                 555                 560

Lys Thr Lys Pro Arg Glu Glu Gln Tyr Asn Ser Thr Tyr Arg Val Val
                565                 570                 575

Ser Val Leu Thr Val Leu His Gln Asp Trp Leu Asn Gly Lys Glu Tyr
            580                 585                 590

Lys Cys Lys Val Ser Asn Lys Ala Leu Pro Ala Pro Ile Glu Lys Thr
        595                 600                 605

Ile Ser Lys Ala Lys Gly Gln Pro Arg Glu Pro Gln Val Tyr Thr Leu
    610                 615                 620

Pro Pro Ser Arg Glu Glu Met Thr Lys Asn Gln Val Ser Leu Thr Cys
625                 630                 635                 640

Leu Val Lys Gly Phe Tyr Pro Ser Asp Ile Ala Val Glu Trp Glu Ser
                645                 650                 655

Asn Gly Gln Pro Glu Asn Asn Tyr Lys Thr Thr Pro Pro Val Leu Asp
            660                 665                 670

Ser Asp Gly Ser Phe Phe Leu Tyr Ser Lys Leu Thr Val Asp Lys Ser
        675                 680                 685

Arg Trp Gln Glu Gly Asn Val Phe Ser Cys Ser Val Met His Glu Ala
    690                 695                 700

Leu His Asn His Tyr Thr Gln Lys Ser Leu Ser Leu Ser Pro
705                 710                 715

<210>  142
<211>  723
<212>  PRT
<213>  Artificial Sequence

<220>
<223>  an artificially synthesized sequence

<400>  142

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Glu Val Gln Leu Val Glu Ser Gly Gly Gly
        355                 360                 365

Leu Val Gln Pro Leu Ser Gly Arg Ser Asp Asn His Gly Gly Ser Leu
    370                 375                 380

Arg Leu Ser Cys Ala Ala Ser Gly Phe Thr Phe Asp Asp Tyr Gly Met
385                 390                 395                 400

Ser Trp Val Arg Gln Ala Pro Gly Lys Ala Leu Glu Trp Val Ser Ala
                405                 410                 415

Ile Ser Trp Asn Gly Asn Asn Thr Tyr Tyr Thr Glu Ser Met Lys Gly
            420                 425                 430

Arg Phe Thr Ile Ser Arg Asp Asn Ala Lys Asn Thr Leu Tyr Leu Gln
        435                 440                 445

Met Asn Ser Leu Arg Pro Glu Asp Thr Ala Val Tyr Tyr Cys Val Lys
    450                 455                 460

Gly Ser Thr Ala Ile Val Gly Val Pro Pro Thr Tyr Pro Asp Glu Tyr
465                 470                 475                 480

Asp Tyr Trp Gly Gln Gly Thr Leu Val Thr Val Ser Ser Glu Pro Lys
                485                 490                 495

Ser Ser Asp Lys Thr His Thr Cys Pro Pro Cys Pro Ala Pro Glu Leu
            500                 505                 510

Leu Gly Gly Pro Ser Val Phe Leu Phe Pro Pro Lys Pro Lys Asp Thr
        515                 520                 525

Leu Met Ile Ser Arg Thr Pro Glu Val Thr Cys Val Val Val Asp Val
    530                 535                 540

Ser His Glu Asp Pro Glu Val Lys Phe Asn Trp Tyr Val Asp Gly Val
545                 550                 555                 560

Glu Val His Asn Ala Lys Thr Lys Pro Arg Glu Glu Gln Tyr Asn Ser
                565                 570                 575

Thr Tyr Arg Val Val Ser Val Leu Thr Val Leu His Gln Asp Trp Leu
            580                 585                 590

Asn Gly Lys Glu Tyr Lys Cys Lys Val Ser Asn Lys Ala Leu Pro Ala
        595                 600                 605

Pro Ile Glu Lys Thr Ile Ser Lys Ala Lys Gly Gln Pro Arg Glu Pro
    610                 615                 620

Gln Val Tyr Thr Leu Pro Pro Ser Arg Glu Glu Met Thr Lys Asn Gln
625                 630                 635                 640

Val Ser Leu Thr Cys Leu Val Lys Gly Phe Tyr Pro Ser Asp Ile Ala
                645                 650                 655

Val Glu Trp Glu Ser Asn Gly Gln Pro Glu Asn Asn Tyr Lys Thr Thr
            660                 665                 670

Pro Pro Val Leu Asp Ser Asp Gly Ser Phe Phe Leu Tyr Ser Lys Leu
        675                 680                 685

Thr Val Asp Lys Ser Arg Trp Gln Glu Gly Asn Val Phe Ser Cys Ser
    690                 695                 700

Val Met His Glu Ala Leu His Asn His Tyr Thr Gln Lys Ser Leu Ser
705                 710                 715                 720

Leu Ser Pro

<210>  143
<211>  728
<212>  PRT
<213>  Artificial Sequence

<220>
<223>  an artificially synthesized sequence

<400>  143

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Gly Gly Gly Gly Ser Glu Val Gln Leu Val
        355                 360                 365

Glu Ser Gly Gly Gly Leu Val Gln Pro Leu Ser Gly Arg Ser Asp Asn
    370                 375                 380

His Gly Gly Ser Leu Arg Leu Ser Cys Ala Ala Ser Gly Phe Thr Phe
385                 390                 395                 400

Asp Asp Tyr Gly Met Ser Trp Val Arg Gln Ala Pro Gly Lys Ala Leu
                405                 410                 415

Glu Trp Val Ser Ala Ile Ser Trp Asn Gly Asn Asn Thr Tyr Tyr Thr
            420                 425                 430

Glu Ser Met Lys Gly Arg Phe Thr Ile Ser Arg Asp Asn Ala Lys Asn
        435                 440                 445

Thr Leu Tyr Leu Gln Met Asn Ser Leu Arg Pro Glu Asp Thr Ala Val
    450                 455                 460

Tyr Tyr Cys Val Lys Gly Ser Thr Ala Ile Val Gly Val Pro Pro Thr
465                 470                 475                 480

Tyr Pro Asp Glu Tyr Asp Tyr Trp Gly Gln Gly Thr Leu Val Thr Val
                485                 490                 495

Ser Ser Glu Pro Lys Ser Ser Asp Lys Thr His Thr Cys Pro Pro Cys
            500                 505                 510

Pro Ala Pro Glu Leu Leu Gly Gly Pro Ser Val Phe Leu Phe Pro Pro

515                 520                 525

Lys Pro Lys Asp Thr Leu Met Ile Ser Arg Thr Pro Glu Val Thr Cys
    530                 535                 540

Val Val Val Asp Val Ser His Glu Asp Pro Glu Val Lys Phe Asn Trp
545                 550                 555                 560

Tyr Val Asp Gly Val Glu Val His Asn Ala Lys Thr Lys Pro Arg Glu
                565                 570                 575

Glu Gln Tyr Asn Ser Thr Tyr Arg Val Val Ser Val Leu Thr Val Leu
            580                 585                 590

His Gln Asp Trp Leu Asn Gly Lys Glu Tyr Lys Cys Lys Val Ser Asn
        595                 600                 605

Lys Ala Leu Pro Ala Pro Ile Glu Lys Thr Ile Ser Lys Ala Lys Gly
    610                 615                 620

Gln Pro Arg Glu Pro Gln Val Tyr Thr Leu Pro Pro Ser Arg Glu Glu
625                 630                 635                 640

Met Thr Lys Asn Gln Val Ser Leu Thr Cys Leu Val Lys Gly Phe Tyr
                645                 650                 655

Pro Ser Asp Ile Ala Val Glu Trp Glu Ser Asn Gly Gln Pro Glu Asn
            660                 665                 670

Asn Tyr Lys Thr Thr Pro Pro Val Leu Asp Ser Asp Gly Ser Phe Phe
        675                 680                 685

Leu Tyr Ser Lys Leu Thr Val Asp Lys Ser Arg Trp Gln Glu Gly Asn
    690                 695                 700

Val Phe Ser Cys Ser Val Met His Glu Ala Leu His Asn His Tyr Thr
705                 710                 715                 720

Gln Lys Ser Leu Ser Leu Ser Pro
                725

<210>  144
<211>  708
<212>  PRT
<213>  Artificial Sequence

<220>
<223>  an artificially synthesized sequence

<400>  144

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His

245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Glu Val Gln Leu Val Glu Ser Gly Gly
            340                 345                 350

Gly Leu Val Gln Pro Gly Leu Ser Gly Arg Ser Asp Asn His Gly Ser
        355                 360                 365

Leu Arg Leu Ser Cys Ala Ala Ser Gly Phe Thr Phe Asp Asp Tyr Gly
    370                 375                 380

Met Ser Trp Val Arg Gln Ala Pro Gly Lys Ala Leu Glu Trp Val Ser
385                 390                 395                 400

Ala Ile Ser Trp Asn Gly Asn Asn Thr Tyr Tyr Thr Glu Ser Met Lys
                405                 410                 415

Gly Arg Phe Thr Ile Ser Arg Asp Asn Ala Lys Asn Thr Leu Tyr Leu
            420                 425                 430

Gln Met Asn Ser Leu Arg Pro Glu Asp Thr Ala Val Tyr Tyr Cys Val
        435                 440                 445

Lys Gly Ser Thr Ala Ile Val Gly Val Pro Pro Thr Tyr Pro Asp Glu
    450                 455                 460

Tyr Asp Tyr Trp Gly Gln Gly Thr Leu Val Thr Val Ser Ser Glu Pro
465                 470                 475                 480

Lys Ser Ser Asp Lys Thr His Thr Cys Pro Pro Cys Pro Ala Pro Glu
                485                 490                 495

Leu Leu Gly Gly Pro Ser Val Phe Leu Phe Pro Pro Lys Pro Lys Asp
                500                 505                 510

Thr Leu Met Ile Ser Arg Thr Pro Glu Val Thr Cys Val Val Val Asp
        515                 520                 525

Val Ser His Glu Asp Pro Glu Val Lys Phe Asn Trp Tyr Val Asp Gly
    530                 535                 540

Val Glu Val His Asn Ala Lys Thr Lys Pro Arg Glu Glu Gln Tyr Asn
545                 550                 555                 560

Ser Thr Tyr Arg Val Val Ser Val Leu Thr Val Leu His Gln Asp Trp
                565                 570                 575

Leu Asn Gly Lys Glu Tyr Lys Cys Lys Val Ser Asn Lys Ala Leu Pro
            580                 585                 590

Ala Pro Ile Glu Lys Thr Ile Ser Lys Ala Lys Gly Gln Pro Arg Glu
        595                 600                 605

Pro Gln Val Tyr Thr Leu Pro Pro Ser Arg Glu Glu Met Thr Lys Asn
    610                 615                 620

Gln Val Ser Leu Thr Cys Leu Val Lys Gly Phe Tyr Pro Ser Asp Ile
625                 630                 635                 640

Ala Val Glu Trp Glu Ser Asn Gly Gln Pro Glu Asn Asn Tyr Lys Thr
                645                 650                 655

Thr Pro Pro Val Leu Asp Ser Asp Gly Ser Phe Phe Leu Tyr Ser Lys
            660                 665                 670

Leu Thr Val Asp Lys Ser Arg Trp Gln Glu Gly Asn Val Phe Ser Cys
        675                 680                 685

Ser Val Met His Glu Ala Leu His Asn His Tyr Thr Gln Lys Ser Leu
    690                 695                 700

Ser Leu Ser Pro
705

<210>  145
<211>  713
<212>  PRT
<213>  Artificial Sequence

<220>
<223>  an artificially synthesized sequence

<400>  145

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Glu Val Gln Leu
            340                 345                 350

Val Glu Ser Gly Gly Gly Leu Val Gln Pro Gly Leu Ser Gly Arg Ser
        355                 360                 365

Asp Asn His Gly Ser Leu Arg Leu Ser Cys Ala Ala Ser Gly Phe Thr
    370                 375                 380

Phe Asp Asp Tyr Gly Met Ser Trp Val Arg Gln Ala Pro Gly Lys Ala
385                 390                 395                 400

Leu Glu Trp Val Ser Ala Ile Ser Trp Asn Gly Asn Asn Thr Tyr Tyr
                405                 410                 415

Thr Glu Ser Met Lys Gly Arg Phe Thr Ile Ser Arg Asp Asn Ala Lys
            420                 425                 430

Asn Thr Leu Tyr Leu Gln Met Asn Ser Leu Arg Pro Glu Asp Thr Ala
        435                 440                 445

Val Tyr Tyr Cys Val Lys Gly Ser Thr Ala Ile Val Gly Val Pro Pro
    450                 455                 460

Thr Tyr Pro Asp Glu Tyr Asp Tyr Trp Gly Gln Gly Thr Leu Val Thr
465                 470                 475                 480

Val Ser Ser Glu Pro Lys Ser Ser Asp Lys Thr His Thr Cys Pro Pro
                485                 490                 495

Cys Pro Ala Pro Glu Leu Leu Gly Gly Pro Ser Val Phe Leu Phe Pro
            500                 505                 510

Pro Lys Pro Lys Asp Thr Leu Met Ile Ser Arg Thr Pro Glu Val Thr
        515                 520                 525

Cys Val Val Val Asp Val Ser His Glu Asp Pro Glu Val Lys Phe Asn
    530                 535                 540

Trp Tyr Val Asp Gly Val Glu Val His Asn Ala Lys Thr Lys Pro Arg
545                 550                 555                 560

Glu Glu Gln Tyr Asn Ser Thr Tyr Arg Val Val Ser Val Leu Thr Val
                565                 570                 575

Leu His Gln Asp Trp Leu Asn Gly Lys Glu Tyr Lys Cys Lys Val Ser
            580                 585                 590

Asn Lys Ala Leu Pro Ala Pro Ile Glu Lys Thr Ile Ser Lys Ala Lys
        595                 600                 605

Gly Gln Pro Arg Glu Pro Gln Val Tyr Thr Leu Pro Pro Ser Arg Glu
    610                 615                 620

Glu Met Thr Lys Asn Gln Val Ser Leu Thr Cys Leu Val Lys Gly Phe
625                 630                 635                 640

Tyr Pro Ser Asp Ile Ala Val Glu Trp Glu Ser Asn Gly Gln Pro Glu
                645                 650                 655

Asn Asn Tyr Lys Thr Thr Pro Pro Val Leu Asp Ser Asp Gly Ser Phe
            660                 665                 670

Phe Leu Tyr Ser Lys Leu Thr Val Asp Lys Ser Arg Trp Gln Glu Gly
        675                 680                 685

Asn Val Phe Ser Cys Ser Val Met His Glu Ala Leu His Asn His Tyr
    690                 695                 700

Thr Gln Lys Ser Leu Ser Leu Ser Pro
705                 710

<210>  146
<211>  718
<212>  PRT
<213>  Artificial Sequence

<220>

<223>  an artificially synthesized sequence

<400>  146

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Glu Val Gln Leu Val Glu Ser Gly Gly Gly Leu Val Gln Pro Gly
        355                 360                 365

Leu Ser Gly Arg Ser Asp Asn His Gly Ser Leu Arg Leu Ser Cys Ala
    370                 375                 380

Ala Ser Gly Phe Thr Phe Asp Asp Tyr Gly Met Ser Trp Val Arg Gln
385                 390                 395                 400

Ala Pro Gly Lys Ala Leu Glu Trp Val Ser Ala Ile Ser Trp Asn Gly
                405                 410                 415

Asn Asn Thr Tyr Tyr Thr Glu Ser Met Lys Gly Arg Phe Thr Ile Ser
            420                 425                 430

Arg Asp Asn Ala Lys Asn Thr Leu Tyr Leu Gln Met Asn Ser Leu Arg
        435                 440                 445

Pro Glu Asp Thr Ala Val Tyr Tyr Cys Val Lys Gly Ser Thr Ala Ile
    450                 455                 460

Val Gly Val Pro Pro Thr Tyr Pro Asp Glu Tyr Asp Tyr Trp Gly Gln
465                 470                 475                 480

Gly Thr Leu Val Thr Val Ser Ser Glu Pro Lys Ser Ser Asp Lys Thr
                485                 490                 495

His Thr Cys Pro Pro Cys Pro Ala Pro Glu Leu Leu Gly Gly Pro Ser
                500                 505                 510

Val Phe Leu Phe Pro Pro Lys Pro Lys Asp Thr Leu Met Ile Ser Arg
        515                 520                 525

Thr Pro Glu Val Thr Cys Val Val Val Asp Val Ser His Glu Asp Pro
    530                 535                 540

Glu Val Lys Phe Asn Trp Tyr Val Asp Gly Val Glu Val His Asn Ala
545                 550                 555                 560

Lys Thr Lys Pro Arg Glu Glu Gln Tyr Asn Ser Thr Tyr Arg Val Val
                565                 570                 575

Ser Val Leu Thr Val Leu His Gln Asp Trp Leu Asn Gly Lys Glu Tyr
                580                 585                 590

Lys Cys Lys Val Ser Asn Lys Ala Leu Pro Ala Pro Ile Glu Lys Thr
        595                 600                 605

Ile Ser Lys Ala Lys Gly Gln Pro Arg Glu Pro Gln Val Tyr Thr Leu
    610                 615                 620

Pro Pro Ser Arg Glu Glu Met Thr Lys Asn Gln Val Ser Leu Thr Cys
625                 630                 635                 640

Leu Val Lys Gly Phe Tyr Pro Ser Asp Ile Ala Val Glu Trp Glu Ser
                645                 650                 655

Asn Gly Gln Pro Glu Asn Asn Tyr Lys Thr Thr Pro Pro Val Leu Asp
                660                 665                 670

Ser Asp Gly Ser Phe Phe Leu Tyr Ser Lys Leu Thr Val Asp Lys Ser
        675                 680                 685

Arg Trp Gln Glu Gly Asn Val Phe Ser Cys Ser Val Met His Glu Ala
    690                 695                 700

Leu His Asn His Tyr Thr Gln Lys Ser Leu Ser Leu Ser Pro
705                 710                 715

<210>  147
<211>  723
<212>  PRT
<213>  Artificial Sequence

<220>
<223>  an artificially synthesized sequence

<400>  147

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Glu Val Gln Leu Val Glu Ser Gly Gly Gly
        355                 360                 365

Leu Val Gln Pro Gly Leu Ser Gly Arg Ser Asp Asn His Gly Ser Leu
    370                 375                 380

Arg Leu Ser Cys Ala Ala Ser Gly Phe Thr Phe Asp Asp Tyr Gly Met
385                 390                 395                 400

Ser Trp Val Arg Gln Ala Pro Gly Lys Ala Leu Glu Trp Val Ser Ala
                405                 410                 415

Ile Ser Trp Asn Gly Asn Asn Thr Tyr Tyr Thr Glu Ser Met Lys Gly
            420                 425                 430

Arg Phe Thr Ile Ser Arg Asp Asn Ala Lys Asn Thr Leu Tyr Leu Gln
        435                 440                 445

Met Asn Ser Leu Arg Pro Glu Asp Thr Ala Val Tyr Tyr Cys Val Lys
    450                 455                 460

Gly Ser Thr Ala Ile Val Gly Val Pro Pro Thr Tyr Pro Asp Glu Tyr
465                 470                 475                 480

Asp Tyr Trp Gly Gln Gly Thr Leu Val Thr Val Ser Ser Glu Pro Lys

                485                 490                 495

Ser Ser Asp Lys Thr His Thr Cys Pro Pro Cys Pro Ala Pro Glu Leu
            500                 505                 510

Leu Gly Gly Pro Ser Val Phe Leu Phe Pro Pro Lys Pro Lys Asp Thr
        515                 520                 525

Leu Met Ile Ser Arg Thr Pro Glu Val Thr Cys Val Val Val Asp Val
    530                 535                 540

Ser His Glu Asp Pro Glu Val Lys Phe Asn Trp Tyr Val Asp Gly Val
545                 550                 555                 560

Glu Val His Asn Ala Lys Thr Lys Pro Arg Glu Glu Gln Tyr Asn Ser
                565                 570                 575

Thr Tyr Arg Val Val Ser Val Leu Thr Val Leu His Gln Asp Trp Leu
            580                 585                 590

Asn Gly Lys Glu Tyr Lys Cys Lys Val Ser Asn Lys Ala Leu Pro Ala
        595                 600                 605

Pro Ile Glu Lys Thr Ile Ser Lys Ala Lys Gly Gln Pro Arg Glu Pro
    610                 615                 620

Gln Val Tyr Thr Leu Pro Pro Ser Arg Glu Glu Met Thr Lys Asn Gln
625                 630                 635                 640

Val Ser Leu Thr Cys Leu Val Lys Gly Phe Tyr Pro Ser Asp Ile Ala
                645                 650                 655

Val Glu Trp Glu Ser Asn Gly Gln Pro Glu Asn Asn Tyr Lys Thr Thr
            660                 665                 670

Pro Pro Val Leu Asp Ser Asp Gly Ser Phe Phe Leu Tyr Ser Lys Leu
        675                 680                 685

Thr Val Asp Lys Ser Arg Trp Gln Glu Gly Asn Val Phe Ser Cys Ser
    690                 695                 700

Val Met His Glu Ala Leu His Asn His Tyr Thr Gln Lys Ser Leu Ser
705                 710                 715                 720

Leu Ser Pro

<210>  148
<211>  728
<212>  PRT
<213>  Artificial Sequence

<220>
<223>  an artificially synthesized sequence

<400>  148

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser

210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Gly Gly Gly Gly Ser Glu Val Gln Leu Val
        355                 360                 365

Glu Ser Gly Gly Gly Leu Val Gln Pro Gly Leu Ser Gly Arg Ser Asp
    370                 375                 380

Asn His Gly Ser Leu Arg Leu Ser Cys Ala Ala Ser Gly Phe Thr Phe
385                 390                 395                 400

Asp Asp Tyr Gly Met Ser Trp Val Arg Gln Ala Pro Gly Lys Ala Leu
                405                 410                 415

Glu Trp Val Ser Ala Ile Ser Trp Asn Gly Asn Asn Thr Tyr Tyr Thr
            420                 425                 430

Glu Ser Met Lys Gly Arg Phe Thr Ile Ser Arg Asp Asn Ala Lys Asn
        435                 440                 445

Thr Leu Tyr Leu Gln Met Asn Ser Leu Arg Pro Glu Asp Thr Ala Val
    450                 455                 460

Tyr Tyr Cys Val Lys Gly Ser Thr Ala Ile Val Gly Val Pro Pro Thr
465                 470                 475                 480

Tyr Pro Asp Glu Tyr Asp Tyr Trp Gly Gln Gly Thr Leu Val Thr Val
                485                 490                 495

Ser Ser Glu Pro Lys Ser Ser Asp Lys Thr His Thr Cys Pro Pro Cys
            500                 505                 510

Pro Ala Pro Glu Leu Leu Gly Gly Pro Ser Val Phe Leu Phe Pro Pro
        515                 520                 525

Lys Pro Lys Asp Thr Leu Met Ile Ser Arg Thr Pro Glu Val Thr Cys
    530                 535                 540

Val Val Val Asp Val Ser His Glu Asp Pro Glu Val Lys Phe Asn Trp
545                 550                 555                 560

Tyr Val Asp Gly Val Glu Val His Asn Ala Lys Thr Lys Pro Arg Glu
                565                 570                 575

Glu Gln Tyr Asn Ser Thr Tyr Arg Val Val Ser Val Leu Thr Val Leu
            580                 585                 590

His Gln Asp Trp Leu Asn Gly Lys Glu Tyr Lys Cys Lys Val Ser Asn
        595                 600                 605

Lys Ala Leu Pro Ala Pro Ile Glu Lys Thr Ile Ser Lys Ala Lys Gly
    610                 615                 620

Gln Pro Arg Glu Pro Gln Val Tyr Thr Leu Pro Pro Ser Arg Glu Glu
625                 630                 635                 640

Met Thr Lys Asn Gln Val Ser Leu Thr Cys Leu Val Lys Gly Phe Tyr
                645                 650                 655

Pro Ser Asp Ile Ala Val Glu Trp Glu Ser Asn Gly Gln Pro Glu Asn
            660                 665                 670

Asn Tyr Lys Thr Thr Pro Pro Val Leu Asp Ser Asp Gly Ser Phe Phe
        675                 680                 685

Leu Tyr Ser Lys Leu Thr Val Asp Lys Ser Arg Trp Gln Glu Gly Asn
    690                 695                 700

Val Phe Ser Cys Ser Val Met His Glu Ala Leu His Asn His Tyr Thr
705                 710                 715                 720

Gln Lys Ser Leu Ser Leu Ser Pro
                725

<210>  149
<211>  709
<212>  PRT
<213>  Artificial Sequence

<220>
<223>  an artificially synthesized sequence

<400>  149

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Glu Val Gln Leu Val Glu Ser Gly Gly
            340                 345                 350

Gly Leu Val Gln Pro Gly Gly Leu Ser Gly Arg Ser Asp Asn His Gly
        355                 360                 365

Ser Leu Arg Leu Ser Cys Ala Ala Ser Gly Phe Thr Phe Asp Asp Tyr
    370                 375                 380

Gly Met Ser Trp Val Arg Gln Ala Pro Gly Lys Ala Leu Glu Trp Val
385                 390                 395                 400

Ser Ala Ile Ser Trp Asn Gly Asn Asn Thr Tyr Tyr Thr Glu Ser Met
                405                 410                 415

Lys Gly Arg Phe Thr Ile Ser Arg Asp Asn Ala Lys Asn Thr Leu Tyr
            420                 425                 430

Leu Gln Met Asn Ser Leu Arg Pro Glu Asp Thr Ala Val Tyr Tyr Cys
        435                 440                 445

Val Lys Gly Ser Thr Ala Ile Val Gly Val Pro Pro Thr Tyr Pro Asp
    450                 455                 460

Glu Tyr Asp Tyr Trp Gly Gln Gly Thr Leu Val Thr Val Ser Ser Glu
465                 470                 475                 480

Pro Lys Ser Ser Asp Lys Thr His Thr Cys Pro Pro Cys Pro Ala Pro
                485                 490                 495

Glu Leu Leu Gly Gly Pro Ser Val Phe Leu Phe Pro Pro Lys Pro Lys
            500                 505                 510

Asp Thr Leu Met Ile Ser Arg Thr Pro Glu Val Thr Cys Val Val Val
        515                 520                 525

Asp Val Ser His Glu Asp Pro Glu Val Lys Phe Asn Trp Tyr Val Asp
    530                 535                 540

Gly Val Glu Val His Asn Ala Lys Thr Lys Pro Arg Glu Glu Gln Tyr
545                 550                 555                 560

Asn Ser Thr Tyr Arg Val Val Ser Val Leu Thr Val Leu His Gln Asp
                565                 570                 575

Trp Leu Asn Gly Lys Glu Tyr Lys Cys Lys Val Ser Asn Lys Ala Leu
            580                 585                 590

Pro Ala Pro Ile Glu Lys Thr Ile Ser Lys Ala Lys Gly Gln Pro Arg
        595                 600                 605

Glu Pro Gln Val Tyr Thr Leu Pro Pro Ser Arg Glu Glu Met Thr Lys
    610                 615                 620

Asn Gln Val Ser Leu Thr Cys Leu Val Lys Gly Phe Tyr Pro Ser Asp
625                 630                 635                 640

Ile Ala Val Glu Trp Glu Ser Asn Gly Gln Pro Glu Asn Asn Tyr Lys
                645                 650                 655

Thr Thr Pro Pro Val Leu Asp Ser Asp Gly Ser Phe Phe Leu Tyr Ser
            660                 665                 670

Lys Leu Thr Val Asp Lys Ser Arg Trp Gln Glu Gly Asn Val Phe Ser
        675                 680                 685

Cys Ser Val Met His Glu Ala Leu His Asn His Tyr Thr Gln Lys Ser
    690                 695                 700

Leu Ser Leu Ser Pro
705

<210> 150
<211> 714
<212> PRT
<213> Artificial Sequence

<220>
<223> an artificially synthesized sequence

<400> 150

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Glu Val Gln Leu
            340                 345                 350

Val Glu Ser Gly Gly Gly Leu Val Gln Pro Gly Gly Leu Ser Gly Arg
        355                 360                 365

Ser Asp Asn His Gly Ser Leu Arg Leu Ser Cys Ala Ala Ser Gly Phe
    370                 375                 380

Thr Phe Asp Asp Tyr Gly Met Ser Trp Val Arg Gln Ala Pro Gly Lys
385                 390                 395                 400

Ala Leu Glu Trp Val Ser Ala Ile Ser Trp Asn Gly Asn Asn Thr Tyr
                405                 410                 415

Tyr Thr Glu Ser Met Lys Gly Arg Phe Thr Ile Ser Arg Asp Asn Ala
            420                 425                 430

Lys Asn Thr Leu Tyr Leu Gln Met Asn Ser Leu Arg Pro Glu Asp Thr
        435                 440                 445

Ala Val Tyr Tyr Cys Val Lys Gly Ser Thr Ala Ile Val Gly Val Pro
    450                 455                 460

Pro Thr Tyr Pro Asp Glu Tyr Asp Tyr Trp Gly Gln Gly Thr Leu Val
465                 470                 475                 480

Thr Val Ser Ser Glu Pro Lys Ser Ser Asp Lys Thr His Thr Cys Pro
                485                 490                 495

Pro Cys Pro Ala Pro Glu Leu Leu Gly Gly Pro Ser Val Phe Leu Phe
            500                 505                 510

Pro Pro Lys Pro Lys Asp Thr Leu Met Ile Ser Arg Thr Pro Glu Val
        515                 520                 525

Thr Cys Val Val Val Asp Val Ser His Glu Asp Pro Glu Val Lys Phe
    530                 535                 540

Asn Trp Tyr Val Asp Gly Val Glu Val His Asn Ala Lys Thr Lys Pro
545                 550                 555                 560

Arg Glu Glu Gln Tyr Asn Ser Thr Tyr Arg Val Val Ser Val Leu Thr
                565                 570                 575

Val Leu His Gln Asp Trp Leu Asn Gly Lys Glu Tyr Lys Cys Lys Val
            580                 585                 590

Ser Asn Lys Ala Leu Pro Ala Pro Ile Glu Lys Thr Ile Ser Lys Ala
        595                 600                 605

Lys Gly Gln Pro Arg Glu Pro Gln Val Tyr Thr Leu Pro Pro Ser Arg
    610                 615                 620

Glu Glu Met Thr Lys Asn Gln Val Ser Leu Thr Cys Leu Val Lys Gly
625                 630                 635                 640

Phe Tyr Pro Ser Asp Ile Ala Val Glu Trp Glu Ser Asn Gly Gln Pro
                645                 650                 655

Glu Asn Asn Tyr Lys Thr Thr Pro Pro Val Leu Asp Ser Asp Gly Ser
            660                 665                 670

Phe Phe Leu Tyr Ser Lys Leu Thr Val Asp Lys Ser Arg Trp Gln Glu
        675                 680                 685

Gly Asn Val Phe Ser Cys Ser Val Met His Glu Ala Leu His Asn His

690                 695                 700

Tyr Thr Gln Lys Ser Leu Ser Leu Ser Pro
705                 710

<210>  151
<211>  719
<212>  PRT
<213>  Artificial Sequence

<220>
<223>  an artificially synthesized sequence

<400>  151

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Glu Val Gln Leu Val Glu Ser Gly Gly Gly Leu Val Gln Pro Gly
        355                 360                 365

Gly Leu Ser Gly Arg Ser Asp Asn His Gly Ser Leu Arg Leu Ser Cys
    370                 375                 380

Ala Ala Ser Gly Phe Thr Phe Asp Asp Tyr Gly Met Ser Trp Val Arg
385                 390                 395                 400

Gln Ala Pro Gly Lys Ala Leu Glu Trp Val Ser Ala Ile Ser Trp Asn
                405                 410                 415

Gly Asn Asn Thr Tyr Tyr Thr Glu Ser Met Lys Gly Arg Phe Thr Ile
            420                 425                 430

Ser Arg Asp Asn Ala Lys Asn Thr Leu Tyr Leu Gln Met Asn Ser Leu

435                 440                 445

Arg Pro Glu Asp Thr Ala Val Tyr Tyr Cys Val Lys Gly Ser Thr Ala
    450                 455                 460

Ile Val Gly Val Pro Pro Thr Tyr Pro Asp Glu Tyr Asp Tyr Trp Gly
465                 470                 475                 480

Gln Gly Thr Leu Val Thr Val Ser Ser Glu Pro Lys Ser Ser Asp Lys
                485                 490                 495

Thr His Thr Cys Pro Pro Cys Pro Ala Pro Glu Leu Leu Gly Gly Pro
            500                 505                 510

Ser Val Phe Leu Phe Pro Pro Lys Pro Lys Asp Thr Leu Met Ile Ser
        515                 520                 525

Arg Thr Pro Glu Val Thr Cys Val Val Val Asp Val Ser His Glu Asp
    530                 535                 540

Pro Glu Val Lys Phe Asn Trp Tyr Val Asp Gly Val Glu Val His Asn
545                 550                 555                 560

Ala Lys Thr Lys Pro Arg Glu Glu Gln Tyr Asn Ser Thr Tyr Arg Val
                565                 570                 575

Val Ser Val Leu Thr Val Leu His Gln Asp Trp Leu Asn Gly Lys Glu
            580                 585                 590

Tyr Lys Cys Lys Val Ser Asn Lys Ala Leu Pro Ala Pro Ile Glu Lys
        595                 600                 605

Thr Ile Ser Lys Ala Lys Gly Gln Pro Arg Glu Pro Gln Val Tyr Thr
    610                 615                 620

Leu Pro Pro Ser Arg Glu Glu Met Thr Lys Asn Gln Val Ser Leu Thr
625                 630                 635                 640

Cys Leu Val Lys Gly Phe Tyr Pro Ser Asp Ile Ala Val Glu Trp Glu
                645                 650                 655

Ser Asn Gly Gln Pro Glu Asn Asn Tyr Lys Thr Thr Pro Pro Val Leu
            660                 665                 670

Asp Ser Asp Gly Ser Phe Phe Leu Tyr Ser Lys Leu Thr Val Asp Lys
        675                 680                 685

Ser Arg Trp Gln Glu Gly Asn Val Phe Ser Cys Ser Val Met His Glu
    690                 695                 700

Ala Leu His Asn His Tyr Thr Gln Lys Ser Leu Ser Leu Ser Pro
705                 710                 715

<210>  152
<211>  724
<212>  PRT
<213>  Artificial Sequence

<220>
<223>  an artificially synthesized sequence

<400>  152

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys

180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Glu Val Gln Leu Val Glu Ser Gly Gly Gly
        355                 360                 365

Leu Val Gln Pro Gly Gly Leu Ser Gly Arg Ser Asp Asn His Gly Ser
    370                 375                 380

Leu Arg Leu Ser Cys Ala Ala Ser Gly Phe Thr Phe Asp Asp Tyr Gly
385                 390                 395                 400

Met Ser Trp Val Arg Gln Ala Pro Gly Lys Ala Leu Glu Trp Val Ser
                405                 410                 415

Ala Ile Ser Trp Asn Gly Asn Asn Thr Tyr Tyr Thr Glu Ser Met Lys
            420                 425                 430

Gly Arg Phe Thr Ile Ser Arg Asp Asn Ala Lys Asn Thr Leu Tyr Leu
        435                 440                 445

Gln Met Asn Ser Leu Arg Pro Glu Asp Thr Ala Val Tyr Tyr Cys Val
    450                 455                 460

Lys Gly Ser Thr Ala Ile Val Gly Val Pro Pro Thr Tyr Pro Asp Glu
465                 470                 475                 480

Tyr Asp Tyr Trp Gly Gln Gly Thr Leu Val Thr Val Ser Ser Glu Pro
                485                 490                 495

Lys Ser Ser Asp Lys Thr His Thr Cys Pro Pro Cys Pro Ala Pro Glu
            500                 505                 510

Leu Leu Gly Gly Pro Ser Val Phe Leu Phe Pro Pro Lys Pro Lys Asp
        515                 520                 525

Thr Leu Met Ile Ser Arg Thr Pro Glu Val Thr Cys Val Val Val Asp
    530                 535                 540

Val Ser His Glu Asp Pro Glu Val Lys Phe Asn Trp Tyr Val Asp Gly
545                 550                 555                 560

Val Glu Val His Asn Ala Lys Thr Lys Pro Arg Glu Glu Gln Tyr Asn
                565                 570                 575

Ser Thr Tyr Arg Val Val Ser Val Leu Thr Val Leu His Gln Asp Trp
            580                 585                 590

Leu Asn Gly Lys Glu Tyr Lys Cys Lys Val Ser Asn Lys Ala Leu Pro
        595                 600                 605

Ala Pro Ile Glu Lys Thr Ile Ser Lys Ala Lys Gly Gln Pro Arg Glu
    610                 615                 620

Pro Gln Val Tyr Thr Leu Pro Pro Ser Arg Glu Glu Met Thr Lys Asn
625                 630                 635                 640

Gln Val Ser Leu Thr Cys Leu Val Lys Gly Phe Tyr Pro Ser Asp Ile
                645                 650                 655

Ala Val Glu Trp Glu Ser Asn Gly Gln Pro Glu Asn Asn Tyr Lys Thr
            660                 665                 670

Thr Pro Pro Val Leu Asp Ser Asp Gly Ser Phe Phe Leu Tyr Ser Lys
        675                 680                 685

Leu Thr Val Asp Lys Ser Arg Trp Gln Glu Gly Asn Val Phe Ser Cys
    690                 695                 700

Ser Val Met His Glu Ala Leu His Asn His Tyr Thr Gln Lys Ser Leu
705                 710                 715                 720

Ser Leu Ser Pro

<210>  153
<211>  729
<212>  PRT
<213>  Artificial Sequence

<220>
<223>  an artificially synthesized sequence

<400>  153

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Gly Gly Gly Gly Ser Glu Val Gln Leu Val
        355                 360                 365

Glu Ser Gly Gly Gly Leu Val Gln Pro Gly Gly Leu Ser Gly Arg Ser
    370                 375                 380

Asp Asn His Gly Ser Leu Arg Leu Ser Cys Ala Ala Ser Gly Phe Thr
385                 390                 395                 400

Phe Asp Asp Tyr Gly Met Ser Trp Val Arg Gln Ala Pro Gly Lys Ala
                405                 410                 415

Leu Glu Trp Val Ser Ala Ile Ser Trp Asn Gly Asn Asn Thr Tyr Tyr
            420                 425                 430

Thr Glu Ser Met Lys Gly Arg Phe Thr Ile Ser Arg Asp Asn Ala Lys
        435                 440                 445

Asn Thr Leu Tyr Leu Gln Met Asn Ser Leu Arg Pro Glu Asp Thr Ala
    450                 455                 460

Val Tyr Tyr Cys Val Lys Gly Ser Thr Ala Ile Val Gly Val Pro Pro
465                 470                 475                 480

Thr Tyr Pro Asp Glu Tyr Asp Tyr Trp Gly Gln Gly Thr Leu Val Thr
                485                 490                 495

Val Ser Ser Glu Pro Lys Ser Ser Asp Lys Thr His Thr Cys Pro Pro
            500                 505                 510

Cys Pro Ala Pro Glu Leu Leu Gly Gly Pro Ser Val Phe Leu Phe Pro
        515                 520                 525

Pro Lys Pro Lys Asp Thr Leu Met Ile Ser Arg Thr Pro Glu Val Thr
    530                 535                 540

Cys Val Val Val Asp Val Ser His Glu Asp Pro Glu Val Lys Phe Asn
545                 550                 555                 560

Trp Tyr Val Asp Gly Val Glu Val His Asn Ala Lys Thr Lys Pro Arg
                565                 570                 575

Glu Glu Gln Tyr Asn Ser Thr Tyr Arg Val Val Ser Val Leu Thr Val
            580                 585                 590

Leu His Gln Asp Trp Leu Asn Gly Lys Glu Tyr Lys Cys Lys Val Ser
        595                 600                 605

Asn Lys Ala Leu Pro Ala Pro Ile Glu Lys Thr Ile Ser Lys Ala Lys
    610                 615                 620

Gly Gln Pro Arg Glu Pro Gln Val Tyr Thr Leu Pro Pro Ser Arg Glu
625                 630                 635                 640

Glu Met Thr Lys Asn Gln Val Ser Leu Thr Cys Leu Val Lys Gly Phe
                645                 650                 655

Tyr Pro Ser Asp Ile Ala Val Glu Trp Glu Ser Asn Gly Gln Pro Glu
            660                 665                 670

Asn Asn Tyr Lys Thr Thr Pro Pro Val Leu Asp Ser Asp Gly Ser Phe
        675                 680                 685

Phe Leu Tyr Ser Lys Leu Thr Val Asp Lys Ser Arg Trp Gln Glu Gly
    690                 695                 700

Asn Val Phe Ser Cys Ser Val Met His Glu Ala Leu His Asn His Tyr
705                 710                 715                 720

Thr Gln Lys Ser Leu Ser Leu Ser Pro
                725

<210>  154
<211>  700
<212>  PRT
<213>  Artificial Sequence

<220>
<223>  an artificially synthesized sequence

<400>  154

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Glu Val Gln Leu Val Glu Ser Gly Gly
            340                 345                 350

Gly Leu Val Gln Pro Gly Gly Ser Leu Arg Leu Ser Cys Ala Ala Ser
        355                 360                 365

Gly Phe Thr Phe Asp Asp Tyr Gly Met Ser Trp Val Arg Gln Ala Pro
    370                 375                 380

Gly Lys Ala Leu Glu Trp Val Ser Ala Ile Ser Trp Asn Gly Asn Asn
385                 390                 395                 400

Thr Tyr Tyr Thr Glu Ser Met Lys Gly Arg Phe Thr Ile Ser Arg Asp
                405                 410                 415

Asn Ala Lys Asn Thr Leu Tyr Leu Gln Met Asn Ser Leu Arg Pro Glu
            420                 425                 430

Asp Thr Ala Val Tyr Tyr Cys Val Lys Gly Ser Thr Ala Ile Val Gly
        435                 440                 445

Val Pro Pro Thr Tyr Pro Asp Glu Tyr Asp Tyr Trp Gly Gln Gly Thr
    450                 455                 460

Leu Val Thr Val Ser Ser Glu Pro Lys Ser Ser Asp Lys Thr His Thr
465                 470                 475                 480

Cys Pro Pro Cys Pro Ala Pro Glu Leu Leu Gly Gly Pro Ser Val Phe
                485                 490                 495

Leu Phe Pro Pro Lys Pro Lys Asp Thr Leu Met Ile Ser Arg Thr Pro
            500                 505                 510

Glu Val Thr Cys Val Val Val Asp Val Ser His Glu Asp Pro Glu Val
        515                 520                 525

Lys Phe Asn Trp Tyr Val Asp Gly Val Glu Val His Asn Ala Lys Thr
    530                 535                 540

Lys Pro Arg Glu Glu Gln Tyr Asn Ser Thr Tyr Arg Val Val Ser Val
545                 550                 555                 560

Leu Thr Val Leu His Gln Asp Trp Leu Asn Gly Lys Glu Tyr Lys Cys
                565                 570                 575

Lys Val Ser Asn Lys Ala Leu Pro Ala Pro Ile Glu Lys Thr Ile Ser
            580                 585                 590

Lys Ala Lys Gly Gln Pro Arg Glu Pro Gln Val Tyr Thr Leu Pro Pro
        595                 600                 605

Ser Arg Glu Glu Met Thr Lys Asn Gln Val Ser Leu Thr Cys Leu Val
    610                 615                 620

Lys Gly Phe Tyr Pro Ser Asp Ile Ala Val Glu Trp Glu Ser Asn Gly
625                 630                 635                 640

Gln Pro Glu Asn Asn Tyr Lys Thr Thr Pro Pro Val Leu Asp Ser Asp

645                 650                 655

Gly Ser Phe Phe Leu Tyr Ser Lys Leu Thr Val Asp Lys Ser Arg Trp
            660                 665                 670

Gln Glu Gly Asn Val Phe Ser Cys Ser Val Met His Glu Ala Leu His
        675                 680                 685

Asn His Tyr Thr Gln Lys Ser Leu Ser Leu Ser Pro
    690                 695                 700

<210>  155
<211>  705
<212>  PRT
<213>  Artificial Sequence

<220>
<223>  an artificially synthesized sequence

<400>  155

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Glu Val Gln Leu
            340                 345                 350

Val Glu Ser Gly Gly Gly Leu Val Gln Pro Gly Gly Ser Leu Arg Leu
        355                 360                 365

Ser Cys Ala Ala Ser Gly Phe Thr Phe Asp Asp Tyr Gly Met Ser Trp
    370                 375                 380

Val Arg Gln Ala Pro Gly Lys Ala Leu Glu Trp Val Ser Ala Ile Ser
385                 390                 395                 400

Trp Asn Gly Asn Asn Thr Tyr Tyr Thr Glu Ser Met Lys Gly Arg Phe

                405                 410                 415

Thr Ile Ser Arg Asp Asn Ala Lys Asn Thr Leu Tyr Leu Gln Met Asn
            420                 425                 430

Ser Leu Arg Pro Glu Asp Thr Ala Val Tyr Tyr Cys Val Lys Gly Ser
        435                 440                 445

Thr Ala Ile Val Gly Val Pro Pro Thr Tyr Pro Asp Glu Tyr Asp Tyr
    450                 455                 460

Trp Gly Gln Gly Thr Leu Val Thr Val Ser Ser Glu Pro Lys Ser Ser
465                 470                 475                 480

Asp Lys Thr His Thr Cys Pro Pro Cys Pro Ala Pro Glu Leu Leu Gly
                485                 490                 495

Gly Pro Ser Val Phe Leu Phe Pro Pro Lys Pro Lys Asp Thr Leu Met
            500                 505                 510

Ile Ser Arg Thr Pro Glu Val Thr Cys Val Val Val Asp Val Ser His
        515                 520                 525

Glu Asp Pro Glu Val Lys Phe Asn Trp Tyr Val Asp Gly Val Glu Val
    530                 535                 540

His Asn Ala Lys Thr Lys Pro Arg Glu Glu Gln Tyr Asn Ser Thr Tyr
545                 550                 555                 560

Arg Val Val Ser Val Leu Thr Val Leu His Gln Asp Trp Leu Asn Gly
                565                 570                 575

Lys Glu Tyr Lys Cys Lys Val Ser Asn Lys Ala Leu Pro Ala Pro Ile
            580                 585                 590

Glu Lys Thr Ile Ser Lys Ala Lys Gly Gln Pro Arg Glu Pro Gln Val
        595                 600                 605

Tyr Thr Leu Pro Pro Ser Arg Glu Glu Met Thr Lys Asn Gln Val Ser
    610                 615                 620

Leu Thr Cys Leu Val Lys Gly Phe Tyr Pro Ser Asp Ile Ala Val Glu
625                 630                 635                 640

Trp Glu Ser Asn Gly Gln Pro Glu Asn Asn Tyr Lys Thr Thr Pro Pro
                645                 650                 655

Val Leu Asp Ser Asp Gly Ser Phe Phe Leu Tyr Ser Lys Leu Thr Val
            660                 665                 670

Asp Lys Ser Arg Trp Gln Glu Gly Asn Val Phe Ser Cys Ser Val Met
        675                 680                 685

His Glu Ala Leu His Asn His Tyr Thr Gln Lys Ser Leu Ser Leu Ser
    690                 695                 700

Pro
705

<210> 156
<211> 710
<212> PRT
<213> Artificial Sequence

<220>
<223> an artificially synthesized sequence

<400> 156

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala

145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Glu Val Gln Leu Val Glu Ser Gly Gly Gly Leu Val Gln Pro Gly
        355                 360                 365

Gly Ser Leu Arg Leu Ser Cys Ala Ala Ser Gly Phe Thr Phe Asp Asp
    370                 375                 380

Tyr Gly Met Ser Trp Val Arg Gln Ala Pro Gly Lys Ala Leu Glu Trp
385                 390                 395                 400

Val Ser Ala Ile Ser Trp Asn Gly Asn Asn Thr Tyr Tyr Thr Glu Ser
                405                 410                 415

Met Lys Gly Arg Phe Thr Ile Ser Arg Asp Asn Ala Lys Asn Thr Leu
            420                 425                 430

Tyr Leu Gln Met Asn Ser Leu Arg Pro Glu Asp Thr Ala Val Tyr Tyr
        435                 440                 445

Cys Val Lys Gly Ser Thr Ala Ile Val Gly Val Pro Pro Thr Tyr Pro
    450                 455                 460

Asp Glu Tyr Asp Tyr Trp Gly Gln Gly Thr Leu Val Thr Val Ser Ser
465                 470                 475                 480

Glu Pro Lys Ser Ser Asp Lys Thr His Thr Cys Pro Pro Cys Pro Ala
                485                 490                 495

Pro Glu Leu Leu Gly Gly Pro Ser Val Phe Leu Phe Pro Pro Lys Pro
            500                 505                 510

Lys Asp Thr Leu Met Ile Ser Arg Thr Pro Glu Val Thr Cys Val Val
        515                 520                 525

Val Asp Val Ser His Glu Asp Pro Glu Val Lys Phe Asn Trp Tyr Val
    530                 535                 540

Asp Gly Val Glu Val His Asn Ala Lys Thr Lys Pro Arg Glu Glu Gln
545                 550                 555                 560

Tyr Asn Ser Thr Tyr Arg Val Val Ser Val Leu Thr Val Leu His Gln
                565                 570                 575

Asp Trp Leu Asn Gly Lys Glu Tyr Lys Cys Lys Val Ser Asn Lys Ala
            580                 585                 590

Leu Pro Ala Pro Ile Glu Lys Thr Ile Ser Lys Ala Lys Gly Gln Pro
        595                 600                 605

Arg Glu Pro Gln Val Tyr Thr Leu Pro Pro Ser Arg Glu Glu Met Thr
    610                 615                 620

Lys Asn Gln Val Ser Leu Thr Cys Leu Val Lys Gly Phe Tyr Pro Ser
625                 630                 635                 640

Asp Ile Ala Val Glu Trp Glu Ser Asn Gly Gln Pro Glu Asn Asn Tyr
                645                 650                 655

Lys Thr Thr Pro Pro Val Leu Asp Ser Asp Gly Ser Phe Phe Leu Tyr
            660                 665                 670

Ser Lys Leu Thr Val Asp Lys Ser Arg Trp Gln Glu Gly Asn Val Phe
        675                 680                 685

Ser Cys Ser Val Met His Glu Ala Leu His Asn His Tyr Thr Gln Lys
    690                 695                 700

Ser Leu Ser Leu Ser Pro
705                 710

<210>  157
<211>  715
<212>  PRT
<213>  Artificial Sequence

<220>
<223>  an artificially synthesized sequence

<400>  157

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Glu Val Gln Leu Val Glu Ser Gly Gly Gly
        355                 360                 365

Leu Val Gln Pro Gly Gly Ser Leu Arg Leu Ser Cys Ala Ala Ser Gly
    370                 375                 380

Phe Thr Phe Asp Asp Tyr Gly Met Ser Trp Val Arg Gln Ala Pro Gly
385                 390                 395                 400

Lys Ala Leu Glu Trp Val Ser Ala Ile Ser Trp Asn Gly Asn Asn Thr
                405                 410                 415

Tyr Tyr Thr Glu Ser Met Lys Gly Arg Phe Thr Ile Ser Arg Asp Asn
            420                 425                 430

Ala Lys Asn Thr Leu Tyr Leu Gln Met Asn Ser Leu Arg Pro Glu Asp
        435                 440                 445

Thr Ala Val Tyr Tyr Cys Val Lys Gly Ser Thr Ala Ile Val Gly Val
    450                 455                 460

Pro Pro Thr Tyr Pro Asp Glu Tyr Asp Tyr Trp Gly Gln Gly Thr Leu
465                 470                 475                 480

Val Thr Val Ser Ser Glu Pro Lys Ser Ser Asp Lys Thr His Thr Cys
                485                 490                 495

Pro Pro Cys Pro Ala Pro Glu Leu Leu Gly Gly Pro Ser Val Phe Leu
            500                 505                 510

Phe Pro Pro Lys Pro Lys Asp Thr Leu Met Ile Ser Arg Thr Pro Glu
        515                 520                 525

Val Thr Cys Val Val Val Asp Val Ser His Glu Asp Pro Glu Val Lys
    530                 535                 540

Phe Asn Trp Tyr Val Asp Gly Val Glu Val His Asn Ala Lys Thr Lys
545                 550                 555                 560

Pro Arg Glu Glu Gln Tyr Asn Ser Thr Tyr Arg Val Val Ser Val Leu
                565                 570                 575

Thr Val Leu His Gln Asp Trp Leu Asn Gly Lys Glu Tyr Lys Cys Lys
            580                 585                 590

Val Ser Asn Lys Ala Leu Pro Ala Pro Ile Glu Lys Thr Ile Ser Lys
        595                 600                 605

Ala Lys Gly Gln Pro Arg Glu Pro Gln Val Tyr Thr Leu Pro Pro Ser
    610                 615                 620

Arg Glu Glu Met Thr Lys Asn Gln Val Ser Leu Thr Cys Leu Val Lys
625                 630                 635                 640

Gly Phe Tyr Pro Ser Asp Ile Ala Val Glu Trp Glu Ser Asn Gly Gln
                645                 650                 655

Pro Glu Asn Asn Tyr Lys Thr Thr Pro Pro Val Leu Asp Ser Asp Gly
            660                 665                 670

Ser Phe Phe Leu Tyr Ser Lys Leu Thr Val Asp Lys Ser Arg Trp Gln
        675                 680                 685

Glu Gly Asn Val Phe Ser Cys Ser Val Met His Glu Ala Leu His Asn
    690                 695                 700

His Tyr Thr Gln Lys Ser Leu Ser Leu Ser Pro
705                 710                 715

<210> 158
<211> 720
<212> PRT
<213> Artificial Sequence

<220>
<223> an artificially synthesized sequence

<400> 158

Leu Ala Pro Arg Arg Cys Pro Ala Gln Glu Val Ala Arg Gly Val Leu
1               5                   10                  15

Thr Ser Leu Pro Gly Asp Ser Val Thr Leu Thr Cys Pro Gly Val Glu
            20                  25                  30

Pro Glu Asp Asn Ala Thr Val His Trp Val Leu Arg Lys Pro Ala Ala
        35                  40                  45

Gly Ser His Pro Ser Arg Trp Ala Gly Met Gly Arg Arg Leu Leu Leu
    50                  55                  60

Arg Ser Val Gln Leu His Asp Ser Gly Asn Tyr Ser Cys Tyr Arg Ala
65                  70                  75                  80

Gly Arg Pro Ala Gly Thr Val His Leu Leu Val Asp Val Pro Pro Glu
                85                  90                  95

Glu Pro Gln Leu Ser Cys Phe Arg Lys Ser Pro Leu Ser Asn Val Val
            100                 105                 110

Cys Glu Trp Gly Pro Arg Ser Thr Pro Ser Leu Thr Thr Lys Ala Val
        115                 120                 125

Leu Leu Val Arg Lys Phe Gln Asn Ser Pro Ala Glu Asp Phe Gln Glu
    130                 135                 140

Pro Cys Gln Tyr Ser Gln Glu Ser Gln Lys Phe Ser Cys Gln Leu Ala
145                 150                 155                 160

Val Pro Glu Gly Asp Ser Ser Phe Tyr Ile Val Ser Met Cys Val Ala
                165                 170                 175

Ser Ser Val Gly Ser Lys Phe Ser Lys Thr Gln Thr Phe Gln Gly Cys
            180                 185                 190

Gly Ile Leu Gln Pro Asp Pro Pro Ala Asn Ile Thr Val Thr Ala Val
        195                 200                 205

Ala Arg Asn Pro Arg Trp Leu Ser Val Thr Trp Gln Asp Pro His Ser
    210                 215                 220

Trp Asn Ser Ser Phe Tyr Arg Leu Arg Phe Glu Leu Arg Tyr Arg Ala
225                 230                 235                 240

Glu Arg Ser Lys Thr Phe Thr Thr Trp Met Val Lys Asp Leu Gln His
                245                 250                 255

His Cys Val Ile His Asp Ala Trp Ser Gly Leu Arg His Val Val Gln
            260                 265                 270

Leu Arg Ala Gln Glu Glu Phe Gly Gln Gly Glu Trp Ser Glu Trp Ser
        275                 280                 285

Pro Glu Ala Met Gly Thr Pro Trp Thr Glu Ser Arg Ser Pro Pro Ala
    290                 295                 300

Glu Asn Glu Val Ser Thr Pro Met Gln Ala Leu Thr Thr Asn Lys Asp
305                 310                 315                 320

Asp Asp Asn Ile Leu Phe Arg Asp Ser Ala Asn Ala Thr Ser Leu Pro
                325                 330                 335

Val Gln Gly Gly Gly Gly Ser Gly Gly Gly Gly Ser Gly Gly Gly Gly
            340                 345                 350

Ser Gly Gly Gly Gly Ser Gly Gly Gly Gly Ser Glu Val Gln Leu Val
        355                 360                 365

Glu Ser Gly Gly Gly Leu Val Gln Pro Gly Gly Ser Leu Arg Leu Ser
    370                 375                 380

Cys Ala Ala Ser Gly Phe Thr Phe Asp Asp Tyr Gly Met Ser Trp Val
385                 390                 395                 400

Arg Gln Ala Pro Gly Lys Ala Leu Glu Trp Val Ser Ala Ile Ser Trp
                405                 410                 415

Asn Gly Asn Asn Thr Tyr Tyr Thr Glu Ser Met Lys Gly Arg Phe Thr
            420                 425                 430

Ile Ser Arg Asp Asn Ala Lys Asn Thr Leu Tyr Leu Gln Met Asn Ser
        435                 440                 445

Leu Arg Pro Glu Asp Thr Ala Val Tyr Tyr Cys Val Lys Gly Ser Thr
    450                 455                 460

Ala Ile Val Gly Val Pro Pro Thr Tyr Pro Asp Glu Tyr Asp Tyr Trp
465                 470                 475                 480

Gly Gln Gly Thr Leu Val Thr Val Ser Ser Glu Pro Lys Ser Ser Asp
                485                 490                 495

Lys Thr His Thr Cys Pro Pro Cys Pro Ala Pro Glu Leu Leu Gly Gly
            500                 505                 510

Pro Ser Val Phe Leu Phe Pro Pro Lys Pro Lys Asp Thr Leu Met Ile
        515                 520                 525

Ser Arg Thr Pro Glu Val Thr Cys Val Val Val Asp Val Ser His Glu
    530                 535                 540

Asp Pro Glu Val Lys Phe Asn Trp Tyr Val Asp Gly Val Glu Val His
545                 550                 555                 560

Asn Ala Lys Thr Lys Pro Arg Glu Glu Gln Tyr Asn Ser Thr Tyr Arg
                565                 570                 575

Val Val Ser Val Leu Thr Val Leu His Gln Asp Trp Leu Asn Gly Lys
            580                 585                 590

Glu Tyr Lys Cys Lys Val Ser Asn Lys Ala Leu Pro Ala Pro Ile Glu
        595                 600                 605

Lys Thr Ile Ser Lys Ala Lys Gly Gln Pro Arg Glu Pro Gln Val Tyr
    610                 615                 620

Thr Leu Pro Pro Ser Arg Glu Glu Met Thr Lys Asn Gln Val Ser Leu
625                 630                 635                 640

Thr Cys Leu Val Lys Gly Phe Tyr Pro Ser Asp Ile Ala Val Glu Trp

645                 650                 655

Glu Ser Asn Gly Gln Pro Glu Asn Asn Tyr Lys Thr Thr Pro Pro Val
            660                 665                 670

Leu Asp Ser Asp Gly Ser Phe Phe Leu Tyr Ser Lys Leu Thr Val Asp
        675                 680                 685

Lys Ser Arg Trp Gln Glu Gly Asn Val Phe Ser Cys Ser Val Met His
    690                 695                 700

Glu Ala Leu His Asn His Tyr Thr Gln Lys Ser Leu Ser Leu Ser Pro
705                 710                 715                 720

<210>  159
<211>  15
<212>  PRT
<213>  Artificial Sequence

<220>
<223>  an artificially synthesized sequence

<400>  159

Gly Leu Asn Asp Ile Phe Glu Ala Gln Lys Ile Glu Trp His Glu
1               5                   10                  15

<210>  160
<211>  376
<212>  PRT
<213>  Artificial Sequence

<220>
<223>  an artificially synthesized sequence

<400>  160

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asp Asp Tyr
            20                  25                  30

Gly Met Ser Trp Val Arg Gln Ala Pro Gly Lys Ala Leu Glu Trp Val
        35                  40                  45

Ser Ala Ile Ser Trp Asn Gly Asn Asn Thr Tyr Tyr Thr Glu Ser Met
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Pro Glu Asp Thr Ala Val Tyr Tyr Cys
                85                  90                  95

Val Lys Gly Ser Thr Ala Ile Val Gly Val Pro Pro Thr Tyr Pro Asp
            100                 105                 110

Glu Tyr Asp Tyr Trp Gly Gln Gly Thr Leu Val Thr Val Ser Ser Pro
        115                 120                 125

Lys Ser Ser Asp Lys Thr His Thr Cys Pro Pro Cys Pro Ala Pro Glu
    130                 135                 140

Leu Leu Gly Gly Pro Ser Val Phe Leu Phe Pro Pro Lys Pro Lys Asp
145                 150                 155                 160

Thr Leu Met Ile Ser Arg Thr Pro Glu Val Thr Cys Val Val Val Asp
                165                 170                 175

Val Ser His Glu Asp Pro Glu Val Lys Phe Asn Trp Tyr Val Asp Gly
            180                 185                 190

Val Glu Val His Asn Ala Lys Thr Lys Pro Arg Glu Glu Gln Tyr Asn
        195                 200                 205

Ser Thr Tyr Arg Val Val Ser Val Leu Thr Val Leu His Gln Asp Trp
    210                 215                 220

Leu Asn Gly Lys Glu Tyr Lys Cys Lys Val Ser Asn Lys Ala Leu Pro
225                 230                 235                 240

Ala Pro Ile Glu Lys Thr Ile Ser Lys Ala Lys Gly Gln Pro Arg Glu
                245                 250                 255

Pro Gln Val Tyr Thr Leu Pro Pro Ser Arg Glu Glu Met Thr Lys Asn
            260                 265                 270

Gln Val Ser Leu Thr Cys Leu Val Lys Gly Phe Tyr Pro Ser Asp Ile
        275                 280                 285

Ala Val Glu Trp Glu Ser Asn Gly Gln Pro Glu Asn Asn Tyr Lys Thr
    290                 295                 300

Thr Pro Pro Val Leu Asp Ser Asp Gly Ser Phe Phe Leu Tyr Ser Lys
305                 310                 315                 320

Leu Thr Val Asp Lys Ser Arg Trp Gln Gln Gly Asn Val Phe Ser Cys
                325                 330                 335

Ser Val Met His Glu Ala Leu His Asn His Tyr Thr Gln Lys Ser Leu
            340                 345                 350

Ser Leu Ser Pro Gly Gly Gly Gly Ser Gly Leu Asn Asp Ile Phe Glu
        355                 360                 365

Ala Gln Lys Ile Glu Trp His Glu
    370                 375

<210> 161
<211> 349
<212> PRT
<213> Artificial Sequence

<220>
<223> an artificially synthesized sequence

<400> 161

Glu Val Gln Leu Val Glu Ser Gly Gly Gly Leu Val Gln Ala Gly Lys
1               5                   10                  15

Ser Leu Arg Leu Ser Cys Ala Ala Ser Gly Ser Ile Phe Ser Ile His
            20                  25                  30

Ala Met Gly Trp Phe Arg Gln Ala Pro Gly Lys Glu Arg Glu Phe Val
        35                  40                  45

Ala Ala Ile Thr Trp Ser Gly Gly Ile Thr Tyr Tyr Glu Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Ile Tyr Tyr Cys
                85                  90                  95

Ala Ala Asp Arg Ala Glu Ser Ser Trp Tyr Asp Tyr Trp Gly Gln Gly
            100                 105                 110

Thr Gln Val Thr Val Ser Ser Glu Pro Lys Ser Ser Asp Lys Thr His
        115                 120                 125

Thr Cys Pro Pro Cys Pro Ala Pro Glu Leu Leu Gly Gly Pro Ser Val
    130                 135                 140

Phe Leu Phe Pro Pro Lys Pro Lys Asp Thr Leu Met Ile Ser Arg Thr
145                 150                 155                 160

Pro Glu Val Thr Cys Val Val Val Asp Val Ser His Glu Asp Pro Glu
                165                 170                 175

Val Lys Phe Asn Trp Tyr Val Asp Gly Val Glu Val His Asn Ala Lys
            180                 185                 190

Thr Lys Pro Arg Glu Glu Gln Tyr Asn Ser Thr Tyr Arg Val Val Ser
        195                 200                 205

Val Leu Thr Val Leu His Gln Asp Trp Leu Asn Gly Lys Glu Tyr Lys
    210                 215                 220

Cys Lys Val Ser Asn Lys Ala Leu Pro Ala Pro Ile Glu Lys Thr Ile
225                 230                 235                 240

Ser Lys Ala Lys Gly Gln Pro Arg Glu Pro Gln Val Tyr Thr Leu Pro
                245                 250                 255

Pro Ser Arg Glu Glu Met Thr Lys Asn Gln Val Ser Leu Thr Cys Leu
            260                 265                 270

Val Lys Gly Phe Tyr Pro Ser Asp Ile Ala Val Glu Trp Glu Ser Asn
        275                 280                 285

Gly Gln Pro Glu Asn Asn Tyr Lys Thr Thr Pro Pro Val Leu Asp Ser
    290                 295                 300

Asp Gly Ser Phe Phe Leu Tyr Ser Lys Leu Thr Val Asp Lys Ser Arg
305                 310                 315                 320

Trp Gln Glu Gly Asn Val Phe Ser Cys Ser Val Met His Glu Ala Leu
                325                 330                 335

His Asn His Tyr Thr Gln Lys Ser Leu Ser Leu Ser Pro
            340                 345

<210>  162
<211>  349
<212>  PRT
<213>  Artificial Sequence

<220>
<223>  an artificially synthesized sequence

<400>  162

Glu Val Gln Leu Val Glu Ser Gly Gly Gly Leu Val Gln Ala Gly Gly
1               5                   10                  15

Ser Leu Arg Leu Ser Cys Ala Ala Ser Gly Ser Ile Ala Ser Ile His

            20                  25                  30

Ala Met Gly Trp Phe Arg Gln Ala Pro Gly Lys Glu Arg Glu Phe Val
        35                  40                  45

Ala Val Ile Thr Trp Ser Gly Gly Ile Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Ile Tyr Tyr Cys
                85                  90                  95

Ala Gly Asp Lys His Gln Ser Ser Trp Tyr Asp Tyr Trp Gly Gln Gly
            100                 105                 110

Thr Gln Val Thr Val Ser Ser Glu Pro Lys Ser Ser Asp Lys Thr His
        115                 120                 125

Thr Cys Pro Pro Cys Pro Ala Pro Glu Leu Leu Gly Gly Pro Ser Val
    130                 135                 140

Phe Leu Phe Pro Pro Lys Pro Lys Asp Thr Leu Met Ile Ser Arg Thr
145                 150                 155                 160

Pro Glu Val Thr Cys Val Val Val Asp Val Ser His Glu Asp Pro Glu
                165                 170                 175

Val Lys Phe Asn Trp Tyr Val Asp Gly Val Glu Val His Asn Ala Lys
            180                 185                 190

Thr Lys Pro Arg Glu Glu Gln Tyr Asn Ser Thr Tyr Arg Val Val Ser
        195                 200                 205

Val Leu Thr Val Leu His Gln Asp Trp Leu Asn Gly Lys Glu Tyr Lys
    210                 215                 220

Cys Lys Val Ser Asn Lys Ala Leu Pro Ala Pro Ile Glu Lys Thr Ile
225                 230                 235                 240

Ser Lys Ala Lys Gly Gln Pro Arg Glu Pro Gln Val Tyr Thr Leu Pro
                245                 250                 255

Pro Ser Arg Glu Glu Met Thr Lys Asn Gln Val Ser Leu Thr Cys Leu
            260                 265                 270

Val Lys Gly Phe Tyr Pro Ser Asp Ile Ala Val Glu Trp Glu Ser Asn
        275                 280                 285

Gly Gln Pro Glu Asn Asn Tyr Lys Thr Thr Pro Pro Val Leu Asp Ser
    290                 295                 300

Asp Gly Ser Phe Phe Leu Tyr Ser Lys Leu Thr Val Asp Lys Ser Arg
305                 310                 315                 320

Trp Gln Gln Gly Asn Val Phe Ser Cys Ser Val Met His Glu Ala Leu
                325                 330                 335

His Asn His Tyr Thr Gln Lys Ser Leu Ser Leu Ser Pro
            340                 345

<210>  163
<211>  147
<212>  PRT
<213>  Homo sapiens

<400>  163

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln
145

<210> 164
<211> 529
<212> PRT
<213> Artificial Sequence

<220>
<223> an artificially synthesized sequence

<400> 164

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Gly Gly Gly Gly Ser Gly Gly Gly Gly Ser Gly Gly Gly
145                 150                 155                 160

Gly Ser Gly Gly Gly Gly Ser Gly Gly Gly Gly Ser Glu Val Gln Leu
                165                 170                 175

Val Glu Ser Gly Gly Gly Leu Val Leu Ser Gly Arg Ser Asp Asn His
            180                 185                 190

Gln Ala Gly Lys Ser Leu Arg Leu Ser Cys Ala Ala Ser Gly Ser Ile

195                 200                 205

Phe Ser Ile His Ala Met Gly Trp Phe Arg Gln Ala Pro Gly Lys Glu
    210                 215                 220

Arg Glu Phe Val Ala Ala Ile Thr Trp Ser Gly Gly Ile Thr Tyr Tyr
225                 230                 235                 240

Glu Asp Ser Val Lys Gly Arg Phe Thr Ile Ser Arg Asp Asn Ala Lys
                245                 250                 255

Asn Thr Val Tyr Leu Gln Met Asn Ser Leu Lys Pro Glu Asp Thr Ala
            260                 265                 270

Ile Tyr Tyr Cys Ala Ala Asp Arg Ala Glu Ser Ser Trp Tyr Asp Tyr
        275                 280                 285

Trp Gly Gln Gly Thr Gln Val Thr Val Ser Ser Glu Pro Lys Ser Ser
    290                 295                 300

Asp Lys Thr His Thr Cys Pro Pro Cys Pro Ala Pro Glu Leu Leu Gly
305                 310                 315                 320

Gly Pro Ser Val Phe Leu Phe Pro Pro Lys Pro Lys Asp Thr Leu Met
                325                 330                 335

Ile Ser Arg Thr Pro Glu Val Thr Cys Val Val Val Asp Val Ser His
            340                 345                 350

Glu Asp Pro Glu Val Lys Phe Asn Trp Tyr Val Asp Gly Val Glu Val
        355                 360                 365

His Asn Ala Lys Thr Lys Pro Arg Glu Glu Gln Tyr Asn Ser Thr Tyr
    370                 375                 380

Arg Val Val Ser Val Leu Thr Val Leu His Gln Asp Trp Leu Asn Gly
385                 390                 395                 400

Lys Glu Tyr Lys Cys Lys Val Ser Asn Lys Ala Leu Pro Ala Pro Ile
                405                 410                 415

Glu Lys Thr Ile Ser Lys Ala Lys Gly Gln Pro Arg Glu Pro Gln Val
            420                 425                 430

Tyr Thr Leu Pro Pro Ser Arg Glu Glu Met Thr Lys Asn Gln Val Ser
        435                 440                 445

Leu Thr Cys Leu Val Lys Gly Phe Tyr Pro Ser Asp Ile Ala Val Glu
    450                 455                 460

Trp Glu Ser Asn Gly Gln Pro Glu Asn Asn Tyr Lys Thr Thr Pro Pro
465                 470                 475                 480

Val Leu Asp Ser Asp Gly Ser Phe Phe Leu Tyr Ser Lys Leu Thr Val
                485                 490                 495

Asp Lys Ser Arg Trp Gln Glu Gly Asn Val Phe Ser Cys Ser Val Met
            500                 505                 510

His Glu Ala Leu His Asn His Tyr Thr Gln Lys Ser Leu Ser Leu Ser
        515                 520                 525

Pro

<210>  165
<211>  529
<212>  PRT
<213>  Artificial Sequence

<220>
<223>  an artificially synthesized sequence

<400>  165

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg

        115                 120                 125

Ala Glu Val Pro Thr Ala His Pro Ser Pro Ser Pro Arg Pro Ala Gly
    130                 135                 140

Gln Phe Gln Gly Gly Gly Gly Ser Gly Gly Gly Gly Ser Gly Gly Gly
145                 150                 155                 160

Gly Ser Gly Gly Gly Gly Ser Gly Gly Gly Gly Ser Glu Val Gln Leu
                165                 170                 175

Val Glu Ser Gly Gly Gly Leu Val Gln Leu Ser Gly Arg Ser Asp Asn
            180                 185                 190

His Ala Gly Lys Ser Leu Arg Leu Ser Cys Ala Ala Ser Gly Ser Ile
        195                 200                 205

Phe Ser Ile His Ala Met Gly Trp Phe Arg Gln Ala Pro Gly Lys Glu
    210                 215                 220

Arg Glu Phe Val Ala Ala Ile Thr Trp Ser Gly Gly Ile Thr Tyr Tyr
225                 230                 235                 240

Glu Asp Ser Val Lys Gly Arg Phe Thr Ile Ser Arg Asp Asn Ala Lys
                245                 250                 255

Asn Thr Val Tyr Leu Gln Met Asn Ser Leu Lys Pro Glu Asp Thr Ala
            260                 265                 270

Ile Tyr Tyr Cys Ala Ala Asp Arg Ala Glu Ser Ser Trp Tyr Asp Tyr
        275                 280                 285

Trp Gly Gln Gly Thr Gln Val Thr Val Ser Ser Glu Pro Lys Ser Ser
    290                 295                 300

Asp Lys Thr His Thr Cys Pro Pro Cys Pro Ala Pro Glu Leu Leu Gly
305                 310                 315                 320

Gly Pro Ser Val Phe Leu Phe Pro Pro Lys Pro Lys Asp Thr Leu Met
                325                 330                 335

Ile Ser Arg Thr Pro Glu Val Thr Cys Val Val Val Asp Val Ser His
            340                 345                 350

Glu Asp Pro Glu Val Lys Phe Asn Trp Tyr Val Asp Gly Val Glu Val
        355                 360                 365

His Asn Ala Lys Thr Lys Pro Arg Glu Glu Gln Tyr Asn Ser Thr Tyr
    370                 375                 380

Arg Val Val Ser Val Leu Thr Val Leu His Gln Asp Trp Leu Asn Gly
385                 390                 395                 400

Lys Glu Tyr Lys Cys Lys Val Ser Asn Lys Ala Leu Pro Ala Pro Ile
                405                 410                 415

Glu Lys Thr Ile Ser Lys Ala Lys Gly Gln Pro Arg Glu Pro Gln Val
            420                 425                 430

Tyr Thr Leu Pro Pro Ser Arg Glu Glu Met Thr Lys Asn Gln Val Ser
        435                 440                 445

Leu Thr Cys Leu Val Lys Gly Phe Tyr Pro Ser Asp Ile Ala Val Glu
    450                 455                 460

Trp Glu Ser Asn Gly Gln Pro Glu Asn Asn Tyr Lys Thr Thr Pro Pro
465                 470                 475                 480

Val Leu Asp Ser Asp Gly Ser Phe Phe Leu Tyr Ser Lys Leu Thr Val
                485                 490                 495

Asp Lys Ser Arg Trp Gln Glu Gly Asn Val Phe Ser Cys Ser Val Met
            500                 505                 510

His Glu Ala Leu His Asn His Tyr Thr Gln Lys Ser Leu Ser Leu Ser
        515                 520                 525

Pro

<210>  166
<211>  529
<212>  PRT
<213>  Artificial Sequence

<220>
<223>  an artificially synthesized sequence

<400>  166

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr

35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Gly Gly Gly Gly Ser Gly Gly Gly Gly Ser Gly Gly Gly
145                 150                 155                 160

Gly Ser Gly Gly Gly Gly Ser Gly Gly Gly Gly Ser Glu Val Gln Leu
                165                 170                 175

Val Glu Ser Gly Gly Gly Leu Val Leu Ser Gly Arg Ser Asp Asn His
            180                 185                 190

Gln Ala Gly Gly Ser Leu Arg Leu Ser Cys Ala Ala Ser Gly Ser Ile
        195                 200                 205

Ala Ser Ile His Ala Met Gly Trp Phe Arg Gln Ala Pro Gly Lys Glu
    210                 215                 220

Arg Glu Phe Val Ala Val Ile Thr Trp Ser Gly Gly Ile Thr Tyr Tyr
225                 230                 235                 240

Ala Asp Ser Val Lys Gly Arg Phe Thr Ile Ser Arg Asp Asn Ala Lys
                245                 250                 255

Asn Thr Val Tyr Leu Gln Met Asn Ser Leu Lys Pro Glu Asp Thr Ala
            260                 265                 270

Ile Tyr Tyr Cys Ala Gly Asp Lys His Gln Ser Ser Trp Tyr Asp Tyr
        275                 280                 285

Trp Gly Gln Gly Thr Gln Val Thr Val Ser Ser Glu Pro Lys Ser Ser
    290                 295                 300

Asp Lys Thr His Thr Cys Pro Pro Cys Pro Ala Pro Glu Leu Leu Gly
305                 310                 315                 320

Gly Pro Ser Val Phe Leu Phe Pro Pro Lys Pro Lys Asp Thr Leu Met
                325                 330                 335

Ile Ser Arg Thr Pro Glu Val Thr Cys Val Val Val Asp Val Ser His
            340                 345                 350

Glu Asp Pro Glu Val Lys Phe Asn Trp Tyr Val Asp Gly Val Glu Val
        355                 360                 365

His Asn Ala Lys Thr Lys Pro Arg Glu Glu Gln Tyr Asn Ser Thr Tyr
    370                 375                 380

Arg Val Val Ser Val Leu Thr Val Leu His Gln Asp Trp Leu Asn Gly
385                 390                 395                 400

Lys Glu Tyr Lys Cys Lys Val Ser Asn Lys Ala Leu Pro Ala Pro Ile
                405                 410                 415

Glu Lys Thr Ile Ser Lys Ala Lys Gly Gln Pro Arg Glu Pro Gln Val
            420                 425                 430

Tyr Thr Leu Pro Pro Ser Arg Glu Glu Met Thr Lys Asn Gln Val Ser
        435                 440                 445

Leu Thr Cys Leu Val Lys Gly Phe Tyr Pro Ser Asp Ile Ala Val Glu
    450                 455                 460

Trp Glu Ser Asn Gly Gln Pro Glu Asn Asn Tyr Lys Thr Thr Pro Pro
465                 470                 475                 480

Val Leu Asp Ser Asp Gly Ser Phe Phe Leu Tyr Ser Lys Leu Thr Val
                485                 490                 495

Asp Lys Ser Arg Trp Gln Glu Gly Asn Val Phe Ser Cys Ser Val Met
            500                 505                 510

His Glu Ala Leu His Asn His Tyr Thr Gln Lys Ser Leu Ser Leu Ser
        515                 520                 525

Pro

<210>  167
<211>  529
<212>  PRT
<213>  Artificial Sequence

<220>
<223>  an artificially synthesized sequence

<400>  167

Pro Gly Trp Phe Leu Asp Ser Pro Asp Arg Pro Trp Asn Pro Pro Thr
1               5                   10                  15

Phe Ser Pro Ala Leu Leu Val Val Thr Glu Gly Asp Asn Ala Thr Phe
            20                  25                  30

Thr Cys Ser Phe Ser Asn Thr Ser Glu Ser Phe Val Leu Asn Trp Tyr
        35                  40                  45

Arg Met Ser Pro Ser Asn Gln Thr Asp Lys Leu Ala Ala Phe Pro Glu
    50                  55                  60

Asp Arg Ser Gln Pro Gly Gln Asp Cys Arg Phe Arg Val Thr Gln Leu
65                  70                  75                  80

Pro Asn Gly Arg Asp Phe His Met Ser Val Val Arg Ala Arg Arg Asn
                85                  90                  95

Asp Ser Gly Thr Tyr Leu Cys Gly Ala Ile Ser Leu Ala Pro Lys Ala
            100                 105                 110

Gln Ile Lys Glu Ser Leu Arg Ala Glu Leu Arg Val Thr Glu Arg Arg
        115                 120                 125

Ala Glu Val Pro Thr Ala His Pro Ser Pro Ser Pro Arg Pro Ala Gly
    130                 135                 140

Gln Phe Gln Gly Gly Gly Gly Ser Gly Gly Gly Gly Ser Gly Gly Gly
145                 150                 155                 160

Gly Ser Gly Gly Gly Gly Ser Gly Gly Gly Gly Ser Glu Val Gln Leu
                165                 170                 175

Val Glu Ser Gly Gly Gly Leu Val Gln Ala Leu Ser Gly Arg Ser Asp
            180                 185                 190

Asn His Gly Gly Ser Leu Arg Leu Ser Cys Ala Ala Ser Gly Ser Ile
        195                 200                 205

Ala Ser Ile His Ala Met Gly Trp Phe Arg Gln Ala Pro Gly Lys Glu
    210                 215                 220

Arg Glu Phe Val Ala Val Ile Thr Trp Ser Gly Gly Ile Thr Tyr Tyr
225                 230                 235                 240

Ala Asp Ser Val Lys Gly Arg Phe Thr Ile Ser Arg Asp Asn Ala Lys
                245                 250                 255

Asn Thr Val Tyr Leu Gln Met Asn Ser Leu Lys Pro Glu Asp Thr Ala
            260                 265                 270

Ile Tyr Tyr Cys Ala Gly Asp Lys His Gln Ser Ser Trp Tyr Asp Tyr
        275                 280                 285

Trp Gly Gln Gly Thr Gln Val Thr Val Ser Ser Glu Pro Lys Ser Ser
    290                 295                 300

Asp Lys Thr His Thr Cys Pro Pro Cys Pro Ala Pro Glu Leu Leu Gly
305                 310                 315                 320

Gly Pro Ser Val Phe Leu Phe Pro Pro Lys Pro Lys Asp Thr Leu Met
                325                 330                 335

Ile Ser Arg Thr Pro Glu Val Thr Cys Val Val Val Asp Val Ser His
            340                 345                 350

Glu Asp Pro Glu Val Lys Phe Asn Trp Tyr Val Asp Gly Val Glu Val
        355                 360                 365

His Asn Ala Lys Thr Lys Pro Arg Glu Glu Gln Tyr Asn Ser Thr Tyr
    370                 375                 380

Arg Val Val Ser Val Leu Thr Val Leu His Gln Asp Trp Leu Asn Gly
385                 390                 395                 400

Lys Glu Tyr Lys Cys Lys Val Ser Asn Lys Ala Leu Pro Ala Pro Ile
                405                 410                 415

Glu Lys Thr Ile Ser Lys Ala Lys Gly Gln Pro Arg Glu Pro Gln Val
            420                 425                 430

Tyr Thr Leu Pro Pro Ser Arg Glu Glu Met Thr Lys Asn Gln Val Ser
        435                 440                 445

Leu Thr Cys Leu Val Lys Gly Phe Tyr Pro Ser Asp Ile Ala Val Glu
    450                 455                 460

Trp Glu Ser Asn Gly Gln Pro Glu Asn Asn Tyr Lys Thr Thr Pro Pro
465                 470                 475                 480

Val Leu Asp Ser Asp Gly Ser Phe Phe Leu Tyr Ser Lys Leu Thr Val
                485                 490                 495

Asp Lys Ser Arg Trp Gln Glu Gly Asn Val Phe Ser Cys Ser Val Met
            500                 505                 510

His Glu Ala Leu His Asn His Tyr Thr Gln Lys Ser Leu Ser Leu Ser
        515                 520                 525

Pro

<210>  168
<211>  368
<212>  PRT
<213>  Artificial Sequence

<220>
<223>  an artificially synthesized sequence

<400>  168

Glu Val Gln Leu Val Glu Ser Gly Gly Gly Leu Val Gln Ala Gly Lys
1               5                   10                  15

Ser Leu Arg Leu Ser Cys Ala Ala Ser Gly Ser Ile Phe Ser Ile His
            20                  25                  30

Ala Met Gly Trp Phe Arg Gln Ala Pro Gly Lys Glu Arg Glu Phe Val
        35                  40                  45

Ala Ala Ile Thr Trp Ser Gly Gly Ile Thr Tyr Tyr Glu Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Ile Tyr Tyr Cys
                85                  90                  95

Ala Ala Asp Arg Ala Glu Ser Ser Trp Tyr Asp Tyr Trp Gly Gln Gly
            100                 105                 110

Thr Gln Val Thr Val Ser Ser Pro Lys Ser Ser Asp Lys Thr His Thr
        115                 120                 125

Cys Pro Pro Cys Pro Ala Pro Glu Leu Leu Gly Gly Pro Ser Val Phe
    130                 135                 140

Leu Phe Pro Pro Lys Pro Lys Asp Thr Leu Met Ile Ser Arg Thr Pro
145                 150                 155                 160

Glu Val Thr Cys Val Val Val Asp Val Ser His Glu Asp Pro Glu Val
                165                 170                 175

Lys Phe Asn Trp Tyr Val Asp Gly Val Glu Val His Asn Ala Lys Thr
            180                 185                 190

Lys Pro Arg Glu Glu Gln Tyr Asn Ser Thr Tyr Arg Val Val Ser Val
        195                 200                 205

Leu Thr Val Leu His Gln Asp Trp Leu Asn Gly Lys Glu Tyr Lys Cys
    210                 215                 220

Lys Val Ser Asn Lys Ala Leu Pro Ala Pro Ile Glu Lys Thr Ile Ser
225                 230                 235                 240

Lys Ala Lys Gly Gln Pro Arg Glu Pro Gln Val Tyr Thr Leu Pro Pro
                245                 250                 255

Ser Arg Glu Glu Met Thr Lys Asn Gln Val Ser Leu Thr Cys Leu Val
            260                 265                 270

Lys Gly Phe Tyr Pro Ser Asp Ile Ala Val Glu Trp Glu Ser Asn Gly
        275                 280                 285

Gln Pro Glu Asn Asn Tyr Lys Thr Thr Pro Pro Val Leu Asp Ser Asp
    290                 295                 300

Gly Ser Phe Phe Leu Tyr Ser Lys Leu Thr Val Asp Lys Ser Arg Trp
305                 310                 315                 320

Gln Gln Gly Asn Val Phe Ser Cys Ser Val Met His Glu Ala Leu His
                325                 330                 335

Asn His Tyr Thr Gln Lys Ser Leu Ser Leu Ser Pro Gly Gly Gly Gly
            340                 345                 350

Ser Gly Leu Asn Asp Ile Phe Glu Ala Gln Lys Ile Glu Trp His Glu
        355                 360                 365

<210>  169
<211>  368
<212>  PRT

<213>  Artificial Sequence

<220>
<223>  an artificially synthesized sequence

<400>  169

Glu Val Gln Leu Val Glu Ser Gly Gly Gly Leu Val Gln Ala Gly Gly
1               5                   10                  15

Ser Leu Arg Leu Ser Cys Ala Ala Ser Gly Ser Ile Ala Ser Ile His
            20                  25                  30

Ala Met Gly Trp Phe Arg Gln Ala Pro Gly Lys Glu Arg Glu Phe Val
        35                  40                  45

Ala Val Ile Thr Trp Ser Gly Gly Ile Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Ile Tyr Tyr Cys
                85                  90                  95

Ala Gly Asp Lys His Gln Ser Ser Trp Tyr Asp Tyr Trp Gly Gln Gly
            100                 105                 110

Thr Gln Val Thr Val Ser Ser Pro Lys Ser Ser Asp Lys Thr His Thr
        115                 120                 125

Cys Pro Pro Cys Pro Ala Pro Glu Leu Leu Gly Gly Pro Ser Val Phe
    130                 135                 140

Leu Phe Pro Pro Lys Pro Lys Asp Thr Leu Met Ile Ser Arg Thr Pro
145                 150                 155                 160

Glu Val Thr Cys Val Val Val Asp Val Ser His Glu Asp Pro Glu Val
                165                 170                 175

Lys Phe Asn Trp Tyr Val Asp Gly Val Glu Val His Asn Ala Lys Thr
            180                 185                 190

Lys Pro Arg Glu Glu Gln Tyr Asn Ser Thr Tyr Arg Val Val Ser Val
        195                 200                 205

Leu Thr Val Leu His Gln Asp Trp Leu Asn Gly Lys Glu Tyr Lys Cys
    210                 215                 220

Lys Val Ser Asn Lys Ala Leu Pro Ala Pro Ile Glu Lys Thr Ile Ser
225                 230                 235                 240

Lys Ala Lys Gly Gln Pro Arg Glu Pro Gln Val Tyr Thr Leu Pro Pro
                245                 250                 255

Ser Arg Glu Glu Met Thr Lys Asn Gln Val Ser Leu Thr Cys Leu Val
            260                 265                 270

Lys Gly Phe Tyr Pro Ser Asp Ile Ala Val Glu Trp Glu Ser Asn Gly
        275                 280                 285

Gln Pro Glu Asn Asn Tyr Lys Thr Thr Pro Pro Val Leu Asp Ser Asp
    290                 295                 300

Gly Ser Phe Phe Leu Tyr Ser Lys Leu Thr Val Asp Lys Ser Arg Trp
305                 310                 315                 320

Gln Gln Gly Asn Val Phe Ser Cys Ser Val Met His Glu Ala Leu His
                325                 330                 335

Asn His Tyr Thr Gln Lys Ser Leu Ser Leu Ser Pro Gly Gly Gly Gly
            340                 345                 350

Ser Gly Leu Asn Asp Ile Phe Glu Ala Gln Lys Ile Glu Trp His Glu
        355                 360                 365

<210>  170
<211>  12
<212>  PRT
<213>  Artificial Sequence

<220>
<223>  an artificially synthesized sequence

<400>  170

Thr Ser Gly Ser Gly Arg Ser Ala Asn Ala Arg Gly
1               5                   10

<210>  171
<211>  12
<212>  PRT
<213>  Artificial Sequence

<220>
<223>  an artificially synthesized sequence

<400>  171

Thr Ser Gln Ser Gly Arg Ser Ala Asn Gln Arg Gly
1               5                   10

<210>  172
<211>  12
<212>  PRT
<213>  Artificial Sequence

<220>
<223>  an artificially synthesized sequence

<400>  172

Thr Ser Pro Ser Gly Arg Ser Ala Tyr Pro Arg Gly
1               5                   10


<210>  173
<211>  12
<212>  PRT
<213>  Artificial Sequence

<220>
<223>  an artificially synthesized sequence

<400>  173

Thr Ser Gly Ser Gly Arg Ser Ala Thr Pro Arg Gly
1               5                   10


<210>  174
<211>  12
<212>  PRT
<213>  Artificial Sequence

<220>
<223>  an artificially synthesized sequence

<400>  174

Thr Ser Gln Ser Gly Arg Ser Ala Thr Pro Arg Gly
1               5                   10


<210>  175
<211>  12
<212>  PRT
<213>  Artificial Sequence

<220>
<223>  an artificially synthesized sequence

<400>  175

Thr Ser Ala Ser Gly Arg Ser Ala Thr Pro Arg Gly
1               5                   10


<210>  176
<211>  12
<212>  PRT
<213>  Artificial Sequence

<220>

<223>  an artificially synthesized sequence

<400>  176

Thr Ser Tyr Ser Gly Arg Ser Ala Val Pro Arg Gly
1               5                   10

<210>  177
<211>  12
<212>  PRT
<213>  Artificial Sequence

<220>
<223>  an artificially synthesized sequence

<400>  177

Thr Ser Tyr Ser Gly Arg Ser Ala Asn Phe Arg Gly
1               5                   10

<210>  178
<211>  12
<212>  PRT
<213>  Artificial Sequence

<220>
<223>  an artificially synthesized sequence

<400>  178

Thr Ser Ser Ser Gly Arg Ser Ala Thr Pro Arg Gly
1               5                   10

<210>  179
<211>  12
<212>  PRT
<213>  Artificial Sequence

<220>
<223>  an artificially synthesized sequence

<400>  179

Thr Ser Thr Thr Gly Arg Ser Ala Ser Pro Arg Gly
1               5                   10

<210>  180
<211>  12
<212>  PRT
<213>  Artificial Sequence

<220>
<223>  an artificially synthesized sequence

<400>  180

Thr Ser Ala Ser Gly Arg Ser Ala Asn Pro Arg Gly
1               5                   10

<210>  181
<211>  12
<212>  PRT
<213>  Artificial Sequence

<220>
<223>  an artificially synthesized sequence

<400>  181

Thr Ser Glu Ser Gly Arg Ser Ala Asn Pro Arg Gly
1               5                   10


<210>  182
<211>  12
<212>  PRT
<213>  Artificial Sequence

<220>
<223>  an artificially synthesized sequence

<400>  182

Thr Ser Phe Ser Gly Arg Ser Ala Asn Pro Arg Gly
1               5                   10


<210>  183
<211>  12
<212>  PRT
<213>  Artificial Sequence

<220>
<223>  an artificially synthesized sequence

<400>  183

Thr Ser Gly Ser Gly Arg Ser Ala Asn Pro Arg Gly
1               5                   10


<210>  184
<211>  12
<212>  PRT
<213>  Artificial Sequence

<220>
<223>  an artificially synthesized sequence

<400>  184

Thr Ser His Ser Gly Arg Ser Ala Asn Pro Arg Gly
1               5                   10


<210>  185
<211>  12
<212>  PRT
<213>  Artificial Sequence

<220>

<223>  an artificially synthesized sequence

<400>  185

Thr Ser Lys Ser Gly Arg Ser Ala Asn Pro Arg Gly
1               5                   10


<210>  186
<211>  12
<212>  PRT
<213>  Artificial Sequence

<220>
<223>  an artificially synthesized sequence

<400>  186

Thr Ser Met Ser Gly Arg Ser Ala Asn Pro Arg Gly
1               5                   10


<210>  187
<211>  12
<212>  PRT
<213>  Artificial Sequence

<220>
<223>  an artificially synthesized sequence

<400>  187

Thr Ser Asn Ser Gly Arg Ser Ala Asn Pro Arg Gly
1               5                   10


<210>  188
<211>  12
<212>  PRT
<213>  Artificial Sequence

<220>
<223>  an artificially synthesized sequence

<400>  188

Thr Ser Pro Ser Gly Arg Ser Ala Asn Pro Arg Gly
1               5                   10


<210>  189
<211>  12
<212>  PRT
<213>  Artificial Sequence

<220>
<223>  an artificially synthesized sequence

<400>  189

Thr Ser Gln Ser Gly Arg Ser Ala Asn Pro Arg Gly
1               5                   10

<210>  190
<211>  12
<212>  PRT
<213>  Artificial Sequence

<220>
<223>  an artificially synthesized sequence

<400>  190

Thr Ser Trp Ser Gly Arg Ser Ala Asn Pro Arg Gly
1               5                   10


<210>  191
<211>  12
<212>  PRT
<213>  Artificial Sequence

<220>
<223>  an artificially synthesized sequence

<400>  191

Thr Ser Tyr Ser Gly Arg Ser Ala Asn Pro Arg Gly
1               5                   10


<210>  192
<211>  12
<212>  PRT
<213>  Artificial Sequence

<220>
<223>  an artificially synthesized sequence

<400>  192

Thr Ser Thr Ala Gly Arg Ser Ala Asn Pro Arg Gly
1               5                   10


<210>  193
<211>  12
<212>  PRT
<213>  Artificial Sequence

<220>
<223>  an artificially synthesized sequence

<400>  193

Thr Ser Thr Asp Gly Arg Ser Ala Asn Pro Arg Gly
1               5                   10


<210>  194
<211>  12
<212>  PRT
<213>  Artificial Sequence

<220>

<223>  an artificially synthesized sequence

<400>  194

Thr Ser Thr Glu Gly Arg Ser Ala Asn Pro Arg Gly
1               5                   10


<210>  195
<211>  12
<212>  PRT
<213>  Artificial Sequence

<220>
<223>  an artificially synthesized sequence

<400>  195

Thr Ser Thr Phe Gly Arg Ser Ala Asn Pro Arg Gly
1               5                   10


<210>  196
<211>  12
<212>  PRT
<213>  Artificial Sequence

<220>
<223>  an artificially synthesized sequence

<400>  196

Thr Ser Thr Leu Gly Arg Ser Ala Asn Pro Arg Gly
1               5                   10


<210>  197
<211>  12
<212>  PRT
<213>  Artificial Sequence

<220>
<223>  an artificially synthesized sequence

<400>  197

Thr Ser Thr Met Gly Arg Ser Ala Asn Pro Arg Gly
1               5                   10


<210>  198
<211>  12
<212>  PRT
<213>  Artificial Sequence

<220>
<223>  an artificially synthesized sequence

<400>  198

Thr Ser Thr Pro Gly Arg Ser Ala Asn Pro Arg Gly
1               5                   10

<210>  199
<211>  12
<212>  PRT
<213>  Artificial Sequence

<220>
<223>  an artificially synthesized sequence

<400>  199

Thr Ser Thr Gln Gly Arg Ser Ala Asn Pro Arg Gly
1               5                   10


<210>  200
<211>  12
<212>  PRT
<213>  Artificial Sequence

<220>
<223>  an artificially synthesized sequence

<400>  200

Thr Ser Thr Val Gly Arg Ser Ala Asn Pro Arg Gly
1               5                   10


<210>  201
<211>  12
<212>  PRT
<213>  Artificial Sequence

<220>
<223>  an artificially synthesized sequence

<400>  201

Thr Ser Thr Trp Gly Arg Ser Ala Asn Pro Arg Gly
1               5                   10


<210>  202
<211>  12
<212>  PRT
<213>  Artificial Sequence

<220>
<223>  an artificially synthesized sequence

<400>  202

Thr Ser Thr Ser Ala Arg Ser Ala Asn Pro Arg Gly
1               5                   10


<210>  203
<211>  12
<212>  PRT
<213>  Artificial Sequence

<220>

<223>  an artificially synthesized sequence

<400>  203

Thr Ser Thr Ser Glu Arg Ser Ala Asn Pro Arg Gly
1               5                   10


<210>  204
<211>  12
<212>  PRT
<213>  Artificial Sequence

<220>
<223>  an artificially synthesized sequence

<400>  204

Thr Ser Thr Ser Phe Arg Ser Ala Asn Pro Arg Gly
1               5                   10


<210>  205
<211>  12
<212>  PRT
<213>  Artificial Sequence

<220>
<223>  an artificially synthesized sequence

<400>  205

Thr Ser Thr Ser His Arg Ser Ala Asn Pro Arg Gly
1               5                   10


<210>  206
<211>  12
<212>  PRT
<213>  Artificial Sequence

<220>
<223>  an artificially synthesized sequence

<400>  206

Thr Ser Thr Ser Ile Arg Ser Ala Asn Pro Arg Gly
1               5                   10


<210>  207
<211>  12
<212>  PRT
<213>  Artificial Sequence

<220>
<223>  an artificially synthesized sequence

<400>  207

Thr Ser Thr Ser Lys Arg Ser Ala Asn Pro Arg Gly
1               5                   10

<210>  208
<211>  12
<212>  PRT
<213>  Artificial Sequence

<220>
<223>  an artificially synthesized sequence

<400>  208

Thr Ser Thr Ser Leu Arg Ser Ala Asn Pro Arg Gly
1               5                   10


<210>  209
<211>  12
<212>  PRT
<213>  Artificial Sequence

<220>
<223>  an artificially synthesized sequence

<400>  209

Thr Ser Thr Ser Met Arg Ser Ala Asn Pro Arg Gly
1               5                   10


<210>  210
<211>  12
<212>  PRT
<213>  Artificial Sequence

<220>
<223>  an artificially synthesized sequence

<400>  210

Thr Ser Thr Ser Asn Arg Ser Ala Asn Pro Arg Gly
1               5                   10


<210>  211
<211>  12
<212>  PRT
<213>  Artificial Sequence

<220>
<223>  an artificially synthesized sequence

<400>  211

Thr Ser Thr Ser Pro Arg Ser Ala Asn Pro Arg Gly
1               5                   10


<210>  212
<211>  12
<212>  PRT
<213>  Artificial Sequence

<220>

<223>  an artificially synthesized sequence

<400>  212

Thr Ser Thr Ser Gln Arg Ser Ala Asn Pro Arg Gly
1               5                   10


<210>  213
<211>  12
<212>  PRT
<213>  Artificial Sequence

<220>
<223>  an artificially synthesized sequence

<400>  213

Thr Ser Thr Ser Arg Arg Ser Ala Asn Pro Arg Gly
1               5                   10


<210>  214
<211>  12
<212>  PRT
<213>  Artificial Sequence

<220>
<223>  an artificially synthesized sequence

<400>  214

Thr Ser Thr Ser Thr Arg Ser Ala Asn Pro Arg Gly
1               5                   10


<210>  215
<211>  12
<212>  PRT
<213>  Artificial Sequence

<220>
<223>  an artificially synthesized sequence

<400>  215

Thr Ser Thr Ser Val Arg Ser Ala Asn Pro Arg Gly
1               5                   10


<210>  216
<211>  12
<212>  PRT
<213>  Artificial Sequence

<220>
<223>  an artificially synthesized sequence

<400>  216

Thr Ser Thr Ser Trp Arg Ser Ala Asn Pro Arg Gly
1               5                   10

<210>  217
<211>  12
<212>  PRT
<213>  Artificial Sequence

<220>
<223>  an artificially synthesized sequence

<400>  217

Thr Ser Thr Ser Tyr Arg Ser Ala Asn Pro Arg Gly
1               5                   10


<210>  218
<211>  12
<212>  PRT
<213>  Artificial Sequence

<220>
<223>  an artificially synthesized sequence

<400>  218

Thr Ser Thr Ser Gly Arg Ala Ala Asn Pro Arg Gly
1               5                   10


<210>  219
<211>  12
<212>  PRT
<213>  Artificial Sequence

<220>
<223>  an artificially synthesized sequence

<400>  219

Thr Ser Thr Ser Gly Arg Asp Ala Asn Pro Arg Gly
1               5                   10


<210>  220
<211>  12
<212>  PRT
<213>  Artificial Sequence

<220>
<223>  an artificially synthesized sequence

<400>  220

Thr Ser Thr Ser Gly Arg Glu Ala Asn Pro Arg Gly
1               5                   10


<210>  221
<211>  12
<212>  PRT
<213>  Artificial Sequence

<220>

<223>  an artificially synthesized sequence

<400>  221

Thr Ser Thr Ser Gly Arg Gly Ala Asn Pro Arg Gly
1               5                   10


<210>  222
<211>  12
<212>  PRT
<213>  Artificial Sequence

<220>
<223>  an artificially synthesized sequence

<400>  222

Thr Ser Thr Ser Gly Arg His Ala Asn Pro Arg Gly
1               5                   10


<210>  223
<211>  12
<212>  PRT
<213>  Artificial Sequence

<220>
<223>  an artificially synthesized sequence

<400>  223

Thr Ser Thr Ser Gly Arg Ile Ala Asn Pro Arg Gly
1               5                   10


<210>  224
<211>  12
<212>  PRT
<213>  Artificial Sequence

<220>
<223>  an artificially synthesized sequence

<400>  224

Thr Ser Thr Ser Gly Arg Lys Ala Asn Pro Arg Gly
1               5                   10


<210>  225
<211>  12
<212>  PRT
<213>  Artificial Sequence

<220>
<223>  an artificially synthesized sequence

<400>  225

Thr Ser Thr Ser Gly Arg Leu Ala Asn Pro Arg Gly
1               5                   10

<210>  226
<211>  12
<212>  PRT
<213>  Artificial Sequence

<220>
<223>  an artificially synthesized sequence

<400>  226

Thr Ser Thr Ser Gly Arg Met Ala Asn Pro Arg Gly
1               5                   10


<210>  227
<211>  12
<212>  PRT
<213>  Artificial Sequence

<220>
<223>  an artificially synthesized sequence

<400>  227

Thr Ser Thr Ser Gly Arg Asn Ala Asn Pro Arg Gly
1               5                   10


<210>  228
<211>  12
<212>  PRT
<213>  Artificial Sequence

<220>
<223>  an artificially synthesized sequence

<400>  228

Thr Ser Thr Ser Gly Arg Pro Ala Asn Pro Arg Gly
1               5                   10


<210>  229
<211>  12
<212>  PRT
<213>  Artificial Sequence

<220>
<223>  an artificially synthesized sequence

<400>  229

Thr Ser Thr Ser Gly Arg Gln Ala Asn Pro Arg Gly
1               5                   10


<210>  230
<211>  12
<212>  PRT
<213>  Artificial Sequence

<220>

<223>  an artificially synthesized sequence

<400>  230

Thr Ser Thr Ser Gly Arg Arg Ala Asn Pro Arg Gly
1               5                   10


<210>  231
<211>  12
<212>  PRT
<213>  Artificial Sequence

<220>
<223>  an artificially synthesized sequence

<400>  231

Thr Ser Thr Ser Gly Arg Thr Ala Asn Pro Arg Gly
1               5                   10


<210>  232
<211>  12
<212>  PRT
<213>  Artificial Sequence

<220>
<223>  an artificially synthesized sequence

<400>  232

Thr Ser Thr Ser Gly Arg Val Ala Asn Pro Arg Gly
1               5                   10


<210>  233
<211>  12
<212>  PRT
<213>  Artificial Sequence

<220>
<223>  an artificially synthesized sequence

<400>  233

Thr Ser Thr Ser Gly Arg Trp Ala Asn Pro Arg Gly
1               5                   10


<210>  234
<211>  12
<212>  PRT
<213>  Artificial Sequence

<220>
<223>  an artificially synthesized sequence

<400>  234

Thr Ser Thr Ser Gly Arg Tyr Ala Asn Pro Arg Gly
1               5                   10

<210>  235
<211>  12
<212>  PRT
<213>  Artificial Sequence

<220>
<223>  an artificially synthesized sequence

<400>  235

Thr Ser Thr Ser Gly Arg Ser Glu Asn Pro Arg Gly
1               5                   10


<210>  236
<211>  12
<212>  PRT
<213>  Artificial Sequence

<220>
<223>  an artificially synthesized sequence

<400>  236

Thr Ser Thr Ser Gly Arg Ser Phe Asn Pro Arg Gly
1               5                   10


<210>  237
<211>  12
<212>  PRT
<213>  Artificial Sequence

<220>
<223>  an artificially synthesized sequence

<400>  237

Thr Ser Thr Ser Gly Arg Ser Lys Asn Pro Arg Gly
1               5                   10


<210>  238
<211>  12
<212>  PRT
<213>  Artificial Sequence

<220>
<223>  an artificially synthesized sequence

<400>  238

Thr Ser Thr Ser Gly Arg Ser Met Asn Pro Arg Gly
1               5                   10


<210>  239
<211>  12
<212>  PRT
<213>  Artificial Sequence

<220>

<223>  an artificially synthesized sequence

<400>  239

Thr Ser Thr Ser Gly Arg Ser Asn Asn Pro Arg Gly
1               5                   10


<210>  240
<211>  12
<212>  PRT
<213>  Artificial Sequence

<220>
<223>  an artificially synthesized sequence

<400>  240

Thr Ser Thr Ser Gly Arg Ser Pro Asn Pro Arg Gly
1               5                   10


<210>  241
<211>  12
<212>  PRT
<213>  Artificial Sequence

<220>
<223>  an artificially synthesized sequence

<400>  241

Thr Ser Thr Ser Gly Arg Ser Gln Asn Pro Arg Gly
1               5                   10


<210>  242
<211>  12
<212>  PRT
<213>  Artificial Sequence

<220>
<223>  an artificially synthesized sequence

<400>  242

Thr Ser Thr Ser Gly Arg Ser Arg Asn Pro Arg Gly
1               5                   10


<210>  243
<211>  12
<212>  PRT
<213>  Artificial Sequence

<220>
<223>  an artificially synthesized sequence

<400>  243

Thr Ser Thr Ser Gly Arg Ser Ser Asn Pro Arg Gly
1               5                   10

<210>  244
<211>  12
<212>  PRT
<213>  Artificial Sequence

<220>
<223>  an artificially synthesized sequence

<400>  244

Thr Ser Thr Ser Gly Arg Ser Trp Asn Pro Arg Gly
1               5                   10


<210>  245
<211>  12
<212>  PRT
<213>  Artificial Sequence

<220>
<223>  an artificially synthesized sequence

<400>  245

Thr Ser Thr Ser Gly Arg Ser Tyr Asn Pro Arg Gly
1               5                   10


<210>  246
<211>  12
<212>  PRT
<213>  Artificial Sequence

<220>
<223>  an artificially synthesized sequence

<400>  246

Thr Ser Thr Ser Gly Arg Ser Ala Ala Pro Arg Gly
1               5                   10


<210>  247
<211>  12
<212>  PRT
<213>  Artificial Sequence

<220>
<223>  an artificially synthesized sequence

<400>  247

Thr Ser Thr Ser Gly Arg Ser Ala Asp Pro Arg Gly
1               5                   10


<210>  248
<211>  12
<212>  PRT
<213>  Artificial Sequence

<220>

<223>  an artificially synthesized sequence

<400>  248

Thr Ser Thr Ser Gly Arg Ser Ala Glu Pro Arg Gly
1               5                   10


<210>  249
<211>  12
<212>  PRT
<213>  Artificial Sequence

<220>
<223>  an artificially synthesized sequence

<400>  249

Thr Ser Thr Ser Gly Arg Ser Ala Phe Pro Arg Gly
1               5                   10


<210>  250
<211>  12
<212>  PRT
<213>  Artificial Sequence

<220>
<223>  an artificially synthesized sequence

<400>  250

Thr Ser Thr Ser Gly Arg Ser Ala Gly Pro Arg Gly
1               5                   10


<210>  251
<211>  12
<212>  PRT
<213>  Artificial Sequence

<220>
<223>  an artificially synthesized sequence

<400>  251

Thr Ser Thr Ser Gly Arg Ser Ala Lys Pro Arg Gly
1               5                   10


<210>  252
<211>  12
<212>  PRT
<213>  Artificial Sequence

<220>
<223>  an artificially synthesized sequence

<400>  252

Thr Ser Thr Ser Gly Arg Ser Ala Leu Pro Arg Gly
1               5                   10

<210>  253
<211>  12
<212>  PRT
<213>  Artificial Sequence

<220>
<223>  an artificially synthesized sequence

<400>  253

Thr Ser Thr Ser Gly Arg Ser Ala Met Pro Arg Gly
1               5                   10


<210>  254
<211>  12
<212>  PRT
<213>  Artificial Sequence

<220>
<223>  an artificially synthesized sequence

<400>  254

Thr Ser Thr Ser Gly Arg Ser Ala Pro Pro Arg Gly
1               5                   10


<210>  255
<211>  12
<212>  PRT
<213>  Artificial Sequence

<220>
<223>  an artificially synthesized sequence

<400>  255

Thr Ser Thr Ser Gly Arg Ser Ala Gln Pro Arg Gly
1               5                   10


<210>  256
<211>  12
<212>  PRT
<213>  Artificial Sequence

<220>
<223>  an artificially synthesized sequence

<400>  256

Thr Ser Thr Ser Gly Arg Ser Ala Val Pro Arg Gly
1               5                   10


<210>  257
<211>  12
<212>  PRT
<213>  Artificial Sequence

<220>

<223>  an artificially synthesized sequence

<400>  257

Thr Ser Thr Ser Gly Arg Ser Ala Trp Pro Arg Gly
1               5                   10


<210>  258
<211>  12
<212>  PRT
<213>  Artificial Sequence

<220>
<223>  an artificially synthesized sequence

<400>  258

Thr Ser Thr Ser Gly Arg Ser Ala Tyr Pro Arg Gly
1               5                   10


<210>  259
<211>  12
<212>  PRT
<213>  Artificial Sequence

<220>
<223>  an artificially synthesized sequence

<400>  259

Thr Ser Thr Ser Gly Arg Ser Ala Asn Ala Arg Gly
1               5                   10


<210>  260
<211>  12
<212>  PRT
<213>  Artificial Sequence

<220>
<223>  an artificially synthesized sequence

<400>  260

Thr Ser Thr Ser Gly Arg Ser Ala Asn Asp Arg Gly
1               5                   10


<210>  261
<211>  12
<212>  PRT
<213>  Artificial Sequence

<220>
<223>  an artificially synthesized sequence

<400>  261

Thr Ser Thr Ser Gly Arg Ser Ala Asn Glu Arg Gly
1               5                   10

<210>  262
<211>  12
<212>  PRT
<213>  Artificial Sequence

<220>
<223>  an artificially synthesized sequence

<400>  262

Thr Ser Thr Ser Gly Arg Ser Ala Asn Phe Arg Gly
1               5                   10


<210>  263
<211>  12
<212>  PRT
<213>  Artificial Sequence

<220>
<223>  an artificially synthesized sequence

<400>  263

Thr Ser Thr Ser Gly Arg Ser Ala Asn Gly Arg Gly
1               5                   10


<210>  264
<211>  12
<212>  PRT
<213>  Artificial Sequence

<220>
<223>  an artificially synthesized sequence

<400>  264

Thr Ser Thr Ser Gly Arg Ser Ala Asn Ile Arg Gly
1               5                   10


<210>  265
<211>  12
<212>  PRT
<213>  Artificial Sequence

<220>
<223>  an artificially synthesized sequence

<400>  265

Thr Ser Thr Ser Gly Arg Ser Ala Asn Lys Arg Gly
1               5                   10


<210>  266
<211>  12
<212>  PRT
<213>  Artificial Sequence

<220>

<223>  an artificially synthesized sequence

<400>  266

Thr Ser Thr Ser Gly Arg Ser Ala Asn Asn Arg Gly
1               5                   10


<210>  267
<211>  12
<212>  PRT
<213>  Artificial Sequence

<220>
<223>  an artificially synthesized sequence

<400>  267

Thr Ser Thr Ser Gly Arg Ser Ala Asn Gln Arg Gly
1               5                   10


<210>  268
<211>  12
<212>  PRT
<213>  Artificial Sequence

<220>
<223>  an artificially synthesized sequence

<400>  268

Thr Ser Thr Ser Gly Arg Ser Ala Asn Ser Arg Gly
1               5                   10


<210>  269
<211>  12
<212>  PRT
<213>  Artificial Sequence

<220>
<223>  an artificially synthesized sequence

<400>  269

Thr Ser Thr Ser Gly Arg Ser Ala Asn Thr Arg Gly
1               5                   10


<210>  270
<211>  12
<212>  PRT
<213>  Artificial Sequence

<220>
<223>  an artificially synthesized sequence

<400>  270

Thr Ser Thr Ser Gly Arg Ser Ala Asn Trp Arg Gly
1               5                   10

<210>  271
<211>  12
<212>  PRT
<213>  Artificial Sequence

<220>
<223>  an artificially synthesized sequence

<400>  271

Thr Ser Asp Ser Gly Arg Ser Ala Asn Pro Arg Gly
1               5                   10


<210>  272
<211>  12
<212>  PRT
<213>  Artificial Sequence

<220>
<223>  an artificially synthesized sequence

<400>  272

Thr Ser Ile Ser Gly Arg Ser Ala Asn Pro Arg Gly
1               5                   10


<210>  273
<211>  12
<212>  PRT
<213>  Artificial Sequence

<220>
<223>  an artificially synthesized sequence

<400>  273

Thr Ser Ser Ser Gly Arg Ser Ala Asn Pro Arg Gly
1               5                   10


<210>  274
<211>  12
<212>  PRT
<213>  Artificial Sequence

<220>
<223>  an artificially synthesized sequence

<400>  274

Thr Ser Thr His Gly Arg Ser Ala Asn Pro Arg Gly
1               5                   10


<210>  275
<211>  12
<212>  PRT
<213>  Artificial Sequence

<220>

<223>  an artificially synthesized sequence

<400>  275

Thr Ser Thr Lys Gly Arg Ser Ala Asn Pro Arg Gly
1               5                   10


<210>  276
<211>  12
<212>  PRT
<213>  Artificial Sequence

<220>
<223>  an artificially synthesized sequence

<400>  276

Thr Ser Thr Thr Gly Arg Ser Ala Asn Pro Arg Gly
1               5                   10


<210>  277
<211>  12
<212>  PRT
<213>  Artificial Sequence

<220>
<223>  an artificially synthesized sequence

<400>  277

Thr Ser Thr Tyr Gly Arg Ser Ala Asn Pro Arg Gly
1               5                   10


<210>  278
<211>  12
<212>  PRT
<213>  Artificial Sequence

<220>
<223>  an artificially synthesized sequence

<400>  278

Thr Ser Thr Ser Asp Arg Ser Ala Asn Pro Arg Gly
1               5                   10


<210>  279
<211>  12
<212>  PRT
<213>  Artificial Sequence

<220>
<223>  an artificially synthesized sequence

<400>  279

Thr Ser Thr Ser Ser Arg Ser Ala Asn Pro Arg Gly
1               5                   10

<210>  280
<211>  12
<212>  PRT
<213>  Artificial Sequence

<220>
<223>  an artificially synthesized sequence

<400>  280

Thr Ser Thr Ser Gly Arg Phe Ala Asn Pro Arg Gly
1               5                   10


<210>  281
<211>  12
<212>  PRT
<213>  Artificial Sequence

<220>
<223>  an artificially synthesized sequence

<400>  281

Thr Ser Thr Ser Gly Arg Ser Asp Asn Pro Arg Gly
1               5                   10


<210>  282
<211>  12
<212>  PRT
<213>  Artificial Sequence

<220>
<223>  an artificially synthesized sequence

<400>  282

Thr Ser Thr Ser Gly Arg Ser His Asn Pro Arg Gly
1               5                   10


<210>  283
<211>  12
<212>  PRT
<213>  Artificial Sequence

<220>
<223>  an artificially synthesized sequence

<400>  283

Thr Ser Thr Ser Gly Arg Ser Ile Asn Pro Arg Gly
1               5                   10


<210>  284
<211>  12
<212>  PRT
<213>  Artificial Sequence

<220>

<223>  an artificially synthesized sequence

<400>  284

Thr Ser Thr Ser Gly Arg Ser Leu Asn Pro Arg Gly
1               5                   10


<210>  285
<211>  12
<212>  PRT
<213>  Artificial Sequence

<220>
<223>  an artificially synthesized sequence

<400>  285

Thr Ser Thr Ser Gly Arg Ser Thr Asn Pro Arg Gly
1               5                   10


<210>  286
<211>  12
<212>  PRT
<213>  Artificial Sequence

<220>
<223>  an artificially synthesized sequence

<400>  286

Thr Ser Thr Ser Gly Arg Ser Val Asn Pro Arg Gly
1               5                   10


<210>  287
<211>  12
<212>  PRT
<213>  Artificial Sequence

<220>
<223>  an artificially synthesized sequence

<400>  287

Thr Ser Thr Ser Gly Arg Ser Ala His Pro Arg Gly
1               5                   10


<210>  288
<211>  12
<212>  PRT
<213>  Artificial Sequence

<220>
<223>  an artificially synthesized sequence

<400>  288

Thr Ser Thr Ser Gly Arg Ser Ala Ile Pro Arg Gly
1               5                   10

<210>  289
<211>  12
<212>  PRT
<213>  Artificial Sequence

<220>
<223>  an artificially synthesized sequence

<400>  289

Thr Ser Thr Ser Gly Arg Ser Ala Arg Pro Arg Gly
1               5                   10


<210>  290
<211>  12
<212>  PRT
<213>  Artificial Sequence

<220>
<223>  an artificially synthesized sequence

<400>  290

Thr Ser Thr Ser Gly Arg Ser Ala Ser Pro Arg Gly
1               5                   10


<210>  291
<211>  12
<212>  PRT
<213>  Artificial Sequence

<220>
<223>  an artificially synthesized sequence

<400>  291

Thr Ser Thr Ser Gly Arg Ser Ala Thr Pro Arg Gly
1               5                   10


<210>  292
<211>  12
<212>  PRT
<213>  Artificial Sequence

<220>
<223>  an artificially synthesized sequence

<400>  292

Thr Ser Thr Ser Gly Arg Ser Ala Asn His Arg Gly
1               5                   10


<210>  293
<211>  12
<212>  PRT
<213>  Artificial Sequence

<220>

<223>  an artificially synthesized sequence

<400>  293

Thr Ser Thr Ser Gly Arg Ser Ala Asn Leu Arg Gly
1               5                   10


<210>  294
<211>  12
<212>  PRT
<213>  Artificial Sequence

<220>
<223>  an artificially synthesized sequence

<400>  294

Thr Ser Thr Ser Gly Arg Ser Ala Asn Met Arg Gly
1               5                   10


<210>  295
<211>  12
<212>  PRT
<213>  Artificial Sequence

<220>
<223>  an artificially synthesized sequence

<400>  295

Thr Ser Thr Ser Gly Arg Ser Ala Asn Arg Arg Gly
1               5                   10


<210>  296
<211>  12
<212>  PRT
<213>  Artificial Sequence

<220>
<223>  an artificially synthesized sequence

<400>  296

Thr Ser Thr Ser Gly Arg Ser Ala Asn Val Arg Gly
1               5                   10


<210>  297
<211>  12
<212>  PRT
<213>  Artificial Sequence

<220>
<223>  an artificially synthesized sequence

<400>  297

Thr Ser Thr Ser Gly Arg Ser Ala Asn Tyr Arg Gly
1               5                   10

<210>  298
<211>  12
<212>  PRT
<213>  Artificial Sequence

<220>
<223>  an artificially synthesized sequence

<400>  298

Thr Ser Gly Ser Gly Arg Ser Ala Val Pro Arg Gly
1               5                   10


<210>  299
<211>  12
<212>  PRT
<213>  Artificial Sequence

<220>
<223>  an artificially synthesized sequence

<400>  299

Thr Ser Gly Ser Gly Arg Ser Ala Tyr Pro Arg Gly
1               5                   10


<210>  300
<211>  12
<212>  PRT
<213>  Artificial Sequence

<220>
<223>  an artificially synthesized sequence

<400>  300

Thr Ser Gly Ser Gly Arg Ser Ala Asn Gln Arg Gly
1               5                   10


<210>  301
<211>  12
<212>  PRT
<213>  Artificial Sequence

<220>
<223>  an artificially synthesized sequence

<400>  301

Thr Ser Gly Ser Gly Arg Ser Ala Asn Ile Arg Gly
1               5                   10


<210>  302
<211>  12
<212>  PRT
<213>  Artificial Sequence

<220>

<223>  an artificially synthesized sequence

<400>  302

Thr Ser Gly Ser Gly Arg Ser Ala Asn Phe Arg Gly
1               5                   10


<210>  303
<211>  12
<212>  PRT
<213>  Artificial Sequence

<220>
<223>  an artificially synthesized sequence

<400>  303

Thr Ser Gly Ser Gly Arg Ser Ala Asn Ser Arg Gly
1               5                   10


<210>  304
<211>  12
<212>  PRT
<213>  Artificial Sequence

<220>
<223>  an artificially synthesized sequence

<400>  304

Thr Ser Gln Ser Gly Arg Ser Ala Val Pro Arg Gly
1               5                   10


<210>  305
<211>  12
<212>  PRT
<213>  Artificial Sequence

<220>
<223>  an artificially synthesized sequence

<400>  305

Thr Ser Gln Ser Gly Arg Ser Ala Tyr Pro Arg Gly
1               5                   10


<210>  306
<211>  12
<212>  PRT
<213>  Artificial Sequence

<220>
<223>  an artificially synthesized sequence

<400>  306

Thr Ser Gln Ser Gly Arg Ser Ala Asn Ala Arg Gly
1               5                   10

<210>  307
<211>  12
<212>  PRT
<213>  Artificial Sequence

<220>
<223>  an artificially synthesized sequence

<400>  307

Thr Ser Gln Ser Gly Arg Ser Ala Asn Ile Arg Gly
1               5                   10


<210>  308
<211>  12
<212>  PRT
<213>  Artificial Sequence

<220>
<223>  an artificially synthesized sequence

<400>  308

Thr Ser Gln Ser Gly Arg Ser Ala Asn Phe Arg Gly
1               5                   10


<210>  309
<211>  12
<212>  PRT
<213>  Artificial Sequence

<220>
<223>  an artificially synthesized sequence

<400>  309

Thr Ser Gln Ser Gly Arg Ser Ala Asn Ser Arg Gly
1               5                   10


<210>  310
<211>  12
<212>  PRT
<213>  Artificial Sequence

<220>
<223>  an artificially synthesized sequence

<400>  310

Thr Ser Pro Ser Gly Arg Ser Ala Val Pro Arg Gly
1               5                   10


<210>  311
<211>  12
<212>  PRT
<213>  Artificial Sequence

<220>

<223>  an artificially synthesized sequence

<400>  311

Thr Ser Pro Ser Gly Arg Ser Ala Asn Gln Arg Gly
1               5                   10


<210>  312
<211>  12
<212>  PRT
<213>  Artificial Sequence

<220>
<223>  an artificially synthesized sequence

<400>  312

Thr Ser Pro Ser Gly Arg Ser Ala Asn Ala Arg Gly
1               5                   10


<210>  313
<211>  12
<212>  PRT
<213>  Artificial Sequence

<220>
<223>  an artificially synthesized sequence

<400>  313

Thr Ser Pro Ser Gly Arg Ser Ala Asn Ile Arg Gly
1               5                   10


<210>  314
<211>  12
<212>  PRT
<213>  Artificial Sequence

<220>
<223>  an artificially synthesized sequence

<400>  314

Thr Ser Pro Ser Gly Arg Ser Ala Asn Phe Arg Gly
1               5                   10


<210>  315
<211>  12
<212>  PRT
<213>  Artificial Sequence

<220>
<223>  an artificially synthesized sequence

<400>  315

Thr Ser Pro Ser Gly Arg Ser Ala Asn Ser Arg Gly
1               5                   10

<210>  316
<211>  12
<212>  PRT
<213>  Artificial Sequence

<220>
<223>  an artificially synthesized sequence

<400>  316

Thr Ser Ala Ser Gly Arg Ser Ala Val Pro Arg Gly
1               5                   10


<210>  317
<211>  12
<212>  PRT
<213>  Artificial Sequence

<220>
<223>  an artificially synthesized sequence

<400>  317

Thr Ser Ala Ser Gly Arg Ser Ala Tyr Pro Arg Gly
1               5                   10


<210>  318
<211>  12
<212>  PRT
<213>  Artificial Sequence

<220>
<223>  an artificially synthesized sequence

<400>  318

Thr Ser Ala Ser Gly Arg Ser Ala Asn Gln Arg Gly
1               5                   10


<210>  319
<211>  12
<212>  PRT
<213>  Artificial Sequence

<220>
<223>  an artificially synthesized sequence

<400>  319

Thr Ser Ala Ser Gly Arg Ser Ala Asn Ala Arg Gly
1               5                   10


<210>  320
<211>  12
<212>  PRT
<213>  Artificial Sequence

<220>

<223>  an artificially synthesized sequence

<400>  320

Thr Ser Ala Ser Gly Arg Ser Ala Asn Ile Arg Gly
1               5                   10


<210>  321
<211>  12
<212>  PRT
<213>  Artificial Sequence

<220>
<223>  an artificially synthesized sequence

<400>  321

Thr Ser Ala Ser Gly Arg Ser Ala Asn Phe Arg Gly
1               5                   10


<210>  322
<211>  12
<212>  PRT
<213>  Artificial Sequence

<220>
<223>  an artificially synthesized sequence

<400>  322

Thr Ser Ala Ser Gly Arg Ser Ala Asn Ser Arg Gly
1               5                   10


<210>  323
<211>  12
<212>  PRT
<213>  Artificial Sequence

<220>
<223>  an artificially synthesized sequence

<400>  323

Thr Ser Tyr Ser Gly Arg Ser Glu Asn Pro Arg Gly
1               5                   10


<210>  324
<211>  12
<212>  PRT
<213>  Artificial Sequence

<220>
<223>  an artificially synthesized sequence

<400>  324

Thr Ser Gly Ser Gly Arg Ser Glu Asn Pro Arg Gly
1               5                   10

<210>  325
<211>  12
<212>  PRT
<213>  Artificial Sequence

<220>
<223>  an artificially synthesized sequence

<400>  325

Thr Ser Gln Ser Gly Arg Ser Glu Asn Pro Arg Gly
1               5                   10


<210>  326
<211>  12
<212>  PRT
<213>  Artificial Sequence

<220>
<223>  an artificially synthesized sequence

<400>  326

Thr Ser Pro Ser Gly Arg Ser Glu Asn Pro Arg Gly
1               5                   10


<210>  327
<211>  12
<212>  PRT
<213>  Artificial Sequence

<220>
<223>  an artificially synthesized sequence

<400>  327

Thr Ser Ala Ser Gly Arg Ser Glu Asn Pro Arg Gly
1               5                   10


<210>  328
<211>  12
<212>  PRT
<213>  Artificial Sequence

<220>
<223>  an artificially synthesized sequence

<400>  328

Thr Ser His Ser Gly Arg Ser Glu Asn Pro Arg Gly
1               5                   10


<210>  329
<211>  12
<212>  PRT
<213>  Artificial Sequence

<220>

<223>  an artificially synthesized sequence

<400>  329

Thr Ser Thr Ser Gly Arg Ser Glu Asn Gln Arg Gly
1               5                   10


<210>  330
<211>  12
<212>  PRT
<213>  Artificial Sequence

<220>
<223>  an artificially synthesized sequence

<400>  330

Thr Ser Thr Ser Gly Arg Ser Glu Asn Ala Arg Gly
1               5                   10


<210>  331
<211>  12
<212>  PRT
<213>  Artificial Sequence

<220>
<223>  an artificially synthesized sequence

<400>  331

Thr Ser Thr Ser Gly Arg Ser Glu Asn Ile Arg Gly
1               5                   10


<210>  332
<211>  12
<212>  PRT
<213>  Artificial Sequence

<220>
<223>  an artificially synthesized sequence

<400>  332

Thr Ser Thr Ser Gly Arg Ser Glu Asn Phe Arg Gly
1               5                   10


<210>  333
<211>  12
<212>  PRT
<213>  Artificial Sequence

<220>
<223>  an artificially synthesized sequence

<400>  333

Thr Ser Thr Ser Gly Arg Ser Glu Asn Ser Arg Gly
1               5                   10

<210>  334
<211>  12
<212>  PRT
<213>  Artificial Sequence

<220>
<223>  an artificially synthesized sequence

<400>  334

Thr Ser Tyr Ser Gly Arg Ser Ala Glu Pro Arg Gly
1               5                   10


<210>  335
<211>  12
<212>  PRT
<213>  Artificial Sequence

<220>
<223>  an artificially synthesized sequence

<400>  335

Thr Ser Gly Ser Gly Arg Ser Ala Glu Pro Arg Gly
1               5                   10


<210>  336
<211>  12
<212>  PRT
<213>  Artificial Sequence

<220>
<223>  an artificially synthesized sequence

<400>  336

Thr Ser Gln Ser Gly Arg Ser Ala Glu Pro Arg Gly
1               5                   10


<210>  337
<211>  12
<212>  PRT
<213>  Artificial Sequence

<220>
<223>  an artificially synthesized sequence

<400>  337

Thr Ser Pro Ser Gly Arg Ser Ala Glu Pro Arg Gly
1               5                   10


<210>  338
<211>  12
<212>  PRT
<213>  Artificial Sequence

<220>

<223>  an artificially synthesized sequence

<400>  338

Thr Ser Ala Ser Gly Arg Ser Ala Glu Pro Arg Gly
1               5                   10


<210>  339
<211>  12
<212>  PRT
<213>  Artificial Sequence

<220>
<223>  an artificially synthesized sequence

<400>  339

Thr Ser His Ser Gly Arg Ser Ala Glu Pro Arg Gly
1               5                   10


<210>  340
<211>  12
<212>  PRT
<213>  Artificial Sequence

<220>
<223>  an artificially synthesized sequence

<400>  340

Thr Ser Thr Ser Gly Arg Ser Ala Glu Gln Arg Gly
1               5                   10


<210>  341
<211>  12
<212>  PRT
<213>  Artificial Sequence

<220>
<223>  an artificially synthesized sequence

<400>  341

Thr Ser Thr Ser Gly Arg Ser Ala Glu Ala Arg Gly
1               5                   10


<210>  342
<211>  12
<212>  PRT
<213>  Artificial Sequence

<220>
<223>  an artificially synthesized sequence

<400>  342

Thr Ser Thr Ser Gly Arg Ser Ala Glu Ile Arg Gly
1               5                   10

<210>  343
<211>  12
<212>  PRT
<213>  Artificial Sequence

<220>
<223>  an artificially synthesized sequence

<400>  343

Thr Ser Thr Ser Gly Arg Ser Ala Glu Phe Arg Gly
1               5                   10


<210>  344
<211>  12
<212>  PRT
<213>  Artificial Sequence

<220>
<223>  an artificially synthesized sequence

<400>  344

Thr Ser Thr Ser Gly Arg Ser Ala Glu Ser Arg Gly
1               5                   10


<210>  345
<211>  12
<212>  PRT
<213>  Artificial Sequence

<220>
<223>  an artificially synthesized sequence

<400>  345

Thr Ser Gly Thr Gly Arg Ser Ala Asn Pro Arg Gly
1               5                   10


<210>  346
<211>  12
<212>  PRT
<213>  Artificial Sequence

<220>
<223>  an artificially synthesized sequence

<400>  346

Thr Ser Gly Lys Gly Arg Ser Ala Asn Pro Arg Gly
1               5                   10


<210>  347
<211>  12
<212>  PRT
<213>  Artificial Sequence

<220>

<223>  an artificially synthesized sequence

<400>  347

Thr Ser Gly Ser Gly Arg Ser Ala Ile Pro Arg Gly
1               5                   10


<210>  348
<211>  12
<212>  PRT
<213>  Artificial Sequence

<220>
<223>  an artificially synthesized sequence

<400>  348

Thr Ser Gly Ser Gly Arg Ser Ala Ser Pro Arg Gly
1               5                   10


<210>  349
<211>  12
<212>  PRT
<213>  Artificial Sequence

<220>
<223>  an artificially synthesized sequence

<400>  349

Thr Ser Gly Ser Gly Arg Ser Ala His Pro Arg Gly
1               5                   10


<210>  350
<211>  12
<212>  PRT
<213>  Artificial Sequence

<220>
<223>  an artificially synthesized sequence

<400>  350

Thr Ser Gly Ser Gly Arg Ser Ala Asn Tyr Arg Gly
1               5                   10


<210>  351
<211>  12
<212>  PRT
<213>  Artificial Sequence

<220>
<223>  an artificially synthesized sequence

<400>  351

Thr Ser Gly Ser Gly Arg Ser Ala Asn Val Arg Gly
1               5                   10

<210>  352
<211>  12
<212>  PRT
<213>  Artificial Sequence

<220>
<223>  an artificially synthesized sequence

<400>  352

Thr Ser Gly Ser Gly Arg Ser Ala Asn His Arg Gly
1               5                   10


<210>  353
<211>  12
<212>  PRT
<213>  Artificial Sequence

<220>
<223>  an artificially synthesized sequence

<400>  353

Thr Ser Gln Thr Gly Arg Ser Ala Asn Pro Arg Gly
1               5                   10


<210>  354
<211>  12
<212>  PRT
<213>  Artificial Sequence

<220>
<223>  an artificially synthesized sequence

<400>  354

Thr Ser Gln Lys Gly Arg Ser Ala Asn Pro Arg Gly
1               5                   10


<210>  355
<211>  12
<212>  PRT
<213>  Artificial Sequence

<220>
<223>  an artificially synthesized sequence

<400>  355

Thr Ser Gln Ser Gly Arg Ser Ala Ile Pro Arg Gly
1               5                   10


<210>  356
<211>  12
<212>  PRT
<213>  Artificial Sequence

<220>

<223>  an artificially synthesized sequence

<400>  356

Thr Ser Gln Ser Gly Arg Ser Ala Ser Pro Arg Gly
1               5                   10


<210>  357
<211>  12
<212>  PRT
<213>  Artificial Sequence

<220>
<223>  an artificially synthesized sequence

<400>  357

Thr Ser Gln Ser Gly Arg Ser Ala His Pro Arg Gly
1               5                   10


<210>  358
<211>  12
<212>  PRT
<213>  Artificial Sequence

<220>
<223>  an artificially synthesized sequence

<400>  358

Thr Ser Gln Ser Gly Arg Ser Ala Asn Tyr Arg Gly
1               5                   10


<210>  359
<211>  12
<212>  PRT
<213>  Artificial Sequence

<220>
<223>  an artificially synthesized sequence

<400>  359

Thr Ser Gln Ser Gly Arg Ser Ala Asn Val Arg Gly
1               5                   10


<210>  360
<211>  12
<212>  PRT
<213>  Artificial Sequence

<220>
<223>  an artificially synthesized sequence

<400>  360

Thr Ser Gln Ser Gly Arg Ser Ala Asn His Arg Gly
1               5                   10

<210>  361
<211>  12
<212>  PRT
<213>  Artificial Sequence

<220>
<223>  an artificially synthesized sequence

<400>  361

Thr Ser Pro Thr Gly Arg Ser Ala Asn Pro Arg Gly
1               5                   10


<210>  362
<211>  12
<212>  PRT
<213>  Artificial Sequence

<220>
<223>  an artificially synthesized sequence

<400>  362

Thr Ser Pro Lys Gly Arg Ser Ala Asn Pro Arg Gly
1               5                   10


<210>  363
<211>  12
<212>  PRT
<213>  Artificial Sequence

<220>
<223>  an artificially synthesized sequence

<400>  363

Thr Ser Pro Ser Gly Arg Ser Ala Ile Pro Arg Gly
1               5                   10


<210>  364
<211>  12
<212>  PRT
<213>  Artificial Sequence

<220>
<223>  an artificially synthesized sequence

<400>  364

Thr Ser Pro Ser Gly Arg Ser Ala Thr Pro Arg Gly
1               5                   10


<210>  365
<211>  12
<212>  PRT
<213>  Artificial Sequence

<220>

<223>  an artificially synthesized sequence

<400>  365

Thr Ser Pro Ser Gly Arg Ser Ala Ser Pro Arg Gly
1               5                   10


<210>  366
<211>  12
<212>  PRT
<213>  Artificial Sequence

<220>
<223>  an artificially synthesized sequence

<400>  366

Thr Ser Pro Ser Gly Arg Ser Ala His Pro Arg Gly
1               5                   10


<210>  367
<211>  12
<212>  PRT
<213>  Artificial Sequence

<220>
<223>  an artificially synthesized sequence

<400>  367

Thr Ser Pro Ser Gly Arg Ser Ala Asn Tyr Arg Gly
1               5                   10


<210>  368
<211>  12
<212>  PRT
<213>  Artificial Sequence

<220>
<223>  an artificially synthesized sequence

<400>  368

Thr Ser Pro Ser Gly Arg Ser Ala Asn Val Arg Gly
1               5                   10


<210>  369
<211>  12
<212>  PRT
<213>  Artificial Sequence

<220>
<223>  an artificially synthesized sequence

<400>  369

Thr Ser Pro Ser Gly Arg Ser Ala Asn His Arg Gly
1               5                   10

<210>  370
<211>  12
<212>  PRT
<213>  Artificial Sequence

<220>
<223>  an artificially synthesized sequence

<400>  370

Thr Ser Ala Thr Gly Arg Ser Ala Asn Pro Arg Gly
1               5                   10


<210>  371
<211>  12
<212>  PRT
<213>  Artificial Sequence

<220>
<223>  an artificially synthesized sequence

<400>  371

Thr Ser Ala Lys Gly Arg Ser Ala Asn Pro Arg Gly
1               5                   10


<210>  372
<211>  12
<212>  PRT
<213>  Artificial Sequence

<220>
<223>  an artificially synthesized sequence

<400>  372

Thr Ser Ala Ser Gly Arg Ser Ala Ile Pro Arg Gly
1               5                   10


<210>  373
<211>  12
<212>  PRT
<213>  Artificial Sequence

<220>
<223>  an artificially synthesized sequence

<400>  373

Thr Ser Ala Ser Gly Arg Ser Ala Ser Pro Arg Gly
1               5                   10


<210>  374
<211>  12
<212>  PRT
<213>  Artificial Sequence

<220>

<223>  an artificially synthesized sequence

<400>  374

Thr Ser Ala Ser Gly Arg Ser Ala His Pro Arg Gly
1               5                   10


<210>  375
<211>  12
<212>  PRT
<213>  Artificial Sequence

<220>
<223>  an artificially synthesized sequence

<400>  375

Thr Ser Ala Ser Gly Arg Ser Ala Asn Tyr Arg Gly
1               5                   10


<210>  376
<211>  12
<212>  PRT
<213>  Artificial Sequence

<220>
<223>  an artificially synthesized sequence

<400>  376

Thr Ser Ala Ser Gly Arg Ser Ala Asn Val Arg Gly
1               5                   10


<210>  377
<211>  12
<212>  PRT
<213>  Artificial Sequence

<220>
<223>  an artificially synthesized sequence

<400>  377

Thr Ser Ala Ser Gly Arg Ser Ala Asn His Arg Gly
1               5                   10


<210>  378
<211>  12
<212>  PRT
<213>  Artificial Sequence

<220>
<223>  an artificially synthesized sequence

<400>  378

Thr Ser Tyr Thr Gly Arg Ser Ala Asn Pro Arg Gly
1               5                   10

<210>  379
<211>  12
<212>  PRT
<213>  Artificial Sequence

<220>
<223>  an artificially synthesized sequence

<400>  379

Thr Ser Tyr Lys Gly Arg Ser Ala Asn Pro Arg Gly
1               5                   10


<210>  380
<211>  12
<212>  PRT
<213>  Artificial Sequence

<220>
<223>  an artificially synthesized sequence

<400>  380

Thr Ser Tyr Ser Gly Arg Ser Ala Ile Pro Arg Gly
1               5                   10


<210>  381
<211>  12
<212>  PRT
<213>  Artificial Sequence

<220>
<223>  an artificially synthesized sequence

<400>  381

Thr Ser Tyr Ser Gly Arg Ser Ala Thr Pro Arg Gly
1               5                   10


<210>  382
<211>  12
<212>  PRT
<213>  Artificial Sequence

<220>
<223>  an artificially synthesized sequence

<400>  382

Thr Ser Tyr Ser Gly Arg Ser Ala Ser Pro Arg Gly
1               5                   10


<210>  383
<211>  12
<212>  PRT
<213>  Artificial Sequence

<220>

<223>  an artificially synthesized sequence

<400>  383

Thr Ser Tyr Ser Gly Arg Ser Ala His Pro Arg Gly
1               5                   10


<210>  384
<211>  12
<212>  PRT
<213>  Artificial Sequence

<220>
<223>  an artificially synthesized sequence

<400>  384

Thr Ser Tyr Ser Gly Arg Ser Ala Asn Ala Arg Gly
1               5                   10


<210>  385
<211>  12
<212>  PRT
<213>  Artificial Sequence

<220>
<223>  an artificially synthesized sequence

<400>  385

Thr Ser Tyr Ser Gly Arg Ser Ala Asn Tyr Arg Gly
1               5                   10


<210>  386
<211>  12
<212>  PRT
<213>  Artificial Sequence

<220>
<223>  an artificially synthesized sequence

<400>  386

Thr Ser Tyr Ser Gly Arg Ser Ala Asn Val Arg Gly
1               5                   10


<210>  387
<211>  12
<212>  PRT
<213>  Artificial Sequence

<220>
<223>  an artificially synthesized sequence

<400>  387

Thr Ser Tyr Ser Gly Arg Ser Ala Asn His Arg Gly
1               5                   10

<210>  388
<211>  12
<212>  PRT
<213>  Artificial Sequence

<220>
<223>  an artificially synthesized sequence

<400>  388

Thr Ser Ser Thr Gly Arg Ser Ala Asn Pro Arg Gly
1               5                   10


<210>  389
<211>  12
<212>  PRT
<213>  Artificial Sequence

<220>
<223>  an artificially synthesized sequence

<400>  389

Thr Ser Ser Lys Gly Arg Ser Ala Asn Pro Arg Gly
1               5                   10


<210>  390
<211>  12
<212>  PRT
<213>  Artificial Sequence

<220>
<223>  an artificially synthesized sequence

<400>  390

Thr Ser Ser Ser Gly Arg Ser Ala Val Pro Arg Gly
1               5                   10


<210>  391
<211>  12
<212>  PRT
<213>  Artificial Sequence

<220>
<223>  an artificially synthesized sequence

<400>  391

Thr Ser Ser Ser Gly Arg Ser Ala Ile Pro Arg Gly
1               5                   10


<210>  392
<211>  12
<212>  PRT
<213>  Artificial Sequence

<220>

<223>  an artificially synthesized sequence

<400>  392

Thr Ser Ser Ser Gly Arg Ser Ala Ser Pro Arg Gly
1               5                   10


<210>  393
<211>  12
<212>  PRT
<213>  Artificial Sequence

<220>
<223>  an artificially synthesized sequence

<400>  393

Thr Ser Ser Ser Gly Arg Ser Ala His Pro Arg Gly
1               5                   10


<210>  394
<211>  12
<212>  PRT
<213>  Artificial Sequence

<220>
<223>  an artificially synthesized sequence

<400>  394

Thr Ser Ser Ser Gly Arg Ser Ala Asn Ala Arg Gly
1               5                   10


<210>  395
<211>  12
<212>  PRT
<213>  Artificial Sequence

<220>
<223>  an artificially synthesized sequence

<400>  395

Thr Ser Ser Ser Gly Arg Ser Ala Asn Phe Arg Gly
1               5                   10


<210>  396
<211>  12
<212>  PRT
<213>  Artificial Sequence

<220>
<223>  an artificially synthesized sequence

<400>  396

Thr Ser Ser Ser Gly Arg Ser Ala Asn Tyr Arg Gly
1               5                   10

<210>  397
<211>  12
<212>  PRT
<213>  Artificial Sequence

<220>
<223>  an artificially synthesized sequence

<400>  397

Thr Ser Ser Ser Gly Arg Ser Ala Asn Val Arg Gly
1               5                   10


<210>  398
<211>  12
<212>  PRT
<213>  Artificial Sequence

<220>
<223>  an artificially synthesized sequence

<400>  398

Thr Ser Ser Ser Gly Arg Ser Ala Asn His Arg Gly
1               5                   10


<210>  399
<211>  12
<212>  PRT
<213>  Artificial Sequence

<220>
<223>  an artificially synthesized sequence

<400>  399

Thr Ser Ile Thr Gly Arg Ser Ala Asn Pro Arg Gly
1               5                   10


<210>  400
<211>  12
<212>  PRT
<213>  Artificial Sequence

<220>
<223>  an artificially synthesized sequence

<400>  400

Thr Ser Ile Lys Gly Arg Ser Ala Asn Pro Arg Gly
1               5                   10


<210>  401
<211>  12
<212>  PRT
<213>  Artificial Sequence

<220>

<223>  an artificially synthesized sequence

<400>  401

Thr Ser Ile Ser Gly Arg Ser Ala Val Pro Arg Gly
1               5                   10


<210>  402
<211>  12
<212>  PRT
<213>  Artificial Sequence

<220>
<223>  an artificially synthesized sequence

<400>  402

Thr Ser Ile Ser Gly Arg Ser Ala Ile Pro Arg Gly
1               5                   10


<210>  403
<211>  12
<212>  PRT
<213>  Artificial Sequence

<220>
<223>  an artificially synthesized sequence

<400>  403

Thr Ser Ile Ser Gly Arg Ser Ala Thr Pro Arg Gly
1               5                   10


<210>  404
<211>  12
<212>  PRT
<213>  Artificial Sequence

<220>
<223>  an artificially synthesized sequence

<400>  404

Thr Ser Ile Ser Gly Arg Ser Ala Ser Pro Arg Gly
1               5                   10


<210>  405
<211>  12
<212>  PRT
<213>  Artificial Sequence

<220>
<223>  an artificially synthesized sequence

<400>  405

Thr Ser Ile Ser Gly Arg Ser Ala His Pro Arg Gly
1               5                   10

<210>  406
<211>  12
<212>  PRT
<213>  Artificial Sequence

<220>
<223>  an artificially synthesized sequence

<400>  406

Thr Ser Ile Ser Gly Arg Ser Ala Asn Ala Arg Gly
1               5                   10


<210>  407
<211>  12
<212>  PRT
<213>  Artificial Sequence

<220>
<223>  an artificially synthesized sequence

<400>  407

Thr Ser Ile Ser Gly Arg Ser Ala Asn Phe Arg Gly
1               5                   10


<210>  408
<211>  12
<212>  PRT
<213>  Artificial Sequence

<220>
<223>  an artificially synthesized sequence

<400>  408

Thr Ser Ile Ser Gly Arg Ser Ala Asn Tyr Arg Gly
1               5                   10


<210>  409
<211>  12
<212>  PRT
<213>  Artificial Sequence

<220>
<223>  an artificially synthesized sequence

<400>  409

Thr Ser Ile Ser Gly Arg Ser Ala Asn Val Arg Gly
1               5                   10


<210>  410
<211>  12
<212>  PRT
<213>  Artificial Sequence

<220>

<223>  an artificially synthesized sequence

<400>  410

Thr Ser Ile Ser Gly Arg Ser Ala Asn His Arg Gly
1               5                   10


<210>  411
<211>  12
<212>  PRT
<213>  Artificial Sequence

<220>
<223>  an artificially synthesized sequence

<400>  411

Thr Ser Thr Thr Gly Arg Ser Ala Val Pro Arg Gly
1               5                   10


<210>  412
<211>  12
<212>  PRT
<213>  Artificial Sequence

<220>
<223>  an artificially synthesized sequence

<400>  412

Thr Ser Thr Thr Gly Arg Ser Ala Ile Pro Arg Gly
1               5                   10


<210>  413
<211>  12
<212>  PRT
<213>  Artificial Sequence

<220>
<223>  an artificially synthesized sequence

<400>  413

Thr Ser Thr Thr Gly Arg Ser Ala Thr Pro Arg Gly
1               5                   10


<210>  414
<211>  12
<212>  PRT
<213>  Artificial Sequence

<220>
<223>  an artificially synthesized sequence

<400>  414

Thr Ser Thr Thr Gly Arg Ser Ala His Pro Arg Gly
1               5                   10

<210>  415
<211>  12
<212>  PRT
<213>  Artificial Sequence

<220>
<223>  an artificially synthesized sequence

<400>  415

Thr Ser Thr Thr Gly Arg Ser Ala Asn Ala Arg Gly
1               5                   10


<210>  416
<211>  12
<212>  PRT
<213>  Artificial Sequence

<220>
<223>  an artificially synthesized sequence

<400>  416

Thr Ser Thr Thr Gly Arg Ser Ala Asn Phe Arg Gly
1               5                   10


<210>  417
<211>  12
<212>  PRT
<213>  Artificial Sequence

<220>
<223>  an artificially synthesized sequence

<400>  417

Thr Ser Thr Thr Gly Arg Ser Ala Asn Tyr Arg Gly
1               5                   10


<210>  418
<211>  12
<212>  PRT
<213>  Artificial Sequence

<220>
<223>  an artificially synthesized sequence

<400>  418

Thr Ser Thr Thr Gly Arg Ser Ala Asn Val Arg Gly
1               5                   10


<210>  419
<211>  12
<212>  PRT
<213>  Artificial Sequence

<220>

<223>  an artificially synthesized sequence

<400>  419

Thr Ser Thr Thr Gly Arg Ser Ala Asn His Arg Gly
1               5                   10


<210>  420
<211>  12
<212>  PRT
<213>  Artificial Sequence

<220>
<223>  an artificially synthesized sequence

<400>  420

Thr Ser Thr Lys Gly Arg Ser Ala Val Pro Arg Gly
1               5                   10


<210>  421
<211>  12
<212>  PRT
<213>  Artificial Sequence

<220>
<223>  an artificially synthesized sequence

<400>  421

Thr Ser Thr Lys Gly Arg Ser Ala Ile Pro Arg Gly
1               5                   10


<210>  422
<211>  12
<212>  PRT
<213>  Artificial Sequence

<220>
<223>  an artificially synthesized sequence

<400>  422

Thr Ser Thr Lys Gly Arg Ser Ala Thr Pro Arg Gly
1               5                   10


<210>  423
<211>  12
<212>  PRT
<213>  Artificial Sequence

<220>
<223>  an artificially synthesized sequence

<400>  423

Thr Ser Thr Lys Gly Arg Ser Ala Ser Pro Arg Gly
1               5                   10

<210>  424
<211>  12
<212>  PRT
<213>  Artificial Sequence

<220>
<223>  an artificially synthesized sequence

<400>  424

Thr Ser Thr Lys Gly Arg Ser Ala His Pro Arg Gly
1               5                   10


<210>  425
<211>  12
<212>  PRT
<213>  Artificial Sequence

<220>
<223>  an artificially synthesized sequence

<400>  425

Thr Ser Thr Lys Gly Arg Ser Ala Asn Ala Arg Gly
1               5                   10


<210>  426
<211>  12
<212>  PRT
<213>  Artificial Sequence

<220>
<223>  an artificially synthesized sequence

<400>  426

Thr Ser Thr Lys Gly Arg Ser Ala Asn Phe Arg Gly
1               5                   10


<210>  427
<211>  12
<212>  PRT
<213>  Artificial Sequence

<220>
<223>  an artificially synthesized sequence

<400>  427

Thr Ser Thr Lys Gly Arg Ser Ala Asn Tyr Arg Gly
1               5                   10


<210>  428
<211>  12
<212>  PRT
<213>  Artificial Sequence

<220>

<223>  an artificially synthesized sequence

<400>  428

Thr Ser Thr Lys Gly Arg Ser Ala Asn Val Arg Gly
1               5                   10


<210>  429
<211>  12
<212>  PRT
<213>  Artificial Sequence

<220>
<223>  an artificially synthesized sequence

<400>  429

Thr Ser Thr Lys Gly Arg Ser Ala Asn His Arg Gly
1               5                   10


<210>  430
<211>  12
<212>  PRT
<213>  Artificial Sequence

<220>
<223>  an artificially synthesized sequence

<400>  430

Thr Ser Thr Ser Gly Arg Ser Ala Val Tyr Arg Gly
1               5                   10


<210>  431
<211>  12
<212>  PRT
<213>  Artificial Sequence

<220>
<223>  an artificially synthesized sequence

<400>  431

Thr Ser Thr Ser Gly Arg Ser Ala Val Val Arg Gly
1               5                   10


<210>  432
<211>  12
<212>  PRT
<213>  Artificial Sequence

<220>
<223>  an artificially synthesized sequence

<400>  432

Thr Ser Thr Ser Gly Arg Ser Ala Val His Arg Gly
1               5                   10

<210>  433
<211>  12
<212>  PRT
<213>  Artificial Sequence

<220>
<223>  an artificially synthesized sequence

<400>  433

Thr Ser Thr Ser Gly Arg Ser Ala Ile Tyr Arg Gly
1               5                   10


<210>  434
<211>  12
<212>  PRT
<213>  Artificial Sequence

<220>
<223>  an artificially synthesized sequence

<400>  434

Thr Ser Thr Ser Gly Arg Ser Ala Ile Val Arg Gly
1               5                   10


<210>  435
<211>  12
<212>  PRT
<213>  Artificial Sequence

<220>
<223>  an artificially synthesized sequence

<400>  435

Thr Ser Thr Ser Gly Arg Ser Ala Ile His Arg Gly
1               5                   10


<210>  436
<211>  12
<212>  PRT
<213>  Artificial Sequence

<220>
<223>  an artificially synthesized sequence

<400>  436

Thr Ser Thr Ser Gly Arg Ser Ala Ser Tyr Arg Gly
1               5                   10


<210>  437
<211>  12
<212>  PRT
<213>  Artificial Sequence

<220>

<223>  an artificially synthesized sequence

<400>  437

Thr Ser Thr Ser Gly Arg Ser Ala Ser Val Arg Gly
1               5                   10


<210>  438
<211>  12
<212>  PRT
<213>  Artificial Sequence

<220>
<223>  an artificially synthesized sequence

<400>  438

Thr Ser Thr Ser Gly Arg Ser Ala Ser His Arg Gly
1               5                   10


<210>  439
<211>  12
<212>  PRT
<213>  Artificial Sequence

<220>
<223>  an artificially synthesized sequence

<400>  439

Thr Ser Thr Ser Gly Arg Ser Ala His Tyr Arg Gly
1               5                   10


<210>  440
<211>  12
<212>  PRT
<213>  Artificial Sequence

<220>
<223>  an artificially synthesized sequence

<400>  440

Thr Ser Thr Ser Gly Arg Ser Ala His Val Arg Gly
1               5                   10


<210>  441
<211>  12
<212>  PRT
<213>  Artificial Sequence

<220>
<223>  an artificially synthesized sequence

<400>  441

Thr Ser Thr Ser Gly Arg Ser Ala His His Arg Gly
1               5                   10

<210>  442
<211>  12
<212>  PRT
<213>  Artificial Sequence

<220>
<223>  an artificially synthesized sequence

<400>  442

Thr Ser Pro Ser Gly Arg Ser Glu Val Pro Arg Gly
1               5                   10


<210>  443
<211>  12
<212>  PRT
<213>  Artificial Sequence

<220>
<223>  an artificially synthesized sequence

<400>  443

Thr Ser Pro Ser Gly Arg Ser Ala Glu Pro Arg Gly
1               5                   10


<210>  444
<211>  12
<212>  PRT
<213>  Artificial Sequence

<220>
<223>  an artificially synthesized sequence

<400>  444

Thr Ser Pro Ser Gly Arg Ser Ala Gly Pro Arg Gly
1               5                   10


<210>  445
<211>  12
<212>  PRT
<213>  Artificial Sequence

<220>
<223>  an artificially synthesized sequence

<400>  445

Thr Ser Ala Ser Gly Arg Ser Glu Asn Ala Arg Gly
1               5                   10


<210>  446
<211>  12
<212>  PRT
<213>  Artificial Sequence

<220>

<223>  an artificially synthesized sequence

<400>  446

Thr Ser Ala Ser Gly Arg Ser Ala Glu Ala Arg Gly
1               5                   10


<210>  447
<211>  12
<212>  PRT
<213>  Artificial Sequence

<220>
<223>  an artificially synthesized sequence

<400>  447

Thr Ser Ala Ser Gly Arg Ser Ala Gly Ala Arg Gly
1               5                   10


<210>  448
<211>  12
<212>  PRT
<213>  Artificial Sequence

<220>
<223>  an artificially synthesized sequence

<400>  448

Thr Ser Gly Thr Gly Arg Ser Ala Thr Pro Arg Gly
1               5                   10


<210>  449
<211>  12
<212>  PRT
<213>  Artificial Sequence

<220>
<223>  an artificially synthesized sequence

<400>  449

Thr Ser Gly Ser Gly Arg Ser Ala Thr Tyr Arg Gly
1               5                   10


<210>  450
<211>  12
<212>  PRT
<213>  Artificial Sequence

<220>
<223>  an artificially synthesized sequence

<400>  450

Thr Ser Gly Ser Gly Arg Ser Ala Thr Val Arg Gly
1               5                   10

<210>  451
<211>  12
<212>  PRT
<213>  Artificial Sequence

<220>
<223>  an artificially synthesized sequence

<400>  451

Thr Ser Gly Ser Gly Arg Ser Ala Thr His Arg Gly
1               5                   10


<210>  452
<211>  12
<212>  PRT
<213>  Artificial Sequence

<220>
<223>  an artificially synthesized sequence

<400>  452

Thr Ser Gly Thr Gly Arg Ser Ala Thr Tyr Arg Gly
1               5                   10


<210>  453
<211>  12
<212>  PRT
<213>  Artificial Sequence

<220>
<223>  an artificially synthesized sequence

<400>  453

Thr Ser Gly Thr Gly Arg Ser Ala Thr Val Arg Gly
1               5                   10


<210>  454
<211>  12
<212>  PRT
<213>  Artificial Sequence

<220>
<223>  an artificially synthesized sequence

<400>  454

Thr Ser Gly Thr Gly Arg Ser Ala Thr His Arg Gly
1               5                   10


<210>  455
<211>  12
<212>  PRT
<213>  Artificial Sequence

<220>

<223>  an artificially synthesized sequence

<400>  455

Thr Ser Gly Ser Gly Arg Ser Glu Thr Pro Arg Gly
1               5                   10


<210>  456
<211>  12
<212>  PRT
<213>  Artificial Sequence

<220>
<223>  an artificially synthesized sequence

<400>  456

Thr Ser Gly Thr Gly Arg Ser Glu Thr Pro Arg Gly
1               5                   10


<210>  457
<211>  12
<212>  PRT
<213>  Artificial Sequence

<220>
<223>  an artificially synthesized sequence

<400>  457

Thr Ser Gly Ser Gly Arg Ser Glu Thr Tyr Arg Gly
1               5                   10


<210>  458
<211>  12
<212>  PRT
<213>  Artificial Sequence

<220>
<223>  an artificially synthesized sequence

<400>  458

Thr Ser Gly Ser Gly Arg Ser Glu Thr Val Arg Gly
1               5                   10


<210>  459
<211>  12
<212>  PRT
<213>  Artificial Sequence

<220>
<223>  an artificially synthesized sequence

<400>  459

Thr Ser Gly Ser Gly Arg Ser Glu Thr His Arg Gly
1               5                   10

<210>  460
<211>  12
<212>  PRT
<213>  Artificial Sequence

<220>
<223>  an artificially synthesized sequence

<400>  460

Thr Ser Tyr Thr Gly Arg Ser Ala Val Pro Arg Gly
1               5                   10


<210>  461
<211>  12
<212>  PRT
<213>  Artificial Sequence

<220>
<223>  an artificially synthesized sequence

<400>  461

Thr Ser Tyr Ser Gly Arg Ser Ala Val Tyr Arg Gly
1               5                   10


<210>  462
<211>  12
<212>  PRT
<213>  Artificial Sequence

<220>
<223>  an artificially synthesized sequence

<400>  462

Thr Ser Tyr Ser Gly Arg Ser Ala Val Val Arg Gly
1               5                   10


<210>  463
<211>  12
<212>  PRT
<213>  Artificial Sequence

<220>
<223>  an artificially synthesized sequence

<400>  463

Thr Ser Tyr Ser Gly Arg Ser Ala Val His Arg Gly
1               5                   10


<210>  464
<211>  12
<212>  PRT
<213>  Artificial Sequence

<220>

<223>  an artificially synthesized sequence

<400>  464

Thr Ser Tyr Thr Gly Arg Ser Ala Val Tyr Arg Gly
1               5                   10


<210>  465
<211>  12
<212>  PRT
<213>  Artificial Sequence

<220>
<223>  an artificially synthesized sequence

<400>  465

Thr Ser Tyr Thr Gly Arg Ser Ala Val Val Arg Gly
1               5                   10


<210>  466
<211>  12
<212>  PRT
<213>  Artificial Sequence

<220>
<223>  an artificially synthesized sequence

<400>  466

Thr Ser Tyr Thr Gly Arg Ser Ala Val His Arg Gly
1               5                   10


<210>  467
<211>  12
<212>  PRT
<213>  Artificial Sequence

<220>
<223>  an artificially synthesized sequence

<400>  467

Thr Ser Tyr Ser Gly Arg Ser Glu Val Pro Arg Gly
1               5                   10


<210>  468
<211>  12
<212>  PRT
<213>  Artificial Sequence

<220>
<223>  an artificially synthesized sequence

<400>  468

Thr Ser Tyr Thr Gly Arg Ser Glu Val Pro Arg Gly
1               5                   10

<210>  469
<211>  12
<212>  PRT
<213>  Artificial Sequence

<220>
<223>  an artificially synthesized sequence

<400>  469

Thr Ser Tyr Ser Gly Arg Ser Glu Val Tyr Arg Gly
1               5                   10


<210>  470
<211>  12
<212>  PRT
<213>  Artificial Sequence

<220>
<223>  an artificially synthesized sequence

<400>  470

Thr Ser Tyr Ser Gly Arg Ser Glu Val Val Arg Gly
1               5                   10


<210>  471
<211>  12
<212>  PRT
<213>  Artificial Sequence

<220>
<223>  an artificially synthesized sequence

<400>  471

Thr Ser Tyr Ser Gly Arg Ser Glu Val His Arg Gly
1               5                   10


<210>  472
<211>  12
<212>  PRT
<213>  Artificial Sequence

<220>
<223>  an artificially synthesized sequence

<400>  472

Thr Ser Tyr Thr Gly Arg Ser Ala Val Pro Gly Gly
1               5                   10


<210>  473
<211>  12
<212>  PRT
<213>  Artificial Sequence

<220>

<223>  an artificially synthesized sequence

<400>  473

Thr Ser Tyr Ser Gly Arg Ser Ala Val Tyr Gly Gly
1               5                   10


<210>  474
<211>  12
<212>  PRT
<213>  Artificial Sequence

<220>
<223>  an artificially synthesized sequence

<400>  474

Thr Ser Tyr Ser Gly Arg Ser Ala Val Val Gly Gly
1               5                   10


<210>  475
<211>  12
<212>  PRT
<213>  Artificial Sequence

<220>
<223>  an artificially synthesized sequence

<400>  475

Thr Ser Tyr Ser Gly Arg Ser Ala Val His Gly Gly
1               5                   10


<210>  476
<211>  12
<212>  PRT
<213>  Artificial Sequence

<220>
<223>  an artificially synthesized sequence

<400>  476

Thr Ser Tyr Thr Gly Arg Ser Ala Val Tyr Gly Gly
1               5                   10


<210>  477
<211>  12
<212>  PRT
<213>  Artificial Sequence

<220>
<223>  an artificially synthesized sequence

<400>  477

Thr Ser Tyr Thr Gly Arg Ser Ala Val Val Gly Gly
1               5                   10

<210>  478
<211>  12
<212>  PRT
<213>  Artificial Sequence

<220>
<223>  an artificially synthesized sequence

<400>  478

Thr Ser Tyr Thr Gly Arg Ser Ala Val His Gly Gly
1               5                   10


<210>  479
<211>  8
<212>  PRT
<213>  Artificial Sequence

<220>
<223>  an artificially synthesized sequence

<400>  479

Ala Ser Gly Arg Ser Ala Asn Pro
1               5


<210>  480
<211>  8
<212>  PRT
<213>  Artificial Sequence

<220>
<223>  an artificially synthesized sequence

<400>  480

Glu Ser Gly Arg Ser Ala Asn Pro
1               5


<210>  481
<211>  8
<212>  PRT
<213>  Artificial Sequence

<220>
<223>  an artificially synthesized sequence

<400>  481

Phe Ser Gly Arg Ser Ala Asn Pro
1               5


<210>  482
<211>  8
<212>  PRT
<213>  Artificial Sequence

<220>

<223>  an artificially synthesized sequence

<400>  482

Gly Ser Gly Arg Ser Ala Asn Pro
1               5


<210>  483
<211>  8
<212>  PRT
<213>  Artificial Sequence

<220>
<223>  an artificially synthesized sequence

<400>  483

His Ser Gly Arg Ser Ala Asn Pro
1               5


<210>  484
<211>  8
<212>  PRT
<213>  Artificial Sequence

<220>
<223>  an artificially synthesized sequence

<400>  484

Lys Ser Gly Arg Ser Ala Asn Pro
1               5


<210>  485
<211>  8
<212>  PRT
<213>  Artificial Sequence

<220>
<223>  an artificially synthesized sequence

<400>  485

Met Ser Gly Arg Ser Ala Asn Pro
1               5


<210>  486
<211>  8
<212>  PRT
<213>  Artificial Sequence

<220>
<223>  an artificially synthesized sequence

<400>  486

Asn Ser Gly Arg Ser Ala Asn Pro
1               5

<210>  487
<211>  8
<212>  PRT
<213>  Artificial Sequence

<220>
<223>  an artificially synthesized sequence

<400>  487

Pro Ser Gly Arg Ser Ala Asn Pro
1               5


<210>  488
<211>  8
<212>  PRT
<213>  Artificial Sequence

<220>
<223>  an artificially synthesized sequence

<400>  488

Gln Ser Gly Arg Ser Ala Asn Pro
1               5


<210>  489
<211>  8
<212>  PRT
<213>  Artificial Sequence

<220>
<223>  an artificially synthesized sequence

<400>  489

Trp Ser Gly Arg Ser Ala Asn Pro
1               5


<210>  490
<211>  8
<212>  PRT
<213>  Artificial Sequence

<220>
<223>  an artificially synthesized sequence

<400>  490

Tyr Ser Gly Arg Ser Ala Asn Pro
1               5


<210>  491
<211>  8
<212>  PRT
<213>  Artificial Sequence

<220>

<223>  an artificially synthesized sequence

<400>  491

Thr Ala Gly Arg Ser Ala Asn Pro
1               5


<210>  492
<211>  8
<212>  PRT
<213>  Artificial Sequence

<220>
<223>  an artificially synthesized sequence

<400>  492

Thr Asp Gly Arg Ser Ala Asn Pro
1               5


<210>  493
<211>  8
<212>  PRT
<213>  Artificial Sequence

<220>
<223>  an artificially synthesized sequence

<400>  493

Thr Glu Gly Arg Ser Ala Asn Pro
1               5


<210>  494
<211>  8
<212>  PRT
<213>  Artificial Sequence

<220>
<223>  an artificially synthesized sequence

<400>  494

Thr Phe Gly Arg Ser Ala Asn Pro
1               5


<210>  495
<211>  8
<212>  PRT
<213>  Artificial Sequence

<220>
<223>  an artificially synthesized sequence

<400>  495

Thr Leu Gly Arg Ser Ala Asn Pro
1               5

<210>  496
<211>  8
<212>  PRT
<213>  Artificial Sequence

<220>
<223>  an artificially synthesized sequence

<400>  496

Thr Met Gly Arg Ser Ala Asn Pro
1               5


<210>  497
<211>  8
<212>  PRT
<213>  Artificial Sequence

<220>
<223>  an artificially synthesized sequence

<400>  497

Thr Pro Gly Arg Ser Ala Asn Pro
1               5


<210>  498
<211>  8
<212>  PRT
<213>  Artificial Sequence

<220>
<223>  an artificially synthesized sequence

<400>  498

Thr Gln Gly Arg Ser Ala Asn Pro
1               5


<210>  499
<211>  8
<212>  PRT
<213>  Artificial Sequence

<220>
<223>  an artificially synthesized sequence

<400>  499

Thr Val Gly Arg Ser Ala Asn Pro
1               5


<210>  500
<211>  8
<212>  PRT
<213>  Artificial Sequence

<220>

<223>  an artificially synthesized sequence

<400>  500

Thr Trp Gly Arg Ser Ala Asn Pro
1               5


<210>  501
<211>  8
<212>  PRT
<213>  Artificial Sequence

<220>
<223>  an artificially synthesized sequence

<400>  501

Thr Ser Ala Arg Ser Ala Asn Pro
1               5


<210>  502
<211>  8
<212>  PRT
<213>  Artificial Sequence

<220>
<223>  an artificially synthesized sequence

<400>  502

Thr Ser Glu Arg Ser Ala Asn Pro
1               5


<210>  503
<211>  8
<212>  PRT
<213>  Artificial Sequence

<220>
<223>  an artificially synthesized sequence

<400>  503

Thr Ser Phe Arg Ser Ala Asn Pro
1               5


<210>  504
<211>  8
<212>  PRT
<213>  Artificial Sequence

<220>
<223>  an artificially synthesized sequence

<400>  504

Thr Ser His Arg Ser Ala Asn Pro
1               5

<210>  505
<211>  8
<212>  PRT
<213>  Artificial Sequence

<220>
<223>  an artificially synthesized sequence

<400>  505

Thr Ser Ile Arg Ser Ala Asn Pro
1               5


<210>  506
<211>  8
<212>  PRT
<213>  Artificial Sequence

<220>
<223>  an artificially synthesized sequence

<400>  506

Thr Ser Lys Arg Ser Ala Asn Pro
1               5


<210>  507
<211>  8
<212>  PRT
<213>  Artificial Sequence

<220>
<223>  an artificially synthesized sequence

<400>  507

Thr Ser Leu Arg Ser Ala Asn Pro
1               5


<210>  508
<211>  8
<212>  PRT
<213>  Artificial Sequence

<220>
<223>  an artificially synthesized sequence

<400>  508

Thr Ser Met Arg Ser Ala Asn Pro
1               5


<210>  509
<211>  8
<212>  PRT
<213>  Artificial Sequence

<220>

<223>  an artificially synthesized sequence

<400>  509

Thr Ser Asn Arg Ser Ala Asn Pro
1               5


<210>  510
<211>  8
<212>  PRT
<213>  Artificial Sequence

<220>
<223>  an artificially synthesized sequence

<400>  510

Thr Ser Pro Arg Ser Ala Asn Pro
1               5


<210>  511
<211>  8
<212>  PRT
<213>  Artificial Sequence

<220>
<223>  an artificially synthesized sequence

<400>  511

Thr Ser Gln Arg Ser Ala Asn Pro
1               5


<210>  512
<211>  8
<212>  PRT
<213>  Artificial Sequence

<220>
<223>  an artificially synthesized sequence

<400>  512

Thr Ser Arg Arg Ser Ala Asn Pro
1               5


<210>  513
<211>  8
<212>  PRT
<213>  Artificial Sequence

<220>
<223>  an artificially synthesized sequence

<400>  513

Thr Ser Thr Arg Ser Ala Asn Pro
1               5

<210>  514
<211>  8
<212>  PRT
<213>  Artificial Sequence

<220>
<223>  an artificially synthesized sequence

<400>  514

Thr Ser Val Arg Ser Ala Asn Pro
1               5


<210>  515
<211>  8
<212>  PRT
<213>  Artificial Sequence

<220>
<223>  an artificially synthesized sequence

<400>  515

Thr Ser Trp Arg Ser Ala Asn Pro
1               5


<210>  516
<211>  8
<212>  PRT
<213>  Artificial Sequence

<220>
<223>  an artificially synthesized sequence

<400>  516

Thr Ser Tyr Arg Ser Ala Asn Pro
1               5


<210>  517
<211>  8
<212>  PRT
<213>  Artificial Sequence

<220>
<223>  an artificially synthesized sequence

<400>  517

Thr Ser Gly Arg Ala Ala Asn Pro
1               5


<210>  518
<211>  8
<212>  PRT
<213>  Artificial Sequence

<220>

<223>  an artificially synthesized sequence

<400>  518

Thr Ser Gly Arg Asp Ala Asn Pro
1               5


<210>  519
<211>  8
<212>  PRT
<213>  Artificial Sequence

<220>
<223>  an artificially synthesized sequence

<400>  519

Thr Ser Gly Arg Glu Ala Asn Pro
1               5


<210>  520
<211>  8
<212>  PRT
<213>  Artificial Sequence

<220>
<223>  an artificially synthesized sequence

<400>  520

Thr Ser Gly Arg Gly Ala Asn Pro
1               5


<210>  521
<211>  8
<212>  PRT
<213>  Artificial Sequence

<220>
<223>  an artificially synthesized sequence

<400>  521

Thr Ser Gly Arg His Ala Asn Pro
1               5


<210>  522
<211>  8
<212>  PRT
<213>  Artificial Sequence

<220>
<223>  an artificially synthesized sequence

<400>  522

Thr Ser Gly Arg Ile Ala Asn Pro
1               5

<210>  523
<211>  8
<212>  PRT
<213>  Artificial Sequence

<220>
<223>  an artificially synthesized sequence

<400>  523

Thr Ser Gly Arg Lys Ala Asn Pro
1               5


<210>  524
<211>  8
<212>  PRT
<213>  Artificial Sequence

<220>
<223>  an artificially synthesized sequence

<400>  524

Thr Ser Gly Arg Leu Ala Asn Pro
1               5


<210>  525
<211>  8
<212>  PRT
<213>  Artificial Sequence

<220>
<223>  an artificially synthesized sequence

<400>  525

Thr Ser Gly Arg Met Ala Asn Pro
1               5


<210>  526
<211>  8
<212>  PRT
<213>  Artificial Sequence

<220>
<223>  an artificially synthesized sequence

<400>  526

Thr Ser Gly Arg Asn Ala Asn Pro
1               5


<210>  527
<211>  8
<212>  PRT
<213>  Artificial Sequence

<220>

<223>  an artificially synthesized sequence

<400>  527

Thr Ser Gly Arg Pro Ala Asn Pro
1               5


<210>  528
<211>  8
<212>  PRT
<213>  Artificial Sequence

<220>
<223>  an artificially synthesized sequence

<400>  528

Thr Ser Gly Arg Gln Ala Asn Pro
1               5


<210>  529
<211>  8
<212>  PRT
<213>  Artificial Sequence

<220>
<223>  an artificially synthesized sequence

<400>  529

Thr Ser Gly Arg Arg Ala Asn Pro
1               5


<210>  530
<211>  8
<212>  PRT
<213>  Artificial Sequence

<220>
<223>  an artificially synthesized sequence

<400>  530

Thr Ser Gly Arg Thr Ala Asn Pro
1               5


<210>  531
<211>  8
<212>  PRT
<213>  Artificial Sequence

<220>
<223>  an artificially synthesized sequence

<400>  531

Thr Ser Gly Arg Val Ala Asn Pro
1               5

<210>  532
<211>  8
<212>  PRT
<213>  Artificial Sequence

<220>
<223>  an artificially synthesized sequence

<400>  532

Thr Ser Gly Arg Trp Ala Asn Pro
1               5


<210>  533
<211>  8
<212>  PRT
<213>  Artificial Sequence

<220>
<223>  an artificially synthesized sequence

<400>  533

Thr Ser Gly Arg Tyr Ala Asn Pro
1               5


<210>  534
<211>  8
<212>  PRT
<213>  Artificial Sequence

<220>
<223>  an artificially synthesized sequence

<400>  534

Thr Ser Gly Arg Ser Glu Asn Pro
1               5


<210>  535
<211>  8
<212>  PRT
<213>  Artificial Sequence

<220>
<223>  an artificially synthesized sequence

<400>  535

Thr Ser Gly Arg Ser Phe Asn Pro
1               5


<210>  536
<211>  8
<212>  PRT
<213>  Artificial Sequence

<220>

<223>  an artificially synthesized sequence

<400>  536

Thr Ser Gly Arg Ser Lys Asn Pro
1               5


<210>  537
<211>  8
<212>  PRT
<213>  Artificial Sequence

<220>
<223>  an artificially synthesized sequence

<400>  537

Thr Ser Gly Arg Ser Met Asn Pro
1               5


<210>  538
<211>  8
<212>  PRT
<213>  Artificial Sequence

<220>
<223>  an artificially synthesized sequence

<400>  538

Thr Ser Gly Arg Ser Asn Asn Pro
1               5


<210>  539
<211>  8
<212>  PRT
<213>  Artificial Sequence

<220>
<223>  an artificially synthesized sequence

<400>  539

Thr Ser Gly Arg Ser Pro Asn Pro
1               5


<210>  540
<211>  8
<212>  PRT
<213>  Artificial Sequence

<220>
<223>  an artificially synthesized sequence

<400>  540

Thr Ser Gly Arg Ser Gln Asn Pro
1               5

<210>  541
<211>  8
<212>  PRT
<213>  Artificial Sequence

<220>
<223>  an artificially synthesized sequence

<400>  541

Thr Ser Gly Arg Ser Arg Asn Pro
1               5


<210>  542
<211>  8
<212>  PRT
<213>  Artificial Sequence

<220>
<223>  an artificially synthesized sequence

<400>  542

Thr Ser Gly Arg Ser Ser Asn Pro
1               5


<210>  543
<211>  8
<212>  PRT
<213>  Artificial Sequence

<220>
<223>  an artificially synthesized sequence

<400>  543

Thr Ser Gly Arg Ser Trp Asn Pro
1               5


<210>  544
<211>  8
<212>  PRT
<213>  Artificial Sequence

<220>
<223>  an artificially synthesized sequence

<400>  544

Thr Ser Gly Arg Ser Tyr Asn Pro
1               5


<210>  545
<211>  8
<212>  PRT
<213>  Artificial Sequence

<220>

<223>  an artificially synthesized sequence

<400>  545

Thr Ser Gly Arg Ser Ala Ala Pro
1               5


<210>  546
<211>  8
<212>  PRT
<213>  Artificial Sequence

<220>
<223>  an artificially synthesized sequence

<400>  546

Thr Ser Gly Arg Ser Ala Asp Pro
1               5


<210>  547
<211>  8
<212>  PRT
<213>  Artificial Sequence

<220>
<223>  an artificially synthesized sequence

<400>  547

Thr Ser Gly Arg Ser Ala Glu Pro
1               5


<210>  548
<211>  8
<212>  PRT
<213>  Artificial Sequence

<220>
<223>  an artificially synthesized sequence

<400>  548

Thr Ser Gly Arg Ser Ala Phe Pro
1               5


<210>  549
<211>  8
<212>  PRT
<213>  Artificial Sequence

<220>
<223>  an artificially synthesized sequence

<400>  549

Thr Ser Gly Arg Ser Ala Gly Pro
1               5

<210>  550
<211>  8
<212>  PRT
<213>  Artificial Sequence

<220>
<223>  an artificially synthesized sequence

<400>  550

Thr Ser Gly Arg Ser Ala Lys Pro
1               5


<210>  551
<211>  8
<212>  PRT
<213>  Artificial Sequence

<220>
<223>  an artificially synthesized sequence

<400>  551

Thr Ser Gly Arg Ser Ala Leu Pro
1               5


<210>  552
<211>  8
<212>  PRT
<213>  Artificial Sequence

<220>
<223>  an artificially synthesized sequence

<400>  552

Thr Ser Gly Arg Ser Ala Met Pro
1               5


<210>  553
<211>  8
<212>  PRT
<213>  Artificial Sequence

<220>
<223>  an artificially synthesized sequence

<400>  553

Thr Ser Gly Arg Ser Ala Pro Pro
1               5


<210>  554
<211>  8
<212>  PRT
<213>  Artificial Sequence

<220>

<223>  an artificially synthesized sequence

<400>  554

Thr Ser Gly Arg Ser Ala Gln Pro
1               5


<210>  555
<211>  8
<212>  PRT
<213>  Artificial Sequence

<220>
<223>  an artificially synthesized sequence

<400>  555

Thr Ser Gly Arg Ser Ala Val Pro
1               5


<210>  556
<211>  8
<212>  PRT
<213>  Artificial Sequence

<220>
<223>  an artificially synthesized sequence

<400>  556

Thr Ser Gly Arg Ser Ala Trp Pro
1               5


<210>  557
<211>  8
<212>  PRT
<213>  Artificial Sequence

<220>
<223>  an artificially synthesized sequence

<400>  557

Thr Ser Gly Arg Ser Ala Tyr Pro
1               5


<210>  558
<211>  8
<212>  PRT
<213>  Artificial Sequence

<220>
<223>  an artificially synthesized sequence

<400>  558

Thr Ser Gly Arg Ser Ala Asn Ala
1               5

<210>  559
<211>  8
<212>  PRT
<213>  Artificial Sequence

<220>
<223>  an artificially synthesized sequence

<400>  559

Thr Ser Gly Arg Ser Ala Asn Asp
1               5


<210>  560
<211>  8
<212>  PRT
<213>  Artificial Sequence

<220>
<223>  an artificially synthesized sequence

<400>  560

Thr Ser Gly Arg Ser Ala Asn Glu
1               5


<210>  561
<211>  8
<212>  PRT
<213>  Artificial Sequence

<220>
<223>  an artificially synthesized sequence

<400>  561

Thr Ser Gly Arg Ser Ala Asn Phe
1               5


<210>  562
<211>  8
<212>  PRT
<213>  Artificial Sequence

<220>
<223>  an artificially synthesized sequence

<400>  562

Thr Ser Gly Arg Ser Ala Asn Gly
1               5


<210>  563
<211>  8
<212>  PRT
<213>  Artificial Sequence

<220>

<223>  an artificially synthesized sequence

<400>  563

Thr Ser Gly Arg Ser Ala Asn Ile
1               5


<210>  564
<211>  8
<212>  PRT
<213>  Artificial Sequence

<220>
<223>  an artificially synthesized sequence

<400>  564

Thr Ser Gly Arg Ser Ala Asn Lys
1               5


<210>  565
<211>  8
<212>  PRT
<213>  Artificial Sequence

<220>
<223>  an artificially synthesized sequence

<400>  565

Thr Ser Gly Arg Ser Ala Asn Asn
1               5


<210>  566
<211>  8
<212>  PRT
<213>  Artificial Sequence

<220>
<223>  an artificially synthesized sequence

<400>  566

Thr Ser Gly Arg Ser Ala Asn Gln
1               5


<210>  567
<211>  8
<212>  PRT
<213>  Artificial Sequence

<220>
<223>  an artificially synthesized sequence

<400>  567

Thr Ser Gly Arg Ser Ala Asn Ser
1               5

<210>  568
<211>  8
<212>  PRT
<213>  Artificial Sequence

<220>
<223>  an artificially synthesized sequence

<400>  568

Thr Ser Gly Arg Ser Ala Asn Thr
1               5


<210>  569
<211>  8
<212>  PRT
<213>  Artificial Sequence

<220>
<223>  an artificially synthesized sequence

<400>  569

Thr Ser Gly Arg Ser Ala Asn Trp
1               5


<210>  570
<211>  8
<212>  PRT
<213>  Artificial Sequence

<220>
<223>  an artificially synthesized sequence

<400>  570

Asp Ser Gly Arg Ser Ala Asn Pro
1               5


<210>  571
<211>  8
<212>  PRT
<213>  Artificial Sequence

<220>
<223>  an artificially synthesized sequence

<400>  571

Ile Ser Gly Arg Ser Ala Asn Pro
1               5


<210>  572
<211>  8
<212>  PRT
<213>  Artificial Sequence

<220>

<223>  an artificially synthesized sequence

<400>  572

Ser Ser Gly Arg Ser Ala Asn Pro
1               5


<210>  573
<211>  8
<212>  PRT
<213>  Artificial Sequence

<220>
<223>  an artificially synthesized sequence

<400>  573

Thr His Gly Arg Ser Ala Asn Pro
1               5


<210>  574
<211>  8
<212>  PRT
<213>  Artificial Sequence

<220>
<223>  an artificially synthesized sequence

<400>  574

Thr Lys Gly Arg Ser Ala Asn Pro
1               5


<210>  575
<211>  8
<212>  PRT
<213>  Artificial Sequence

<220>
<223>  an artificially synthesized sequence

<400>  575

Thr Thr Gly Arg Ser Ala Asn Pro
1               5


<210>  576
<211>  8
<212>  PRT
<213>  Artificial Sequence

<220>
<223>  an artificially synthesized sequence

<400>  576

Thr Tyr Gly Arg Ser Ala Asn Pro
1               5

<210>  577
<211>  8
<212>  PRT
<213>  Artificial Sequence

<220>
<223>  an artificially synthesized sequence

<400>  577

Thr Ser Asp Arg Ser Ala Asn Pro
1               5


<210>  578
<211>  8
<212>  PRT
<213>  Artificial Sequence

<220>
<223>  an artificially synthesized sequence

<400>  578

Thr Ser Ser Arg Ser Ala Asn Pro
1               5


<210>  579
<211>  8
<212>  PRT
<213>  Artificial Sequence

<220>
<223>  an artificially synthesized sequence

<400>  579

Thr Ser Gly Arg Phe Ala Asn Pro
1               5


<210>  580
<211>  8
<212>  PRT
<213>  Artificial Sequence

<220>
<223>  an artificially synthesized sequence

<400>  580

Thr Ser Gly Arg Ser Asp Asn Pro
1               5


<210>  581
<211>  8
<212>  PRT
<213>  Artificial Sequence

<220>

<223>  an artificially synthesized sequence

<400>  581

Thr Ser Gly Arg Ser His Asn Pro
1               5


<210>  582
<211>  8
<212>  PRT
<213>  Artificial Sequence

<220>
<223>  an artificially synthesized sequence

<400>  582

Thr Ser Gly Arg Ser Ile Asn Pro
1               5


<210>  583
<211>  8
<212>  PRT
<213>  Artificial Sequence

<220>
<223>  an artificially synthesized sequence

<400>  583

Thr Ser Gly Arg Ser Leu Asn Pro
1               5


<210>  584
<211>  8
<212>  PRT
<213>  Artificial Sequence

<220>
<223>  an artificially synthesized sequence

<400>  584

Thr Ser Gly Arg Ser Thr Asn Pro
1               5


<210>  585
<211>  8
<212>  PRT
<213>  Artificial Sequence

<220>
<223>  an artificially synthesized sequence

<400>  585

Thr Ser Gly Arg Ser Val Asn Pro
1               5

<210>  586
<211>  8
<212>  PRT
<213>  Artificial Sequence

<220>
<223>  an artificially synthesized sequence

<400>  586

Thr Ser Gly Arg Ser Ala His Pro
1               5


<210>  587
<211>  8
<212>  PRT
<213>  Artificial Sequence

<220>
<223>  an artificially synthesized sequence

<400>  587

Thr Ser Gly Arg Ser Ala Ile Pro
1               5


<210>  588
<211>  8
<212>  PRT
<213>  Artificial Sequence

<220>
<223>  an artificially synthesized sequence

<400>  588

Thr Ser Gly Arg Ser Ala Arg Pro
1               5


<210>  589
<211>  8
<212>  PRT
<213>  Artificial Sequence

<220>
<223>  an artificially synthesized sequence

<400>  589

Thr Ser Gly Arg Ser Ala Ser Pro
1               5


<210>  590
<211>  8
<212>  PRT
<213>  Artificial Sequence

<220>

<223>  an artificially synthesized sequence

<400>  590

Thr Ser Gly Arg Ser Ala Thr Pro
1               5


<210>  591
<211>  8
<212>  PRT
<213>  Artificial Sequence

<220>
<223>  an artificially synthesized sequence

<400>  591

Thr Ser Gly Arg Ser Ala Asn His
1               5


<210>  592
<211>  8
<212>  PRT
<213>  Artificial Sequence

<220>
<223>  an artificially synthesized sequence

<400>  592

Thr Ser Gly Arg Ser Ala Asn Leu
1               5


<210>  593
<211>  8
<212>  PRT
<213>  Artificial Sequence

<220>
<223>  an artificially synthesized sequence

<400>  593

Thr Ser Gly Arg Ser Ala Asn Met
1               5


<210>  594
<211>  8
<212>  PRT
<213>  Artificial Sequence

<220>
<223>  an artificially synthesized sequence

<400>  594

Thr Ser Gly Arg Ser Ala Asn Arg
1               5

<210>  595
<211>  8
<212>  PRT
<213>  Artificial Sequence

<220>
<223>  an artificially synthesized sequence

<400>  595

Thr Ser Gly Arg Ser Ala Asn Val
1               5


<210>  596
<211>  8
<212>  PRT
<213>  Artificial Sequence

<220>
<223>  an artificially synthesized sequence

<400>  596

Thr Ser Gly Arg Ser Ala Asn Tyr
1               5


<210>  597
<211>  8
<212>  PRT
<213>  Artificial Sequence

<220>
<223>  an artificially synthesized sequence

<400>  597

Gly Ser Gly Arg Ser Ala Val Pro
1               5


<210>  598
<211>  8
<212>  PRT
<213>  Artificial Sequence

<220>
<223>  an artificially synthesized sequence

<400>  598

Gly Ser Gly Arg Ser Ala Tyr Pro
1               5


<210>  599
<211>  8
<212>  PRT
<213>  Artificial Sequence

<220>

<223>  an artificially synthesized sequence

<400>  599

Gly Ser Gly Arg Ser Ala Asn Gln
1               5


<210>  600
<211>  8
<212>  PRT
<213>  Artificial Sequence

<220>
<223>  an artificially synthesized sequence

<400>  600

Gly Ser Gly Arg Ser Ala Asn Ala
1               5


<210>  601
<211>  8
<212>  PRT
<213>  Artificial Sequence

<220>
<223>  an artificially synthesized sequence

<400>  601

Gly Ser Gly Arg Ser Ala Asn Ile
1               5


<210>  602
<211>  8
<212>  PRT
<213>  Artificial Sequence

<220>
<223>  an artificially synthesized sequence

<400>  602

Gly Ser Gly Arg Ser Ala Asn Phe
1               5


<210>  603
<211>  8
<212>  PRT
<213>  Artificial Sequence

<220>
<223>  an artificially synthesized sequence

<400>  603

Gly Ser Gly Arg Ser Ala Asn Ser
1               5

<210>  604
<211>  8
<212>  PRT
<213>  Artificial Sequence

<220>
<223>  an artificially synthesized sequence

<400>  604

Gln Ser Gly Arg Ser Ala Val Pro
1               5


<210>  605
<211>  8
<212>  PRT
<213>  Artificial Sequence

<220>
<223>  an artificially synthesized sequence

<400>  605

Gln Ser Gly Arg Ser Ala Tyr Pro
1               5


<210>  606
<211>  8
<212>  PRT
<213>  Artificial Sequence

<220>
<223>  an artificially synthesized sequence

<400>  606

Gln Ser Gly Arg Ser Ala Asn Gln
1               5


<210>  607
<211>  8
<212>  PRT
<213>  Artificial Sequence

<220>
<223>  an artificially synthesized sequence

<400>  607

Gln Ser Gly Arg Ser Ala Asn Ala
1               5


<210>  608
<211>  8
<212>  PRT
<213>  Artificial Sequence

<220>

<223>  an artificially synthesized sequence

<400>  608

Gln Ser Gly Arg Ser Ala Asn Ile
1               5


<210>  609
<211>  8
<212>  PRT
<213>  Artificial Sequence

<220>
<223>  an artificially synthesized sequence

<400>  609

Gln Ser Gly Arg Ser Ala Asn Phe
1               5


<210>  610
<211>  8
<212>  PRT
<213>  Artificial Sequence

<220>
<223>  an artificially synthesized sequence

<400>  610

Gln Ser Gly Arg Ser Ala Asn Ser
1               5


<210>  611
<211>  8
<212>  PRT
<213>  Artificial Sequence

<220>
<223>  an artificially synthesized sequence

<400>  611

Pro Ser Gly Arg Ser Ala Val Pro
1               5


<210>  612
<211>  8
<212>  PRT
<213>  Artificial Sequence

<220>
<223>  an artificially synthesized sequence

<400>  612

Pro Ser Gly Arg Ser Ala Tyr Pro
1               5

<210>  613
<211>  8
<212>  PRT
<213>  Artificial Sequence

<220>
<223>  an artificially synthesized sequence

<400>  613

Pro Ser Gly Arg Ser Ala Asn Gln
1               5


<210>  614
<211>  8
<212>  PRT
<213>  Artificial Sequence

<220>
<223>  an artificially synthesized sequence

<400>  614

Pro Ser Gly Arg Ser Ala Asn Ala
1               5


<210>  615
<211>  8
<212>  PRT
<213>  Artificial Sequence

<220>
<223>  an artificially synthesized sequence

<400>  615

Pro Ser Gly Arg Ser Ala Asn Ile
1               5


<210>  616
<211>  8
<212>  PRT
<213>  Artificial Sequence

<220>
<223>  an artificially synthesized sequence

<400>  616

Pro Ser Gly Arg Ser Ala Asn Phe
1               5


<210>  617
<211>  8
<212>  PRT
<213>  Artificial Sequence

<220>

<223>  an artificially synthesized sequence

<400>  617

Pro Ser Gly Arg Ser Ala Asn Ser
1               5


<210>  618
<211>  8
<212>  PRT
<213>  Artificial Sequence

<220>
<223>  an artificially synthesized sequence

<400>  618

Ala Ser Gly Arg Ser Ala Val Pro
1               5


<210>  619
<211>  8
<212>  PRT
<213>  Artificial Sequence

<220>
<223>  an artificially synthesized sequence

<400>  619

Ala Ser Gly Arg Ser Ala Tyr Pro
1               5


<210>  620
<211>  8
<212>  PRT
<213>  Artificial Sequence

<220>
<223>  an artificially synthesized sequence

<400>  620

Ala Ser Gly Arg Ser Ala Asn Gln
1               5


<210>  621
<211>  8
<212>  PRT
<213>  Artificial Sequence

<220>
<223>  an artificially synthesized sequence

<400>  621

Ala Ser Gly Arg Ser Ala Asn Ala
1               5

<210>  622
<211>  8
<212>  PRT
<213>  Artificial Sequence

<220>
<223>  an artificially synthesized sequence

<400>  622

Ala Ser Gly Arg Ser Ala Asn Ile
1               5


<210>  623
<211>  8
<212>  PRT
<213>  Artificial Sequence

<220>
<223>  an artificially synthesized sequence

<400>  623

Ala Ser Gly Arg Ser Ala Asn Phe
1               5


<210>  624
<211>  8
<212>  PRT
<213>  Artificial Sequence

<220>
<223>  an artificially synthesized sequence

<400>  624

Ala Ser Gly Arg Ser Ala Asn Ser
1               5


<210>  625
<211>  8
<212>  PRT
<213>  Artificial Sequence

<220>
<223>  an artificially synthesized sequence

<400>  625

Tyr Ser Gly Arg Ser Glu Asn Pro
1               5


<210>  626
<211>  8
<212>  PRT
<213>  Artificial Sequence

<220>

<223>  an artificially synthesized sequence

<400>  626

Gly Ser Gly Arg Ser Glu Asn Pro
1               5


<210>  627
<211>  8
<212>  PRT
<213>  Artificial Sequence

<220>
<223>  an artificially synthesized sequence

<400>  627

Gln Ser Gly Arg Ser Glu Asn Pro
1               5


<210>  628
<211>  8
<212>  PRT
<213>  Artificial Sequence

<220>
<223>  an artificially synthesized sequence

<400>  628

Pro Ser Gly Arg Ser Glu Asn Pro
1               5


<210>  629
<211>  8
<212>  PRT
<213>  Artificial Sequence

<220>
<223>  an artificially synthesized sequence

<400>  629

Ala Ser Gly Arg Ser Glu Asn Pro
1               5


<210>  630
<211>  8
<212>  PRT
<213>  Artificial Sequence

<220>
<223>  an artificially synthesized sequence

<400>  630

His Ser Gly Arg Ser Glu Asn Pro
1               5

<210>  631
<211>  8
<212>  PRT
<213>  Artificial Sequence

<220>
<223>  an artificially synthesized sequence

<400>  631

Thr Ser Gly Arg Ser Glu Asn Gln
1               5


<210>  632
<211>  8
<212>  PRT
<213>  Artificial Sequence

<220>
<223>  an artificially synthesized sequence

<400>  632

Thr Ser Gly Arg Ser Glu Asn Ala
1               5


<210>  633
<211>  8
<212>  PRT
<213>  Artificial Sequence

<220>
<223>  an artificially synthesized sequence

<400>  633

Thr Ser Gly Arg Ser Glu Asn Ile
1               5


<210>  634
<211>  8
<212>  PRT
<213>  Artificial Sequence

<220>
<223>  an artificially synthesized sequence

<400>  634

Thr Ser Gly Arg Ser Glu Asn Phe
1               5


<210>  635
<211>  8
<212>  PRT
<213>  Artificial Sequence

<220>

<223>  an artificially synthesized sequence

<400>  635

Thr Ser Gly Arg Ser Glu Asn Ser
1               5


<210>  636
<211>  8
<212>  PRT
<213>  Artificial Sequence

<220>
<223>  an artificially synthesized sequence

<400>  636

Tyr Ser Gly Arg Ser Ala Glu Pro
1               5


<210>  637
<211>  8
<212>  PRT
<213>  Artificial Sequence

<220>
<223>  an artificially synthesized sequence

<400>  637

Gly Ser Gly Arg Ser Ala Glu Pro
1               5


<210>  638
<211>  8
<212>  PRT
<213>  Artificial Sequence

<220>
<223>  an artificially synthesized sequence

<400>  638

Gln Ser Gly Arg Ser Ala Glu Pro
1               5


<210>  639
<211>  8
<212>  PRT
<213>  Artificial Sequence

<220>
<223>  an artificially synthesized sequence

<400>  639

Pro Ser Gly Arg Ser Ala Glu Pro
1               5

<210>  640
<211>  8
<212>  PRT
<213>  Artificial Sequence

<220>
<223>  an artificially synthesized sequence

<400>  640

Ala Ser Gly Arg Ser Ala Glu Pro
1               5


<210>  641
<211>  8
<212>  PRT
<213>  Artificial Sequence

<220>
<223>  an artificially synthesized sequence

<400>  641

His Ser Gly Arg Ser Ala Glu Pro
1               5


<210>  642
<211>  8
<212>  PRT
<213>  Artificial Sequence

<220>
<223>  an artificially synthesized sequence

<400>  642

Thr Ser Gly Arg Ser Ala Glu Gln
1               5


<210>  643
<211>  8
<212>  PRT
<213>  Artificial Sequence

<220>
<223>  an artificially synthesized sequence

<400>  643

Thr Ser Gly Arg Ser Ala Glu Ala
1               5


<210>  644
<211>  8
<212>  PRT
<213>  Artificial Sequence

<220>

<223>  an artificially synthesized sequence

<400>  644

Thr Ser Gly Arg Ser Ala Glu Ile
1               5


<210>  645
<211>  8
<212>  PRT
<213>  Artificial Sequence

<220>
<223>  an artificially synthesized sequence

<400>  645

Thr Ser Gly Arg Ser Ala Glu Phe
1               5


<210>  646
<211>  8
<212>  PRT
<213>  Artificial Sequence

<220>
<223>  an artificially synthesized sequence

<400>  646

Thr Ser Gly Arg Ser Ala Glu Ser
1               5


<210>  647
<211>  8
<212>  PRT
<213>  Artificial Sequence

<220>
<223>  an artificially synthesized sequence

<400>  647

Gly Thr Gly Arg Ser Ala Asn Pro
1               5


<210>  648
<211>  8
<212>  PRT
<213>  Artificial Sequence

<220>
<223>  an artificially synthesized sequence

<400>  648

Gly Lys Gly Arg Ser Ala Asn Pro
1               5

<210>  649
<211>  8
<212>  PRT
<213>  Artificial Sequence

<220>
<223>  an artificially synthesized sequence

<400>  649

Gly Ser Gly Arg Ser Ala Ile Pro
1               5


<210>  650
<211>  8
<212>  PRT
<213>  Artificial Sequence

<220>
<223>  an artificially synthesized sequence

<400>  650

Gly Ser Gly Arg Ser Ala Thr Pro
1               5


<210>  651
<211>  8
<212>  PRT
<213>  Artificial Sequence

<220>
<223>  an artificially synthesized sequence

<400>  651

Gly Ser Gly Arg Ser Ala Ser Pro
1               5


<210>  652
<211>  8
<212>  PRT
<213>  Artificial Sequence

<220>
<223>  an artificially synthesized sequence

<400>  652

Gly Ser Gly Arg Ser Ala His Pro
1               5


<210>  653
<211>  8
<212>  PRT
<213>  Artificial Sequence

<220>

<223>  an artificially synthesized sequence

<400>  653

Gly Ser Gly Arg Ser Ala Asn Tyr
1               5


<210>  654
<211>  8
<212>  PRT
<213>  Artificial Sequence

<220>
<223>  an artificially synthesized sequence

<400>  654

Gly Ser Gly Arg Ser Ala Asn Val
1               5


<210>  655
<211>  8
<212>  PRT
<213>  Artificial Sequence

<220>
<223>  an artificially synthesized sequence

<400>  655

Gly Ser Gly Arg Ser Ala Asn His
1               5


<210>  656
<211>  8
<212>  PRT
<213>  Artificial Sequence

<220>
<223>  an artificially synthesized sequence

<400>  656

Gln Thr Gly Arg Ser Ala Asn Pro
1               5


<210>  657
<211>  8
<212>  PRT
<213>  Artificial Sequence

<220>
<223>  an artificially synthesized sequence

<400>  657

Gln Lys Gly Arg Ser Ala Asn Pro
1               5

<210>  658
<211>  8
<212>  PRT
<213>  Artificial Sequence

<220>
<223>  an artificially synthesized sequence

<400>  658

Gln Ser Gly Arg Ser Ala Ile Pro
1               5


<210>  659
<211>  8
<212>  PRT
<213>  Artificial Sequence

<220>
<223>  an artificially synthesized sequence

<400>  659

Gln Ser Gly Arg Ser Ala Thr Pro
1               5


<210>  660
<211>  8
<212>  PRT
<213>  Artificial Sequence

<220>
<223>  an artificially synthesized sequence

<400>  660

Gln Ser Gly Arg Ser Ala Ser Pro
1               5


<210>  661
<211>  8
<212>  PRT
<213>  Artificial Sequence

<220>
<223>  an artificially synthesized sequence

<400>  661

Gln Ser Gly Arg Ser Ala His Pro
1               5


<210>  662
<211>  8
<212>  PRT
<213>  Artificial Sequence

<220>

<223>  an artificially synthesized sequence

<400>  662

Gln Ser Gly Arg Ser Ala Asn Tyr
1               5


<210>  663
<211>  8
<212>  PRT
<213>  Artificial Sequence

<220>
<223>  an artificially synthesized sequence

<400>  663

Gln Ser Gly Arg Ser Ala Asn Val
1               5


<210>  664
<211>  8
<212>  PRT
<213>  Artificial Sequence

<220>
<223>  an artificially synthesized sequence

<400>  664

Gln Ser Gly Arg Ser Ala Asn His
1               5


<210>  665
<211>  8
<212>  PRT
<213>  Artificial Sequence

<220>
<223>  an artificially synthesized sequence

<400>  665

Pro Thr Gly Arg Ser Ala Asn Pro
1               5


<210>  666
<211>  8
<212>  PRT
<213>  Artificial Sequence

<220>
<223>  an artificially synthesized sequence

<400>  666

Pro Lys Gly Arg Ser Ala Asn Pro
1               5

<210>  667
<211>  8
<212>  PRT
<213>  Artificial Sequence

<220>
<223>  an artificially synthesized sequence

<400>  667

Pro Ser Gly Arg Ser Ala Ile Pro
1               5


<210>  668
<211>  8
<212>  PRT
<213>  Artificial Sequence

<220>
<223>  an artificially synthesized sequence

<400>  668

Pro Ser Gly Arg Ser Ala Thr Pro
1               5


<210>  669
<211>  8
<212>  PRT
<213>  Artificial Sequence

<220>
<223>  an artificially synthesized sequence

<400>  669

Pro Ser Gly Arg Ser Ala Ser Pro
1               5


<210>  670
<211>  8
<212>  PRT
<213>  Artificial Sequence

<220>
<223>  an artificially synthesized sequence

<400>  670

Pro Ser Gly Arg Ser Ala His Pro
1               5


<210>  671
<211>  8
<212>  PRT
<213>  Artificial Sequence

<220>

<223>  an artificially synthesized sequence

<400>  671

Pro Ser Gly Arg Ser Ala Asn Tyr
1               5


<210>  672
<211>  8
<212>  PRT
<213>  Artificial Sequence

<220>
<223>  an artificially synthesized sequence

<400>  672

Pro Ser Gly Arg Ser Ala Asn Val
1               5


<210>  673
<211>  8
<212>  PRT
<213>  Artificial Sequence

<220>
<223>  an artificially synthesized sequence

<400>  673

Pro Ser Gly Arg Ser Ala Asn His
1               5


<210>  674
<211>  8
<212>  PRT
<213>  Artificial Sequence

<220>
<223>  an artificially synthesized sequence

<400>  674

Ala Thr Gly Arg Ser Ala Asn Pro
1               5


<210>  675
<211>  8
<212>  PRT
<213>  Artificial Sequence

<220>
<223>  an artificially synthesized sequence

<400>  675

Ala Lys Gly Arg Ser Ala Asn Pro
1               5

<210>  676
<211>  8
<212>  PRT
<213>  Artificial Sequence

<220>
<223>  an artificially synthesized sequence

<400>  676

Ala Ser Gly Arg Ser Ala Ile Pro
1               5


<210>  677
<211>  8
<212>  PRT
<213>  Artificial Sequence

<220>
<223>  an artificially synthesized sequence

<400>  677

Ala Ser Gly Arg Ser Ala Thr Pro
1               5


<210>  678
<211>  8
<212>  PRT
<213>  Artificial Sequence

<220>
<223>  an artificially synthesized sequence

<400>  678

Ala Ser Gly Arg Ser Ala Ser Pro
1               5


<210>  679
<211>  8
<212>  PRT
<213>  Artificial Sequence

<220>
<223>  an artificially synthesized sequence

<400>  679

Ala Ser Gly Arg Ser Ala His Pro
1               5


<210>  680
<211>  8
<212>  PRT
<213>  Artificial Sequence

<220>

<223>  an artificially synthesized sequence

<400>  680

Ala Ser Gly Arg Ser Ala Asn Tyr
1               5


<210>  681
<211>  8
<212>  PRT
<213>  Artificial Sequence

<220>
<223>  an artificially synthesized sequence

<400>  681

Ala Ser Gly Arg Ser Ala Asn Val
1               5


<210>  682
<211>  8
<212>  PRT
<213>  Artificial Sequence

<220>
<223>  an artificially synthesized sequence

<400>  682

Ala Ser Gly Arg Ser Ala Asn His
1               5


<210>  683
<211>  8
<212>  PRT
<213>  Artificial Sequence

<220>
<223>  an artificially synthesized sequence

<400>  683

Tyr Thr Gly Arg Ser Ala Asn Pro
1               5


<210>  684
<211>  8
<212>  PRT
<213>  Artificial Sequence

<220>
<223>  an artificially synthesized sequence

<400>  684

Tyr Lys Gly Arg Ser Ala Asn Pro
1               5

<210>  685
<211>  8
<212>  PRT
<213>  Artificial Sequence

<220>
<223>  an artificially synthesized sequence

<400>  685

Tyr Ser Gly Arg Ser Ala Val Pro
1               5


<210>  686
<211>  8
<212>  PRT
<213>  Artificial Sequence

<220>
<223>  an artificially synthesized sequence

<400>  686

Tyr Ser Gly Arg Ser Ala Ile Pro
1               5


<210>  687
<211>  8
<212>  PRT
<213>  Artificial Sequence

<220>
<223>  an artificially synthesized sequence

<400>  687

Tyr Ser Gly Arg Ser Ala Thr Pro
1               5


<210>  688
<211>  8
<212>  PRT
<213>  Artificial Sequence

<220>
<223>  an artificially synthesized sequence

<400>  688

Tyr Ser Gly Arg Ser Ala Ser Pro
1               5


<210>  689
<211>  8
<212>  PRT
<213>  Artificial Sequence

<220>

<223>  an artificially synthesized sequence

<400>  689

Tyr Ser Gly Arg Ser Ala His Pro
1               5


<210>  690
<211>  8
<212>  PRT
<213>  Artificial Sequence

<220>
<223>  an artificially synthesized sequence

<400>  690

Tyr Ser Gly Arg Ser Ala Asn Ala
1               5


<210>  691
<211>  8
<212>  PRT
<213>  Artificial Sequence

<220>
<223>  an artificially synthesized sequence

<400>  691

Tyr Ser Gly Arg Ser Ala Asn Phe
1               5


<210>  692
<211>  8
<212>  PRT
<213>  Artificial Sequence

<220>
<223>  an artificially synthesized sequence

<400>  692

Tyr Ser Gly Arg Ser Ala Asn Tyr
1               5


<210>  693
<211>  8
<212>  PRT
<213>  Artificial Sequence

<220>
<223>  an artificially synthesized sequence

<400>  693

Tyr Ser Gly Arg Ser Ala Asn Val
1               5

<210>  694
<211>  8
<212>  PRT
<213>  Artificial Sequence

<220>
<223>  an artificially synthesized sequence

<400>  694

Tyr Ser Gly Arg Ser Ala Asn His
1               5


<210>  695
<211>  8
<212>  PRT
<213>  Artificial Sequence

<220>
<223>  an artificially synthesized sequence

<400>  695

Ser Thr Gly Arg Ser Ala Asn Pro
1               5


<210>  696
<211>  8
<212>  PRT
<213>  Artificial Sequence

<220>
<223>  an artificially synthesized sequence

<400>  696

Ser Lys Gly Arg Ser Ala Asn Pro
1               5


<210>  697
<211>  8
<212>  PRT
<213>  Artificial Sequence

<220>
<223>  an artificially synthesized sequence

<400>  697

Ser Ser Gly Arg Ser Ala Val Pro
1               5


<210>  698
<211>  8
<212>  PRT
<213>  Artificial Sequence

<220>

<223>  an artificially synthesized sequence

<400>  698

Ser Ser Gly Arg Ser Ala Ile Pro
1               5


<210>  699
<211>  8
<212>  PRT
<213>  Artificial Sequence

<220>
<223>  an artificially synthesized sequence

<400>  699

Ser Ser Gly Arg Ser Ala Thr Pro
1               5


<210>  700
<211>  8
<212>  PRT
<213>  Artificial Sequence

<220>
<223>  an artificially synthesized sequence

<400>  700

Ser Ser Gly Arg Ser Ala Ser Pro
1               5


<210>  701
<211>  8
<212>  PRT
<213>  Artificial Sequence

<220>
<223>  an artificially synthesized sequence

<400>  701

Ser Ser Gly Arg Ser Ala His Pro
1               5


<210>  702
<211>  8
<212>  PRT
<213>  Artificial Sequence

<220>
<223>  an artificially synthesized sequence

<400>  702

Ser Ser Gly Arg Ser Ala Asn Ala
1               5

<210>  703
<211>  8
<212>  PRT
<213>  Artificial Sequence

<220>
<223>  an artificially synthesized sequence

<400>  703

Ser Ser Gly Arg Ser Ala Asn Phe
1               5


<210>  704
<211>  8
<212>  PRT
<213>  Artificial Sequence

<220>
<223>  an artificially synthesized sequence

<400>  704

Ser Ser Gly Arg Ser Ala Asn Tyr
1               5


<210>  705
<211>  8
<212>  PRT
<213>  Artificial Sequence

<220>
<223>  an artificially synthesized sequence

<400>  705

Ser Ser Gly Arg Ser Ala Asn Val
1               5


<210>  706
<211>  8
<212>  PRT
<213>  Artificial Sequence

<220>
<223>  an artificially synthesized sequence

<400>  706

Ser Ser Gly Arg Ser Ala Asn His
1               5


<210>  707
<211>  8
<212>  PRT
<213>  Artificial Sequence

<220>

<223>  an artificially synthesized sequence

<400>  707

Ile Thr Gly Arg Ser Ala Asn Pro
1               5


<210>  708
<211>  8
<212>  PRT
<213>  Artificial Sequence

<220>
<223>  an artificially synthesized sequence

<400>  708

Ile Lys Gly Arg Ser Ala Asn Pro
1               5


<210>  709
<211>  8
<212>  PRT
<213>  Artificial Sequence

<220>
<223>  an artificially synthesized sequence

<400>  709

Ile Ser Gly Arg Ser Ala Val Pro
1               5


<210>  710
<211>  8
<212>  PRT
<213>  Artificial Sequence

<220>
<223>  an artificially synthesized sequence

<400>  710

Ile Ser Gly Arg Ser Ala Ile Pro
1               5


<210>  711
<211>  8
<212>  PRT
<213>  Artificial Sequence

<220>
<223>  an artificially synthesized sequence

<400>  711

Ile Ser Gly Arg Ser Ala Thr Pro
1               5

<210>  712
<211>  8
<212>  PRT
<213>  Artificial Sequence

<220>
<223>  an artificially synthesized sequence

<400>  712

Ile Ser Gly Arg Ser Ala Ser Pro
1               5


<210>  713
<211>  8
<212>  PRT
<213>  Artificial Sequence

<220>
<223>  an artificially synthesized sequence

<400>  713

Ile Ser Gly Arg Ser Ala His Pro
1               5


<210>  714
<211>  8
<212>  PRT
<213>  Artificial Sequence

<220>
<223>  an artificially synthesized sequence

<400>  714

Ile Ser Gly Arg Ser Ala Asn Ala
1               5


<210>  715
<211>  8
<212>  PRT
<213>  Artificial Sequence

<220>
<223>  an artificially synthesized sequence

<400>  715

Ile Ser Gly Arg Ser Ala Asn Phe
1               5


<210>  716
<211>  8
<212>  PRT
<213>  Artificial Sequence

<220>

<223>  an artificially synthesized sequence

<400>  716

Ile Ser Gly Arg Ser Ala Asn Tyr
1               5


<210>  717
<211>  8
<212>  PRT
<213>  Artificial Sequence

<220>
<223>  an artificially synthesized sequence

<400>  717

Ile Ser Gly Arg Ser Ala Asn Val
1               5


<210>  718
<211>  8
<212>  PRT
<213>  Artificial Sequence

<220>
<223>  an artificially synthesized sequence

<400>  718

Ile Ser Gly Arg Ser Ala Asn His
1               5


<210>  719
<211>  8
<212>  PRT
<213>  Artificial Sequence

<220>
<223>  an artificially synthesized sequence

<400>  719

Thr Thr Gly Arg Ser Ala Val Pro
1               5


<210>  720
<211>  8
<212>  PRT
<213>  Artificial Sequence

<220>
<223>  an artificially synthesized sequence

<400>  720

Thr Thr Gly Arg Ser Ala Ile Pro
1               5

<210>  721
<211>  8
<212>  PRT
<213>  Artificial Sequence

<220>
<223>  an artificially synthesized sequence

<400>  721

Thr Thr Gly Arg Ser Ala Thr Pro
1               5


<210>  722
<211>  8
<212>  PRT
<213>  Artificial Sequence

<220>
<223>  an artificially synthesized sequence

<400>  722

Thr Thr Gly Arg Ser Ala Ser Pro
1               5


<210>  723
<211>  8
<212>  PRT
<213>  Artificial Sequence

<220>
<223>  an artificially synthesized sequence

<400>  723

Thr Thr Gly Arg Ser Ala His Pro
1               5


<210>  724
<211>  8
<212>  PRT
<213>  Artificial Sequence

<220>
<223>  an artificially synthesized sequence

<400>  724

Thr Thr Gly Arg Ser Ala Asn Ala
1               5


<210>  725
<211>  8
<212>  PRT
<213>  Artificial Sequence

<220>

<223>  an artificially synthesized sequence

<400>  725

Thr Thr Gly Arg Ser Ala Asn Phe
1               5

<210>  726
<211>  8
<212>  PRT
<213>  Artificial Sequence

<220>
<223>  an artificially synthesized sequence

<400>  726

Thr Thr Gly Arg Ser Ala Asn Tyr
1               5

<210>  727
<211>  8
<212>  PRT
<213>  Artificial Sequence

<220>
<223>  an artificially synthesized sequence

<400>  727

Thr Thr Gly Arg Ser Ala Asn Val
1               5

<210>  728
<211>  8
<212>  PRT
<213>  Artificial Sequence

<220>
<223>  an artificially synthesized sequence

<400>  728

Thr Thr Gly Arg Ser Ala Asn His
1               5

<210>  729
<211>  8
<212>  PRT
<213>  Artificial Sequence

<220>
<223>  an artificially synthesized sequence

<400>  729

Thr Lys Gly Arg Ser Ala Val Pro
1               5

<210>  730
<211>  8
<212>  PRT
<213>  Artificial Sequence

<220>
<223>  an artificially synthesized sequence

<400>  730

Thr Lys Gly Arg Ser Ala Ile Pro
1               5


<210>  731
<211>  8
<212>  PRT
<213>  Artificial Sequence

<220>
<223>  an artificially synthesized sequence

<400>  731

Thr Lys Gly Arg Ser Ala Thr Pro
1               5


<210>  732
<211>  8
<212>  PRT
<213>  Artificial Sequence

<220>
<223>  an artificially synthesized sequence

<400>  732

Thr Lys Gly Arg Ser Ala Ser Pro
1               5


<210>  733
<211>  8
<212>  PRT
<213>  Artificial Sequence

<220>
<223>  an artificially synthesized sequence

<400>  733

Thr Lys Gly Arg Ser Ala His Pro
1               5


<210>  734
<211>  8
<212>  PRT
<213>  Artificial Sequence

<220>

<223>  an artificially synthesized sequence

<400>  734

Thr Lys Gly Arg Ser Ala Asn Ala
1               5


<210>  735
<211>  8
<212>  PRT
<213>  Artificial Sequence

<220>
<223>  an artificially synthesized sequence

<400>  735

Thr Lys Gly Arg Ser Ala Asn Phe
1               5


<210>  736
<211>  8
<212>  PRT
<213>  Artificial Sequence

<220>
<223>  an artificially synthesized sequence

<400>  736

Thr Lys Gly Arg Ser Ala Asn Tyr
1               5


<210>  737
<211>  8
<212>  PRT
<213>  Artificial Sequence

<220>
<223>  an artificially synthesized sequence

<400>  737

Thr Lys Gly Arg Ser Ala Asn Val
1               5


<210>  738
<211>  8
<212>  PRT
<213>  Artificial Sequence

<220>
<223>  an artificially synthesized sequence

<400>  738

Thr Lys Gly Arg Ser Ala Asn His
1               5

<210>  739
<211>  8
<212>  PRT
<213>  Artificial Sequence

<220>
<223>  an artificially synthesized sequence

<400>  739

Thr Ser Gly Arg Ser Ala Val Tyr
1               5


<210>  740
<211>  8
<212>  PRT
<213>  Artificial Sequence

<220>
<223>  an artificially synthesized sequence

<400>  740

Thr Ser Gly Arg Ser Ala Val Val
1               5


<210>  741
<211>  8
<212>  PRT
<213>  Artificial Sequence

<220>
<223>  an artificially synthesized sequence

<400>  741

Thr Ser Gly Arg Ser Ala Val His
1               5


<210>  742
<211>  8
<212>  PRT
<213>  Artificial Sequence

<220>
<223>  an artificially synthesized sequence

<400>  742

Thr Ser Gly Arg Ser Ala Ile Tyr
1               5


<210>  743
<211>  8
<212>  PRT
<213>  Artificial Sequence

<220>

<223>  an artificially synthesized sequence

<400>  743

Thr Ser Gly Arg Ser Ala Ile Val
1               5


<210>  744
<211>  8
<212>  PRT
<213>  Artificial Sequence

<220>
<223>  an artificially synthesized sequence

<400>  744

Thr Ser Gly Arg Ser Ala Ile His
1               5


<210>  745
<211>  8
<212>  PRT
<213>  Artificial Sequence

<220>
<223>  an artificially synthesized sequence

<400>  745

Thr Ser Gly Arg Ser Ala Ser Tyr
1               5


<210>  746
<211>  8
<212>  PRT
<213>  Artificial Sequence

<220>
<223>  an artificially synthesized sequence

<400>  746

Thr Ser Gly Arg Ser Ala Ser Val
1               5


<210>  747
<211>  8
<212>  PRT
<213>  Artificial Sequence

<220>
<223>  an artificially synthesized sequence

<400>  747

Thr Ser Gly Arg Ser Ala Ser His
1               5

<210>  748
<211>  8
<212>  PRT
<213>  Artificial Sequence

<220>
<223>  an artificially synthesized sequence

<400>  748

Thr Ser Gly Arg Ser Ala His Tyr
1               5


<210>  749
<211>  8
<212>  PRT
<213>  Artificial Sequence

<220>
<223>  an artificially synthesized sequence

<400>  749

Thr Ser Gly Arg Ser Ala His Val
1               5


<210>  750
<211>  8
<212>  PRT
<213>  Artificial Sequence

<220>
<223>  an artificially synthesized sequence

<400>  750

Thr Ser Gly Arg Ser Ala His His
1               5


<210>  751
<211>  8
<212>  PRT
<213>  Artificial Sequence

<220>
<223>  an artificially synthesized sequence

<400>  751

Pro Ser Gly Arg Ser Glu Val Pro
1               5


<210>  752
<211>  8
<212>  PRT
<213>  Artificial Sequence

<220>

<223>  an artificially synthesized sequence

<400>  752

Pro Ser Gly Arg Ser Ala Glu Pro
1               5


<210>  753
<211>  8
<212>  PRT
<213>  Artificial Sequence

<220>
<223>  an artificially synthesized sequence

<400>  753

Pro Ser Gly Arg Ser Ala Gly Pro
1               5


<210>  754
<211>  8
<212>  PRT
<213>  Artificial Sequence

<220>
<223>  an artificially synthesized sequence

<400>  754

Ala Ser Gly Arg Ser Glu Asn Ala
1               5


<210>  755
<211>  8
<212>  PRT
<213>  Artificial Sequence

<220>
<223>  an artificially synthesized sequence

<400>  755

Ala Ser Gly Arg Ser Ala Glu Ala
1               5


<210>  756
<211>  8
<212>  PRT
<213>  Artificial Sequence

<220>
<223>  an artificially synthesized sequence

<400>  756

Ala Ser Gly Arg Ser Ala Gly Ala
1               5

<210>  757
<211>  8
<212>  PRT
<213>  Artificial Sequence

<220>
<223>  an artificially synthesized sequence

<400>  757

Gly Thr Gly Arg Ser Ala Thr Pro
1               5


<210>  758
<211>  8
<212>  PRT
<213>  Artificial Sequence

<220>
<223>  an artificially synthesized sequence

<400>  758

Gly Ser Gly Arg Ser Ala Thr Tyr
1               5


<210>  759
<211>  8
<212>  PRT
<213>  Artificial Sequence

<220>
<223>  an artificially synthesized sequence

<400>  759

Gly Ser Gly Arg Ser Ala Thr Val
1               5


<210>  760
<211>  8
<212>  PRT
<213>  Artificial Sequence

<220>
<223>  an artificially synthesized sequence

<400>  760

Gly Ser Gly Arg Ser Ala Thr His
1               5


<210>  761
<211>  8
<212>  PRT
<213>  Artificial Sequence

<220>

<223>  an artificially synthesized sequence

<400>  761

Gly Thr Gly Arg Ser Ala Thr Tyr
1               5


<210>  762
<211>  8
<212>  PRT
<213>  Artificial Sequence

<220>
<223>  an artificially synthesized sequence

<400>  762

Gly Thr Gly Arg Ser Ala Thr Val
1               5


<210>  763
<211>  8
<212>  PRT
<213>  Artificial Sequence

<220>
<223>  an artificially synthesized sequence

<400>  763

Gly Thr Gly Arg Ser Ala Thr His
1               5


<210>  764
<211>  8
<212>  PRT
<213>  Artificial Sequence

<220>
<223>  an artificially synthesized sequence

<400>  764

Gly Ser Gly Arg Ser Glu Thr Pro
1               5


<210>  765
<211>  8
<212>  PRT
<213>  Artificial Sequence

<220>
<223>  an artificially synthesized sequence

<400>  765

Gly Thr Gly Arg Ser Glu Thr Pro
1               5

<210>  766
<211>  8
<212>  PRT
<213>  Artificial Sequence

<220>
<223>  an artificially synthesized sequence

<400>  766

Gly Ser Gly Arg Ser Glu Thr Tyr
1               5


<210>  767
<211>  8
<212>  PRT
<213>  Artificial Sequence

<220>
<223>  an artificially synthesized sequence

<400>  767

Gly Ser Gly Arg Ser Glu Thr Val
1               5


<210>  768
<211>  8
<212>  PRT
<213>  Artificial Sequence

<220>
<223>  an artificially synthesized sequence

<400>  768

Gly Ser Gly Arg Ser Glu Thr His
1               5


<210>  769
<211>  8
<212>  PRT
<213>  Artificial Sequence

<220>
<223>  an artificially synthesized sequence

<400>  769

Tyr Thr Gly Arg Ser Ala Val Pro
1               5


<210>  770
<211>  8
<212>  PRT
<213>  Artificial Sequence

<220>

<223>  an artificially synthesized sequence

<400>  770

Tyr Ser Gly Arg Ser Ala Val Tyr
1               5

<210>  771
<211>  8
<212>  PRT
<213>  Artificial Sequence

<220>
<223>  an artificially synthesized sequence

<400>  771

Tyr Ser Gly Arg Ser Ala Val Val
1               5

<210>  772
<211>  8
<212>  PRT
<213>  Artificial Sequence

<220>
<223>  an artificially synthesized sequence

<400>  772

Tyr Ser Gly Arg Ser Ala Val His
1               5

<210>  773
<211>  8
<212>  PRT
<213>  Artificial Sequence

<220>
<223>  an artificially synthesized sequence

<400>  773

Tyr Thr Gly Arg Ser Ala Val Tyr
1               5

<210>  774
<211>  8
<212>  PRT
<213>  Artificial Sequence

<220>
<223>  an artificially synthesized sequence

<400>  774

Tyr Thr Gly Arg Ser Ala Val Val
1               5

<210>  775
<211>  8
<212>  PRT
<213>  Artificial Sequence

<220>
<223>  an artificially synthesized sequence

<400>  775

Tyr Thr Gly Arg Ser Ala Val His
1               5


<210>  776
<211>  8
<212>  PRT
<213>  Artificial Sequence

<220>
<223>  an artificially synthesized sequence

<400>  776

Tyr Ser Gly Arg Ser Glu Val Pro
1               5


<210>  777
<211>  8
<212>  PRT
<213>  Artificial Sequence

<220>
<223>  an artificially synthesized sequence

<400>  777

Tyr Thr Gly Arg Ser Glu Val Pro
1               5


<210>  778
<211>  8
<212>  PRT
<213>  Artificial Sequence

<220>
<223>  an artificially synthesized sequence

<400>  778

Tyr Ser Gly Arg Ser Glu Val Tyr
1               5


<210>  779
<211>  8
<212>  PRT
<213>  Artificial Sequence

<220>

<223>  an artificially synthesized sequence

<400>  779

Tyr Ser Gly Arg Ser Glu Val Val
1               5


<210>  780
<211>  8
<212>  PRT
<213>  Artificial Sequence

<220>
<223>  an artificially synthesized sequence

<400>  780

Tyr Ser Gly Arg Ser Glu Val His
1               5


<210>  781
<211>  8
<212>  PRT
<213>  Artificial Sequence

<220>
<223>  an artificially synthesized sequence

<400>  781

Tyr Thr Gly Arg Ser Ala Val Pro
1               5


<210>  782
<211>  8
<212>  PRT
<213>  Artificial Sequence

<220>
<223>  an artificially synthesized sequence

<400>  782

Tyr Ser Gly Arg Ser Ala Val Tyr
1               5


<210>  783
<211>  8
<212>  PRT
<213>  Artificial Sequence

<220>
<223>  an artificially synthesized sequence

<400>  783

Tyr Ser Gly Arg Ser Ala Val Val
1               5

<210>  784
<211>  8
<212>  PRT
<213>  Artificial Sequence

<220>
<223>  an artificially synthesized sequence

<400>  784

Tyr Ser Gly Arg Ser Ala Val His
1               5


<210>  785
<211>  8
<212>  PRT
<213>  Artificial Sequence

<220>
<223>  an artificially synthesized sequence

<400>  785

Tyr Thr Gly Arg Ser Ala Val Tyr
1               5


<210>  786
<211>  8
<212>  PRT
<213>  Artificial Sequence

<220>
<223>  an artificially synthesized sequence

<400>  786

Tyr Thr Gly Arg Ser Ala Val Val
1               5


<210>  787
<211>  8
<212>  PRT
<213>  Artificial Sequence

<220>
<223>  an artificially synthesized sequence

<400>  787

Tyr Thr Gly Arg Ser Ala Val His
1               5


<210>  788

<400>  788
000

<210>  789

<400> 789
000

<210> 790

<400> 790
000

<210> 791

<400> 791
000

<210> 792

<400> 792
000

<210> 793

<400> 793
000

<210> 794

<400> 794
000

<210> 795

<400> 795
000

<210> 796

<400> 796
000

<210> 797

<400> 797
000

<210> 798

<400> 798
000

<210> 799

<400> 799
000

<210> 800

<400> 800
000

<210> 801

<400> 801
000

<210> 802

<400> 802
000

<210> 803

<400> 803
000

<210> 804

<400> 804
000

<210> 805

<400> 805
000

<210> 806

<400> 806
000

<210> 807

<400> 807
000

<210> 808

<400> 808
000

<210> 809

<400> 809
000

<210> 810

<400> 810
000

<210> 811

<400> 811
000

<210> 812

<400> 812
000

<210> 813

<400> 813
000

<210> 814

<400> 814
000

<210> 815

<400> 815
000

<210> 816

<400> 816
000

<210> 817

<400> 817
000

<210> 818

<400> 818
000

<210> 819

<400> 819
000

<210> 820

<400> 820
000

<210> 821

<400> 821
000

<210> 822

<400> 822
000

<210> 823

<400> 823
000

<210> 824

<400> 824
000

<210> 825

<400> 825
000

<210> 826

<400> 826
000

<210>  827

<400>  827
000

<210>  828
<211>  12
<212>  PRT
<213>  Artificial sequence

<220>
<223>  an artificially synthesized sequence

<400>  828

Thr Ser Thr Ser Gly Arg Ser Ala Asn Pro Ala Gly
1               5                   10


<210>  829
<211>  12
<212>  PRT
<213>  Artificial sequence

<220>
<223>  an artificially synthesized sequence

<400>  829

Thr Ser Thr Ser Gly Arg Ser Ala Asn Pro His Gly
1               5                   10


<210>  830
<211>  12
<212>  PRT
<213>  Artificial sequence

<220>
<223>  an artificially synthesized sequence

<400>  830

Thr Ser Thr Ser Gly Arg Ser Ala Asn Pro Ile Gly
1               5                   10


<210>  831
<211>  12
<212>  PRT
<213>  Artificial sequence

<220>
<223>  an artificially synthesized sequence

<400>  831

Thr Ser Thr Ser Gly Arg Ser Ala Asn Pro Leu Gly
1               5                   10


<210>  832
<211>  12

<212>  PRT
<213>  Artificial sequence

<220>
<223>  an artificially synthesized sequence

<400>  832

Thr Ser Thr Ser Gly Arg Ser Ala Asn Pro Ser Gly
1               5                   10


<210>  833
<211>  12
<212>  PRT
<213>  Artificial sequence

<220>
<223>  an artificially synthesized sequence

<400>  833

Ile Ser Thr Ser Gly Arg Ser Ala Asn Pro Ile Gly
1               5                   10


<210>  834
<211>  12
<212>  PRT
<213>  Artificial sequence

<220>
<223>  an artificially synthesized sequence

<400>  834

Tyr Ser Thr Ser Gly Arg Ser Ala Asn Pro Ile Gly
1               5                   10


<210>  835
<211>  12
<212>  PRT
<213>  Artificial sequence

<220>
<223>  an artificially synthesized sequence

<400>  835

Thr Ser Tyr Ser Gly Arg Ser Ala Val Pro Ala Gly
1               5                   10


<210>  836
<211>  12
<212>  PRT
<213>  Artificial sequence

<220>
<223>  an artificially synthesized sequence

<400>  836

Thr Ser Pro Ser Gly Arg Ser Ala Asn Ile Ala Gly
1               5                   10

<210>  837
<211>  12
<212>  PRT
<213>  Artificial sequence

<220>
<223>  an artificially synthesized sequence

<400>  837

Thr Ser Pro Ser Gly Arg Ser Ala Asn Phe Ala Gly
1               5                   10

<210>  838
<211>  12
<212>  PRT
<213>  Artificial sequence

<220>
<223>  an artificially synthesized sequence

<400>  838

Thr Ser Pro Thr Gly Arg Ser Ala Asn Pro Ala Gly
1               5                   10

<210>  839
<211>  12
<212>  PRT
<213>  Artificial sequence

<220>
<223>  an artificially synthesized sequence

<400>  839

Thr Ser Pro Ser Gly Arg Ser Ala Ile Pro Ala Gly
1               5                   10

<210>  840
<211>  12
<212>  PRT
<213>  Artificial sequence

<220>
<223>  an artificially synthesized sequence

<400>  840

Thr Ser Tyr Thr Gly Arg Ser Ala Asn Pro Ala Gly
1               5                   10

<210>  841
<211>  12

<212>  PRT
<213>  Artificial sequence

<220>
<223>  an artificially synthesized sequence

<400>  841

Thr Ser Tyr Ser Gly Arg Ser Ala Ile Pro Ala Gly
1               5                   10


<210>  842
<211>  12
<212>  PRT
<213>  Artificial sequence

<220>
<223>  an artificially synthesized sequence

<400>  842

Thr Ser Ile Ser Gly Arg Ser Ala Asn Tyr Ala Gly
1               5                   10


<210>  843
<211>  12
<212>  PRT
<213>  Artificial sequence

<220>
<223>  an artificially synthesized sequence

<400>  843

Thr Ser Pro Ser Gly Arg Ser Ala Gly Pro Ala Gly
1               5                   10


<210>  844
<211>  12
<212>  PRT
<213>  Artificial sequence

<220>
<223>  an artificially synthesized sequence

<400>  844

Thr Ser Tyr Thr Gly Arg Ser Ala Val Pro Ala Gly
1               5                   10


<210>  845
<211>  12
<212>  PRT
<213>  Artificial sequence

<220>
<223>  an artificially synthesized sequence

<400>  845

Thr Ser Tyr Thr Gly Arg Ser Ala Val Tyr Ala Gly
1               5                   10

<210>  846
<211>  12
<212>  PRT
<213>  Artificial sequence

<220>
<223>  an artificially synthesized sequence

<400>  846

Thr Ser Tyr Thr Gly Arg Ser Ala Val Val Ala Gly
1               5                   10

<210>  847
<211>  12
<212>  PRT
<213>  Artificial sequence

<220>
<223>  an artificially synthesized sequence

<400>  847

Thr Ser Tyr Thr Gly Arg Ser Ala Val His Ala Gly
1               5                   10

<210>  848
<211>  12
<212>  PRT
<213>  Artificial sequence

<220>
<223>  an artificially synthesized sequence

<400>  848

Thr Ser Tyr Ser Gly Arg Ser Ala Val Pro His Gly
1               5                   10

<210>  849
<211>  12
<212>  PRT
<213>  Artificial sequence

<220>
<223>  an artificially synthesized sequence

<400>  849

Thr Ser Pro Ser Gly Arg Ser Ala Asn Ile His Gly
1               5                   10

<210>  850
<211>  12

<212>  PRT
<213>  Artificial sequence

<220>
<223>  an artificially synthesized sequence

<400>  850

Thr Ser Pro Ser Gly Arg Ser Ala Asn Phe His Gly
1               5                   10


<210>  851
<211>  12
<212>  PRT
<213>  Artificial sequence

<220>
<223>  an artificially synthesized sequence

<400>  851

Thr Ser Pro Thr Gly Arg Ser Ala Asn Pro His Gly
1               5                   10


<210>  852
<211>  12
<212>  PRT
<213>  Artificial sequence

<220>
<223>  an artificially synthesized sequence

<400>  852

Thr Ser Pro Ser Gly Arg Ser Ala Ile Pro His Gly
1               5                   10


<210>  853
<211>  12
<212>  PRT
<213>  Artificial sequence

<220>
<223>  an artificially synthesized sequence

<400>  853

Thr Ser Tyr Thr Gly Arg Ser Ala Asn Pro His Gly
1               5                   10


<210>  854
<211>  12
<212>  PRT
<213>  Artificial sequence

<220>
<223>  an artificially synthesized sequence

<400>  854

Thr Ser Tyr Ser Gly Arg Ser Ala Ile Pro His Gly
1               5                   10

<210>  855
<211>  12
<212>  PRT
<213>  Artificial sequence

<220>
<223>  an artificially synthesized sequence

<400>  855

Thr Ser Ile Ser Gly Arg Ser Ala Asn Tyr His Gly
1               5                   10

<210>  856
<211>  12
<212>  PRT
<213>  Artificial sequence

<220>
<223>  an artificially synthesized sequence

<400>  856

Thr Ser Pro Ser Gly Arg Ser Ala Gly Pro His Gly
1               5                   10

<210>  857
<211>  12
<212>  PRT
<213>  Artificial sequence

<220>
<223>  an artificially synthesized sequence

<400>  857

Thr Ser Tyr Thr Gly Arg Ser Ala Val Pro His Gly
1               5                   10

<210>  858
<211>  12
<212>  PRT
<213>  Artificial sequence

<220>
<223>  an artificially synthesized sequence

<400>  858

Thr Ser Tyr Thr Gly Arg Ser Ala Val Tyr His Gly
1               5                   10

<210>  859
<211>  12

<212>  PRT
<213>  Artificial sequence

<220>
<223>  an artificially synthesized sequence

<400>  859

Thr Ser Tyr Thr Gly Arg Ser Ala Val Val His Gly
1               5                   10


<210>  860
<211>  12
<212>  PRT
<213>  Artificial sequence

<220>
<223>  an artificially synthesized sequence

<400>  860

Thr Ser Tyr Thr Gly Arg Ser Ala Val His His Gly
1               5                   10


<210>  861
<211>  12
<212>  PRT
<213>  Artificial sequence

<220>
<223>  an artificially synthesized sequence

<400>  861

Thr Ser Tyr Ser Gly Arg Ser Ala Val Pro Ile Gly
1               5                   10


<210>  862
<211>  12
<212>  PRT
<213>  Artificial sequence

<220>
<223>  an artificially synthesized sequence

<400>  862

Thr Ser Pro Ser Gly Arg Ser Ala Asn Ile Ile Gly
1               5                   10


<210>  863
<211>  12
<212>  PRT
<213>  Artificial sequence

<220>
<223>  an artificially synthesized sequence

<400>  863

Thr Ser Pro Ser Gly Arg Ser Ala Asn Phe Ile Gly
1               5                   10

<210>  864
<211>  12
<212>  PRT
<213>  Artificial sequence

<220>
<223>  an artificially synthesized sequence

<400>  864

Thr Ser Pro Thr Gly Arg Ser Ala Asn Pro Ile Gly
1               5                   10

<210>  865
<211>  12
<212>  PRT
<213>  Artificial sequence

<220>
<223>  an artificially synthesized sequence

<400>  865

Thr Ser Pro Ser Gly Arg Ser Ala Ile Pro Ile Gly
1               5                   10

<210>  866
<211>  12
<212>  PRT
<213>  Artificial sequence

<220>
<223>  an artificially synthesized sequence

<400>  866

Thr Ser Tyr Thr Gly Arg Ser Ala Asn Pro Ile Gly
1               5                   10

<210>  867
<211>  12
<212>  PRT
<213>  Artificial sequence

<220>
<223>  an artificially synthesized sequence

<400>  867

Thr Ser Tyr Ser Gly Arg Ser Ala Ile Pro Ile Gly
1               5                   10

<210>  868
<211>  12

<212>  PRT
<213>  Artificial sequence

<220>
<223>  an artificially synthesized sequence

<400>  868

Thr Ser Ile Ser Gly Arg Ser Ala Asn Tyr Ile Gly
1               5                   10


<210>  869
<211>  12
<212>  PRT
<213>  Artificial sequence

<220>
<223>  an artificially synthesized sequence

<400>  869

Thr Ser Pro Ser Gly Arg Ser Ala Gly Pro Ile Gly
1               5                   10


<210>  870
<211>  12
<212>  PRT
<213>  Artificial sequence

<220>
<223>  an artificially synthesized sequence

<400>  870

Thr Ser Tyr Thr Gly Arg Ser Ala Val Pro Ile Gly
1               5                   10


<210>  871
<211>  12
<212>  PRT
<213>  Artificial sequence

<220>
<223>  an artificially synthesized sequence

<400>  871

Thr Ser Tyr Thr Gly Arg Ser Ala Val Tyr Ile Gly
1               5                   10


<210>  872
<211>  12
<212>  PRT
<213>  Artificial sequence

<220>
<223>  an artificially synthesized sequence

<400>  872

Thr Ser Tyr Thr Gly Arg Ser Ala Val Val Ile Gly
1               5                   10

<210>  873
<211>  12
<212>  PRT
<213>  Artificial sequence

<220>
<223>  an artificially synthesized sequence

<400>  873

Thr Ser Tyr Thr Gly Arg Ser Ala Val His Ile Gly
1               5                   10

<210>  874
<211>  12
<212>  PRT
<213>  Artificial sequence

<220>
<223>  an artificially synthesized sequence

<400>  874

Thr Ser Tyr Ser Gly Arg Ser Ala Val Pro Leu Gly
1               5                   10

<210>  875
<211>  12
<212>  PRT
<213>  Artificial sequence

<220>
<223>  an artificially synthesized sequence

<400>  875

Thr Ser Pro Ser Gly Arg Ser Ala Asn Ile Leu Gly
1               5                   10

<210>  876
<211>  12
<212>  PRT
<213>  Artificial sequence

<220>
<223>  an artificially synthesized sequence

<400>  876

Thr Ser Pro Ser Gly Arg Ser Ala Asn Phe Leu Gly
1               5                   10

<210>  877
<211>  12

<212>  PRT
<213>  Artificial sequence

<220>
<223>  an artificially synthesized sequence

<400>  877

Thr Ser Pro Thr Gly Arg Ser Ala Asn Pro Leu Gly
1               5                   10


<210>  878
<211>  12
<212>  PRT
<213>  Artificial sequence

<220>
<223>  an artificially synthesized sequence

<400>  878

Thr Ser Pro Ser Gly Arg Ser Ala Ile Pro Leu Gly
1               5                   10


<210>  879
<211>  12
<212>  PRT
<213>  Artificial sequence

<220>
<223>  an artificially synthesized sequence

<400>  879

Thr Ser Tyr Thr Gly Arg Ser Ala Asn Pro Leu Gly
1               5                   10


<210>  880
<211>  12
<212>  PRT
<213>  Artificial sequence

<220>
<223>  an artificially synthesized sequence

<400>  880

Thr Ser Tyr Ser Gly Arg Ser Ala Ile Pro Leu Gly
1               5                   10


<210>  881
<211>  12
<212>  PRT
<213>  Artificial sequence

<220>
<223>  an artificially synthesized sequence

<400>  881

Thr Ser Ile Ser Gly Arg Ser Ala Asn Tyr Leu Gly
1               5                   10

<210>  882
<211>  12
<212>  PRT
<213>  Artificial sequence

<220>
<223>  an artificially synthesized sequence

<400>  882

Thr Ser Pro Ser Gly Arg Ser Ala Gly Pro Leu Gly
1               5                   10

<210>  883
<211>  12
<212>  PRT
<213>  Artificial sequence

<220>
<223>  an artificially synthesized sequence

<400>  883

Thr Ser Tyr Thr Gly Arg Ser Ala Val Pro Leu Gly
1               5                   10

<210>  884
<211>  12
<212>  PRT
<213>  Artificial sequence

<220>
<223>  an artificially synthesized sequence

<400>  884

Thr Ser Tyr Thr Gly Arg Ser Ala Val Tyr Leu Gly
1               5                   10

<210>  885
<211>  12
<212>  PRT
<213>  Artificial sequence

<220>
<223>  an artificially synthesized sequence

<400>  885

Thr Ser Tyr Thr Gly Arg Ser Ala Val Val Leu Gly
1               5                   10

<210>  886
<211>  12

<212>  PRT
<213>  Artificial sequence

<220>
<223>  an artificially synthesized sequence

<400>  886

Thr Ser Tyr Thr Gly Arg Ser Ala Val His Leu Gly
1               5                   10


<210>  887
<211>  12
<212>  PRT
<213>  Artificial sequence

<220>
<223>  an artificially synthesized sequence

<400>  887

Thr Ser Tyr Ser Gly Arg Ser Ala Val Pro Ser Gly
1               5                   10


<210>  888
<211>  12
<212>  PRT
<213>  Artificial sequence

<220>
<223>  an artificially synthesized sequence

<400>  888

Thr Ser Pro Ser Gly Arg Ser Ala Asn Ile Ser Gly
1               5                   10


<210>  889
<211>  12
<212>  PRT
<213>  Artificial sequence

<220>
<223>  an artificially synthesized sequence

<400>  889

Thr Ser Pro Ser Gly Arg Ser Ala Asn Phe Ser Gly
1               5                   10


<210>  890
<211>  12
<212>  PRT
<213>  Artificial sequence

<220>
<223>  an artificially synthesized sequence

<400>  890

Thr Ser Pro Thr Gly Arg Ser Ala Asn Pro Ser Gly
1               5                   10

<210>  891
<211>  12
<212>  PRT
<213>  Artificial sequence

<220>
<223>  an artificially synthesized sequence

<400>  891

Thr Ser Pro Ser Gly Arg Ser Ala Ile Pro Ser Gly
1               5                   10

<210>  892
<211>  12
<212>  PRT
<213>  Artificial sequence

<220>
<223>  an artificially synthesized sequence

<400>  892

Thr Ser Tyr Thr Gly Arg Ser Ala Asn Pro Ser Gly
1               5                   10

<210>  893
<211>  12
<212>  PRT
<213>  Artificial sequence

<220>
<223>  an artificially synthesized sequence

<400>  893

Thr Ser Tyr Ser Gly Arg Ser Ala Ile Pro Ser Gly
1               5                   10

<210>  894
<211>  12
<212>  PRT
<213>  Artificial sequence

<220>
<223>  an artificially synthesized sequence

<400>  894

Thr Ser Ile Ser Gly Arg Ser Ala Asn Tyr Ser Gly
1               5                   10

<210>  895
<211>  12

<212>  PRT
<213>  Artificial sequence

<220>
<223>  an artificially synthesized sequence

<400>  895

Thr Ser Pro Ser Gly Arg Ser Ala Gly Pro Ser Gly
1               5                   10


<210>  896
<211>  12
<212>  PRT
<213>  Artificial sequence

<220>
<223>  an artificially synthesized sequence

<400>  896

Thr Ser Tyr Thr Gly Arg Ser Ala Val Pro Ser Gly
1               5                   10


<210>  897
<211>  12
<212>  PRT
<213>  Artificial sequence

<220>
<223>  an artificially synthesized sequence

<400>  897

Thr Ser Tyr Thr Gly Arg Ser Ala Val Tyr Ser Gly
1               5                   10


<210>  898
<211>  12
<212>  PRT
<213>  Artificial sequence

<220>
<223>  an artificially synthesized sequence

<400>  898

Thr Ser Tyr Thr Gly Arg Ser Ala Val Val Ser Gly
1               5                   10


<210>  899
<211>  12
<212>  PRT
<213>  Artificial sequence

<220>
<223>  an artificially synthesized sequence

<400>  899

Thr Ser Tyr Thr Gly Arg Ser Ala Val His Ser Gly
1               5                   10

<210>  900
<211>  12
<212>  PRT
<213>  Artificial sequence

<220>
<223>  an artificially synthesized sequence

<400>  900

Ile Ser Tyr Ser Gly Arg Ser Ala Val Pro Ile Gly
1               5                   10

<210>  901
<211>  12
<212>  PRT
<213>  Artificial sequence

<220>
<223>  an artificially synthesized sequence

<400>  901

Ile Ser Pro Ser Gly Arg Ser Ala Asn Ile Ile Gly
1               5                   10

<210>  902
<211>  12
<212>  PRT
<213>  Artificial sequence

<220>
<223>  an artificially synthesized sequence

<400>  902

Ile Ser Pro Ser Gly Arg Ser Ala Asn Phe Ile Gly
1               5                   10

<210>  903
<211>  12
<212>  PRT
<213>  Artificial sequence

<220>
<223>  an artificially synthesized sequence

<400>  903

Ile Ser Pro Thr Gly Arg Ser Ala Asn Pro Ile Gly
1               5                   10

<210>  904
<211>  12

<212>  PRT
<213>  Artificial sequence

<220>
<223>  an artificially synthesized sequence

<400>  904

Ile Ser Pro Ser Gly Arg Ser Ala Ile Pro Ile Gly
1               5                   10


<210>  905
<211>  12
<212>  PRT
<213>  Artificial sequence

<220>
<223>  an artificially synthesized sequence

<400>  905

Ile Ser Tyr Thr Gly Arg Ser Ala Asn Pro Ile Gly
1               5                   10


<210>  906
<211>  12
<212>  PRT
<213>  Artificial sequence

<220>
<223>  an artificially synthesized sequence

<400>  906

Ile Ser Tyr Ser Gly Arg Ser Ala Ile Pro Ile Gly
1               5                   10


<210>  907
<211>  12
<212>  PRT
<213>  Artificial sequence

<220>
<223>  an artificially synthesized sequence

<400>  907

Ile Ser Ile Ser Gly Arg Ser Ala Asn Tyr Ile Gly
1               5                   10


<210>  908
<211>  12
<212>  PRT
<213>  Artificial sequence

<220>
<223>  an artificially synthesized sequence

<400>  908

Ile Ser Pro Ser Gly Arg Ser Ala Gly Pro Ile Gly
1               5                   10

<210>  909
<211>  12
<212>  PRT
<213>  Artificial sequence

<220>
<223>  an artificially synthesized sequence

<400>  909

Ile Ser Tyr Thr Gly Arg Ser Ala Val Pro Ile Gly
1               5                   10

<210>  910
<211>  12
<212>  PRT
<213>  Artificial sequence

<220>
<223>  an artificially synthesized sequence

<400>  910

Ile Ser Tyr Thr Gly Arg Ser Ala Val Tyr Ile Gly
1               5                   10

<210>  911
<211>  12
<212>  PRT
<213>  Artificial sequence

<220>
<223>  an artificially synthesized sequence

<400>  911

Ile Ser Tyr Thr Gly Arg Ser Ala Val Val Ile Gly
1               5                   10

<210>  912
<211>  12
<212>  PRT
<213>  Artificial sequence

<220>
<223>  an artificially synthesized sequence

<400>  912

Ile Ser Tyr Thr Gly Arg Ser Ala Val His Ile Gly
1               5                   10

<210>  913
<211>  12

<212>  PRT
<213>  Artificial sequence

<220>
<223>  an artificially synthesized sequence

<400>  913

Tyr Ser Tyr Ser Gly Arg Ser Ala Val Pro Ile Gly
1               5                   10


<210>  914
<211>  12
<212>  PRT
<213>  Artificial sequence

<220>
<223>  an artificially synthesized sequence

<400>  914

Tyr Ser Pro Ser Gly Arg Ser Ala Asn Ile Ile Gly
1               5                   10


<210>  915
<211>  12
<212>  PRT
<213>  Artificial sequence

<220>
<223>  an artificially synthesized sequence

<400>  915

Tyr Ser Pro Ser Gly Arg Ser Ala Asn Phe Ile Gly
1               5                   10


<210>  916
<211>  12
<212>  PRT
<213>  Artificial sequence

<220>
<223>  an artificially synthesized sequence

<400>  916

Tyr Ser Pro Thr Gly Arg Ser Ala Asn Pro Ile Gly
1               5                   10


<210>  917
<211>  12
<212>  PRT
<213>  Artificial sequence

<220>
<223>  an artificially synthesized sequence

<400>  917

Tyr Ser Pro Ser Gly Arg Ser Ala Ile Pro Ile Gly
1               5                   10

<210>  918
<211>  12
<212>  PRT
<213>  Artificial sequence

<220>
<223>  an artificially synthesized sequence

<400>  918

Tyr Ser Tyr Thr Gly Arg Ser Ala Asn Pro Ile Gly
1               5                   10

<210>  919
<211>  12
<212>  PRT
<213>  Artificial sequence

<220>
<223>  an artificially synthesized sequence

<400>  919

Tyr Ser Tyr Ser Gly Arg Ser Ala Ile Pro Ile Gly
1               5                   10

<210>  920
<211>  12
<212>  PRT
<213>  Artificial sequence

<220>
<223>  an artificially synthesized sequence

<400>  920

Tyr Ser Ile Ser Gly Arg Ser Ala Asn Tyr Ile Gly
1               5                   10

<210>  921
<211>  12
<212>  PRT
<213>  Artificial sequence

<220>
<223>  an artificially synthesized sequence

<400>  921

Tyr Ser Pro Ser Gly Arg Ser Ala Gly Pro Ile Gly
1               5                   10

<210>  922
<211>  12

<212>  PRT
<213>  Artificial sequence

<220>
<223>  an artificially synthesized sequence

<400>  922

Tyr Ser Tyr Thr Gly Arg Ser Ala Val Pro Ile Gly
1               5                   10


<210>  923
<211>  12
<212>  PRT
<213>  Artificial sequence

<220>
<223>  an artificially synthesized sequence

<400>  923

Tyr Ser Tyr Thr Gly Arg Ser Ala Val Tyr Ile Gly
1               5                   10


<210>  924
<211>  12
<212>  PRT
<213>  Artificial sequence

<220>
<223>  an artificially synthesized sequence

<400>  924

Tyr Ser Tyr Thr Gly Arg Ser Ala Val Val Ile Gly
1               5                   10


<210>  925
<211>  12
<212>  PRT
<213>  Artificial sequence

<220>
<223>  an artificially synthesized sequence

<400>  925

Tyr Ser Tyr Thr Gly Arg Ser Ala Val His Ile Gly
1               5                   10